# EUROPÄISCHE PATENTANMELDUNG

(11) **EP 4 094 580 A1**
(43) Veröffentlichungstag der Anmeldung: **30.11.2022**
(21) Anmeldenummer: 22177160.3
(22) Anmeldetag: 22.10.2014
(51) Int. Cl.: A01N 43/82, A01P 13/00, A01N 33/22, A01N 37/40, A01N 41/10, A01N 43/08, A01N 43/40, A01N 43/54, A01N 43/56, A01N 43/66, A01N 43/707, A01N 43/76, A01N 43/80, A01N 43/90, A01N 47/30, A01N 47/36, A01N 57/20

(54) **HERBIZIDE ZUSAMMENSETZUNGEN ENTHALTEND N-(1,3,4-OXADIAZOL-2-YL)ARYLCARBONSÄUREAMIDE**

(30) Priorität: 25.10.2013 EP 13190182
(62) Teilanmeldung aus: 14789235.0
(71) Anmelder: Bayer CropScience Aktiengesellschaft, 40789 Monheim (DE)
(72) Erfinder: Köhn, Arnim, 51373 Leverkusen (DE); Waldraff, Christian, 51373 Leverkusen (DE); Gatzweiler, Elmar, 51373 Leverkusen (DE); Trabold, Klaus, 51373 Leverkusen (DE); Menne, Hubert, 51373 Leverkusen (DE); Ahrens, Hartmut, 51373 Leverkusen (DE); Dörner-Rieping, Simon, 51373 Leverkusen (DE); Braun, Ralf, 51373 Leverkusen (DE); Heinemann, Ines, 51373 Leverkusen (DE)
(74) Vertreter: BIP Patents

(57) **Zusammenfassung**

Es werden herbizide Zusammensetzungen beschrieben, die Wirkstoffe aus der Gruppe der N-(1,3,4-Oxadiazol-2-yl)arylcarbon-säureamidesowie weitere Herbizide und gegebenenfalls Safener enthalten. Diese herbiziden Zusammensetzungen sind für den Einsatz gegen Schadpflanzen in Nutzpflanzenkulturen besonders geeignet.

## Beschreibung

Die vorliegende Erfindung betrifft agrochemisch wirksame herbizide Zusammensetzungen, Verfahren zu deren Herstellung sowie deren Verwendung zur Bekämpfung von Schadpflanzen.

WO 2012/126932 A1 offenbart bestimmte N-(1,3,4-Oxadiazol-2-yl)arylcarbonsäureamide mit herbiziden Eigenschaften. Allerdings zeigen diese Wirkstoffe nicht immer eine ausreichende Wirkung gegen Schadpflanzen und/oder sie sind zum Teil nicht voll verträglich mit einigen wichtigen Kulturpflanzen, wie Getreidearten, Mais oder Reis.

Aufgabe der vorliegenden Erfindung ist es daher, herbizide Zusammensetzungen bereitzustellen, in welchen die Wirksamkeit gegenüber Schadpflanzen und/oder Selektivität der oben genannten Herbizide gegenüber wichtigen Kulturpflanzen erhöht ist. Diese Aufgabe wird durch die nachfolgend beschriebenen erfindungsgemäßen herbiziden Zusammensetzungen enthaltend bestimmte N-(1,3,4-Oxadiazol-2-yl)arylcarbonsäureamide, weitere Herbizide und gegebenenfalls Safener, gelöst.

Gegenstand der vorliegenden Erfindung sind herbizide Zusammensetzungen, enthaltend
(A) eine oder mehrere Verbindungen der Formel (I) (Komponente A) oder deren Salze, 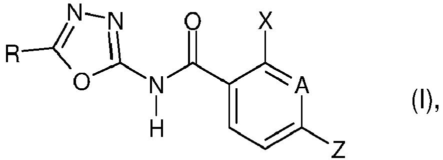 worin die Substituenten folgende Bedeutungen haben:
   A bedeutet N oder CY,
   R bedeutet Wasserstoff, (C₁-C₆)-Alkyl, R¹O-(C₁-C₆)-Alkyl, CH₂R⁶, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, OR¹, NHR¹, Methoxycarbonyl, Ethoxycarbonyl, Methoxycarbonylmethyl, Ethoxycarbonylmethyl, Methylcarbonyl, Trifluormethylcarbonyl, Dimethylamino, Acetylamino, Methylsulfenyl, Methylsulfinyl, Methylsulfonyl oder jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes Heteroaryl, Heterocyclyl, Benzyl oder Phenyl,
   X bedeutet Nitro, Halogen, Cyano, Formyl, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OR¹, OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, CH₂P(O)(OR⁵)₂, (C₁-C₆)-Alkyl-Heteroaryl, (C₁-C₆)-Alkyl-Heterocyclyl, wobei die beiden letzt genannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy, und wobei Heterocyclyl n Oxogruppen trägt,
   Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹,OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂ (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
   Z bedeutet Wasserstoff, Halogen, Cyano, Rhodano, (C₁-C₆)-Alkyl, (C₁-C₆)-Alkoxy, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹,C(O)N(R¹)₂, NR¹C(O)N(R¹)₂, OC(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, Heteroaryl, Heterocyclyl oder Phenyl, wobei die letzten drei Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy oder Halogen-(C₁-C₆)-alkoxy substituiert sind, und wobei Heterocyclyl n Oxogruppen trägt,
   R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
   R² bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
   R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
   R⁴ bedeutet (C₁-C₆)-Alkyl, (C₂-C₆)-Alkenyl oder (C₂-C₆)-Alkinyl,
   R⁵ bedeutet Methyl oder Ethyl,
   R⁶ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₁-C₆)-Alkoxy, (C₃-C₆)-Cycloalkyl oder jeweils durch s Reste aus der Gruppe Methyl, Ethyl, Methoxy, Trifluormethyl und Halogen substituiertes Heteroaryl, Heterocyclyl oder Phenyl,
   n bedeutet 0, 1 oder 2,
   s bedeutet 0, 1, 2 oder 3,
   sowie
(B) ein oder mehrere Herbizide (Komponente B) ausgewählt aus den Gruppen (B1) bis(B11):
   B1 1,3-Diketoverbindungen, umfassend
      prohexadione, prohexadione-calcium, trinexapac-ethyl,
      alloxydim, alloxydim-sodium, butroxydim, clethodim, cycloxydim, ketospiradox, profoxydim, sethoxydim, tepraloxydim, tralkoxydim,
      mesotrione, sulcotrione, tefuryltrione, tembotrione, bicyclopyrone, pinoxaden,
   B2 (Sulfon)Amide, umfassend
      beflubutamide, bromobutide, dimethenamide, dimethenamide-P, diphenamide, napropamide, pethoxamid, N-[3-Chlor-4-(1-methylethyl)-phenyl]-2-methylpentanamid, propyzamide,
      diflufenican, etobenzanid, flufenacet, mefenacet, mefluidide, picolinafen, propanil, N-phenylphtalamic acid,
      acetochlor, alachlor, amidochlor, butachlor, butenachlor, dimethachlor, metazachlor, metolachlor, S-metolachlor, pretilachlor, propachlor, propisochlor, (2-chloro-6'-ethyl-N-isopropoxymethylaceto-o-toluidide), thenylchlor,
      asulam, carbaryl, carbetamide, chlorpropham, desmedipham, phenmedipham, propham,
      butylate, cycloate, dimepiperate, EPTC, esprocarb, methasulfocarb, molinate, orbencarb, pebulate, prosulfocarb, pyributicarb, thiobencarb, tri-allate, vernolate, amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron-methyl-sodium, foramsulfuron, halosulfuron-methyl, imazosulfuron, iodosulfuron, iodosulfuron-methyl-sodium, mesosulfuron, mesosulfuron-methyl, metazosulfuron, methiopyrsulfuron, metsulfuron, metsulfuron-methyl, monosulfuron, monosulfuronester, nicosulfuron, orthosulfamuron, oxasulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, trifloxysulfuron (sodium), triflusulfuron, triflusulfuron-methyl, tritosulfuron, (benzoic acid, 2-[[[[[4-methoxy-6-(methylthio)-2-pyrimidinyl]amino]carbonyl]amino]-sulfonyl]methyl-ester), flucarbazone, flucarbazone-sodium, ipfencarbazone, propoxycarbazone, propoxycarbazone-sodium, thiencarbazone, thiencarbazone-methyl,
      cloransulam, cloransulam-methyl, diclosulam, florasulam, flumetsulam, metosulam, penoxsulam, pyroxsulam,
      3-chloro-N-[(4,6-dimethoxypyrimidin-2-yl)carbamoyl]-1-methyl-4-(5-methyl-5,6-dihydro-1,4,2-dioxazin-3-yl)-1H-pyrazole-5-sulfonamide,
   B3 Arylnitrile, umfassend
      bromoxynil, bromoxynil-butyrate, bromoxynil-potassium, bromoxynil-heptanoate, bromoxynil-octanoate, detosyl-pyrazolate (DTP), dichlobenil, ioxynil, ioxynil-octanoate, ioxynil-potassium, ioxynil-sodium, pyraclonil,
   B4 Azole, umfassend
      benzofenap , pyrazolynate (pyrazolate), pyrazoxyfen, pyroxasulfone, topramezone, pyrasulfotole, 3-(3-chloro-5-{[1-methyl-3-(trifluormethyl)-1H-pyrazol-5-yl]oxy}phenoxy)-1-methyl-5-(trifluormethyl)-1H-pyrazole, 3-(3-iodo-5-{[1-methyl-3-(trifluormethyl)-1H-pyrazol-5-yl]oxy}phenoxy)-1-methyl-5-(trifluormethyl)-1H-pyrazole, 1-ethyl-3-(3-fluoro-5-{[1-methyl-3-(trifluormethyl)-1H-pyrazol-5-yl]oxy}phenoxy)-5-(trifluormethyl)-1H-pyrazole, pyraflufen, pyraflufen-ethyl, fenoxasulfone, isouron, isoxaben, isoxaflutole, imazamethabenz, imazamethabenz-methyl, imazamox, imazamox-ammonium, imazapic, imazapic-ammonium, imazapyr, imazapyr-isopropyl-ammonium, imazaquin, imazaquin-ammonium, imazethapyr, imazethapyr-immonium, azafenidin, oxadiargyl, oxadiazon, amicarbazone, carfentrazone, carfentrazone-ethyl, sulfentrazone, amitrole, paclobutrazol, uniconazole, uniconazole-P, cafenstrole,
      fentrazamide,
   B5 weitere Herbizide, umfassend
      allidochlor, aminocyclopyrachlor, aminocyclopyrachlor-potassium, aminocyclopyrachlor-methyl, N-acetylthiazolidine-4-carboxylic acid, aminopyralid, ammonium pelargonate, ammonium sulfamat, aviglycine, benazolin, benazolin-ethyl, benfluralin, benfuresate, bentazone, benzobicyclon, 6-benzylaminopurine, brassinolide, bromofenoxim, butralin, chlorfenac, chlorfenac-sodium, chlorfenprop, chlorflurenol, chlorflurenol-methyl, chloridazon, chlormequat chloride, chlorphthalim, chlorthal-dimethyl, cinidon, cinidon-ethyl, cinmethylin, clofencet, clomazone, cloxyfonac, cyanamide, cyclanilide, cyclopyrimorate, 6-isopentylamino-purin, kinetin, zeatin, dalapon, daminozide, dazomet, n-decanol, difenzoquat metilsulfate, 2,6-diisopropylnaphtalene, dikegulac, dikegulac-sodium, dimethipin, dinitramine, dinoterb, diquat, diquat dibromide, dithiopyr, DNOC, endothal, endothal-dipotassium, endothaldisodium, endothal-mono(N,N-dimethylalkylammonium), ethafluralin, ethofumesate, ethylchlozate, flamprop, flamprop-M-isopropyl, flamprop-M-methyl, fluchloralin, flufenpyr, flufenpyr-ethyl, flumetralin, flumichlorac, flumiclorac-pentyl, flumioxazin, flupropanate, flurenol, flurenol-butyl, flurenol-dimethylammonium-methyl, fluridone, flurochloridone, flurtamone, fluthiacet, fluthiacet-methyl, gibberillic acid, halauxifen, indanofan, isoprothiolane, maleic hydrazide, mepiquat chloride, metam, methiozolin, methylarsonic acid, 1-methylcyclopropene, methyl isothiocyanate, nitrophenolate mixture, nonanoic acid, norflurazon, oleic acid, oryzalin, oxaziclomefone, paraquat, paraquat dichloride, pendimethalin, pentachlorophenol, pentoxazone, petroleum oils, prodiamine, n-propyl-dihydrojasmonate, pyridafol, pyridate, quinoclamine, sintofen, TCA, TCA sodium, tecnazene, thiazopyr, triacontanol, triafamone, trifluralin, urea sulfate,
   B6 (Het)Arylcarbonsäuren, umfassend
      chloramben, dicamba, 2,3,6-TBA, clopyralid, fluroxypyr, fluroxypyr-meptyl, inabenfide, picloram, triclopyr, quinclorac, quinmerac, indol-3-ylacetic acid, 4-indol-3-yl-butyric acid, 2-(1-naphthyl)acetamide, 1-naphtylacetic acid, 2-naphthyloxyacetic acid,
   B7 organische Phosphorverbindungen, umfassend
      anilofos, bensulide, bilanafos, bilanafos-sodium, butamifos, clacyfos, fosamine, glufosinate, glufosinate-Salze, glufosinate-ammonium, glufosinate-sodium, glufosinate-P, L-glufosinate-ammonium, L-glufosinate-sodium, glyphosate, glyphosate-Salze, glyphosate-isopropyl-ammonium, glyphosate-ammonium, glyphosatedimethylammonium, glyphosate-trimesium (=sulfosate), glyphosate-diammonium, glyphosate-potassium, glyphosate-sodium, piperophos, ethephon, tribufos,
   B8 Phenylether, umfassend
      acifluorfen-sodium, aclonifen, fluoroglycofen, fluoroglycofen-ethyl, fomesafen, fomesafen-sodium, halosafen, lactofen, oxyfluorfen, acifluorfen, bifenox, ethoxyfenethyl, clomeprop, cloprop, dichlorprop, dichlorprop-P, mecoprop, mecoprop-sodium, mecoprop-butotyl, mecoprop-P, mecoprop-P-butotyl, mecoprop-P-dimethylammonium, mecoprop-P-2-ethylhexyl, mecoprop-P-patassium, 4-CPA, 2,4-D, 2,4-D-butotyl, 2,4-D-butyl, 2,4-D-dimethylammonium, 2,4-D-diolamin, 2,4-D-ethyl, 2,4-D-2-ethylhexyl, 2,4-D-isobutyl, 2,4-D-isooctyl, 2,4-D- iso-propyl-ammonium, 2,4-D-potassium, 2,4-D-triisopropanolammonium, 2,4-D-trolamine, MCPA, MCPA-butotyl, MCPAdimethylammonium, MCPA-2-ethylhexyl. MCPA-isopropylammonium, MCPApotassium, MCPA-sodium, 2,4-DB, MCPB, MCPB-methyl, MCPB-ethyl-sodium, clodinafop-ethyl, clodinafop-propargyl, cyhalofop, cyhalofop-butyl, diclofop, diclofop-P, diclofop-methyl, diclofop-P-methyl, fenoxaprop, fenoxaprop-P, fenoxaprop-P-ethyl, fluazifop, fluazifop-P, fluazifop-butyl, fluazifop-P-butyl, haloxyfop, haloxyfop-P, metamifop, propaquizafop quizalofop, quizalofop-ethyl, quizalofop-P, quizalofop-P-ethyl, quizalofop-P-tefuryl,
   B9 Pyrimidine, umfassend
      ancymidol, flurprimidol, pyrimisulfan, bispyribac, bispyribac-sodium, pyribenzoxim, pyriminobac, pyriminobac-methyl, pyribambenz, pyribambenz-isopropyl, pyribambenzpropyl, pyriftalid, pyrithiobac, pyrithiobac-sodium, bromacil, butafenacil, lenacil, saflufenacil, terbacil, tifenacil, 2-chloro-4-fluoro-5-[3-methyl-2,6-dioxo-4-(trifluoromethyl)-3,6-dihydropyrimidin-1(2H)-yl]-N-[methyl(1-methylethyl)-sulfamoyl]benzamide, ethyl[(3-{2-chloro-5-[2,6-dioxo-4-(trifluoromethyl)-3,6-dihydropyrimidin-1 (2H)-yl]-4-fluorophenoxy}pyridin-2-yl)oxy]acetate,
   B10 (Thio)harnstoffe, umfassend
      cumyluron, chlorbromuron, chlorotoluron, daimuron, diflufenzopyr, diflufenzopyrsodium, dimefuron, diuron, fluometuron, forchlorfenuron, isoproturon, karbutilate, linuron, metobromuron, metoxuron, monolinuron, neburon, siduron, terbucarb, thidiazuron, tebuthiuron, methabenzthiazuron,
   B11 Triazine, umfassend
      triaziflam, indaziflam, atrazine, cyanazine, cyprazine, propazine, simazine, terbumeton, terbuthylazine, trietazine, prometon, ametryn, dimethametryn, prometryn, simetryn, terbutryn, ethiozin, hexazinon, metamitron, metribuzin.

In einer weiteren Ausführungsform enthalten diese herbiziden Zusammensetzungen (C) einen oder mehrere Safener (Komponente C) aus der Gruppe bestehend aus benoxacor (C1), cloquintocet-mexyl (C2), cyprosulfamide (C3), dichlormid (C4), fenclorim (C5), fenchlorazole (C6), furilazole (C7), isoxadifen-ethyl (C8), mefenpyr-diethyl (C9), 4-(dichloroacetyl)-1-oxa-4-azaspiro[4.5]decane der CAS 71526-07-3 (C10), 2,2,5-trimethyl-3-(dechloroacetyl)-1,3-oxazolidine der CAS 52836-31-4 (C11), 2-methoxy-N-({4-[(methylcarbamoyl)amino]phenyl}sulfonyl)benzamid der CAS 129531-12-0 (C12).

Die Komponenten B) und C) sind beispielsweise aus "The Pesticide Manual", 16th edition (2012), The British Crop Protection Council and the Royal Soc. of Chemistry bekannt.

Die erfindungsgemäßen herbiziden Zusammensetzungen können zusätzliche weitere Komponenten, beispielsweise Pflanzenschutzmittelwirkstoffe anderer Art und/oder im Pflanzenschutz übliche Zusatzstoffe und/oder Formulierungshilfsmittel, enthalten oder zusammen mit diesen eingesetzt werden.

Die Herbizide (A), (B) und gegebenenfalls die Safener (C) können auf bekannte Weise angewendet werden, beispielsweise gemeinsam (beispielsweise als Co-Formulierung oder als Tank-Mischung) oder auch zeitlich kurz hintereinander versetzt (Splitting), z.B. auf die Pflanzen, Pflanzenteile, Pflanzensamen oder die Fläche, auf der die Pflanzen wachsen. Möglich ist z.B. die Anwendung der Einzelwirkstoffe oder der Herbizid-Safener-Kombination in mehreren Portionen (Sequenzanwendung), z. B. nach Anwendungen im Vorauflauf, gefolgt von Nachauflauf-Applikationen oder nach frühen Nachauflaufanwendungen, gefolgt von Applikationen im mittleren oder späten Nachauflauf. Bevorzugt ist dabei die gemeinsame oder die zeitnahe Anwendung der Wirkstoffe der jeweiligen Kombination. Möglich ist auch die Anwendung der Einzelwirkstoffe oder der Herbizid-Safener-Kombination zur Saatgutbehandlung.

Bevorzugt sind solche erfindungsgemäße Zusammensetzungen, die als Herbizid (A) Verbindungen der allgemeinen Formel (I) und deren Salze enthalten, worin
A bedeutet N oder CY,
R bedeutet Wasserstoff, (C₁-C₆)-Alkyl, (C₃-C₇)-Cycloalkyl, Halogen-(C₁-C₆)-alkyl, (C₃-C₇)-Cycloalkylmethyl, Methoxycarbonylmethyl, Ethoxycarbonylmethyl, Acetylmethyl, Methoxymethyl, Methoxyethyl, Benzyl, Pyrazin.2-yl, Furan-2-yl, Tetrahydrofuran-2-yl, Morpholin, Dimethylamino oder durch s Reste aus der Gruppe Methyl, Methoxy, Trifluormethyl und Halogen substituiertes Phenyl;
X bedeutet Nitro, Halogen, Cyano, (C₁-C₆)-Alkyl, Halogen-(C₁-C₆)-alkyl, (C₃-C₆)-Cycloalkyl, OR¹, S(O)ₙR², (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Heteroaryl, (C₁-C₆)-Alkyl-Heterocyclyl, wobei die beiden letzt genannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy substituiert sind, und wobei Heterocyclyl n Oxogruppen trägt,
Y Wasserstoff, Nitro, Halogen, Cyano, (C₁-C₆)-Alkyl, (C₁-C₆)-Halogenalkyl, OR¹, S(O)ₙR², SO₂N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Nitro, Methyl, Halogen-(C₁-C₆)-alkyl, (C₃-C₆)-Cycloalkyl, S(O)ₙR², 1,2,4-Triazol-1-yl, Pyrazol-1-yl, oder Z kann auch Wasserstoff bedeuten, falls Y für den Rest S(O)ₙR² steht,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 16 letztgenannten Reste durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₃-C₆)-Cycloalkyl oder (C₃-C₆)-Cycloalkyl-(C₁-C₆)-alkyl, wobei diese drei vorstehend genannten Reste jeweils durch s Reste aus der Gruppe bestehend aus Halogen und OR³ substituiert sind,
R³ bedeutet Wasserstoff oder (C₁-C₆)-Alkyl,
R⁴ bedeutet (C₁-C₆)-Alkyl,
n bedeutet 0, 1 oder 2,
s bedeutet 0, 1, 2 oder 3.

Besonders bevorzugt sind erfindungsgemäße Zusammensetzungen, die als Herbizid (A) Verbindungen der allgemeinen Formel (I) und deren Salze enthalten, worin
A bedeutet N oder CY,
R bedeutet Wasserstoff, (C₁-C₄)-Alkyl, Cyclopropyl, Halogen-(C₁-C₄)-alkyl, (C₃-C₆)-Cycloalkylmethyl, Methoxymethyl, Methoxyethyl, Benzyl,
X bedeutet Nitro, Halogen, Cyano, (C₁-C₆)-Alkyl, Halogen-(C₁-C₆)-alkyl, Cyclopropyl, OR¹, S(O)ₙR², (C₁-C₆)-Alkyl-S(O)ₙR², (C₁-C₆)-Alkyl-OR¹, (C₁-C₂)-Alkyl-Heteroaryl, (C₁- C₂)-Alkyl-Heterocyclyl, wobei die beiden letzt genannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy substituiert sind, und wobei Heterocyclyl n Oxogruppen trägt,
Y Wasserstoff, Nitro, Halogen, Cyano, (C₁-C₆)-Alkyl, (C₁-C₆)-Halogenalkyl, OR¹, S(O)ₙR², SO₂N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Nitro, Methyl, Halogen-(C₁-C₆)-alkyl, (C₃-C₆)-Cycloalkyl, S(O)ₙR², 1,2,4-Triazol-1-yl, Pyrazol-1-yl, oder
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 16 letztgenannten Reste durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₃-C₆)-Cycloalkyl oder (C₃-C₆)-Cycloalkyl-(C₁-C₆)-alkyl, wobei diese drei vorstehend genannten Reste jeweils durch s Reste aus der Gruppe bestehend aus Halogen und OR³ substituiert sind,
R³ bedeutet Wasserstoff oder (C₁-C₆)-Alkyl,
R⁴ bedeutet (C₁-C₆)-Alkyl,
n bedeutet 0, 1 oder 2,
s bedeutet 0, 1, 2 oder 3.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in- 1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder Iod.
Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,
Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Heteroaryl steht beispielsweise für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, dass diese Gruppe durch ein oder mehrere gleiche oder verschiedene der genannten Reste substituiert ist. Analoges gilt für den Aufbau von Ringsystemen durch verschiedene Atome und Elemente. Dabei sollen solche Verbindungen vom Anspruchsbegehren ausgenommen sein, von denen der Fachmann weiß, dass sie unter Normalbedingungen chemisch instabil sind.

Gegenstand der vorliegenden Erfindung sind auch herbizide Zusammensetzungen, umfassend Stereoisomere und deren Gemische, die von Formel (I) oder von den Formeln der Komponente B umfasst sind. Solche Verbindungen der Formel (I) oder der Formeln der Komponente B enthalten beispielsweise ein oder mehrere asymmetrisch substituierte Kohlenstoff-Atome oder Sulfoxide. Die durch ihre spezifische Raumform definierten möglichen Stereoisomeren, wie Enantiomere und Diastereomere, sind alle von der Formel (I) oder von den Komponenten (B) und (C) umfasst; insbesondere auch die racemischen Gemische und - soweit Enantiomere möglich sind - beide Enantiomere und insbesondere das jeweils biologisch wirksame Enantiomer. Die einzelnen Stereoisomeren können nach üblichen Methoden aus Gemischen der Stereoisomeren erhalten oder auch durch stereoselektive Reaktionen in Kombination mit dem Einsatz von stereochemisch reinen Ausgangs- oder Hilfsstoffen hergestellt werden.

Beispiele für als Herbizid (A) ganz besonders bevorzugte Verbindungen sind in den folgenden Tabellen aufgeführt.

Darin bedeuten die verwendeten Abkürzungen:

| | | | |
|---|---|---|---|
| Et = Ethyl | Me = Methyl | n-Pr = n-Propyl | i-Pr = Isopropyl |
| c-Pr = Cyclopropyl | Ph = Phenyl | Ac = Acetyl | i-Bu = Isobutyl |

**Tabelle 1: Verbindungen der allgemeinen Formel (I), worin A für CY steht**

| 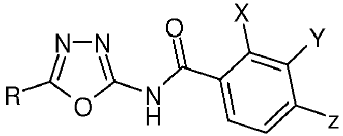 | | | | |
|---|---|---|---|---|
| Bsp.-Nr. | R | X | Y | Z |
| A1-1 | Me | Cl | CH₂OCH₂CF₃ | SO₂Me |
| A1-2 | c-Pr | Cl | SO₂Me | CF₃ |
| A1-3 | CH₂OMe | Me | SO₂Me | CF₃ |
| A1-4 | Me | Me | OEt | SO₂Me |
| A1-5 | Me | Cl | OEt | SO₂Me |
| A1-6 | Me | Cl | SEt | CF₃ |
| A1-7 | Me | Cl | SO₂Et | CF₃ |

| Bsp.-Nr. | R | X | Y | Z |
|---|---|---|---|---|
| A1-8 | Et | Me | SO₂Me | CF₃ |
| A1-9 | Me | Cl | SOMe | c-Pr |
| A1-10 | Me | Cl | 5-Cyano-4,5-dihydro-1,2-oxazol-3-yl | SO₂Et |
| A1-11 | CH₂OMe | Me | SMe | CF₃ |
| A1-12 | Et | Cl | SO₂Me | CF₃ |
| A1-13 | Me | Me | SO₂Me | CF₃ |
| A1-14 | Me | Cl | SOMe | CF₃ |
| A1-15 | CH₂OEt | Cl | SO₂Me | CF₃ |
| A1-16 | Me | Cl | 2H-1,2,3-Triazol-2-yl | SO₂Me |
| A1-17 | Me | Cl | O-CH₂CH₂SMe | Cl |
| A1-18 | Me | Cl | OCH₂CH₂OCF₃ | SO₂Et |
| A1-19 | Me | Cl | OCH₂CF₃ | SO₂Me |
| A1-20 | Me | Cl | SO₂Me | Me |
| A1-21 | Me | CH₂OMe | SO₂Me | CF₃ |
| A1-22 | Me | Cl | OCH₂CH₂OEt | SO₂Et |
| A1-23 | Me | Cl | OCH₂CH₂Cl | SO₂Et |
| A1-24 | Et | Cl | 5-Cyano-4,5-dihydro-1,2-oxazol-3-yl | SO₂Et |
| A1-25 | Me | Me | SMe | CF₃ |
| A1-26 | Me | Cl | OCH₂CH₂Cl | SO₂Me |
| A1-27 | Me | Cl | OCH₂CH₂CH₂SMe | Cl |
| A1-28 | Me | Cl | SCH₂₋c-Pr | CF₃ |
| A1-29 | CH₂F | Me | SO₂Me | CF₃ |
| A1-30 | Me | Cl | O-Propargyl | SO₂Me |
| A1-31 | Me | Cl | 1H-1,2,3-Triazol-1-yl | CF₃ |
| A1-32 | H | Cl | CH₂OCH₂CF₃ | SO₂Me |
| A1-33 | Me | Cl | 3-Br-1-H-Pyrazol-1-yl | SO₂Me |
| A1-34 | c-Pr | Me | SOMe | CF₃ |
| A1-35 | Et | Cl | SO₂Me | SO₂Me |
| A1-36 | Me | Cl | SO₂Me | CF₃ |
| A1-37 | Me | Me | 4,5-dihydro-1,2-oxazol-3-yl | SO₂Me |
| A1-38 | Me | Me | 1H-Pyrazol-1-yl | SO₂Me |
| A1-39 | Me | Cl | O-nPr | SO₂Me |
| A1-40 | Me | Cl | OCH₂CH₂CH₂OMe | SO₂Et |
| A1-41 | Me | Cl | O-nPr | SO₂Et |
| A1-42 | CH₂OMe | Cl | SOMe | CF₃ |
| A1-43 | CH₂OMe | Cl | SO₂Me | CF₃ |
| A1-44 | Me | Cl | 5-Ethyl-4,5-dihydro-1,2-oxazol-3-yl | Cl |
| A1-45 | Me | Me | N(Me)CHO | CF₃ |
| A1-46 | Me | Cl | N(CO)-Pyrrolidin | Cl |
| A1-47 | CF₃ | Me | SO₂Me | SO₂Me |
| A1-48 | Me | Me | 4-OMe-1H-Pyrazol-1-yl | SO₂Me |
| A1-49 | Me | Me | 1H-1,2,3-Triazol-1-yl | SO₂Me |
| A1-50 | Me | Cl | OCH₂CH₂CH₂OMe | SO₂Me |
| A1-51 | Me | Cl | OCHF₂ | SO₂Me |
| A1-52 | Me | Me | 1H-Pyrazol-1-yl | CF₃ |
| A1-53 | Me | Cl | OCH₂CH₂CH₂SCF₃ | SO₂Me |
| A1-54 | CHF₂ | Me | SO₂Me | CF₃ |
| A1-55 | Me | Cl | 1H-Pyrazol-1-yl | CF₃ |
| A1-56 | Me | Cl | OCH₂-cPr | SO₂Me |
| A1-57 | Me | Cl | OCH₂CH₂F | SO₂Me |
| A1-58 | Me | Me | SMe | C₂F₅ |
| A1-59 | Me | Cl | OMe | SO₂Me |
| A1-60 | Me | Me | SOMe | CF₃ |
| A1-61 | Me | Br | 1-H-Pyrazol-1-yl | C₂F₅ |

**Tabelle 2: Verbindungen der allgemeinen Formel (I), worin A für Stickstoff steht**

| 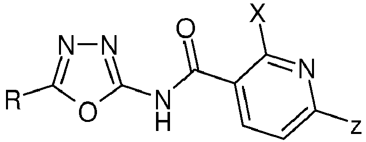 | | | |
|---|---|---|---|
| Bsp.-Nr. | R | X | Z |
| A2-1 | Me | Me | CF₃ |
| A2-2 | Pyrazin-2-yl | Cl | CF₃ |
| A2-3 | Me | Br | CF₃ |
| A2-4 | Me | Cl | CF₃ |

Bevorzugte Herbizide der Gruppe B1 sind clethodim, sethoxydim, tepraloxydim, mesotrione, sulcotrione, tefuryltrione, tembotrione, bicyclopyrone, pinoxaden, tralkoxydim.

Besonders bevorzugte Herbizide der Gruppe B1 sind clethodim (B1-1), sulcotrione (B1-2), tefuryltrione (B1-3), tembotrione (B1-4), bicyclopyrone (B1-5), pinoxaden (B1-6).

Bevorzugte Herbizide der Gruppe B2 sind dimethenamide, dimethenamide-P, napropamide, pethoxamid, propyzamide, diflufenican, flufenacet, mefenacet, picolinafen, propanil, acetochlor, alachlor, butachlor, metazachlor, metolachlor, S-metolachlor, pretilachlor, thenylchlor, asulam, carbetamide, desmedipham, phenmedipham, esprocarb, molinate, prosulfocarb, thiobencarb, amidosulfuron, chlorimuron-ethyl, cyclosulfamuron, ethoxysulfuron, flupyrsulfuron-methyl-sodium, foramsulfuron, iodosulfuron-methyl-sodium, mesosulfuron-methyl, nicosulfuron, orthosulfamuron, prosulfuron, pyrazosulfuron-ethyl, rimsulfuron, trifloxysulfuron (sodium), flucarbazone-sodium, propoxycarbazone-sodium, thiencarbazone-methyl, florasulam, metosulam, penoxsulam, metsulfuron-methyl, sulfosulfuron, thifensulfuron-methyl, tribenuron-methyl, tritosulfuron, pyroxsulam.

Besonders bevorzugte Herbizide der Gruppe B2 sind dimethenamide-P (B2-1), napropamide (B2-2), diflufenican (B2-3), flufenacet (B2-4), mefenacet (B2-5), acetochlor (B2-6), metazachlor (B2-7), S-metolachlor (B2-8), asulam (B2-9), desmedipham (B2-10), phenmedipham (B2-11), molinate (B2-12), prosulfocarb (B2-13), amidosulfuron (B2-14), ethoxysulfuron (B2-15), foramsulfuron (B2-16), iodosulfuron-methyl-sodium (B2-17), mesosulfuron-methyl (B2-18), flucarbazone-sodium (B2-19), propoxycarbazone-sodium (B2-20), thiencarbazone-methyl (B2-21), florasulam (B2-22), metosulam (B2-23), metsulfuron-methyl (B2-24), sulfosulfuron (B2-25), thifensulfuron-methyl (B2-26), tribenuron-methyl (B2-27), tritosulfuron (B2-28), pyroxsulam (B2-29).

Bevorzugte Herbizide der Gruppe B3 sind bromoxynil (B3-1) und ioxynil (B3-2).

Bevorzugte Herbizide der Gruppe B4 sind benzofenap, topramezone, pyrasulfotole, isoxaflutole, imazamox, imazethapyr, oxadiargyl, oxadiazon, amicarbazone, carfentrazone-ethyl, sulfentrazone, uniconazole, cafenstrole, fentrazamide, 3-(3-chloro-5-{[1-methyl-3-(trifluormethyl)-1H-pyrazol-5-yl]oxy}phenoxy)-1-methyl-5-(trifluormethyl)-1H-pyrazole, 3-(3-iodo-5-{[1-methyl-3-(trifluormethyl)-1H-pyrazol-5-yl]oxy}phenoxy)-1-methyl-5-(trifluormethyl)-1H-pyrazole, 1-ethyl-3-(3-fluoro-5-{[1-methyl-3-(trifluormethyl)-1H-pyrazol-5-yl]oxy}phenoxy)-5-(trifluormethyl)-1H-pyrazole, pyraflufen-ethyl.

Besonders bevorzugte Herbizide der Gruppe B4 sind pyrasulfotole (B4-1), isoxaflutole (B4-2), oxadiargyl (B4-3), oxadiazon (B4-4), amicarbazone (B4-5), fentrazamide (B4-6), 3-(3-chloro-5-{[1-methyl-3-(trifluormethyl)-1H-pyrazol-5-yl]oxy}phenoxy)-1-methyl-5-(trifluormethyl)-1H-pyrazole (B4-7), 3-(3-iodo-5-{[1-methyl-3-(trifluormethyl)-1H-pyrazol-5-yl]oxy}phenoxy)-1-methyl-5-(trifluormethyl)-1H-pyrazole (B4.8), 1-ethyl-3-(3-fluoro-5-{[1-methyl-3-(trifluormethyl)-1H-pyrazol-5-yl]oxy}phenoxy)-5-(trifluormethyl)-1H-pyrazole (B4-9), pyraflufen-ethyl (B4-10), imazamox (B4-11).

Bevorzugte Herbizide der Gruppe B5 sind aminopyralid, benazolin, benfuresate, bentazone, cinidon-ethyl, clomazone, diquat dibromide, ethofumesate, flumiclorac-pentyl, flumioxazin, flurtamone, oxaziclomefone, pendimethalin, pyridate und trifluralin. Besonders bevorzugte Herbizide der Gruppe B5 sind aminopyralid (B5-1), benfuresate (B5-2), ethofumesate (B5-3), flurtamone (B5-4) und oxaziclomefone (B5-5).

Bevorzugte Herbizide der Gruppe B6 sind dicamba (B6-1), clopyralid (B6-2), fluroxypyr (B6-3), picloram (B6-4), triclopyr (B6-5), quinclorac (B6-6).

Bevorzugte Herbizide der Gruppe B7 sind anilofos (B7-1), glufosinate-ammonium (B7-2), L-glufosinate-ammonium (B7-3), glyphosate (B7-4), glyphosate-isopropyl-ammonium (B7-5), glyphosate-ammonium (B7-6), glyphosate-trimesium (=sulfosate (B7-7)), glyphosate-diammonium (B7-7), glyphosate-potassium (B7-8).

Bevorzugte Herbizide der Gruppe B8 sind acifluorfen-sodium, aclonifen, fluoroglycofen-ethyl, oxyfluorfen, bifenox, dichlorprop-P, mecoprop-P, 2,4-D, MCPA, clodinafop-propargyl, cyhalofop-butyl, diclofop-methyl, diclofop-P-methyl, fenoxaprop-P-ethyl, fluazifop-P-butyl, quizalofop-P.

Besonders bevorzugte Herbizide der Gruppe B8 sind aclonifen (B8-1), diclofop-methyl (B8-2), diclofop-P-methyl (B8-3), fenoxaprop-P-ethyl (B8-4), MCPA (B8-5), 2,4-D (B8-6), clodinafop-ethyl (B8-7).

Bevorzugte Herbizide der Gruppe B9- sind bispyribac (sodium), pyriftalid, bromacil, lenacil, 2-chloro-4-fluoro-5-[3-methyl-2,6-dioxo-4-(trifluoromethyl)-3,6-dihydropyrimidin-1 (2H)-yl]-N-[methyl(1-methylethyl)-sulfamoyl]benzamide,

Besonders bevorzugte Herbizide der Gruppe B9 sind bispyribac (sodium) (B9-1), bromacil (B9-2).

Bevorzugte Herbizide der Gruppe B10 sind cumyluron (B10-1), daimuron (B10-2), diuron (B10-3), isoproturon (B10-4), diflufenzopyr (B10-5).

Bevorzugte Herbizide der Gruppe B11 sind atrazine, simazine, terbuthylazine, ametryn, terbutryn, metamitron, metribuzin.

Besonders bevorzugte Herbizide der Gruppe B11 sind metamitron (B11-1), metribuzin (B11-2), terbuthylazine (B11-2).

Beispiele für bevorzugte Zusammensetzungen von Herbiziden (A) und Herbiziden (B) sind nachfolgend genannt.
(A1-1)+(B1-1), (A1-1)+(B1-2), (A1-1)+(B1-3), (A1-1)+(B1-4), (A1-1)+(B1-5), (A1-1)+(B1-6), (A1-1)+(B2-1), (A1-1)+(B2-2), (A1-1)+(B2-3), (A1-1)+(B2-4), (A1-1)+(B2-5), (A1-1)+(B2-6), (A1-1)+(B2-7), (A1-1)+(B2-8), (A1-1)+(B2-9), (A1-1)+(B2-10), (A1-1)+(B2-11), (A1-1)+(B2-12), (A1-1)+(B2-13), (A1-1)+(B2-14), (A1-1)+(B2-15), (A1-1)+(B2-16), (A1-1)+(B2-17), (A1-1)+(B2-18), (A1-1)+(B2-19), (A1-1)+(B2-20), (A1-1)+(B2-21), (A1-1)+(B2-22), (A1-1)+(B2-23), (A1-1)+(B2-24), (A1-1)+(B2-25), (A1-1)+(B2-26), (A1-1)+(B2-27), (A1-1)+(B2-28), (A1-1)+(B2-29), (A1-1)+(B3-1), (A1-1)+(B3-2), (A1-1)+(B4-1), (A1-1)+(B4-2), (A1-1)+(B4-3), (A1-1)+(B4-4), (A1-1)+(B4-5), (A1-1)+(B4-6), (A1-1)+(B4-7), (A1-1)+(B4-8), (A1-1)+(B4-9), (A1-1)+(B4-10), (A1-1)+(B4-11), (A1-1)+(B5-1), (A1-1)+(B5-2), (A1-1)+(B5-3), (A1-1)+(B5-4), (A1-1)+(B5-5), (A1-1)+(B6-1), (A1-1)+(B6-2), (A1-1)+(B6-3), (A1-1)+(B6-4), (A1-1)+(B6-5), (A1-1)+(B6-6), (A1-1)+(B7-1), (A1-1)+(B7-2), (A1-1)+(B7-3), (A1-1)+(B7-4), (A1-1)+(B7-5), (A1-1)+(B7-6), (A1-1)+(B7-7)), (A1-1)+(B7-7), (A1-1)+(B7-8), (A1-1)+(B8-1), (A1-1)+(B8-2), (A1-1)+(B8-3), (A1-1)+(B8-4), (A1-1)+(B8-5), (A1-1)+(B8-6), (A1-1)+(B8-7), (A1-1)+(B9-1), (A1-1)+(B9-2), (A1-1)+(B10-1), (A1-1)+(B10-2), (A1-1)+(B10-3), (A1-1)+(B10-4), (A1-1)+(B10-5), (A1-1)+(B11-1), (A1-1)+(B11-2), (A1-1)+(B11-2),
(A1-2)+(B1-1), (A1-2)+(B1-2), (A1-2)+(B1-3), (A1-2)+(B1-4), (A1-2)+(B1-5), (A1-2)+(B1-6), (A1-2)+(B2-1), (A1-2)+(B2-2), (A1-2)+(B2-3), (A1-2)+(B2-4), (A1-2)+(B2-5), (A1-2)+(B2-6), (A1-2)+(B2-7), (A1-2)+(B2-8), (A1-2)+(B2-9), (A1-2)+(B2-10), (A1-2)+(B2-11), (A1-2)+(B2-12), (A1-2)+(B2-13), (A1-2)+(B2-14), (A1-2)+(B2-15), (A1-2)+(B2-16), (A1-2)+(B2-17), (A1-2)+(B2-18), (A1-2)+(B2-19), (A1-2)+(B2-20), (A1-2)+(B2-21), (A1-2)+(B2-22), (A1-2)+(B2-23), (A1-2)+(B2-24), (A1-2)+(B2-25), (A1-2)+(B2-26), (A1-2)+(B2-27), (A1-2)+(B2-28), (A1-2)+(B2-29), (A1-2)+(B3-1), (A1-2)+(B3-2), (A1-2)+(B4-1), (A1-2)+(B4-2), (A1-2)+(B4-3), (A1-2)+(B4-4), (A1-2)+(B4-5), (A1-2)+(B4-6), (A1-2)+(B4-7), (A1-2)+(B4-8), (A1-2)+(B4-9), (A1-2)+(B4-10), (A1-2)+(B4-11), (A1-2)+(B5-1), (A1-2)+(B5-2), (A1-2)+(B5-3), (A1-2)+(B5-4), (A1-2)+(B5-5), (A1-2)+(B6-1), (A1-2)+(B6-2), (A1-2)+(B6-3), (A1-2)+(B6-4), (A1-2)+(B6-5), (A1-2)+(B6-6), (A1-2)+(B7-1), (A1-2)+(B7-2), (A1-2)+(B7-3), (A1-2)+(B7-4), (A1-2)+(B7-5), (A1-2)+(B7-6), (A1-2)+(B7-7)), (A1-2)+(B7-7), (A1-2)+(B7-8), (A1-2)+(B8-1), (A1-2)+(B8-2), (A1-2)+(B8-3), (A1-2)+(B8-4), (A1-2)+(B8-5), (A1-2)+(B8-6), (A1-2)+(B8-7), (A1-2)+(B9-1), (A1-2)+(B9-2), (A1-2)+(B10-1), (A1-2)+(B10-2), (A1-2)+(B10-3), (A1-2)+(B10-4), (A1-2)+(B10-5), (A1-2)+(B11-1), (A1-2)+(B11-2), (A1-2)+(B11-2),
(A1-3)+(B1-1), (A1-3)+(B1-2), (A1-3)+(B1-3), (A1-3)+(B1-4), (A1-3)+(B1-5), (A1-3)+(B1-6), (A1-3)+(B2-1), (A1-3)+(B2-2), (A1-3)+(B2-3), (A1-3)+(B2-4), (A1-3)+(B2-5), (A1-3)+(B2-6), (A1-3)+(B2-7), (A1-3)+(B2-8), (A1-3)+(B2-9), (A1-3)+(B2-10), (A1-3)+(B2-11), (A1-3)+(B2-12), (A1-3)+(B2-13), (A1-3)+(B2-14), (A1-3)+(B2-15), (A1-3)+(B2-16), (A1-3)+(B2-17), (A1-3)+(B2-18), (A1-3)+(B2-19), (A1-3)+(B2-20), (A1-3)+(B2-21), (A1-3)+(B2-22), (A1-3)+(B2-23), (A1-3)+(B2-24), (A1-3)+(B2-25), (A1-3)+(B2-26), (A1-3)+(B2-27), (A1-3)+(B2-28), (A1-3)+(B2-29), (A1-3)+(B3-1), (A1-3)+(B3-2), (A1-3)+(B4-1), (A1-3)+(B4-2), (A1-3)+(B4-3), (A1-3)+(B4-4), (A1-3)+(B4-5), (A1-3)+(B4-6), (A1-3)+(B4-7), (A1-3)+(B4-8), (A1-3)+(B4-9), (A1-3)+(B4-10), (A1-3)+(B4-11), (A1-3)+(B5-1), (A1-3)+(B5-2), (A1-3)+(B5-3), (A1-3)+(B5-4), (A1-3)+(B5-5), (A1-3)+(B6-1), (A1-3)+(B6-2), (A1-3)+(B6-3), (A1-3)+(B6-4), (A1-3)+(B6-5), (A1-3)+(B6-6), (A1-3)+(B7-1), (A1-3)+(B7-2), (A1-3)+(B7-3), (A1-3)+(B7-4), (A1-3)+(B7-5), (A1-3)+(B7-6), (A1-3)+(B7-7)), (A1-3)+(B7-7), (A1-3)+(B7-8), (A1-3)+(B8-1), (A1-3)+(B8-2), (A1-3)+(B8-3), (A1-3)+(B8-4), (A1-3)+(B8-5), (A1-3)+(B8-6), (A1-3)+(B8-7), (A1-3)+(B9-1), (A1-3)+(B9-2), (A1-3)+(B10-1), (A1-3)+(B10-2), (A1-3)+(B10-3), (A1-3)+(B10-4), (A1-3)+(B10-5), (A1-3)+(B11-1), (A1-3)+(B11-2), (A1-3)+(B11-2),
(A1-4)+(B1-1), (A1-4)+(B1-2), (A1-4)+(B1-3), (A1-4)+(B1-4), (A1-4)+(B1-5), (A1-4)+(B1-6), (A1-4)+(B2-1), (A1-4)+(B2-2), (A1-4)+(B2-3), (A1-4)+(B2-4), (A1-4)+(B2-5), (A1-4)+(B2-6), (A1-4)+(B2-7), (A1-4)+(B2-8), (A1-4)+(B2-9), (A1-4)+(B2-10), (A1-4)+(B2-11), (A1-4)+(B2-12), (A1-4)+(B2-13), (A1-4)+(B2-14), (A1-4)+(B2-15), (A1-4)+(B2-16), (A1-4)+(B2-17), (A1-4)+(B2-18), (A1-4)+(B2-19), (A1-4)+(B2-20), (A1-4)+(B2-21), (A1-4)+(B2-22), (A1-4)+(B2-23), (A1-4)+(B2-24), (A1-4)+(B2-25), (A1-4)+(B2-26), (A1-4)+(B2-27), (A1-4)+(B2-28), (A1-4)+(B2-29), (A1-4)+(B3-1), (A1-4)+(B3-2), (A1-4)+(B4-1), (A1-4)+(B4-2), (A1-4)+(B4-3), (A1-4)+(B4-4), (A1-4)+(B4-5), (A1-4)+(B4-6), (A1-4)+(B4-7), (A1-4)+(B4-8), (A1-4)+(B4-9), (A1-4)+(B4-10), (A1-4)+(B4-11), (A1-4)+(B5-1), (A1-4)+(B5-2), (A1-4)+(B5-3), (A1-4)+(B5-4), (A1-4)+(B5-5), (A1-4)+(B6-1), (A1-4)+(B6-2), (A1-4)+(B6-3), (A1-4)+(B6-4), (A1-4)+(B6-5), (A1-4)+(B6-6), (A1-4)+(B7-1), (A1-4)+(B7-2), (A1-4)+(B7-3), (A1-4)+(B7-4), (A1-4)+(B7-5), (A1-4)+(B7-6), (A1-4)+(B7-7)), (A1-4)+(B7-7), (A1-4)+(B7-8), (A1-4)+(B8-1), (A1-4)+(B8-2), (A1-4)+(B8-3), (A1-4)+(B8-4), (A1-4)+(B8-5), (A1-4)+(B8-6), (A1-4)+(B8-7), (A1-4)+(B9-1), (A1-4)+(B9-2), (A1-4)+(B10-1), (A1-4)+(B10-2), (A1-4)+(B10-3), (A1-4)+(B10-4), (A1-4)+(B10-5), (A1-4)+(B11-1), (A1-4)+(B11-2), (A1-4)+(B11-2),
(A1-5)+(B1-1), (A1-5)+(B1-2), (A1-5)+(B1-3), (A1-5)+(B1-4), (A1-5)+(B1-5), (A1-5)+(B1-6), (A1-5)+(B2-1), (A1-5)+(B2-2), (A1-5)+(B2-3), (A1-5)+(B2-4), (A1-5)+(B2-5), (A1-5)+(B2-6), (A1-5)+(B2-7), (A1-5)+(B2-8), (A1-5)+(B2-9), (A1-5)+(B2-10), (A1-5)+(B2-11), (A1-5)+(B2-12), (A1-5)+(B2-13), (A1-5)+(B2-14), (A1-5)+(B2-15), (A1-5)+(B2-16), (A1-5)+(B2-17), (A1-5)+(B2-18), (A1-5)+(B2-19), (A1-5)+(B2-20), (A1-5)+(B2-21), (A1-5)+(B2-22), (A1-5)+(B2-23), (A1-5)+(B2-24), (A1-5)+(B2-25), (A1-5)+(B2-26), (A1-5)+(B2-27), (A1-5)+(B2-28), (A1-5)+(B2-29), (A1-5)+(B3-1), (A1-5)+(B3-2), (A1-5)+(B4-1), (A1-5)+(B4-2), (A1-5)+(B4-3), (A1-5)+(B4-4), (A1-5)+(B4-5), (A1-5)+(B4-6), (A1-5)+(B4-7), (A1-5)+(B4-8), (A1-5)+(B4-9), (A1-5)+(B4-10), (A1-5)+(B4-11), (A1-5)+(B5-1), (A1-5)+(B5-2), (A1-5)+(B5-3), (A1-5)+(B5-4), (A1-5)+(B5-5), (A1-5)+(B6-1), (A1-5)+(B6-2), (A1-5)+(B6-3), (A1-5)+(B6-4), (A1-5)+(B6-5), (A1-5)+(B6-6), (A1-5)+(B7-1), (A1-5)+(B7-2), (A1-5)+(B7-3), (A1-5)+(B7-4), (A1-5)+(B7-5), (A1-5)+(B7-6), (A1-5)+(B7-7)), (A1-5)+(B7-7), (A1-5)+(B7-8), (A1-5)+(B8-1), (A1-5)+(B8-2), (A1-5)+(B8-3), (A1-5)+(B8-4), (A1-5)+(B8-5), (A1-5)+(B8-6), (A1-5)+(B8-7), (A1-5)+(B9-1), (A1-5)+(B9-2), (A1-5)+(B10-1), (A1-5)+(B10-2), (A1-5)+(B10-3), (A1-5)+(B10-4), (A1-5)+(B10-5), (A1-5)+(B11-1), (A1-5)+(B11-2), (A1-5)+(B11-2),
(A1-6)+(B1-1), (A1-6)+(B1-2), (A1-6)+(B1-3), (A1-6)+(B1-4), (A1-6)+(B1-5), (A1-6)+(B1-6), (A1-6)+(B2-1), (A1-6)+(B2-2), (A1-6)+(B2-3), (A1-6)+(B2-4), (A1-6)+(B2-5), (A1-6)+(B2-6), (A1-6)+(B2-7), (A1-6)+(B2-8), (A1-6)+(B2-9), (A1-6)+(B2-10), (A1-6)+(B2-11), (A1-6)+(B2-12), (A1-6)+(B2-13), (A1-6)+(B2-14), (A1-6)+(B2-15), (A1-6)+(B2-16), (A1-6)+(B2-17), (A1-6)+(B2-18), (A1-6)+(B2-19), (A1-6)+(B2-20), (A1-6)+(B2-21), (A1-6)+(B2-22), (A1-6)+(B2-23), (A1-6)+(B2-24), (A1-6)+(B2-25), (A1-6)+(B2-26), (A1-6)+(B2-27), (A1-6)+(B2-28), (A1-6)+(B2-29), (A1-6)+(B3-1), (A1-6)+(B3-2), (A1-6)+(B4-1), (A1-6)+(B4-2), (A1-6)+(B4-3), (A1-6)+(B4-4), (A1-6)+(B4-5), (A1-6)+(B4-6), (A1-6)+(B4-7), (A1-6)+(B4-8), (A1-6)+(B4-9), (A1-6)+(B4-10), (A1-6)+(B4-11), (A1-6)+(B5-1), (A1-6)+(B5-2), (A1-6)+(B5-3), (A1-6)+(B5-4), (A1-6)+(B5-5), (A1-6)+(B6-1), (A1-6)+(B6-2), (A1-6)+(B6-3), (A1-6)+(B6-4), (A1-6)+(B6-5), (A1-6)+(B6-6), (A1-6)+(B7-1), (A1-6)+(B7-2), (A1-6)+(B7-3), (A1-6)+(B7-4), (A1-6)+(B7-5), (A1-6)+(B7-6), (A1-6)+(B7-7)), (A1-6)+(B7-7), (A1-6)+(B7-8), (A1-6)+(B8-1), (A1-6)+(B8-2), (A1-6)+(B8-3), (A1-6)+(B8-4), (A1-6)+(B8-5), (A1-6)+(B8-6), (A1-6)+(B8-7), (A1-6)+(B9-1), (A1-6)+(B9-2), (A1-6)+(B10-1), (A1-6)+(B10-2), (A1-6)+(B10-3), (A1-6)+(B10-4), (A1-6)+(B10-5), (A1-6)+(B11-1), (A1-6)+(B11-2), (A1-6)+(B11-2),
(A1-7)+(B1-1), (A1-7)+(B1-2), (A1-7)+(B1-3), (A1-7)+(B1-4), (A1-7)+(B1-5), (A1-7)+(B1-6), (A1-7)+(B2-1), (A1-7)+(B2-2), (A1-7)+(B2-3), (A1-7)+(B2-4), (A1-7)+(B2-5), (A1-7)+(B2-6), (A1-7)+(B2-7), (A1-7)+(B2-8), (A1-7)+(B2-9), (A1-7)+(B2-10), (A1-7)+(B2-11), (A1-7)+(B2-12), (A1-7)+(B2-13), (A1-7)+(B2-14), (A1-7)+(B2-15), (A1-7)+(B2-16), (A1-7)+(B2-17), (A1-7)+(B2-18), (A1-7)+(B2-19), (A1-7)+(B2-20), (A1-7)+(B2-21), (A1-7)+(B2-22), (A1-7)+(B2-23), (A1-7)+(B2-24), (A1-7)+(B2-25), (A1-7)+(B2-26), (A1-7)+(B2-27), (A1-7)+(B2-28), (A1-7)+(B2-29), (A1-7)+(B3-1), (A1-7)+(B3-2), (A1-7)+(B4-1), (A1-7)+(B4-2), (A1-7)+(B4-3), (A1-7)+(B4-4), (A1-7)+(B4-5), (A1-7)+(B4-6), (A1-7)+(B4-7), (A1-7)+(B4-8), (A1-7)+(B4-9), (A1-7)+(B4-10), (A1-7)+(B4-11), (A1-7)+(B5-1), (A1-7)+(B5-2), (A1-7)+(B5-3), (A1-7)+(B5-4), (A1-7)+(B5-5), (A1-7)+(B6-1), (A1-7)+(B6-2), (A1-7)+(B6-3), (A1-7)+(B6-4), (A1-7)+(B6-5), (A1-7)+(B6-6), (A1-7)+(B7-1), (A1-7)+(B7-2), (A1-7)+(B7-3), (A1-7)+(B7-4), (A1-7)+(B7-5), (A1-7)+(B7-6), (A1-7)+(B7-7)), (A1-7)+(B7-7), (A1-7)+(B7-8), (A1-7)+(B8-1), (A1-7)+(B8-2), (A1-7)+(B8-3), (A1-7)+(B8-4), (A1-7)+(B8-5), (A1-7)+(B8-6), (A1-7)+(B8-7), (A1-7)+(B9-1), (A1-7)+(B9-2), (A1-7)+(B10-1), (A1-7)+(B10-2), (A1-7)+(B10-3), (A1-7)+(B10-4), (A1-7)+(B10-5), (A1-7)+(B11-1), (A1-7)+(B11-2), (A1-7)+(B11-2),
(A1-8)+(B1-1), (A1-8)+(B1-2), (A1-8)+(B1-3), (A1-8)+(B1-4), (A1-8)+(B1-5), (A1-8)+(B1-6), (A1-8)+(B2-1), (A1-8)+(B2-2), (A1-8)+(B2-3), (A1-8)+(B2-4), (A1-8)+(B2-5), (A1-8)+(B2-6), (A1-8)+(B2-7), (A1-8)+(B2-8), (A1-8)+(B2-9), (A1-8)+(B2-10), (A1-8)+(B2-11), (A1-8)+(B2-12), (A1-8)+(B2-13), (A1-8)+(B2-14), (A1-8)+(B2-15), (A1-8)+(B2-16), (A1-8)+(B2-17), (A1-8)+(B2-18), (A1-8)+(B2-19), (A1-8)+(B2-20), (A1-8)+(B2-21), (A1-8)+(B2-22), (A1-8)+(B2-23), (A1-8)+(B2-24), (A1-8)+(B2-25), (A1-8)+(B2-26), (A1-8)+(B2-27), (A1-8)+(B2-28), (A1-8)+(B2-29), (A1-8)+(B3-1), (A1-8)+(B3-2), (A1-8)+(B4-1), (A1-8)+(B4-2), (A1-8)+(B4-3), (A1-8)+(B4-4), (A1-8)+(B4-5), (A1-8)+(B4-6), (A1-8)+(B4-7), (A1-8)+(B4-8), (A1-8)+(B4-9), (A1-8)+(B4-10), (A1-8)+(B4-11), (A1-8)+(B5-1), (A1-8)+(B5-2), (A1-8)+(B5-3), (A1-8)+(B5-4), (A1-8)+(B5-5), (A1-8)+(B6-1), (A1-8)+(B6-2), (A1-8)+(B6-3), (A1-8)+(B6-4), (A1-8)+(B6-5), (A1-8)+(B6-6), (A1-8)+(B7-1), (A1-8)+(B7-2), (A1-8)+(B7-3), (A1-8)+(B7-4), (A1-8)+(B7-5), (A1-8)+(B7-6), (A1-8)+(B7-7)), (A1-8)+(B7-7), (A1-8)+(B7-8), (A1-8)+(B8-1), (A1-8)+(B8-2), (A1-8)+(B8-3), (A1-8)+(B8-4), (A1-8)+(B8-5), (A1-8)+(B8-6), (A1-8)+(B8-7), (A1-8)+(B9-1), (A1-8)+(B9-2), (A1-8)+(B10-1), (A1-8)+(B10-2), (A1-8)+(B10-3), (A1-8)+(B10-4), (A1-8)+(B10-5), (A1-8)+(B11-1), (A1-8)+(B11-2), (A1-8)+(B11-2),
(A1-9)+(B1-1), (A1-9)+(B1-2), (A1-9)+(B1-3), (A1-9)+(B1-4), (A1-9)+(B1-5), (A1-9)+(B1-6), (A1-9)+(B2-1), (A1-9)+(B2-2), (A1-9)+(B2-3), (A1-9)+(B2-4), (A1-9)+(B2-5), (A1-9)+(B2-6), (A1-9)+(B2-7), (A1-9)+(B2-8), (A1-9)+(B2-9), (A1-9)+(B2-10), (A1-9)+(B2-11), (A1-9)+(B2-12), (A1-9)+(B2-13), (A1-9)+(B2-14), (A1-9)+(B2-15), (A1-9)+(B2-16), (A1-9)+(B2-17), (A1-9)+(B2-18), (A1-9)+(B2-19), (A1-9)+(B2-20), (A1-9)+(B2-21), (A1-9)+(B2-22), (A1-9)+(B2-23), (A1-9)+(B2-24), (A1-9)+(B2-25), (A1-9)+(B2-26), (A1-9)+(B2-27), (A1-9)+(B2-28), (A1-9)+(B2-29), (A1-9)+(B3-1), (A1-9)+(B3-2), (A1-9)+(B4-1), (A1-9)+(B4-2), (A1-9)+(B4-3), (A1-9)+(B4-4), (A1-9)+(B4-5), (A1-9)+(B4-6), (A1-9)+(B4-7), (A1-9)+(B4-8), (A1-9)+(B4-9), (A1-9)+(B4-10), (A1-9)+(B4-11), (A1-9)+(B5-1), (A1-9)+(B5-2), (A1-9)+(B5-3), (A1-9)+(B5-4), (A1-9)+(B5-5), (A1-9)+(B6-1), (A1-9)+(B6-2), (A1-9)+(B6-3), (A1-9)+(B6-4), (A1-9)+(B6-5), (A1-9)+(B6-6), (A1-9)+(B7-1), (A1-9)+(B7-2), (A1-9)+(B7-3), (A1-9)+(B7-4), (A1-9)+(B7-5), (A1-9)+(B7-6), (A1-9)+(B7-7)), (A1-9)+(B7-7), (A1-9)+(B7-8), (A1-9)+(B8-1), (A1-9)+(B8-2), (A1-9)+(B8-3), (A1-9)+(B8-4), (A1-9)+(B8-5), (A1-9)+(B8-6), (A1-9)+(B8-7), (A1-9)+(B9-1), (A1-9)+(B9-2), (A1-9)+(B10-1), (A1-9)+(B10-2), (A1-9)+(B10-3), (A1-9)+(B10-4), (A1-9)+(B10-5), (A1-9)+(B11-1), (A1-9)+(B11-2), (A1-9)+(B11-2),
(A1-10)+(B1-1), (A1-10)+(B1-2), (A1-10)+(B1-3), (A1-10)+(B1-4), (A1-10)+(B1-5), (A1-10)+(B1-6), (A1-10)+(B2-1), (A1-10)+(B2-2), (A1-10)+(B2-3), (A1-10)+(B2-4), (A1-10)+(B2-5), (A1-10)+(B2-6), (A1-10)+(B2-7), (A1-10)+(B2-8), (A1-10)+(B2-9), (A1-10)+(B2-1 0), (A1-10)+(B2-11), (A1-10)+(B2-12), (A1-10)+(B2-13), (A1-10)+(B2-14), (A1-10)+(B2-15), (A1-10)+(B2-16), (A1-10)+(B2-17), (A1-10)+(B2-18), (A1-10)+(B2-19), (A1-10)+(B2-20), (A1-10)+(B2-21), (A1-10)+(B2-22), (A1-10)+(B2-23), (A1-10)+(B2-24), (A1-10)+(B2-25), (A1-10)+(B2-26), (A1-10)+(B2-27), (A1-10)+(B2-28), (A1-10)+(B2-29), (A1-10)+(B3-1), (A1-10)+(B3-2), (A1-10)+(B4-1), (A1-10)+(B4-2), (A1-10)+(B4-3), (A1-10)+(B4-4), (A1-10)+(B4-5), (A1-10)+(B4-6), (A1-10)+(B4-7), (A1-10)+(B4-8), (A1-10)+(B4-9), (A1-10)+(B4-1 0), (A1-10)+(B4-11), (A1-10)+(B5-1), (A1-10)+(B5-2), (A1-10)+(B5-3), (A1-10)+(B5-4), (A1-10)+(B5-5), (A1-10)+(B6-1), (A1-10)+(B6-2), (A1-10)+(B6-3), (A1-10)+(B6-4), (A1-10)+(B6-5), (A1-10)+(B6-6), (A1-10)+(B7-1), (A1-10)+(B7-2), (A1-10)+(B7-3), (A1-10)+(B7-4), (A1-10)+(B7-5), (A1-10)+(B7-6), (A1-10)+(B7-7)), (A1-10)+(B7-7), (A1-10)+(B7-8), (A1-10)+(B8-1), (A1-10)+(B8-2), (A1-10)+(B8-3), (A1-10)+(B8-4), (A1-10)+(B8-5), (A1-10)+(B8-6), (A1-10)+(B8-7), (A1 -10)+(B9-1), (A1-10)+(B9-2), (A1-10)+(B10-1), (A1-10)+(B10-2), (A1-10)+(B10-3), (A1-10)+(B10-4), (A1-10)+(B10-5), (A1-10)+(B11-1), (A1-10)+(B11-2), (A1-10)+(B11-2),
(A1-11)+(B1-1), (A1-11)+(B1-2), (A1-11)+(B1-3), (A1-11)+(B1-4), (A1-11)+(B1-5), (A1-11)+(B1-6), (A1-11)+(B2-1), (A1-11)+(B2-2), (A1-11)+(B2-3), (A1-11)+(B2-4), (A1-11)+(B2-5), (A1-11)+(B2-6), (A1-11)+(B2-7), (A1-11)+(B2-8), (A1-11)+(B2-9), (A1-11)+(B2-10), (A1-11)+(B2-11), (A1-11)+(B2-12), (A1-11)+(B2-13), (A1-11)+(B2-14), (A1-11)+(B2-15), (A1-11)+(B2-16), (A1-11)+(B2-17), (A1-11)+(B2-18), (A1-11)+(B2-19), (A1-11)+(B2-20), (A1-11)+(B2-21), (A1-11)+(B2-22), (A1-11)+(B2-23), (A1-11)+(B2-24), (A1-1 1)+(B2-25), (A1-11)+(B2-26), (A1-11)+(B2-27), (A1-11)+(B2-28), (A1-1 1)+(B2-29), (A1-11)+(B3-1), (A1-11)+(B3-2), (A1-11)+(B4-1), (A1-11)+(B4-2), (A1-11)+(B4-3), (A1-11)+(B4-4), (A1-11)+(B4-5), (A1-11)+(B4-6), (A1-11)+(B4-7), (A1-11)+(B4-8), (A1-11)+(B4-9), (A1-11)+(B4-10), (A1-11)+(B4-11), (A1-11)+(B5-1), (A1-11)+(B5-2), (A1-11)+(B5-3), (A1-11)+(B5-4), (A1-11)+(B5-5), (A1-11)+(B6-1), (A1-11)+(B6-2), (A1-11)+(B6-3), (A1-11)+(B6-4), (A1-11)+(B6-5), (A1-11)+(B6-6), (A1-11)+(B7-1), (A1-11)+(B7-2), (A1-11)+(B7-3), (A1-11)+(B7-4), (A1-11)+(B7-5), (A1-11)+(B7-6), (A1-11)+(B7-7)), (A1-11)+(B7-7), (A1-11)+(B7-8), (A1-11)+(B8-1), (A1-11)+(B8-2), (A1-11)+(B8-3), (A1-11)+(B8-4), (A1-11)+(B8-5), (A1-11)+(B8-6), (A1-11)+(B8-7), (A1-11)+(B9-1), (A1-11)+(B9-2), (A1-11)+(B10-1), (A1-11)+(B10-2), (A1-11)+(B10-3), (A1-11)+(B10-4), (A1-11)+(B10-5), (A1-11)+(B11-1), (A1-11)+(B11-2), (A1-11)+(B11-2),
(A1-12)+(B1-1), (A1-12)+(B1-2), (A1-12)+(B1-3), (A1-12)+(B1-4), (A1-12)+(B1-5), (A1-12)+(B1-6), (A1-12)+(B2-1), (A1-12)+(B2-2), (A1-12)+(B2-3), (A1-12)+(B2-4), (A1-12)+(B2-5), (A1-12)+(B2-6), (A1-12)+(B2-7), (A1-12)+(B2-8), (A1-12)+(B2-9), (A1-12)+(B2-10), (A1-12)+(B2-11), (A1-12)+(B2-12), (A1-12)+(B2-13), (A1-12)+(B2-14), (A1-12)+(B2-15), (A1-12)+(B2-16), (A1-12)+(B2-17), (A1-12)+(B2-18), (A1-12)+(B2-19), (A1-12)+(B2-20), (A1-12)+(B2-21), (A1-12)+(B2-22), (A1-12)+(B2-23), (A1-12)+(B2-24), (A1-12)+(B2-25), (A1-12)+(B2-26), (A1-12)+(B2-27), (A1-12)+(B2-28), (A1-12)+(B2-29), (A1-12)+(B3-1), (A1-12)+(B3-2), (A1-12)+(B4-1), (A1-12)+(B4-2), (A1-12)+(B4-3), (A1-12)+(B4-4), (A1-12)+(B4-5), (A1-12)+(B4-6), (A1-12)+(B4-7), (A1-12)+(B4-8), (A1-12)+(B4-9), (A1-12)+(B4-10), (A1-12)+(B4-11), (A1-12)+(B5-1), (A1-12)+(B5-2), (A1-12)+(B5-3), (A1-12)+(B5-4), (A1-12)+(B5-5), (A1-12)+(B6-1), (A1-12)+(B6-2), (A1-12)+(B6-3), (A1-12)+(B6-4), (A1-12)+(B6-5), (A1-12)+(B6-6), (A1-12)+(B7-1), (A1-12)+(B7-2), (A1-12)+(B7-3), (A1-12)+(B7-4), (A1-12)+(B7-5), (A1-12)+(B7-6), (A1-12)+(B7-7)), (A1-12)+(B7-7), (A1-12)+(B7-8), (A1-12)+(B8-1), (A1-12)+(B8-2), (A1-12)+(B8-3), (A1-12)+(B8-4), (A1-12)+(B8-5), (A1-12)+(B8-6), (A1-12)+(B8-7), (A1-12)+(B9-1), (A1-12)+(B9-2), (A1-12)+(B10-1), (A1-12)+(B10-2), (A1-12)+(B10-3), (A1-12)+(B10-4), (A1-12)+(B10-5), (A1-12)+(B11-1), (A1-12)+(B11-2), (A1-12)+(B11-2),
(A1-13)+(B1-1), (A1-13)+(B1-2), (A1-13)+(B1-3), (A1-13)+(B1-4), (A1-13)+(B1-5), (A1-13)+(B1-6), (A1-13)+(B2-1), (A1-13)+(B2-2), (A1-13)+(B2-3), (A1-13)+(B2-4), (A1-13)+(B2-5), (A1-13)+(B2-6), (A1-13)+(B2-7), (A1-13)+(B2-8), (A1-13)+(B2-9), (A1-13)+(B2-10), (A1-13)+(B2-11), (A1-13)+(B2-12), (A1-13)+(B2-13), (A1-13)+(B2-14), (A1-13)+(B2-15), (A1-13)+(B2-16), (A1-13)+(B2-17), (A1-13)+(B2-18), (A1-13)+(B2-19), (A1-13)+(B2-20), (A1-13)+(B2-21), (A1-13)+(B2-22), (A1-13)+(B2-23), (A1-13)+(B2-24), (A1-13)+(B2-25), (A1-13)+(B2-26), (A1-13)+(B2-27), (A1-13)+(B2-28), (A1-13)+(B2-29), (A1-13)+(B3-1), (A1-13)+(B3-2), (A1-13)+(B4-1), (A1-13)+(B4-2), (A1-13)+(B4-3), (A1-13)+(B4-4), (A1-13)+(B4-5), (A1-13)+(B4-6), (A1-13)+(B4-7), (A1-13)+(B4-8), (A1-13)+(B4-9), (A1-13)+(B4-10), (A1-13)+(B4-11), (A1-13)+(B5-1), (A1-13)+(B5-2), (A1-13)+(B5-3), (A1-13)+(B5-4), (A1-13)+(B5-5), (A1-13)+(B6-1), (A1-13)+(B6-2), (A1-13)+(B6-3), (A1-13)+(B6-4), (A1-13)+(B6-5), (A1-13)+(B6-6), (A1-13)+(B7-1), (A1-13)+(B7-2), (A1-13)+(B7-3), (A1-13)+(B7-4), (A1-13)+(B7-5), (A1-13)+(B7-6), (A1-13)+(B7-7)), (A1-13)+(B7-7), (A1-13)+(B7-8), (A1-13)+(B8-1), (A1-13)+(B8-2), (A1-13)+(B8-3), (A1-13)+(B8-4), (A1-13)+(B8-5), (A1-13)+(B8-6), (A1-13)+(B8-7), (A1-13)+(B9-1), (A1-13)+(B9-2), (A1-13)+(B10-1), (A1-13)+(B10-2), (A1-13)+(B10-3), (A1-13)+(B10-4), (A1-13)+(B10-5), (A1-13)+(B11-1), (A1-13)+(B11-2), (A1-13)+(B11-2),
(A1-14)+(B1-1), (A1-14)+(B1-2), (A1-14)+(B1-3), (A1-14)+(B1-4), (A1-14)+(B1-5), (A1-14)+(B1-6), (A1-14)+(B2-1), (A1-14)+(B2-2), (A1-14)+(B2-3), (A1-14)+(B2-4), (A1-14)+(B2-5), (A1-14)+(B2-6), (A1-14)+(B2-7), (A1-14)+(B2-8), (A1-14)+(B2-9), (A1-14)+(B2-10), (A1-14)+(B2-11), (A1-14)+(B2-12), (A1-14)+(B2-13), (A1-14)+(B2-14), (A1-14)+(B2-15), (A1-14)+(B2-16), (A1-14)+(B2-17), (A1-14)+(B2-18), (A1-14)+(B2-19), (A1-14)+(B2-20), (A1-14)+(B2-21), (A1-14)+(B2-22), (A1-14)+(B2-23), (A1-14)+(B2-24), (A1-14)+(B2-25), (A1-14)+(B2-26), (A1-14)+(B2-27), (A1-14)+(B2-28), (A1-14)+(B2-29), (A1-14)+(B3-1), (A1-14)+(B3-2), (A1-14)+(B4-1), (A1-14)+(B4-2), (A1-14)+(B4-3), (A1-14)+(B4-4), (A1-14)+(B4-5), (A1-14)+(B4-6), (A1-14)+(B4-7), (A1-14)+(B4-8), (A1-14)+(B4-9), (A1-14)+(B4-10), (A1-14)+(B4-11), (A1-14)+(B5-1), (A1-14)+(B5-2), (A1-14)+(B5-3), (A1-14)+(B5-4), (A1-14)+(B5-5), (A1-14)+(B6-1), (A1-14)+(B6-2), (A1-14)+(B6-3), (A1-14)+(B6-4), (A1-14)+(B6-5), (A1-14)+(B6-6), (A1-14)+(B7-1), (A1-14)+(B7-2), (A1-14)+(B7-3), (A1-14)+(B7-4), (A1-14)+(B7-5), (A1-14)+(B7-6), (A1-14)+(B7-7)), (A1-14)+(B7-7), (A1-14)+(B7-8), (A1-14)+(B8-1), (A1-14)+(B8-2), (A1-14)+(B8-3), (A1-14)+(B8-4), (A1-14)+(B8-5), (A1-14)+(B8-6), (A1-14)+(B8-7), (A1-14)+(B9-1), (A1-14)+(B9-2), (A1-14)+(B10-1), (A1-14)+(B10-2), (A1-14)+(B10-3), (A1-14)+(B10-4), (A1-14)+(B10-5), (A1-14)+(B11-1), (A1-14)+(B11-2), (A1-14)+(B11-2),
(A1-15)+(B1-1), (A1-15)+(B1-2), (A1-15)+(B1-3), (A1-15)+(B1-4), (A1-15)+(B1-5), (A1-15)+(B1-6), (A1-15)+(B2-1), (A1-15)+(B2-2), (A1-15)+(B2-3), (A1-15)+(B2-4), (A1-15)+(B2-5), (A1-15)+(B2-6), (A1-15)+(B2-7), (A1-15)+(B2-8), (A1-15)+(B2-9), (A1-15)+(B2-10), (A1-15)+(B2-11), (A1-15)+(B2-12), (A1-15)+(B2-13), (A1-15)+(B2-14), (A1-15)+(B2-15), (A1-15)+(B2-16), (A1-15)+(B2-17), (A1-15)+(B2-18), (A1-15)+(B2-19), (A1-15)+(B2-20), (A1-15)+(B2-21), (A1-15)+(B2-22), (A1-15)+(B2-23), (A1-15)+(B2-24), (A1-15)+(B2-25), (A1-15)+(B2-26), (A1-15)+(B2-27), (A1-15)+(B2-28), (A1-15)+(B2-29), (A1-15)+(B3-1), (A1-15)+(B3-2), (A1-15)+(B4-1), (A1-15)+(B4-2), (A1-15)+(B4-3), (A1-15)+(B4-4), (A1-15)+(B4-5), (A1-15)+(B4-6), (A1-15)+(B4-7), (A1-15)+(B4-8), (A1-15)+(B4-9), (A1-15)+(B4-10), (A1-15)+(B4-11), (A1-15)+(B5-1), (A1-15)+(B5-2), (A1-15)+(B5-3), (A1-15)+(B5-4), (A1-15)+(B5-5), (A1-15)+(B6-1), (A1-15)+(B6-2), (A1-15)+(B6-3), (A1-15)+(B6-4), (A1-15)+(B6-5), (A1-15)+(B6-6), (A1-15)+(B7-1), (A1-15)+(B7-2), (A1-15)+(B7-3), (A1-15)+(B7-4), (A1-15)+(B7-5), (A1-15)+(B7-6), (A1-15)+(B7-7)), (A1-15)+(B7-7), (A1-15)+(B7-8), (A1-15)+(B8-1), (A1-15)+(B8-2), (A1-15)+(B8-3), (A1-15)+(B8-4), (A1-15)+(B8-5), (A1-15)+(B8-6), (A1-15)+(B8-7), (A1-15)+(B9-1), (A1-15)+(B9-2), (A1-15)+(B10-1), (A1-15)+(B10-2), (A1-15)+(B10-3), (A1-15)+(B10-4), (A1-15)+(B10-5), (A1-15)+(B11-1), (A1-15)+(B11-2), (A1-15)+(B11-2),
(A1-16)+(B1-1), (A1-16)+(B1-2), (A1-16)+(B1-3), (A1-16)+(B1-4), (A1-16)+(B1-5), (A1-16)+(B1-6), (A1-16)+(B2-1), (A1-16)+(B2-2), (A1-16)+(B2-3), (A1-16)+(B2-4), (A1-16)+(B2-5), (A1-16)+(B2-6), (A1-16)+(B2-7), (A1-16)+(B2-8), (A1-16)+(B2-9), (A1-16)+(B2-10), (A1-16)+(B2-11), (A1-16)+(B2-12), (A1-16)+(B2-13), (A1-16)+(B2-14), (A1-16)+(B2-15), (A1-16)+(B2-16), (A1-16)+(B2-17), (A1-16)+(B2-18), (A1-16)+(B2-19), (A1-16)+(B2-20), (A1-16)+(B2-21), (A1-16)+(B2-22), (A1-16)+(B2-23), (A1-16)+(B2-24), (A1-16)+(B2-25), (A1-16)+(B2-26), (A1-16)+(B2-27), (A1-16)+(B2-28), (A1-16)+(B2-29), (A1-16)+(B3-1), (A1-16)+(B3-2), (A1-16)+(B4-1), (A1-16)+(B4-2), (A1-16)+(B4-3), (A1-16)+(B4-4), (A1-16)+(B4-5), (A1-16)+(B4-6), (A1-16)+(B4-7), (A1-16)+(B4-8), (A1-16)+(B4-9), (A1-16)+(B4-10), (A1-16)+(B4-11), (A1-16)+(B5-1), (A1-16)+(B5-2), (A1-16)+(B5-3), (A1-16)+(B5-4), (A1-16)+(B5-5), (A1-16)+(B6-1), (A1-16)+(B6-2), (A1-16)+(B6-3), (A1-16)+(B6-4), (A1-16)+(B6-5), (A1-16)+(B6-6), (A1-16)+(B7-1), (A1-16)+(B7-2), (A1-16)+(B7-3), (A1-16)+(B7-4), (A1-16)+(B7-5), (A1-16)+(B7-6), (A1-16)+(B7-7)), (A1-16)+(B7-7), (A1-16)+(B7-8), (A1-16)+(B8-1), (A1-16)+(B8-2), (A1-16)+(B8-3), (A1-16)+(B8-4), (A1-16)+(B8-5), (A1-16)+(B8-6), (A1-16)+(B8-7), (A1-16)+(B9-1), (A1-16)+(B9-2), (A1-16)+(B10-1), (A1-16)+(B10-2), (A1-16)+(B10-3), (A1-16)+(B10-4), (A1-16)+(B10-5), (A1-16)+(B11-1), (A1-16)+(B11-2), (A1-16)+(B11-2),
(A1-17)+(B1-1), (A1-17)+(B1-2), (A1-17)+(B1-3), (A1-17)+(B1-4), (A1-17)+(B1-5), (A1-17)+(B1-6), (A1-17)+(B2-1), (A1-17)+(B2-2), (A1-17)+(B2-3), (A1-17)+(B2-4), (A1-17)+(B2-5), (A1-17)+(B2-6), (A1-17)+(B2-7), (A1-17)+(B2-8), (A1-17)+(B2-9), (A1-17)+(B2-10), (A1-17)+(B2-11), (A1-17)+(B2-12), (A1-17)+(B2-13), (A1-17)+(B2-14), (A1-17)+(B2-15), (A1-17)+(B2-16), (A1-17)+(B2-17), (A1-17)+(B2-18), (A1-17)+(B2-19), (A1-17)+(B2-20), (A1-17)+(B2-21), (A1-17)+(B2-22), (A1-17)+(B2-23), (A1-17)+(B2-24), (A1-17)+(B2-25), (A1-17)+(B2-26), (A1-17)+(B2-27), (A1-17)+(B2-28), (A1-17)+(B2-29), (A1-17)+(B3-1), (A1-17)+(B3-2), (A1-17)+(B4-1), (A1-17)+(B4-2), (A1-17)+(B4-3), (A1-17)+(B4-4), (A1-17)+(B4-5), (A1-17)+(B4-6), (A1-17)+(B4-7), (A1-17)+(B4-8), (A1-17)+(B4-9), (A1-17)+(B4-1 0), (A1-17)+(B4-11), (A1-17)+(B5-1), (A1-17)+(B5-2), (A1-17)+(B5-3), (A1-17)+(B5-4), (A1-17)+(B5-5), (A1-17)+(B6-1), (A1-17)+(B6-2), (A1-17)+(B6-3), (A1-17)+(B6-4), (A1-17)+(B6-5), (A1-17)+(B6-6), (A1-17)+(B7-1), (A1-17)+(B7-2), (A1-17)+(B7-3), (A1-17)+(B7-4), (A1-17)+(B7-5), (A1-17)+(B7-6), (A1-17)+(B7-7)), (A1-17)+(B7-7), (A1-17)+(B7-8), (A1-17)+(B8-1), (A1-17)+(B8-2), (A1-17)+(B8-3), (A1-17)+(B8-4), (A1-17)+(B8-5), (A1-17)+(B8-6), (A1-17)+(B8-7), (A1-17)+(B9-1), (A1-17)+(B9-2), (A1-17)+(B10-1), (A1-17)+(B10-2), (A1-17)+(B10-3), (A1-17)+(B10-4), (A1-17)+(B10-5), (A1-17)+(B11-1), (A1-17)+(B11-2), (A1-17)+(B11-2),
(A1-18)+(B1-1), (A1-18)+(B1-2), (A1-18)+(B1-3), (A1-18)+(B1-4), (A1-18)+(B1-5), (A1-18)+(B1-6), (A1-18)+(B2-1), (A1-18)+(B2-2), (A1-18)+(B2-3), (A1-18)+(B2-4), (A1-18)+(B2-5), (A1-18)+(B2-6), (A1-18)+(B2-7), (A1-18)+(B2-8), (A1-18)+(B2-9), (A1-18)+(B2-10), (A1-18)+(B2-11), (A1-18)+(B2-12), (A1-18)+(B2-13), (A1-18)+(B2-14), (A1-18)+(B2-15), (A1-18)+(B2-16), (A1-18)+(B2-17), (A1-18)+(B2-18), (A1-18)+(B2-19), (A1-18)+(B2-20), (A1-18)+(B2-21), (A1-18)+(B2-22), (A1-18)+(B2-23), (A1-18)+(B2-24), (A1-18)+(B2-25), (A1-18)+(B2-26), (A1-18)+(B2-27), (A1-18)+(B2-28), (A1-18)+(B2-29), (A1-18)+(B3-1), (A1-18)+(B3-2), (A1-18)+(B4-1), (A1-18)+(B4-2), (A1-18)+(B4-3), (A1-18)+(B4-4), (A1-18)+(B4-5), (A1-18)+(B4-6), (A1-18)+(B4-7), (A1-18)+(B4-8), (A1-18)+(B4-9), (A1-18)+(B4-10), (A1-18)+(B4-11), (A1-18)+(B5-1), (A1-18)+(B5-2), (A1-18)+(B5-3), (A1-18)+(B5-4), (A1-18)+(B5-5), (A1-18)+(B6-1), (A1-18)+(B6-2), (A1-18)+(B6-3), (A1-18)+(B6-4), (A1-18)+(B6-5), (A1-18)+(B6-6), (A1-18)+(B7-1), (A1-18)+(B7-2), (A1-18)+(B7-3), (A1-18)+(B7-4), (A1-18)+(B7-5), (A1-18)+(B7-6), (A1-18)+(B7-7)), (A1-18)+(B7-7), (A1-18)+(B7-8), (A1-18)+(B8-1), (A1-18)+(B8-2), (A1-18)+(B8-3), (A1-18)+(B8-4), (A1-18)+(B8-5), (A1-18)+(B8-6), (A1-18)+(B8-7), (A1-18)+(B9-1), (A1-18)+(B9-2), (A1-18)+(B10-1), (A1-18)+(B10-2), (A1-18)+(B10-3), (A1-18)+(B10-4), (A1-18)+(B10-5), (A1-18)+(B11-1), (A1-18)+(B11-2), (A1-18)+(B11-2),
(A1-19)+(B1-1), (A1-19)+(B1-2), (A1-19)+(B1-3), (A1-19)+(B1-4), (A1-19)+(B1-5), (A1-19)+(B1-6), (A1-19)+(B2-1), (A1-19)+(B2-2), (A1-19)+(B2-3), (A1-19)+(B2-4), (A1-19)+(B2-5), (A1-19)+(B2-6), (A1-19)+(B2-7), (A1-19)+(B2-8), (A1-19)+(B2-9), (A1-19)+(B2-10), (A1-19)+(B2-11), (A1-19)+(B2-12), (A1-19)+(B2-13), (A1-19)+(B2-14), (A1-19)+(B2-15), (A1-19)+(B2-16), (A1-19)+(B2-17), (A1-19)+(B2-18), (A1-19)+(B2-19), (A1-19)+(B2-20), (A1-19)+(B2-21), (A1-19)+(B2-22), (A1-19)+(B2-23), (A1-19)+(B2-24), (A1-19)+(B2-25), (A1-19)+(B2-26), (A1-19)+(B2-27), (A1-19)+(B2-28), (A1-19)+(B2-29), (A1-19)+(B3-1), (A1-19)+(B3-2), (A1-19)+(B4-1), (A1-19)+(B4-2), (A1-19)+(B4-3), (A1-19)+(B4-4), (A1-19)+(B4-5), (A1-19)+(B4-6), (A1-19)+(B4-7), (A1-19)+(B4-8), (A1-19)+(B4-9), (A1-19)+(B4-10), (A1-19)+(B4-11), (A1-19)+(B5-1), (A1-19)+(B5-2), (A1-19)+(B5-3), (A1-19)+(B5-4), (A1-19)+(B5-5), (A1-19)+(B6-1), (A1-19)+(B6-2), (A1-19)+(B6-3), (A1-19)+(B6-4), (A1-19)+(B6-5), (A1-19)+(B6-6), (A1-19)+(B7-1), (A1-19)+(B7-2), (A1-19)+(B7-3), (A1-19)+(B7-4), (A1-19)+(B7-5), (A1-19)+(B7-6), (A1-19)+(B7-7)), (A1-19)+(B7-7), (A1-19)+(B7-8), (A1-19)+(B8-1), (A1-19)+(B8-2), (A1-19)+(B8-3), (A1-19)+(B8-4), (A1-19)+(B8-5), (A1-19)+(B8-6), (A1-19)+(B8-7), (A1-19)+(B9-1), (A1-19)+(B9-2), (A1-19)+(B10-1), (A1-19)+(B10-2), (A1-19)+(B10-3), (A1-19)+(B10-4), (A1-19)+(B10-5), (A1-19)+(B11-1), (A1-19)+(B11-2), (A1-19)+(B11-2),
(A1-20)+(B1-1), (A1-20)+(B1-2), (A1-20)+(B1-3), (A1-20)+(B1-4), (A1-20)+(B1-5), (A1-20)+(B1-6), (A1-20)+(B2-1), (A1-20)+(B2-2), (A1-20)+(B2-3), (A1-20)+(B2-4), (A1-20)+(B2-5), (A1-20)+(B2-6), (A1-20)+(B2-7), (A1-20)+(B2-8), (A1-20)+(B2-9), (A1-20)+(B2-10), (A1-20)+(B2-11), (A1-20)+(B2-12), (A1-20)+(B2-13), (A1-20)+(B2-14), (A1-20)+(B2-15), (A1-20)+(B2-16), (A1-20)+(B2-17), (A1-20)+(B2-18), (A1-20)+(B2-19), (A1-20)+(B2-20), (A1-20)+(B2-21), (A1-20)+(B2-22), (A1-20)+(B2-23), (A1-20)+(B2-24), (A1-20)+(B2-25), (A1-20)+(B2-26), (A1-20)+(B2-27), (A1-20)+(B2-28), (A1-20)+(B2-29), (A1-20)+(B3-1), (A1-20)+(B3-2), (A1-20)+(B4-1), (A1-20)+(B4-2), (A1-20)+(B4-3), (A1-20)+(B4-4), (A1-20)+(B4-5), (A1-20)+(B4-6), (A1-20)+(B4-7), (A1-20)+(B4-8), (A1-20)+(B4-9), (A1-20)+(B4-10), (A1-20)+(B4-11), (A1-20)+(B5-1), (A1-20)+(B5-2), (A1-20)+(B5-3), (A1-20)+(B5-4), (A1-20)+(B5-5), (A1-20)+(B6-1), (A1-20)+(B6-2), (A1-20)+(B6-3), (A1-20)+(B6-4), (A1-20)+(B6-5), (A1-20)+(B6-6), (A1-20)+(B7-1), (A1-20)+(B7-2), (A1-20)+(B7-3), (A1-20)+(B7-4), (A1-20)+(B7-5), (A1-20)+(B7-6), (A1-20)+(B7-7)), (A1-20)+(B7-7), (A1-20)+(B7-8), (A1-20)+(B8-1), (A1-20)+(B8-2), (A1-20)+(B8-3), (A1-20)+(B8-4), (A1-20)+(B8-5), (A1-20)+(B8-6), (A1-20)+(B8-7), (A1-20)+(B9-1), (A1-20)+(B9-2), (A1-20)+(B10-1), (A1-20)+(B10-2), (A1-20)+(B10-3), (A1-20)+(B10-4), (A1-20)+(B10-5), (A1-20)+(B11-1), (A1-20)+(B11-2), (A1-20)+(B11-2),
(A1-21)+(B1-1), (A1-21)+(B1-2), (A1-21)+(B1-3), (A1-21)+(B1-4), (A1-21)+(B1-5), (A1-21)+(B1-6), (A1-21)+(B2-1), (A1-21)+(B2-2), (A1-21)+(B2-3), (A1-21)+(B2-4), (A1-21)+(B2-5), (A1-21)+(B2-6), (A1-21)+(B2-7), (A1-21)+(B2-8), (A1-21)+(B2-9), (A1-21)+(B2-10), (A1-21)+(B2-11), (A1-21)+(B2-12), (A1-21)+(B2-13), (A1-21)+(B2-14), (A1-21)+(B2-15), (A1-21)+(B2-16), (A1-21)+(B2-17), (A1-21)+(B2-18), (A1-21)+(B2-19), (A1-21)+(B2-20), (A1-21)+(B2-21), (A1-21)+(B2-22), (A1-21)+(B2-23), (A1-21)+(B2-24), (A1-21)+(B2-25), (A1-21)+(B2-26), (A1-21)+(B2-27), (A1-21)+(B2-28), (A1-21)+(B2-29), (A1-21)+(B3-1), (A1-21)+(B3-2), (A1-21)+(B4-1), (A1-21)+(B4-2), (A1-21)+(B4-3), (A1-21)+(B4-4), (A1-21)+(B4-5), (A1-21)+(B4-6), (A1-21)+(B4-7), (A1-21)+(B4-8), (A1-21)+(B4-9), (A1-21)+(B4-10), (A1-21)+(B4-11), (A1-21)+(B5-1), (A1-21)+(B5-2), (A1-21)+(B5-3), (A1-21)+(B5-4), (A1-21)+(B5-5), (A1-21)+(B6-1), (A1-21)+(B6-2), (A1-21)+(B6-3), (A1-21)+(B6-4), (A1-21)+(B6-5), (A1-21)+(B6-6), (A1-21)+(B7-1), (A1-21)+(B7-2), (A1-21)+(B7-3), (A1-21)+(B7-4), (A1-21)+(B7-5), (A1-21)+(B7-6), (A1-21)+(B7-7)), (A1-21)+(B7-7), (A1-21)+(B7-8), (A1-21)+(B8-1), (A1-21)+(B8-2), (A1-21)+(B8-3), (A1-21)+(B8-4), (A1-21)+(B8-5), (A1-21)+(B8-6), (A1-21)+(B8-7), (A1-21)+(B9-1), (A1-21)+(B9-2), (A1-21)+(B10-1), (A1-21)+(B10-2), (A1-21)+(B10-3), (A1-21)+(B10-4), (A1-21)+(B10-5), (A1-21)+(B11-1), (A1-21)+(B11-2), (A1-21)+(B11-2),
(A1-22)+(B1-1), (A1-22)+(B1-2), (A1-22)+(B1-3), (A1-22)+(B1-4), (A1-22)+(B1-5), (A1-22)+(B1-6), (A1-22)+(B2-1), (A1-22)+(B2-2), (A1-22)+(B2-3), (A1-22)+(B2-4), (A1-22)+(B2-5), (A1-22)+(B2-6), (A1-22)+(B2-7), (A1-22)+(B2-8), (A1-22)+(B2-9), (A1-22)+(B2-10), (A1-22)+(B2-11), (A1-22)+(B2-12), (A1-22)+(B2-13), (A1-22)+(B2-14), (A1-22)+(B2-15), (A1-22)+(B2-16), (A1-22)+(B2-17), (A1-22)+(B2-18), (A1-22)+(B2-19), (A1-22)+(B2-20), (A1-22)+(B2-21), (A1-22)+(B2-22), (A1-22)+(B2-23), (A1-22)+(B2-24), (A1-22)+(B2-25), (A1-22)+(B2-26), (A1-22)+(B2-27), (A1-22)+(B2-28), (A1-22)+(B2-29), (A1-22)+(B3-1), (A1-22)+(B3-2), (A1-22)+(B4-1), (A1-22)+(B4-2), (A1-22)+(B4-3), (A1-22)+(B4-4), (A1-22)+(B4-5), (A1-22)+(B4-6), (A1-22)+(B4-7), (A1-22)+(B4-8), (A1-22)+(B4-9), (A1-22)+(B4-10), (A1-22)+(B4-11), (A1-22)+(B5-1), (A1-22)+(B5-2), (A1-22)+(B5-3), (A1-22)+(B5-4), (A1-22)+(B5-5), (A1-22)+(B6-1), (A1-22)+(B6-2), (A1-22)+(B6-3), (A1-22)+(B6-4), (A1-22)+(B6-5), (A1-22)+(B6-6), (A1-22)+(B7-1), (A1-22)+(B7-2), (A1-22)+(B7-3), (A1-22)+(B7-4), (A1-22)+(B7-5), (A1-22)+(B7-6), (A1-22)+(B7-7)), (A1-22)+(B7-7), (A1-22)+(B7-8), (A1-22)+(B8-1), (A1-22)+(B8-2), (A1-22)+(B8-3), (A1-22)+(B8-4), (A1-22)+(B8-5), (A1-22)+(B8-6), (A1-22)+(B8-7), (A1-22)+(B9-1), (A1-22)+(B9-2), (A1-22)+(B10-1), (A1-22)+(B10-2), (A1-22)+(B10-3), (A1-22)+(B10-4), (A1-22)+(B10-5), (A1-22)+(B11-1), (A1-22)+(B11-2), (A1-22)+(B11-2),
(A1-23)+(B1-1), (A1-23)+(B1-2), (A1-23)+(B1-3), (A1-23)+(B1-4), (A1-23)+(B1-5), (A1-23)+(B1-6), (A1-23)+(B2-1), (A1-23)+(B2-2), (A1-23)+(B2-3), (A1-23)+(B2-4), (A1-23)+(B2-5), (A1-23)+(B2-6), (A1-23)+(B2-7), (A1-23)+(B2-8), (A1-23)+(B2-9), (A1-23)+(B2-10), (A1-23)+(B2-11), (A1-23)+(B2-12), (A1-23)+(B2-13), (A1-23)+(B2-14), (A1-23)+(B2-15), (A1-23)+(B2-16), (A1-23)+(B2-17), (A1-23)+(B2-18), (A1-23)+(B2-19), (A1-23)+(B2-20), (A1-23)+(B2-21), (A1-23)+(B2-22), (A1-23)+(B2-23), (A1-23)+(B2-24), (A1-23)+(B2-25), (A1-23)+(B2-26), (A1-23)+(B2-27), (A1-23)+(B2-28), (A1-23)+(B2-29), (A1-23)+(B3-1), (A1-23)+(B3-2), (A1-23)+(B4-1), (A1-23)+(B4-2), (A1-23)+(B4-3), (A1-23)+(B4-4), (A1-23)+(B4-5), (A1-23)+(B4-6), (A1-23)+(B4-7), (A1-23)+(B4-8), (A1-23)+(B4-9), (A1-23)+(B4-10), (A1-23)+(B4-11), (A1-23)+(B5-1), (A1-23)+(B5-2), (A1-23)+(B5-3), (A1-23)+(B5-4), (A1-23)+(B5-5), (A1-23)+(B6-1), (A1-23)+(B6-2), (A1-23)+(B6-3), (A1-23)+(B6-4), (A1-23)+(B6-5), (A1-23)+(B6-6), (A1-23)+(B7-1), (A1-23)+(B7-2), (A1-23)+(B7-3), (A1-23)+(B7-4), (A1-23)+(B7-5), (A1-23)+(B7-6), (A1-23)+(B7-7)), (A1-23)+(B7-7), (A1-23)+(B7-8), (A1-23)+(B8-1), (A1-23)+(B8-2), (A1-23)+(B8-3), (A1-23)+(B8-4), (A1-23)+(B8-5), (A1-23)+(B8-6), (A1-23)+(B8-7), (A1-23)+(B9-1), (A1-23)+(B9-2), (A1-23)+(B10-1), (A1-23)+(B10-2), (A1-23)+(B10-3), (A1-23)+(B10-4), (A1-23)+(B10-5), (A1-23)+(B11-1), (A1-23)+(B11-2), (A1-23)+(B11-2),
(A1-24)+(B1-1), (A1-24)+(B1-2), (A1-24)+(B1-3), (A1-24)+(B1-4), (A1-24)+(B1-5), (A1-24)+(B1-6), (A1-24)+(B2-1), (A1-24)+(B2-2), (A1-24)+(B2-3), (A1-24)+(B2-4), (A1-24)+(B2-5), (A1-24)+(B2-6), (A1-24)+(B2-7), (A1-24)+(B2-8), (A1-24)+(B2-9), (A1-24)+(B2-10), (A1-24)+(B2-11), (A1-24)+(B2-12), (A1-24)+(B2-13), (A1-24)+(B2-14), (A1-24)+(B2-15), (A1-24)+(B2-16), (A1-24)+(B2-17), (A1-24)+(B2-18), (A1-24)+(B2-19), (A1-24)+(B2-20), (A1-24)+(B2-21), (A1-24)+(B2-22), (A1-24)+(B2-23), (A1-24)+(B2-24), (A1-24)+(B2-25), (A1-24)+(B2-26), (A1-24)+(B2-27), (A1-24)+(B2-28), (A1-24)+(B2-29), (A1-24)+(B3-1), (A1-24)+(B3-2), (A1-24)+(B4-1), (A1-24)+(B4-2), (A1-24)+(B4-3), (A1-24)+(B4-4), (A1-24)+(B4-5), (A1-24)+(B4-6), (A1-24)+(B4-7), (A1-24)+(B4-8), (A1-24)+(B4-9), (A1-24)+(B4-10), (A1-24)+(B4-11), (A1-24)+(B5-1), (A1-24)+(B5-2), (A1-24)+(B5-3), (A1-24)+(B5-4), (A1-24)+(B5-5), (A1-24)+(B6-1), (A1-24)+(B6-2), (A1-24)+(B6-3), (A1-24)+(B6-4), (A1-24)+(B6-5), (A1-24)+(B6-6), (A1-24)+(B7-1), (A1-24)+(B7-2), (A1-24)+(B7-3), (A1-24)+(B7-4), (A1-24)+(B7-5), (A1-24)+(B7-6), (A1-24)+(B7-7)), (A1-24)+(B7-7), (A1-24)+(B7-8), (A1-24)+(B8-1), (A1-24)+(B8-2), (A1-24)+(B8-3), (A1-24)+(B8-4), (A1-24)+(B8-5), (A1-24)+(B8-6), (A1-24)+(B8-7), (A1-24)+(B9-1), (A1-24)+(B9-2), (A1-24)+(B10-1), (A1-24)+(B10-2), (A1-24)+(B10-3), (A1-24)+(B10-4), (A1-24)+(B10-5), (A1-24)+(B11-1), (A1-24)+(B11-2), (A1-24)+(B11-2),
(A1-25)+(B1-1), (A1-25)+(B1-2), (A1-25)+(B1-3), (A1-25)+(B1-4), (A1-25)+(B1-5), (A1-25)+(B1-6), (A1-25)+(B2-1), (A1-25)+(B2-2), (A1-25)+(B2-3), (A1-25)+(B2-4), (A1-25)+(B2-5), (A1-25)+(B2-6), (A1-25)+(B2-7), (A1-25)+(B2-8), (A1-25)+(B2-9), (A1-25)+(B2-10), (A1-25)+(B2-11), (A1-25)+(B2-12), (A1-25)+(B2-13), (A1-25)+(B2-14), (A1-25)+(B2-15), (A1-25)+(B2-16), (A1-25)+(B2-17), (A1-25)+(B2-18), (A1-25)+(B2-19), (A1-25)+(B2-20), (A1-25)+(B2-21), (A1-25)+(B2-22), (A1-25)+(B2-23), (A1-25)+(B2-24), (A1-25)+(B2-25), (A1-25)+(B2-26), (A1-25)+(B2-27), (A1-25)+(B2-28), (A1-25)+(B2-29), (A1-25)+(B3-1), (A1-25)+(B3-2), (A1-25)+(B4-1), (A1-25)+(B4-2), (A1-25)+(B4-3), (A1-25)+(B4-4), (A1-25)+(B4-5), (A1-25)+(B4-6), (A1-25)+(B4-7), (A1-25)+(B4-8), (A1-25)+(B4-9), (A1-25)+(B4-10), (A1-25)+(B4-11), (A1-25)+(B5-1), (A1-25)+(B5-2), (A1-25)+(B5-3), (A1-25)+(B5-4), (A1-25)+(B5-5), (A1-25)+(B6-1), (A1-25)+(B6-2), (A1-25)+(B6-3), (A1-25)+(B6-4), (A1-25)+(B6-5), (A1-25)+(B6-6), (A1-25)+(B7-1), (A1-25)+(B7-2), (A1-25)+(B7-3), (A1-25)+(B7-4), (A1-25)+(B7-5), (A1-25)+(B7-6), (A1-25)+(B7-7)), (A1-25)+(B7-7), (A1-25)+(B7-8), (A1-25)+(B8-1), (A1-25)+(B8-2), (A1-25)+(B8-3), (A1-25)+(B8-4), (A1-25)+(B8-5), (A1-25)+(B8-6), (A1-25)+(B8-7), (A1-25)+(B9-1), (A1-25)+(B9-2), (A1-25)+(B1 0-1), (A1-25)+(B1 0-2), (A1-25)+(B1 0-3), (A1-25)+(B10-4), (A1-25)+(B10-5), (A1-25)+(B11-1), (A1-25)+(B11-2), (A1-25)+(B11-2),
(A1-26)+(B1-1), (A1-26)+(B1-2), (A1-26)+(B1-3), (A1-26)+(B1-4), (A1-26)+(B1-5), (A1-26)+(B1-6), (A1-26)+(B2-1), (A1-26)+(B2-2), (A1-26)+(B2-3), (A1-26)+(B2-4), (A1-26)+(B2-5), (A1-26)+(B2-6), (A1-26)+(B2-7), (A1-26)+(B2-8), (A1-26)+(B2-9), (A1-26)+(B2-10), (A1-26)+(B2-11), (A1-26)+(B2-12), (A1-26)+(B2-13), (A1-26)+(B2-14), (A1-26)+(B2-15), (A1-26)+(B2-16), (A1-26)+(B2-17), (A1-26)+(B2-18), (A1-26)+(B2-19), (A1-26)+(B2-20), (A1-26)+(B2-21), (A1-26)+(B2-22), (A1-26)+(B2-23), (A1-26)+(B2-24), (A1-26)+(B2-25), (A1-26)+(B2-26), (A1-26)+(B2-27), (A1-26)+(B2-28), (A1-26)+(B2-29), (A1-26)+(B3-1), (A1-26)+(B3-2), (A1-26)+(B4-1), (A1-26)+(B4-2), (A1-26)+(B4-3), (A1-26)+(B4-4), (A1-26)+(B4-5), (A1-26)+(B4-6), (A1-26)+(B4-7), (A1-26)+(B4-8), (A1-26)+(B4-9), (A1-26)+(B4-10), (A1-26)+(B4-11), (A1-26)+(B5-1), (A1-26)+(B5-2), (A1-26)+(B5-3), (A1-26)+(B5-4), (A1-26)+(B5-5), (A1-26)+(B6-1), (A1-26)+(B6-2), (A1-26)+(B6-3), (A1-26)+(B6-4), (A1-26)+(B6-5), (A1-26)+(B6-6), (A1-26)+(B7-1), (A1-26)+(B7-2), (A1-26)+(B7-3), (A1-26)+(B7-4), (A1-26)+(B7-5), (A1-26)+(B7-6), (A1-26)+(B7-7)), (A1-26)+(B7-7), (A1-26)+(B7-8), (A1-26)+(B8-1), (A1-26)+(B8-2), (A1-26)+(B8-3), (A1-26)+(B8-4), (A1-26)+(B8-5), (A1-26)+(B8-6), (A1-26)+(B8-7), (A1-26)+(B9-1), (A1-26)+(B9-2), (A1-26)+(B10-1), (A1-26)+(B10-2), (A1-26)+(B10-3), (A1-26)+(B10-4), (A1-26)+(B10-5), (A1-26)+(B11-1), (A1-26)+(B11-2), (A1-26)+(B11-2),
(A1-27)+(B1-1), (A1-27)+(B1-2), (A1-27)+(B1-3), (A1-27)+(B1-4), (A1-27)+(B1-5), (A1-27)+(B1-6), (A1-27)+(B2-1), (A1-27)+(B2-2), (A1-27)+(B2-3), (A1-27)+(B2-4), (A1-27)+(B2-5), (A1-27)+(B2-6), (A1-27)+(B2-7), (A1-27)+(B2-8), (A1-27)+(B2-9), (A1-27)+(B2-10), (A1-27)+(B2-11), (A1-27)+(B2-12), (A1-27)+(B2-13), (A1-27)+(B2-14), (A1-27)+(B2-15), (A1-27)+(B2-16), (A1-27)+(B2-17), (A1-27)+(B2-18), (A1-27)+(B2-19), (A1-27)+(B2-20), (A1-27)+(B2-21), (A1-27)+(B2-22), (A1-27)+(B2-23), (A1-27)+(B2-24), (A1-27)+(B2-25), (A1-27)+(B2-26), (A1-27)+(B2-27), (A1-27)+(B2-28), (A1-27)+(B2-29), (A1-27)+(B3-1), (A1-27)+(B3-2), (A1-27)+(B4-1), (A1-27)+(B4-2), (A1-27)+(B4-3), (A1-27)+(B4-4), (A1-27)+(B4-5), (A1-27)+(B4-6), (A1-27)+(B4-7), (A1-27)+(B4-8), (A1-27)+(B4-9), (A1-27)+(B4-10), (A1-27)+(B4-11), (A1-27)+(B5-1), (A1-27)+(B5-2), (A1-27)+(B5-3), (A1-27)+(B5-4), (A1-27)+(B5-5), (A1-27)+(B6-1), (A1-27)+(B6-2), (A1-27)+(B6-3), (A1-27)+(B6-4), (A1-27)+(B6-5), (A1-27)+(B6-6), (A1-27)+(B7-1), (A1-27)+(B7-2), (A1-27)+(B7-3), (A1-27)+(B7-4), (A1-27)+(B7-5), (A1-27)+(B7-6), (A1-27)+(B7-7)), (A1-27)+(B7-7), (A1-27)+(B7-8), (A1-27)+(B8-1), (A1-27)+(B8-2), (A1-27)+(B8-3), (A1-27)+(B8-4), (A1-27)+(B8-5), (A1-27)+(B8-6), (A1-27)+(B8-7), (A1-27)+(B9-1), (A1-27)+(B9-2), (A1-27)+(B10-1), (A1-27)+(B10-2), (A1-27)+(B10-3), (A1-27)+(B10-4), (A1-27)+(B10-5), (A1-27)+(B11-1), (A1-27)+(B11-2), (A1-27)+(B11-2),
(A1-28)+(B1-1), (A1-28)+(B1-2), (A1-28)+(B1-3), (A1-28)+(B1-4), (A1-28)+(B1-5), (A1-28)+(B1-6), (A1-28)+(B2-1), (A1-28)+(B2-2), (A1-28)+(B2-3), (A1-28)+(B2-4), (A1-28)+(B2-5), (A1-28)+(B2-6), (A1-28)+(B2-7), (A1-28)+(B2-8), (A1-28)+(B2-9), (A1-28)+(B2-10), (A1-28)+(B2-11), (A1-28)+(B2-12), (A1-28)+(B2-13), (A1-28)+(B2-14), (A1-28)+(B2-15), (A1-28)+(B2-16), (A1-28)+(B2-17), (A1-28)+(B2-18), (A1-28)+(B2-19), (A1-28)+(B2-20), (A1-28)+(B2-21), (A1-28)+(B2-22), (A1-28)+(B2-23), (A1-28)+(B2-24), (A1-28)+(B2-25), (A1-28)+(B2-26), (A1-28)+(B2-27), (A1-28)+(B2-28), (A1-28)+(B2-29), (A1-28)+(B3-1), (A1-28)+(B3-2), (A1-28)+(B4-1), (A1-28)+(B4-2), (A1-28)+(B4-3), (A1-28)+(B4-4), (A1-28)+(B4-5), (A1-28)+(B4-6), (A1-28)+(B4-7), (A1-28)+(B4-8), (A1-28)+(B4-9), (A1-28)+(B4-10), (A1-28)+(B4-11), (A1-28)+(B5-1), (A1-28)+(B5-2), (A1-28)+(B5-3), (A1-28)+(B5-4), (A1-28)+(B5-5), (A1-28)+(B6-1), (A1-28)+(B6-2), (A1-28)+(B6-3), (A1-28)+(B6-4), (A1-28)+(B6-5), (A1-28)+(B6-6), (A1-28)+(B7-1), (A1-28)+(B7-2), (A1-28)+(B7-3), (A1-28)+(B7-4), (A1-28)+(B7-5), (A1-28)+(B7-6), (A1-28)+(B7-7)), (A1-28)+(B7-7), (A1-28)+(B7-8), (A1-28)+(B8-1), (A1-28)+(B8-2), (A1-28)+(B8-3), (A1-28)+(B8-4), (A1-28)+(B8-5), (A1-28)+(B8-6), (A1-28)+(B8-7), (A1-28)+(B9-1), (A1-28)+(B9-2), (A1-28)+(B10-1), (A1-28)+(B10-2), (A1-28)+(B10-3), (A1-28)+(B10-4), (A1-28)+(B10-5), (A1-28)+(B11-1), (A1-28)+(B11-2), (A1-28)+(B11-2),
(A1-29)+(B1-1), (A1-29)+(B1-2), (A1-29)+(B1-3), (A1-29)+(B1-4), (A1-29)+(B1-5), (A1-29)+(B1 -6), (A1-29)+(B2-1), (A1-29)+(B2-2), (A1-29)+(B2-3), (A1-29)+(B2-4), (A1-29)+(B2-5), (A1-29)+(B2-6), (A1-29)+(B2-7), (A1-29)+(B2-8), (A1-29)+(B2-9), (A1-29)+(B2-10), (A1-29)+(B2-11), (A1-29)+(B2-12), (A1-29)+(B2-13), (A1-29)+(B2-14), (A1-29)+(B2-15), (A1-29)+(B2-16), (A1-29)+(B2-17), (A1-29)+(B2-18), (A1-29)+(B2-19), (A1-29)+(B2-20), (A1-29)+(B2-21), (A1-29)+(B2-22), (A1-29)+(B2-23), (A1-29)+(B2-24), (A1-29)+(B2-25), (A1-29)+(B2-26), (A1-29)+(B2-27), (A1-29)+(B2-28), (A1-29)+(B2-29), (A1-29)+(B3-1), (A1-29)+(B3-2), (A1-29)+(B4-1), (A1-29)+(B4-2), (A1-29)+(B4-3), (A1-29)+(B4-4), (A1-29)+(B4-5), (A1-29)+(B4-6), (A1-29)+(B4-7), (A1-29)+(B4-8), (A1-29)+(B4-9), (A1-29)+(B4-10), (A1-29)+(B4-11), (A1-29)+(B5-1), (A1-29)+(B5-2), (A1-29)+(B5-3), (A1-29)+(B5-4), (A1-29)+(B5-5), (A1-29)+(B6-1), (A1-29)+(B6-2), (A1-29)+(B6-3), (A1-29)+(B6-4), (A1-29)+(B6-5), (A1-29)+(B6-6), (A1-29)+(B7-1), (A1-29)+(B7-2), (A1-29)+(B7-3), (A1-29)+(B7-4), (A1-29)+(B7-5), (A1-29)+(B7-6), (A1-29)+(B7-7)), (A1-29)+(B7-7), (A1-29)+(B7-8), (A1-29)+(B8-1), (A1-29)+(B8-2), (A1-29)+(B8-3), (A1-29)+(B8-4), (A1-29)+(B8-5), (A1-29)+(B8-6), (A1-29)+(B8-7), (A1-29)+(B9-1), (A1-29)+(B9-2), (A1-29)+(B10-1), (A1-29)+(B10-2), (A1-29)+(B10-3), (A1-29)+(B10-4), (A1-29)+(B10-5), (A1-29)+(B11-1), (A1-29)+(B11-2), (A1-29)+(B11-2),
(A1-30)+(B1-1), (A1-30)+(B1-2), (A1-30)+(B1-3), (A1-30)+(B1-4), (A1-30)+(B1-5), (A1-30)+(B1-6), (A1-30)+(B2-1), (A1-30)+(B2-2), (A1-30)+(B2-3), (A1-30)+(B2-4), (A1-30)+(B2-5), (A1-30)+(B2-6), (A1-30)+(B2-7), (A1-30)+(B2-8), (A1-30)+(B2-9), (A1-30)+(B2-10), (A1-30)+(B2-11), (A1-30)+(B2-12), (A1-30)+(B2-13), (A1-30)+(B2-14), (A1-30)+(B2-15), (A1-30)+(B2-16), (A1-30)+(B2-17), (A1-30)+(B2-18), (A1-30)+(B2-19), (A1-30)+(B2-20), (A1-30)+(B2-21), (A1-30)+(B2-22), (A1-30)+(B2-23), (A1-30)+(B2-24), (A1-30)+(B2-25), (A1-30)+(B2-26), (A1-30)+(B2-27), (A1-30)+(B2-28), (A1-30)+(B2-29), (A1-30)+(B3-1), (A1-30)+(B3-2), (A1-30)+(B4-1), (A1-30)+(B4-2), (A1-30)+(B4-3), (A1-30)+(B4-4), (A1-30)+(B4-5), (A1-30)+(B4-6), (A1-30)+(B4-7), (A1-30)+(B4-8), (A1-30)+(B4-9), (A1-30)+(B4-10), (A1-30)+(B4-11), (A1-30)+(B5-1), (A1-30)+(B5-2), (A1-30)+(B5-3), (A1-30)+(B5-4), (A1-30)+(B5-5), (A1-30)+(B6-1), (A1-30)+(B6-2), (A1-30)+(B6-3), (A1-30)+(B6-4), (A1-30)+(B6-5), (A1-30)+(B6-6), (A1-30)+(B7-1), (A1-30)+(B7-2), (A1-30)+(B7-3), (A1-30)+(B7-4), (A1-30)+(B7-5), (A1-30)+(B7-6), (A1-30)+(B7-7)), (A1-30)+(B7-7), (A1-30)+(B7-8), (A1-30)+(B8-1), (A1-30)+(B8-2), (A1-30)+(B8-3), (A1-30)+(B8-4), (A1-30)+(B8-5), (A1-30)+(B8-6), (A1-30)+(B8-7), (A1-30)+(B9-1), (A1-30)+(B9-2), (A1-30)+(B10-1), (A1-30)+(B10-2), (A1-30)+(B10-3), (A1-30)+(B10-4), (A1-30)+(B10-5), (A1-30)+(B11-1), (A1-30)+(B11-2), (A1-30)+(B11-2),
(A1-31)+(B1-1), (A1-31)+(B1-2), (A1-31)+(B1-3), (A1-31)+(B1-4), (A1-31)+(B1-5), (A1-31)+(B1-6), (A1-31)+(B2-1), (A1-31)+(B2-2), (A1-31)+(B2-3), (A1-31)+(B2-4), (A1-31)+(B2-5), (A1-31)+(B2-6), (A1-31)+(B2-7), (A1-31)+(B2-8), (A1-31)+(B2-9), (A1-31)+(B2-10), (A1-31)+(B2-11), (A1-31)+(B2-12), (A1-31)+(B2-13), (A1-31)+(B2-14), (A1-31)+(B2-15), (A1-31)+(B2-16), (A1-31)+(B2-17), (A1-31)+(B2-18), (A1-31)+(B2-19), (A1-31)+(B2-20), (A1-31)+(B2-21), (A1-31)+(B2-22), (A1-31)+(B2-23), (A1-31)+(B2-24), (A1-31)+(B2-25), (A1-31)+(B2-26), (A1-31)+(B2-27), (A1-31)+(B2-28), (A1-31)+(B2-29), (A1-31)+(B3-1), (A1-31)+(B3-2), (A1-31)+(B4-1), (A1-31)+(B4-2), (A1-31)+(B4-3), (A1-31)+(B4-4), (A1-31)+(B4-5), (A1-31)+(B4-6), (A1-31)+(B4-7), (A1-31)+(B4-8), (A1-31)+(B4-9), (A1-31)+(B4-10), (A1-31)+(B4-11), (A1-31)+(B5-1), (A1-31)+(B5-2), (A1-31)+(B5-3), (A1-31)+(B5-4), (A1-31)+(B5-5), (A1-31)+(B6-1), (A1-31)+(B6-2), (A1-31)+(B6-3), (A1-31)+(B6-4), (A1-31)+(B6-5), (A1-31)+(B6-6), (A1-31)+(B7-1), (A1-31)+(B7-2), (A1-31)+(B7-3), (A1-31)+(B7-4), (A1-31)+(B7-5), (A1-31)+(B7-6), (A1-31)+(B7-7)), (A1-31)+(B7-7), (A1-31)+(B7-8), (A1-31)+(B8-1), (A1-31)+(B8-2), (A1-31)+(B8-3), (A1-31)+(B8-4), (A1-31)+(B8-5), (A1-31)+(B8-6), (A1-31)+(B8-7), (A1-31)+(B9-1), (A1-31)+(B9-2), (A1-31)+(B10-1), (A1-31)+(B10-2), (A1-31)+(B10-3), (A1-31)+(B10-4), (A1-31)+(B10-5), (A1-31)+(B11-1), (A1-31)+(B11-2), (A1-31)+(B11-2),
(A1-32)+(B1-1), (A1-32)+(B1-2), (A1-32)+(B1-3), (A1-32)+(B1-4), (A1-32)+(B1-5), (A1-32)+(B1-6), (A1-32)+(B2-1), (A1-32)+(B2-2), (A1-32)+(B2-3), (A1-32)+(B2-4), (A1-32)+(B2-5), (A1-32)+(B2-6), (A1-32)+(B2-7), (A1-32)+(B2-8), (A1-32)+(B2-9), (A1-32)+(B2-10), (A1-32)+(B2-11), (A1-32)+(B2-12), (A1-32)+(B2-13), (A1-32)+(B2-14), (A1-32)+(B2-15), (A1-32)+(B2-16), (A1-32)+(B2-17), (A1-32)+(B2-18), (A1-32)+(B2-19), (A1-32)+(B2-20), (A1-32)+(B2-21), (A1-32)+(B2-22), (A1-32)+(B2-23), (A1-32)+(B2-24), (A1-32)+(B2-25), (A1-32)+(B2-26), (A1-32)+(B2-27), (A1-32)+(B2-28), (A1-32)+(B2-29), (A1-32)+(B3-1), (A1-32)+(B3-2), (A1-32)+(B4-1), (A1-32)+(B4-2), (A1-32)+(B4-3), (A1-32)+(B4-4), (A1-32)+(B4-5), (A1-32)+(B4-6), (A1-32)+(B4-7), (A1-32)+(B4-8), (A1-32)+(B4-9), (A1-32)+(B4-10), (A1-32)+(B4-11), (A1-32)+(B5-1), (A1-32)+(B5-2), (A1-32)+(B5-3), (A1-32)+(B5-4), (A1-32)+(B5-5), (A1-32)+(B6-1), (A1-32)+(B6-2), (A1-32)+(B6-3), (A1-32)+(B6-4), (A1-32)+(B6-5), (A1-32)+(B6-6), (A1-32)+(B7-1), (A1-32)+(B7-2), (A1-32)+(B7-3), (A1-32)+(B7-4), (A1-32)+(B7-5), (A1-32)+(B7-6), (A1-32)+(B7-7)), (A1-32)+(B7-7), (A1-32)+(B7-8), (A1-32)+(B8-1), (A1-32)+(B8-2), (A1-32)+(B8-3), (A1-32)+(B8-4), (A1-32)+(B8-5), (A1-32)+(B8-6), (A1-32)+(B8-7), (A1-32)+(B9-1), (A1-32)+(B9-2), (A1-32)+(B10-1), (A1-32)+(B10-2), (A1-32)+(B10-3), (A1-32)+(B10-4), (A1-32)+(B10-5), (A1-32)+(B11-1), (A1-32)+(B11-2), (A1-32)+(B11-2),
(A1-33)+(B1-1), (A1-33)+(B1-2), (A1-33)+(B1-3), (A1-33)+(B1-4), (A1-33)+(B1-5), (A1-33)+(B1-6), (A1-33)+(B2-1), (A1-33)+(B2-2), (A1-33)+(B2-3), (A1-33)+(B2-4), (A1-33)+(B2-5), (A1-33)+(B2-6), (A1-33)+(B2-7), (A1-33)+(B2-8), (A1-33)+(B2-9), (A1-33)+(B2-10), (A1-33)+(B2-11), (A1-33)+(B2-12), (A1-33)+(B2-13), (A1-33)+(B2-14), (A1-33)+(B2-15), (A1-33)+(B2-16), (A1-33)+(B2-17), (A1-33)+(B2-18), (A1-33)+(B2-19), (A1-33)+(B2-20), (A1-33)+(B2-21), (A1-33)+(B2-22), (A1-33)+(B2-23), (A1-33)+(B2-24), (A1-33)+(B2-25), (A1-33)+(B2-26), (A1-33)+(B2-27), (A1-33)+(B2-28), (A1-33)+(B2-29), (A1-33)+(B3-1), (A1-33)+(B3-2), (A1-33)+(B4-1), (A1-33)+(B4-2), (A1-33)+(B4-3), (A1-33)+(B4-4), (A1-33)+(B4-5), (A1-33)+(B4-6), (A1-33)+(B4-7), (A1-33)+(B4-8), (A1-33)+(B4-9), (A1-33)+(B4-10), (A1-33)+(B4-11), (A1-33)+(B5-1), (A1-33)+(B5-2), (A1-33)+(B5-3), (A1-33)+(B5-4), (A1-33)+(B5-5), (A1-33)+(B6-1), (A1-33)+(B6-2), (A1-33)+(B6-3), (A1-33)+(B6-4), (A1-33)+(B6-5), (A1-33)+(B6-6), (A1-33)+(B7-1), (A1-33)+(B7-2), (A1-33)+(B7-3), (A1-33)+(B7-4), (A1-33)+(B7-5), (A1-33)+(B7-6), (A1-33)+(B7-7)), (A1-33)+(B7-7), (A1-33)+(B7-8), (A1-33)+(B8-1), (A1-33)+(B8-2), (A1-33)+(B8-3), (A1-33)+(B8-4), (A1-33)+(B8-5), (A1-33)+(B8-6), (A1-33)+(B8-7), (A1-33)+(B9-1), (A1-33)+(B9-2), (A1-33)+(B10-1), (A1-33)+(B10-2), (A1-33)+(B10-3), (A1-33)+(B10-4), (A1-33)+(B10-5), (A1-33)+(B11-1), (A1-33)+(B11-2), (A1-33)+(B11-2),
(A1-34)+(B1-1), (A1-34)+(B1-2), (A1-34)+(B1-3), (A1-34)+(B1-4), (A1-34)+(B1-5), (A1-34)+(B1-6), (A1-34)+(B2-1), (A1-34)+(B2-2), (A1-34)+(B2-3), (A1-34)+(B2-4), (A1-34)+(B2-5), (A1-34)+(B2-6), (A1-34)+(B2-7), (A1-34)+(B2-8), (A1-34)+(B2-9), (A1-34)+(B2-10), (A1-34)+(B2-11), (A1-34)+(B2-12), (A1-34)+(B2-13), (A1-34)+(B2-14), (A1-34)+(B2-15), (A1-34)+(B2-16), (A1-34)+(B2-17), (A1-34)+(B2-18), (A1-34)+(B2-19), (A1-34)+(B2-20), (A1-34)+(B2-21), (A1-34)+(B2-22), (A1-34)+(B2-23), (A1-34)+(B2-24), (A1-34)+(B2-25), (A1-34)+(B2-26), (A1-34)+(B2-27), (A1-34)+(B2-28), (A1-34)+(B2-29), (A1-34)+(B3-1), (A1-34)+(B3-2), (A1-34)+(B4-1), (A1-34)+(B4-2), (A1-34)+(B4-3), (A1-34)+(B4-4), (A1-34)+(B4-5), (A1-34)+(B4-6), (A1-34)+(B4-7), (A1-34)+(B4-8), (A1-34)+(B4-9), (A1-34)+(B4-10), (A1-34)+(B4-11), (A1-34)+(B5-1), (A1-34)+(B5-2), (A1-34)+(B5-3), (A1-34)+(B5-4), (A1-34)+(B5-5), (A1-34)+(B6-1), (A1-34)+(B6-2), (A1-34)+(B6-3), (A1-34)+(B6-4), (A1-34)+(B6-5), (A1-34)+(B6-6), (A1-34)+(B7-1), (A1-34)+(B7-2), (A1-34)+(B7-3), (A1-34)+(B7-4), (A1-34)+(B7-5), (A1-34)+(B7-6), (A1-34)+(B7-7)), (A1-34)+(B7-7), (A1-34)+(B7-8), (A1-34)+(B8-1), (A1-34)+(B8-2), (A1-34)+(B8-3), (A1-34)+(B8-4), (A1-34)+(B8-5), (A1-34)+(B8-6), (A1-34)+(B8-7), (A1-34)+(B9-1), (A1-34)+(B9-2), (A1-34)+(B10-1), (A1-34)+(B10-2), (A1-34)+(B10-3), (A1-34)+(B10-4), (A1-34)+(B10-5), (A1-34)+(B11-1), (A1-34)+(B11-2), (A1-34)+(B11-2),
(A1-35)+(B1-1), (A1-35)+(B1-2), (A1-35)+(B1-3), (A1-35)+(B1-4), (A1-35)+(B1-5), (A1-35)+(B1-6), (A1-35)+(B2-1), (A1-35)+(B2-2), (A1-35)+(B2-3), (A1-35)+(B2-4), (A1-35)+(B2-5), (A1-35)+(B2-6), (A1-35)+(B2-7), (A1-35)+(B2-8), (A1-35)+(B2-9), (A1-35)+(B2-10), (A1-35)+(B2-11), (A1-35)+(B2-12), (A1-35)+(B2-13), (A1-35)+(B2-14), (A1-35)+(B2-15), (A1-35)+(B2-16), (A1-35)+(B2-17), (A1-35)+(B2-18), (A1-35)+(B2-19), (A1-35)+(B2-20), (A1-35)+(B2-21), (A1-35)+(B2-22), (A1-35)+(B2-23), (A1-35)+(B2-24), (A1-35)+(B2-25), (A1-35)+(B2-26), (A1-35)+(B2-27), (A1-35)+(B2-28), (A1-35)+(B2-29), (A1-35)+(B3-1), (A1-35)+(B3-2), (A1-35)+(B4-1), (A1-35)+(B4-2), (A1-35)+(B4-3), (A1-35)+(B4-4), (A1-35)+(B4-5), (A1-35)+(B4-6), (A1-35)+(B4-7), (A1-35)+(B4-8), (A1-35)+(B4-9), (A1-35)+(B4-10), (A1-35)+(B4-11), (A1-35)+(B5-1), (A1-35)+(B5-2), (A1-35)+(B5-3), (A1-35)+(B5-4), (A1-35)+(B5-5), (A1-35)+(B6-1), (A1-35)+(B6-2), (A1-35)+(B6-3), (A1-35)+(B6-4), (A1-35)+(B6-5), (A1-35)+(B6-6), (A1-35)+(B7-1), (A1-35)+(B7-2), (A1-35)+(B7-3), (A1-35)+(B7-4), (A1-35)+(B7-5), (A1-35)+(B7-6), (A1-35)+(B7-7)), (A1-35)+(B7-7), (A1-35)+(B7-8), (A1-35)+(B8-1), (A1-35)+(B8-2), (A1-35)+(B8-3), (A1-35)+(B8-4), (A1-35)+(B8-5), (A1-35)+(B8-6), (A1-35)+(B8-7), (A1-35)+(B9-1), (A1-35)+(B9-2), (A1-35)+(B10-1), (A1-35)+(B10-2), (A1-35)+(B10-3), (A1-35)+(B10-4), (A1-35)+(B10-5), (A1-35)+(B1 1-1), (A1-35)+(B1 1-2), (A1-35)+(B11-2),
(A1-36)+(B1-1), (A1-36)+(B1-2), (A1-36)+(B1-3), (A1-36)+(B1-4), (A1-36)+(B1-5), (A1-36)+(B1-6), (A1-36)+(B2-1), (A1-36)+(B2-2), (A1-36)+(B2-3), (A1-36)+(B2-4), (A1-36)+(B2-5), (A1-36)+(B2-6), (A1-36)+(B2-7), (A1-36)+(B2-8), (A1-36)+(B2-9), (A1-36)+(B2-10), (A1-36)+(B2-11), (A1-36)+(B2-12), (A1-36)+(B2-13), (A1-36)+(B2-14), (A1-36)+(B2-15), (A1-36)+(B2-16), (A1-36)+(B2-17), (A1-36)+(B2-18), (A1-36)+(B2-19), (A1-36)+(B2-20), (A1-36)+(B2-21), (A1-36)+(B2-22), (A1-36)+(B2-23), (A1-36)+(B2-24), (A1-36)+(B2-25), (A1-36)+(B2-26), (A1-36)+(B2-27), (A1-36)+(B2-28), (A1-36)+(B2-29), (A1-36)+(B3-1), (A1-36)+(B3-2), (A1-36)+(B4-1), (A1-36)+(B4-2), (A1-36)+(B4-3), (A1-36)+(B4-4), (A1-36)+(B4-5), (A1-36)+(B4-6), (A1-36)+(B4-7), (A1-36)+(B4-8), (A1-36)+(B4-9), (A1-36)+(B4-10), (A1-36)+(B4-11), (A1-36)+(B5-1), (A1-36)+(B5-2), (A1-36)+(B5-3), (A1-36)+(B5-4), (A1-36)+(B5-5), (A1-36)+(B6-1), (A1-36)+(B6-2), (A1-36)+(B6-3), (A1-36)+(B6-4), (A1-36)+(B6-5), (A1-36)+(B6-6), (A1-36)+(B7-1), (A1-36)+(B7-2), (A1-36)+(B7-3), (A1-36)+(B7-4), (A1-36)+(B7-5), (A1-36)+(B7-6), (A1-36)+(B7-7)), (A1-36)+(B7-7), (A1-36)+(B7-8), (A1-36)+(B8-1), (A1-36)+(B8-2), (A1-36)+(B8-3), (A1-36)+(B8-4), (A1-36)+(B8-5), (A1-36)+(B8-6), (A1-36)+(B8-7), (A1-36)+(B9-1), (A1-36)+(B9-2), (A1-36)+(B10-1), (A1-36)+(B10-2), (A1-36)+(B10-3), (A1-36)+(B10-4), (A1-36)+(B10-5), (A1-36)+(B11-1), (A1-36)+(B11-2), (A1-36)+(B11-2),
(A1-37)+(B1-1), (A1-37)+(B1-2), (A1-37)+(B1-3), (A1-37)+(B1-4), (A1-37)+(B1-5), (A1-37)+(B1-6), (A1-37)+(B2-1), (A1-37)+(B2-2), (A1-37)+(B2-3), (A1-37)+(B2-4), (A1-37)+(B2-5), (A1-37)+(B2-6), (A1-37)+(B2-7), (A1-37)+(B2-8), (A1-37)+(B2-9), (A1-37)+(B2-10), (A1-37)+(B2-11), (A1-37)+(B2-12), (A1-37)+(B2-13), (A1-37)+(B2-14), (A1-37)+(B2-15), (A1-37)+(B2-16), (A1-37)+(B2-17), (A1-37)+(B2-18), (A1-37)+(B2-19), (A1-37)+(B2-20), (A1-37)+(B2-21), (A1-37)+(B2-22), (A1-37)+(B2-23), (A1-37)+(B2-24), (A1-37)+(B2-25), (A1-37)+(B2-26), (A1-37)+(B2-27), (A1-37)+(B2-28), (A1-37)+(B2-29), (A1-37)+(B3-1), (A1-37)+(B3-2), (A1-37)+(B4-1), (A1-37)+(B4-2), (A1-37)+(B4-3), (A1-37)+(B4-4), (A1-37)+(B4-5), (A1-37)+(B4-6), (A1-37)+(B4-7), (A1-37)+(B4-8), (A1-37)+(B4-9), (A1-37)+(B4-10), (A1-37)+(B4-11), (A1-37)+(B5-1), (A1-37)+(B5-2), (A1-37)+(B5-3), (A1-37)+(B5-4), (A1-37)+(B5-5), (A1-37)+(B6-1), (A1-37)+(B6-2), (A1-37)+(B6-3), (A1-37)+(B6-4), (A1-37)+(B6-5), (A1-37)+(B6-6), (A1-37)+(B7-1), (A1-37)+(B7-2), (A1-37)+(B7-3), (A1-37)+(B7-4), (A1-37)+(B7-5), (A1-37)+(B7-6), (A1-37)+(B7-7)), (A1-37)+(B7-7), (A1-37)+(B7-8), (A1-37)+(B8-1), (A1-37)+(B8-2), (A1-37)+(B8-3), (A1-37)+(B8-4), (A1-37)+(B8-5), (A1-37)+(B8-6), (A1-37)+(B8-7), (A1-37)+(B9-1), (A1-37)+(B9-2), (A1-37)+(B10-1), (A1-37)+(B10-2), (A1-37)+(B10-3), (A1-37)+(B10-4), (A1-37)+(B10-5), (A1-37)+(B11-1), (A1-37)+(B11-2), (A1-37)+(B11-2),
(A1-38)+(B1-1), (A1-38)+(B1-2), (A1-38)+(B1-3), (A1-38)+(B1-4), (A1-38)+(B1-5), (A1-38)+(B1-6), (A1-38)+(B2-1), (A1-38)+(B2-2), (A1-38)+(B2-3), (A1-38)+(B2-4), (A1-38)+(B2-5), (A1-38)+(B2-6), (A1-38)+(B2-7), (A1-38)+(B2-8), (A1-38)+(B2-9), (A1-38)+(B2-10), (A1-38)+(B2-11), (A1-38)+(B2-12), (A1-38)+(B2-13), (A1-38)+(B2-14), (A1-38)+(B2-15), (A1-38)+(B2-16), (A1-38)+(B2-17), (A1-38)+(B2-18), (A1-38)+(B2-19), (A1-38)+(B2-20), (A1-38)+(B2-21), (A1-38)+(B2-22), (A1-38)+(B2-23), (A1-38)+(B2-24), (A1-38)+(B2-25), (A1-38)+(B2-26), (A1-38)+(B2-27), (A1-38)+(B2-28), (A1-38)+(B2-29), (A1-38)+(B3-1), (A1-38)+(B3-2), (A1-38)+(B4-1), (A1-38)+(B4-2), (A1-38)+(B4-3), (A1-38)+(B4-4), (A1-38)+(B4-5), (A1-38)+(B4-6), (A1-38)+(B4-7), (A1-38)+(B4-8), (A1-38)+(B4-9), (A1-38)+(B4-10), (A1-38)+(B4-11), (A1-38)+(B5-1), (A1-38)+(B5-2), (A1-38)+(B5-3), (A1-38)+(B5-4), (A1-38)+(B5-5), (A1-38)+(B6-1), (A1-38)+(B6-2), (A1-38)+(B6-3), (A1-38)+(B6-4), (A1-38)+(B6-5), (A1-38)+(B6-6), (A1-38)+(B7-1), (A1-38)+(B7-2), (A1-38)+(B7-3), (A1-38)+(B7-4), (A1-38)+(B7-5), (A1-38)+(B7-6), (A1-38)+(B7-7)), (A1-38)+(B7-7), (A1-38)+(B7-8), (A1-38)+(B8-1), (A1-38)+(B8-2), (A1-38)+(B8-3), (A1-38)+(B8-4), (A1-38)+(B8-5), (A1-38)+(B8-6), (A1-38)+(B8-7), (A1-38)+(B9-1), (A1-38)+(B9-2), (A1-38)+(B10-1), (A1-38)+(B10-2), (A1-38)+(B10-3), (A1-38)+(B10-4), (A1-38)+(B10-5), (A1-38)+(B11-1), (A1-38)+(B11-2), (A1-38)+(B11-2),
(A1-39)+(B1-1), (A1-39)+(B1-2), (A1-39)+(B1-3), (A1-39)+(B1-4), (A1-39)+(B1-5), (A1-39)+(B1-6), (A1-39)+(B2-1), (A1-39)+(B2-2), (A1-39)+(B2-3), (A1-39)+(B2-4), (A1-39)+(B2-5), (A1-39)+(B2-6), (A1-39)+(B2-7), (A1-39)+(B2-8), (A1-39)+(B2-9), (A1-39)+(B2-10), (A1-39)+(B2-11), (A1-39)+(B2-12), (A1-39)+(B2-13), (A1-39)+(B2-14), (A1-39)+(B2-15), (A1-39)+(B2-16), (A1-39)+(B2-17), (A1-39)+(B2-18), (A1-39)+(B2-19), (A1-39)+(B2-20), (A1-39)+(B2-21), (A1-39)+(B2-22), (A1-39)+(B2-23), (A1-39)+(B2-24), (A1-39)+(B2-25), (A1-39)+(B2-26), (A1-39)+(B2-27), (A1-39)+(B2-28), (A1-39)+(B2-29), (A1-39)+(B3-1), (A1-39)+(B3-2), (A1-39)+(B4-1), (A1-39)+(B4-2), (A1-39)+(B4-3), (A1-39)+(B4-4), (A1-39)+(B4-5), (A1-39)+(B4-6), (A1-39)+(B4-7), (A1-39)+(B4-8), (A1-39)+(B4-9), (A1-39)+(B4-10), (A1-39)+(B4-11), (A1-39)+(B5-1), (A1-39)+(B5-2), (A1-39)+(B5-3), (A1-39)+(B5-4), (A1-39)+(B5-5), (A1-39)+(B6-1), (A1-39)+(B6-2), (A1-39)+(B6-3), (A1-39)+(B6-4), (A1-39)+(B6-5), (A1-39)+(B6-6), (A1-39)+(B7-1), (A1-39)+(B7-2), (A1-39)+(B7-3), (A1-39)+(B7-4), (A1-39)+(B7-5), (A1-39)+(B7-6), (A1-39)+(B7-7)), (A1-39)+(B7-7), (A1-39)+(B7-8), (A1-39)+(B8-1), (A1-39)+(B8-2), (A1-39)+(B8-3), (A1-39)+(B8-4), (A1-39)+(B8-5), (A1-39)+(B8-6), (A1-39)+(B8-7), (A1-39)+(B9-1), (A1-39)+(B9-2), (A1-39)+(B10-1), (A1-39)+(B10-2), (A1-39)+(B10-3), (A1-39)+(B10-4), (A1-39)+(B10-5), (A1-39)+(B11-1), (A1-39)+(B11-2), (A1-39)+(B11-2),
(A1-40)+(B1-1), (A1-40)+(B1-2), (A1-40)+(B1-3), (A1-40)+(B1-4), (A1-40)+(B1-5), (A1-40)+(B1-6), (A1-40)+(B2-1), (A1-40)+(B2-2), (A1-40)+(B2-3), (A1-40)+(B2-4), (A1-40)+(B2-5), (A1-40)+(B2-6), (A1-40)+(B2-7), (A1-40)+(B2-8), (A1-40)+(B2-9), (A1-40)+(B2-10), (A1-40)+(B2-11), (A1-40)+(B2-12), (A1-40)+(B2-13), (A1-40)+(B2-14), (A1-40)+(B2-15), (A1-40)+(B2-16), (A1-40)+(B2-17), (A1-40)+(B2-18), (A1-40)+(B2-19), (A1-40)+(B2-20), (A1-40)+(B2-21), (A1-40)+(B2-22), (A1-40)+(B2-23), (A1-40)+(B2-24), (A1-40)+(B2-25), (A1-40)+(B2-26), (A1-40)+(B2-27), (A1-40)+(B2-28), (A1-40)+(B2-29), (A1-40)+(B3-1), (A1-40)+(B3-2), (A1-40)+(B4-1), (A1-40)+(B4-2), (A1-40)+(B4-3), (A1-40)+(B4-4), (A1-40)+(B4-5), (A1-40)+(B4-6), (A1-40)+(B4-7), (A1-40)+(B4-8), (A1-40)+(B4-9), (A1-40)+(B4-10), (A1-40)+(B4-11), (A1-40)+(B5-1), (A1-40)+(B5-2), (A1-40)+(B5-3), (A1-40)+(B5-4), (A1-40)+(B5-5), (A1-40)+(B6-1), (A1-40)+(B6-2), (A1-40)+(B6-3), (A1-40)+(B6-4), (A1-40)+(B6-5), (A1-40)+(B6-6), (A1-40)+(B7-1), (A1-40)+(B7-2), (A1-40)+(B7-3), (A1-40)+(B7-4), (A1-40)+(B7-5), (A1-40)+(B7-6), (A1-40)+(B7-7)), (A1-40)+(B7-7), (A1-40)+(B7-8), (A1-40)+(B8-1), (A1-40)+(B8-2), (A1-40)+(B8-3), (A1-40)+(B8-4), (A1-40)+(B8-5), (A1-40)+(B8-6), (A1-40)+(B8-7), (A1-40)+(B9-1), (A1-40)+(B9-2), (A1-40)+(B10-1), (A1-40)+(B10-2), (A1-40)+(B10-3), (A1-40)+(B10-4), (A1-40)+(B10-5), (A1-40)+(B11-1), (A1-40)+(B11-2), (A1-40)+(B11-2),
(A1-41)+(B1-1), (A1-41)+(B1-2), (A1-41)+(B1-3), (A1-41)+(B1-4), (A1-41)+(B1-5), (A1-41)+(B1-6), (A1-41)+(B2-1), (A1-41)+(B2-2), (A1-41)+(B2-3), (A1-41)+(B2-4), (A1-41)+(B2-5), (A1-41)+(B2-6), (A1-41)+(B2-7), (A1-41)+(B2-8), (A1-41)+(B2-9), (A1-41)+(B2-10), (A1-41)+(B2-11), (A1-41)+(B2-12), (A1-41)+(B2-13), (A1-41)+(B2-14), (A1-41)+(B2-15), (A1-41)+(B2-16), (A1-41)+(B2-17), (A1-41)+(B2-18), (A1-41)+(B2-19), (A1-41)+(B2-20), (A1-41)+(B2-21), (A1-41)+(B2-22), (A1-41)+(B2-23), (A1-41)+(B2-24), (A1-41)+(B2-25), (A1-41)+(B2-26), (A1-41)+(B2-27), (A1-41)+(B2-28), (A1-41)+(B2-29), (A1-41)+(B3-1), (A1-41)+(B3-2), (A1-41)+(B4-1), (A1-41)+(B4-2), (A1-41)+(B4-3), (A1-41)+(B4-4), (A1-41)+(B4-5), (A1-41)+(B4-6), (A1-41)+(B4-7), (A1-41)+(B4-8), (A1-41)+(B4-9), (A1-41)+(B4-10), (A1-41)+(B4-11), (A1-41)+(B5-1), (A1-41)+(B5-2), (A1-41)+(B5-3), (A1-41)+(B5-4), (A1-41)+(B5-5), (A1-41)+(B6-1), (A1-41)+(B6-2), (A1-41)+(B6-3), (A1-41)+(B6-4), (A1-41)+(B6-5), (A1-41)+(B6-6), (A1-41)+(B7-1), (A1-41)+(B7-2), (A1-41)+(B7-3), (A1-41)+(B7-4), (A1-41)+(B7-5), (A1-41)+(B7-6), (A1-41)+(B7-7)), (A1-41)+(B7-7), (A1-41)+(B7-8), (A1-41)+(B8-1), (A1-41)+(B8-2), (A1-41)+(B8-3), (A1-41)+(B8-4), (A1-41)+(B8-5), (A1-41)+(B8-6), (A1-41)+(B8-7), (A1-41)+(B9-1), (A1-41)+(B9-2), (A1-41)+(B10-1), (A1-41)+(B10-2), (A1-41)+(B10-3), (A1-41)+(B10-4), (A1-41)+(B10-5), (A1-41)+(B11-1), (A1-41)+(B11-2), (A1-41)+(B11-2),
(A1-42)+(B1-1), (A1-42)+(B1-2), (A1-42)+(B1-3), (A1-42)+(B1-4), (A1-42)+(B1-5), (A1-42)+(B1-6), (A1-42)+(B2-1), (A1-42)+(B2-2), (A1-42)+(B2-3), (A1-42)+(B2-4), (A1-42)+(B2-5), (A1-42)+(B2-6), (A1-42)+(B2-7), (A1-42)+(B2-8), (A1-42)+(B2-9), (A1-42)+(B2-10), (A1-42)+(B2-11), (A1-42)+(B2-12), (A1-42)+(B2-13), (A1-42)+(B2-14), (A1-42)+(B2-15), (A1-42)+(B2-16), (A1-42)+(B2-17), (A1-42)+(B2-18), (A1-42)+(B2-19), (A1-42)+(B2-20), (A1-42)+(B2-21), (A1-42)+(B2-22), (A1-42)+(B2-23), (A1-42)+(B2-24), (A1-42)+(B2-25), (A1-42)+(B2-26), (A1-42)+(B2-27), (A1-42)+(B2-28), (A1-42)+(B2-29), (A1-42)+(B3-1), (A1-42)+(B3-2), (A1-42)+(B4-1), (A1-42)+(B4-2), (A1-42)+(B4-3), (A1-42)+(B4-4), (A1-42)+(B4-5), (A1-42)+(B4-6), (A1-42)+(B4-7), (A1-42)+(B4-8), (A1-42)+(B4-9), (A1-42)+(B4-10), (A1-42)+(B4-11), (A1-42)+(B5-1), (A1-42)+(B5-2), (A1-42)+(B5-3), (A1-42)+(B5-4), (A1-42)+(B5-5), (A1-42)+(B6-1), (A1-42)+(B6-2), (A1-42)+(B6-3), (A1-42)+(B6-4), (A1-42)+(B6-5), (A1-42)+(B6-6), (A1-42)+(B7-1), (A1-42)+(B7-2), (A1-42)+(B7-3), (A1-42)+(B7-4), (A1-42)+(B7-5), (A1-42)+(B7-6), (A1-42)+(B7-7)), (A1-42)+(B7-7), (A1-42)+(B7-8), (A1-42)+(B8-1), (A1-42)+(B8-2), (A1-42)+(B8-3), (A1-42)+(B8-4), (A1-42)+(B8-5), (A1-42)+(B8-6), (A1-42)+(B8-7), (A1-42)+(B9-1), (A1-42)+(B9-2), (A1-42)+(B10-1), (A1-42)+(B10-2), (A1-42)+(B10-3), (A1-42)+(B10-4), (A1-42)+(B10-5), (A1-42)+(B11-1), (A1-42)+(B11-2), (A1-42)+(B11-2),
(A1-43)+(B1-1), (A1-43)+(B1-2), (A1-43)+(B1-3), (A1-43)+(B1-4), (A1-43)+(B1-5), (A1-43)+(B1-6), (A1-43)+(B2-1), (A1-43)+(B2-2), (A1-43)+(B2-3), (A1-43)+(B2-4), (A1-43)+(B2-5), (A1-43)+(B2-6), (A1-43)+(B2-7), (A1-43)+(B2-8), (A1-43)+(B2-9), (A1-43)+(B2-10), (A1-43)+(B2-11), (A1-43)+(B2-12), (A1-43)+(B2-13), (A1-43)+(B2-14), (A1-43)+(B2-15), (A1-43)+(B2-16), (A1-43)+(B2-17), (A1-43)+(B2-18), (A1-43)+(B2-19), (A1-43)+(B2-20), (A1-43)+(B2-21), (A1-43)+(B2-22), (A1-43)+(B2-23), (A1-43)+(B2-24), (A1-43)+(B2-25), (A1-43)+(B2-26), (A1-43)+(B2-27), (A1-43)+(B2-28), (A1-43)+(B2-29), (A1-43)+(B3-1), (A1-43)+(B3-2), (A1-43)+(B4-1), (A1-43)+(B4-2), (A1-43)+(B4-3), (A1-43)+(B4-4), (A1-43)+(B4-5), (A1-43)+(B4-6), (A1-43)+(B4-7), (A1-43)+(B4-8), (A1-43)+(B4-9), (A1-43)+(B4-10), (A1-43)+(B4-11), (A1-43)+(B5-1), (A1-43)+(B5-2), (A1-43)+(B5-3), (A1-43)+(B5-4), (A1-43)+(B5-5), (A1-43)+(B6-1), (A1-43)+(B6-2), (A1-43)+(B6-3), (A1-43)+(B6-4), (A1-43)+(B6-5), (A1-43)+(B6-6), (A1-43)+(B7-1), (A1-43)+(B7-2), (A1-43)+(B7-3), (A1-43)+(B7-4), (A1-43)+(B7-5), (A1-43)+(B7-6), (A1-43)+(B7-7)), (A1-43)+(B7-7), (A1-43)+(B7-8), (A1-43)+(B8-1), (A1-43)+(B8-2), (A1-43)+(B8-3), (A1-43)+(B8-4), (A1-43)+(B8-5), (A1-43)+(B8-6), (A1-43)+(B8-7), (A1-43)+(B9-1), (A1-43)+(B9-2), (A1-43)+(B10-1), (A1-43)+(B10-2), (A1-43)+(B10-3), (A1-43)+(B10-4), (A1-43)+(B10-5), (A1-43)+(B11-1), (A1-43)+(B11-2), (A1-43)+(B11-2),
(A1-44)+(B1-1), (A1-44)+(B1-2), (A1-44)+(B1-3), (A1-44)+(B1-4), (A1-44)+(B1-5), (A1-44)+(B1-6), (A1-44)+(B2-1), (A1-44)+(B2-2), (A1-44)+(B2-3), (A1-44)+(B2-4), (A1-44)+(B2-5), (A1-44)+(B2-6), (A1-44)+(B2-7), (A1-44)+(B2-8), (A1-44)+(B2-9), (A1-44)+(B2-10), (A1-44)+(B2-11), (A1-44)+(B2-12), (A1-44)+(B2-13), (A1-44)+(B2-14), (A1-44)+(B2-15), (A1-44)+(B2-16), (A1-44)+(B2-17), (A1-44)+(B2-18), (A1-44)+(B2-19), (A1-44)+(B2-20), (A1-44)+(B2-21), (A1-44)+(B2-22), (A1-44)+(B2-23), (A1-44)+(B2-24), (A1-44)+(B2-25), (A1-44)+(B2-26), (A1-44)+(B2-27), (A1-44)+(B2-28), (A1-44)+(B2-29), (A1-44)+(B3-1), (A1-44)+(B3-2), (A1-44)+(B4-1), (A1-44)+(B4-2), (A1-44)+(B4-3), (A1-44)+(B4-4), (A1-44)+(B4-5), (A1-44)+(B4-6), (A1-44)+(B4-7), (A1-44)+(B4-8), (A1-44)+(B4-9), (A1-44)+(B4-10), (A1-44)+(B4-11), (A1-44)+(B5-1), (A1-44)+(B5-2), (A1-44)+(B5-3), (A1-44)+(B5-4), (A1-44)+(B5-5), (A1-44)+(B6-1), (A1-44)+(B6-2), (A1-44)+(B6-3), (A1-44)+(B6-4), (A1-44)+(B6-5), (A1-44)+(B6-6), (A1-44)+(B7-1), (A1-44)+(B7-2), (A1-44)+(B7-3), (A1-44)+(B7-4), (A1-44)+(B7-5), (A1-44)+(B7-6), (A1-44)+(B7-7)), (A1-44)+(B7-7), (A1-44)+(B7-8), (A1-44)+(B8-1), (A1-44)+(B8-2), (A1-44)+(B8-3), (A1-44)+(B8-4), (A1-44)+(B8-5), (A1-44)+(B8-6), (A1-44)+(B8-7), (A1-44)+(B9-1), (A1-44)+(B9-2), (A1-44)+(B10-1), (A1-44)+(B10-2), (A1-44)+(B10-3), (A1-44)+(B10-4), (A1-44)+(B10-5), (A1-44)+(B11-1), (A1-44)+(B11-2), (A1-44)+(B11-2),
(A1-45)+(B1-1), (A1-45)+(B1-2), (A1-45)+(B1-3), (A1-45)+(B1-4), (A1-45)+(B1-5), (A1-45)+(B1-6), (A1-45)+(B2-1), (A1-45)+(B2-2), (A1-45)+(B2-3), (A1-45)+(B2-4), (A1-45)+(B2-5), (A1-45)+(B2-6), (A1-45)+(B2-7), (A1-45)+(B2-8), (A1-45)+(B2-9), (A1-45)+(B2-10), (A1-45)+(B2-11), (A1-45)+(B2-12), (A1-45)+(B2-13), (A1-45)+(B2-14), (A1-45)+(B2-15), (A1-45)+(B2-16), (A1-45)+(B2-17), (A1-45)+(B2-18), (A1-45)+(B2-19), (A1-45)+(B2-20), (A1-45)+(B2-21), (A1-45)+(B2-22), (A1-45)+(B2-23), (A1-45)+(B2-24), (A1-45)+(B2-25), (A1-45)+(B2-26), (A1-45)+(B2-27), (A1-45)+(B2-28), (A1-45)+(B2-29), (A1-45)+(B3-1), (A1-45)+(B3-2), (A1-45)+(B4-1), (A1-45)+(B4-2), (A1-45)+(B4-3), (A1-45)+(B4-4), (A1-45)+(B4-5), (A1-45)+(B4-6), (A1-45)+(B4-7), (A1-45)+(B4-8), (A1-45)+(B4-9), (A1-45)+(B4-10), (A1-45)+(B4-11), (A1-45)+(B5-1), (A1-45)+(B5-2), (A1-45)+(B5-3), (A1-45)+(B5-4), (A1-45)+(B5-5), (A1-45)+(B6-1), (A1-45)+(B6-2), (A1-45)+(B6-3), (A1-45)+(B6-4), (A1-45)+(B6-5), (A1-45)+(B6-6), (A1-45)+(B7-1), (A1-45)+(B7-2), (A1-45)+(B7-3), (A1-45)+(B7-4), (A1-45)+(B7-5), (A1-45)+(B7-6), (A1-45)+(B7-7)), (A1-45)+(B7-7), (A1-45)+(B7-8), (A1-45)+(B8-1), (A1-45)+(B8-2), (A1-45)+(B8-3), (A1-45)+(B8-4), (A1-45)+(B8-5), (A1-45)+(B8-6), (A1-45)+(B8-7), (A1-45)+(B9-1), (A1-45)+(B9-2), (A1-45)+(B10-1), (A1-45)+(B10-2), (A1-45)+(B10-3), (A1-45)+(B10-4), (A1-45)+(B10-5), (A1-45)+(B11-1), (A1-45)+(B11-2), (A1-45)+(B11-2),
(A1-46)+(B1-1), (A1-46)+(B1-2), (A1-46)+(B1-3), (A1-46)+(B1-4), (A1-46)+(B1-5), (A1-46)+(B1-6), (A1-46)+(B2-1), (A1-46)+(B2-2), (A1-46)+(B2-3), (A1-46)+(B2-4), (A1-46)+(B2-5), (A1-46)+(B2-6), (A1-46)+(B2-7), (A1-46)+(B2-8), (A1-46)+(B2-9), (A1-46)+(B2-10), (A1-46)+(B2-11), (A1-46)+(B2-12), (A1-46)+(B2-13), (A1-46)+(B2-14), (A1-46)+(B2-15), (A1-46)+(B2-16), (A1-46)+(B2-17), (A1-46)+(B2-18), (A1-46)+(B2-19), (A1-46)+(B2-20), (A1-46)+(B2-21), (A1-46)+(B2-22), (A1-46)+(B2-23), (A1-46)+(B2-24), (A1-46)+(B2-25), (A1-46)+(B2-26), (A1-46)+(B2-27), (A1-46)+(B2-28), (A1-46)+(B2-29), (A1-46)+(B3-1), (A1-46)+(B3-2), (A1-46)+(B4-1), (A1-46)+(B4-2), (A1-46)+(B4-3), (A1-46)+(B4-4), (A1-46)+(B4-5), (A1-46)+(B4-6), (A1-46)+(B4-7), (A1-46)+(B4-8), (A1-46)+(B4-9), (A1-46)+(B4-10), (A1-46)+(B4-11), (A1-46)+(B5-1), (A1-46)+(B5-2), (A1-46)+(B5-3), (A1-46)+(B5-4), (A1-46)+(B5-5), (A1-46)+(B6-1), (A1-46)+(B6-2), (A1-46)+(B6-3), (A1-46)+(B6-4), (A1-46)+(B6-5), (A1-46)+(B6-6), (A1-46)+(B7-1), (A1-46)+(B7-2), (A1-46)+(B7-3), (A1-46)+(B7-4), (A1-46)+(B7-5), (A1-46)+(B7-6), (A1-46)+(B7-7)), (A1-46)+(B7-7), (A1-46)+(B7-8), (A1-46)+(B8-1), (A1-46)+(B8-2), (A1-46)+(B8-3), (A1-46)+(B8-4), (A1-46)+(B8-5), (A1-46)+(B8-6), (A1-46)+(B8-7), (A1-46)+(B9-1), (A1-46)+(B9-2), (A1-46)+(B10-1), (A1-46)+(B10-2), (A1-46)+(B10-3), (A1-46)+(B10-4), (A1-46)+(B10-5), (A1-46)+(B11-1), (A1-46)+(B11-2), (A1-46)+(B11-2),
(A1-47)+(B1-1), (A1-47)+(B1-2), (A1-47)+(B1-3), (A1-47)+(B1-4), (A1-47)+(B1-5), (A1-47)+(B1-6), (A1-47)+(B2-1), (A1-47)+(B2-2), (A1-47)+(B2-3), (A1-47)+(B2-4), (A1-47)+(B2-5), (A1-47)+(B2-6), (A1-47)+(B2-7), (A1-47)+(B2-8), (A1-47)+(B2-9), (A1-47)+(B2-10), (A1-47)+(B2-11), (A1-47)+(B2-12), (A1-47)+(B2-13), (A1-47)+(B2-14), (A1-47)+(B2-15), (A1-47)+(B2-16), (A1-47)+(B2-17), (A1-47)+(B2-18), (A1-47)+(B2-19), (A1-47)+(B2-20), (A1-47)+(B2-21), (A1-47)+(B2-22), (A1-47)+(B2-23), (A1-47)+(B2-24), (A1-47)+(B2-25), (A1-47)+(B2-26), (A1-47)+(B2-27), (A1-47)+(B2-28), (A1-47)+(B2-29), (A1-47)+(B3-1), (A1-47)+(B3-2), (A1-47)+(B4-1), (A1-47)+(B4-2), (A1-47)+(B4-3), (A1-47)+(B4-4), (A1-47)+(B4-5), (A1-47)+(B4-6), (A1-47)+(B4-7), (A1-47)+(B4-8), (A1-47)+(B4-9), (A1-47)+(B4-10), (A1-47)+(B4-11), (A1-47)+(B5-1), (A1-47)+(B5-2), (A1-47)+(B5-3), (A1-47)+(B5-4), (A1-47)+(B5-5), (A1-47)+(B6-1), (A1-47)+(B6-2), (A1-47)+(B6-3), (A1-47)+(B6-4), (A1-47)+(B6-5), (A1-47)+(B6-6), (A1-47)+(B7-1), (A1-47)+(B7-2), (A1-47)+(B7-3), (A1-47)+(B7-4), (A1-47)+(B7-5), (A1-47)+(B7-6), (A1-47)+(B7-7)), (A1-47)+(B7-7), (A1-47)+(B7-8), (A1-47)+(B8-1), (A1-47)+(B8-2), (A1-47)+(B8-3), (A1-47)+(B8-4), (A1-47)+(B8-5), (A1-47)+(B8-6), (A1-47)+(B8-7), (A1-47)+(B9-1), (A1-47)+(B9-2), (A1-47)+(B10-1), (A1-47)+(B10-2), (A1-47)+(B10-3), (A1-47)+(B10-4), (A1-47)+(B10-5), (A1-47)+(B11-1), (A1-47)+(B11-2), (A1-47)+(B11-2),
(A1-48)+(B1-1), (A1-48)+(B1-2), (A1-48)+(B1-3), (A1-48)+(B1-4), (A1-48)+(B1-5), (A1-48)+(B1-6), (A1-48)+(B2-1), (A1-48)+(B2-2), (A1-48)+(B2-3), (A1-48)+(B2-4), (A1-48)+(B2-5), (A1-48)+(B2-6), (A1-48)+(B2-7), (A1-48)+(B2-8), (A1-48)+(B2-9), (A1-48)+(B2-10), (A1-48)+(B2-11), (A1-48)+(B2-12), (A1-48)+(B2-13), (A1-48)+(B2-14), (A1-48)+(B2-15), (A1-48)+(B2-16), (A1-48)+(B2-17), (A1-48)+(B2-18), (A1-48)+(B2-19), (A1-48)+(B2-20), (A1-48)+(B2-21), (A1-48)+(B2-22), (A1-48)+(B2-23), (A1-48)+(B2-24), (A1-48)+(B2-25), (A1-48)+(B2-26), (A1-48)+(B2-27), (A1-48)+(B2-28), (A1-48)+(B2-29), (A1-48)+(B3-1), (A1-48)+(B3-2), (A1-48)+(B4-1), (A1-48)+(B4-2), (A1-48)+(B4-3), (A1-48)+(B4-4), (A1-48)+(B4-5), (A1-48)+(B4-6), (A1-48)+(B4-7), (A1-48)+(B4-8), (A1-48)+(B4-9), (A1-48)+(B4-10), (A1-48)+(B4-11), (A1-48)+(B5-1), (A1-48)+(B5-2), (A1-48)+(B5-3), (A1-48)+(B5-4), (A1-48)+(B5-5), (A1-48)+(B6-1), (A1-48)+(B6-2), (A1-48)+(B6-3), (A1-48)+(B6-4), (A1-48)+(B6-5), (A1-48)+(B6-6), (A1-48)+(B7-1), (A1-48)+(B7-2), (A1-48)+(B7-3), (A1-48)+(B7-4), (A1-48)+(B7-5), (A1-48)+(B7-6), (A1-48)+(B7-7)), (A1-48)+(B7-7), (A1-48)+(B7-8), (A1-48)+(B8-1), (A1-48)+(B8-2), (A1-48)+(B8-3), (A1-48)+(B8-4), (A1-48)+(B8-5), (A1-48)+(B8-6), (A1-48)+(B8-7), (A1-48)+(B9-1), (A1-48)+(B9-2), (A1-48)+(B10-1), (A1-48)+(B10-2), (A1-48)+(B10-3), (A1-48)+(B10-4), (A1-48)+(B10-5), (A1-48)+(B11-1), (A1-48)+(B1 1-2), (A1-48)+(B11-2),
(A1-49)+(B1-1), (A1-49)+(B1-2), (A1-49)+(B1-3), (A1-49)+(B1-4), (A1-49)+(B1-5), (A1-49)+(B1-6), (A1-49)+(B2-1), (A1-49)+(B2-2), (A1-49)+(B2-3), (A1-49)+(B2-4), (A1-49)+(B2-5), (A1-49)+(B2-6), (A1-49)+(B2-7), (A1-49)+(B2-8), (A1-49)+(B2-9), (A1-49)+(B2-10), (A1-49)+(B2-11), (A1-49)+(B2-12), (A1-49)+(B2-13), (A1-49)+(B2-14), (A1-49)+(B2-15), (A1-49)+(B2-16), (A1-49)+(B2-17), (A1-49)+(B2-18), (A1-49)+(B2-19), (A1-49)+(B2-20), (A1-49)+(B2-21), (A1-49)+(B2-22), (A1-49)+(B2-23), (A1-49)+(B2-24), (A1-49)+(B2-25), (A1-49)+(B2-26), (A1-49)+(B2-27), (A1-49)+(B2-28), (A1-49)+(B2-29), (A1-49)+(B3-1), (A1-49)+(B3-2), (A1-49)+(B4-1), (A1-49)+(B4-2), (A1-49)+(B4-3), (A1-49)+(B4-4), (A1-49)+(B4-5), (A1-49)+(B4-6), (A1-49)+(B4-7), (A1-49)+(B4-8), (A1-49)+(B4-9), (A1-49)+(B4-10), (A1-49)+(B4-11), (A1-49)+(B5-1), (A1-49)+(B5-2), (A1-49)+(B5-3), (A1-49)+(B5-4), (A1-49)+(B5-5), (A1-49)+(B6-1), (A1-49)+(B6-2), (A1-49)+(B6-3), (A1-49)+(B6-4), (A1-49)+(B6-5), (A1-49)+(B6-6), (A1-49)+(B7-1), (A1-49)+(B7-2), (A1-49)+(B7-3), (A1-49)+(B7-4), (A1-49)+(B7-5), (A1-49)+(B7-6), (A1-49)+(B7-7)), (A1-49)+(B7-7), (A1-49)+(B7-8), (A1-49)+(B8-1), (A1-49)+(B8-2), (A1-49)+(B8-3), (A1-49)+(B8-4), (A1-49)+(B8-5), (A1-49)+(B8-6), (A1-49)+(B8-7), (A1-49)+(B9-1), (A1-49)+(B9-2), (A1-49)+(B10-1), (A1-49)+(B10-2), (A1-49)+(B10-3), (A1-49)+(B10-4), (A1-49)+(B10-5), (A1-49)+(B11-1), (A1-49)+(B11-2), (A1-49)+(B11-2),
(A1-50)+(B1-1), (A1-50)+(B1-2), (A1-50)+(B1-3), (A1-50)+(B1-4), (A1-50)+(B1-5), (A1-50)+(B1-6), (A1-50)+(B2-1), (A1-50)+(B2-2), (A1-50)+(B2-3), (A1-50)+(B2-4), (A1-50)+(B2-5), (A1-50)+(B2-6), (A1-50)+(B2-7), (A1-50)+(B2-8), (A1-50)+(B2-9), (A1-50)+(B2-10), (A1-50)+(B2-11), (A1-50)+(B2-12), (A1-50)+(B2-13), (A1-50)+(B2-14), (A1-50)+(B2-15), (A1-50)+(B2-16), (A1-50)+(B2-17), (A1-50)+(B2-18), (A1-50)+(B2-19), (A1-50)+(B2-20), (A1-50)+(B2-21), (A1-50)+(B2-22), (A1-50)+(B2-23), (A1-50)+(B2-24), (A1-50)+(B2-25), (A1-50)+(B2-26), (A1-50)+(B2-27), (A1-50)+(B2-28), (A1-50)+(B2-29), (A1-50)+(B3-1), (A1-50)+(B3-2), (A1-50)+(B4-1), (A1-50)+(B4-2), (A1-50)+(B4-3), (A1-50)+(B4-4), (A1-50)+(B4-5), (A1-50)+(B4-6), (A1-50)+(B4-7), (A1-50)+(B4-8), (A1-50)+(B4-9), (A1-50)+(B4-10), (A1-50)+(B4-11), (A1-50)+(B5-1), (A1-50)+(B5-2), (A1-50)+(B5-3), (A1-50)+(B5-4), (A1-50)+(B5-5), (A1-50)+(B6-1), (A1-50)+(B6-2), (A1-50)+(B6-3), (A1-50)+(B6-4), (A1-50)+(B6-5), (A1-50)+(B6-6), (A1-50)+(B7-1), (A1-50)+(B7-2), (A1-50)+(B7-3), (A1-50)+(B7-4), (A1-50)+(B7-5), (A1-50)+(B7-6), (A1-50)+(B7-7)), (A1-50)+(B7-7), (A1-50)+(B7-8), (A1-50)+(B8-1), (A1-50)+(B8-2), (A1-50)+(B8-3), (A1-50)+(B8-4), (A1-50)+(B8-5), (A1-50)+(B8-6), (A1-50)+(B8-7), (A1-50)+(B9-1), (A1-50)+(B9-2), (A1-50)+(B10-1), (A1-50)+(B10-2), (A1-50)+(B10-3), (A1-50)+(B10-4), (A1-50)+(B10-5), (A1-50)+(B11-1), (A1-50)+(B11-2), (A1-50)+(B11-2),
(A1-51)+(B1-1), (A1-51)+(B1-2), (A1-51)+(B1-3), (A1-51)+(B1-4), (A1-51)+(B1-5), (A1-51)+(B1-6), (A1-51)+(B2-1), (A1-51)+(B2-2), (A1-51)+(B2-3), (A1-51)+(B2-4), (A1-51)+(B2-5), (A1-51)+(B2-6), (A1-51)+(B2-7), (A1-51)+(B2-8), (A1-51)+(B2-9), (A1-51)+(B2-10), (A1-51)+(B2-11), (A1-51)+(B2-12), (A1-51)+(B2-13), (A1-51)+(B2-14), (A1-51)+(B2-15), (A1-51)+(B2-16), (A1-51)+(B2-17), (A1-51)+(B2-18), (A1-51)+(B2-19), (A1-51)+(B2-20), (A1-51)+(B2-21), (A1-51)+(B2-22), (A1-51)+(B2-23), (A1-51)+(B2-24), (A1-51)+(B2-25), (A1-51)+(B2-26), (A1-51)+(B2-27), (A1-51)+(B2-28), (A1-51)+(B2-29), (A1-51)+(B3-1), (A1-51)+(B3-2), (A1-51)+(B4-1), (A1-51)+(B4-2), (A1-51)+(B4-3), (A1-51)+(B4-4), (A1-51)+(B4-5), (A1-51)+(B4-6), (A1-51)+(B4-7), (A1-51)+(B4-8), (A1-51)+(B4-9), (A1-51)+(B4-10), (A1-51)+(B4-11), (A1-51)+(B5-1), (A1-51)+(B5-2), (A1-51)+(B5-3), (A1-51)+(B5-4), (A1-51)+(B5-5), (A1-51)+(B6-1), (A1-51)+(B6-2), (A1-51)+(B6-3), (A1-51)+(B6-4), (A1-51)+(B6-5), (A1-51)+(B6-6), (A1-51)+(B7-1), (A1-51)+(B7-2), (A1-51)+(B7-3), (A1-51)+(B7-4), (A1-51)+(B7-5), (A1-51)+(B7-6), (A1-51)+(B7-7)), (A1-51)+(B7-7), (A1-51)+(B7-8), (A1-51)+(B8-1), (A1-51)+(B8-2), (A1-51)+(B8-3), (A1-51)+(B8-4), (A1-51)+(B8-5), (A1-51)+(B8-6), (A1-51)+(B8-7), (A1-51)+(B9-1), (A1-51)+(B9-2), (A1-51)+(B10-1), (A1-51)+(B10-2), (A1-51)+(B10-3), (A1-51)+(B10-4), (A1-51)+(B10-5), (A1-51)+(B11-1), (A1-51)+(B11-2), (A1-51)+(B11-2),
(A1-52)+(B1-1), (A1-52)+(B1-2), (A1-52)+(B1-3), (A1-52)+(B1-4), (A1-52)+(B1-5), (A1-52)+(B1-6), (A1-52)+(B2-1), (A1-52)+(B2-2), (A1-52)+(B2-3), (A1-52)+(B2-4), (A1-52)+(B2-5), (A1-52)+(B2-6), (A1-52)+(B2-7), (A1-52)+(B2-8), (A1-52)+(B2-9), (A1-52)+(B2-10), (A1-52)+(B2-11), (A1-52)+(B2-12), (A1-52)+(B2-13), (A1-52)+(B2-14), (A1-52)+(B2-15), (A1-52)+(B2-16), (A1-52)+(B2-17), (A1-52)+(B2-18), (A1-52)+(B2-19), (A1-52)+(B2-20), (A1-52)+(B2-21), (A1-52)+(B2-22), (A1-52)+(B2-23), (A1-52)+(B2-24), (A1-52)+(B2-25), (A1-52)+(B2-26), (A1-52)+(B2-27), (A1-52)+(B2-28), (A1-52)+(B2-29), (A1-52)+(B3-1), (A1-52)+(B3-2), (A1-52)+(B4-1), (A1-52)+(B4-2), (A1-52)+(B4-3), (A1-52)+(B4-4), (A1-52)+(B4-5), (A1-52)+(B4-6), (A1-52)+(B4-7), (A1-52)+(B4-8), (A1-52)+(B4-9), (A1-52)+(B4-10), (A1-52)+(B4-11), (A1-52)+(B5-1), (A1-52)+(B5-2), (A1-52)+(B5-3), (A1-52)+(B5-4), (A1-52)+(B5-5), (A1-52)+(B6-1), (A1-52)+(B6-2), (A1-52)+(B6-3), (A1-52)+(B6-4), (A1-52)+(B6-5), (A1-52)+(B6-6), (A1-52)+(B7-1), (A1-52)+(B7-2), (A1-52)+(B7-3), (A1-52)+(B7-4), (A1-52)+(B7-5), (A1-52)+(B7-6), (A1-52)+(B7-7)), (A1-52)+(B7-7), (A1-52)+(B7-8), (A1-52)+(B8-1), (A1-52)+(B8-2), (A1-52)+(B8-3), (A1-52)+(B8-4), (A1-52)+(B8-5), (A1-52)+(B8-6), (A1-52)+(B8-7), (A1-52)+(B9-1), (A1-52)+(B9-2), (A1-52)+(B10-1), (A1-52)+(B10-2), (A1-52)+(B10-3), (A1-52)+(B10-4), (A1-52)+(B10-5), (A1-52)+(B11-1), (A1-52)+(B11-2), (A1-52)+(B11-2),
(A1-53)+(B1-1), (A1-53)+(B1-2), (A1-53)+(B1-3), (A1-53)+(B1-4), (A1-53)+(B1-5), (A1-53)+(B1-6), (A1-53)+(B2-1), (A1-53)+(B2-2), (A1-53)+(B2-3), (A1-53)+(B2-4), (A1-53)+(B2-5), (A1-53)+(B2-6), (A1-53)+(B2-7), (A1-53)+(B2-8), (A1-53)+(B2-9), (A1-53)+(B2-10), (A1-53)+(B2-11), (A1-53)+(B2-12), (A1-53)+(B2-13), (A1-53)+(B2-14), (A1-53)+(B2-15), (A1-53)+(B2-16), (A1-53)+(B2-17), (A1-53)+(B2-18), (A1-53)+(B2-19), (A1-53)+(B2-20), (A1-53)+(B2-21), (A1-53)+(B2-22), (A1-53)+(B2-23), (A1-53)+(B2-24), (A1-53)+(B2-25), (A1-53)+(B2-26), (A1-53)+(B2-27), (A1-53)+(B2-28), (A1-53)+(B2-29), (A1-53)+(B3-1), (A1-53)+(B3-2), (A1-53)+(B4-1), (A1-53)+(B4-2), (A1-53)+(B4-3), (A1-53)+(B4-4), (A1-53)+(B4-5), (A1-53)+(B4-6), (A1-53)+(B4-7), (A1-53)+(B4-8), (A1-53)+(B4-9), (A1-53)+(B4-10), (A1-53)+(B4-11), (A1-53)+(B5-1), (A1-53)+(B5-2), (A1-53)+(B5-3), (A1-53)+(B5-4), (A1-53)+(B5-5), (A1-53)+(B6-1), (A1-53)+(B6-2), (A1-53)+(B6-3), (A1-53)+(B6-4), (A1-53)+(B6-5), (A1-53)+(B6-6), (A1-53)+(B7-1), (A1-53)+(B7-2), (A1-53)+(B7-3), (A1-53)+(B7-4), (A1-53)+(B7-5), (A1-53)+(B7-6), (A1-53)+(B7-7)), (A1-53)+(B7-7), (A1-53)+(B7-8), (A1-53)+(B8-1), (A1-53)+(B8-2), (A1-53)+(B8-3), (A1-53)+(B8-4), (A1-53)+(B8-5), (A1-53)+(B8-6), (A1-53)+(B8-7), (A1-53)+(B9-1), (A1-53)+(B9-2), (A1-53)+(B10-1), (A1-53)+(B10-2), (A1-53)+(B10-3), (A1-53)+(B10-4), (A1-53)+(B10-5), (A1-53)+(B11-1), (A1-53)+(B1 1-2), (A1-53)+(B11-2),
(A1-54)+(B1-1), (A1-54)+(B1-2), (A1-54)+(B1-3), (A1-54)+(B1-4), (A1-54)+(B1-5), (A1-54)+(B1-6), (A1-54)+(B2-1), (A1-54)+(B2-2), (A1-54)+(B2-3), (A1-54)+(B2-4), (A1-54)+(B2-5), (A1-54)+(B2-6), (A1-54)+(B2-7), (A1-54)+(B2-8), (A1-54)+(B2-9), (A1-54)+(B2-10), (A1-54)+(B2-11), (A1-54)+(B2-12), (A1-54)+(B2-13), (A1-54)+(B2-14), (A1-54)+(B2-15), (A1-54)+(B2-16), (A1-54)+(B2-17), (A1-54)+(B2-18), (A1-54)+(B2-19), (A1-54)+(B2-20), (A1-54)+(B2-21), (A1-54)+(B2-22), (A1-54)+(B2-23), (A1-54)+(B2-24), (A1-54)+(B2-25), (A1-54)+(B2-26), (A1-54)+(B2-27), (A1-54)+(B2-28), (A1-54)+(B2-29), (A1-54)+(B3-1), (A1-54)+(B3-2), (A1-54)+(B4-1), (A1-54)+(B4-2), (A1-54)+(B4-3), (A1-54)+(B4-4), (A1-54)+(B4-5), (A1-54)+(B4-6), (A1-54)+(B4-7), (A1-54)+(B4-8), (A1-54)+(B4-9), (A1-54)+(B4-10), (A1-54)+(B4-11), (A1-54)+(B5-1), (A1-54)+(B5-2), (A1-54)+(B5-3), (A1-54)+(B5-4), (A1-54)+(B5-5), (A1-54)+(B6-1), (A1-54)+(B6-2), (A1-54)+(B6-3), (A1-54)+(B6-4), (A1-54)+(B6-5), (A1-54)+(B6-6), (A1-54)+(B7-1), (A1-54)+(B7-2), (A1-54)+(B7-3), (A1-54)+(B7-4), (A1-54)+(B7-5), (A1-54)+(B7-6), (A1-54)+(B7-7)), (A1-54)+(B7-7), (A1-54)+(B7-8), (A1-54)+(B8-1), (A1-54)+(B8-2), (A1-54)+(B8-3), (A1-54)+(B8-4), (A1-54)+(B8-5), (A1-54)+(B8-6), (A1-54)+(B8-7), (A1-54)+(B9-1), (A1-54)+(B9-2), (A1-54)+(B10-1), (A1-54)+(B10-2), (A1-54)+(B10-3), (A1-54)+(B10-4), (A1-54)+(B10-5), (A1-54)+(B11-1), (A1-54)+(B11-2), (A1-54)+(B11-2),
(A1-55)+(B1-1), (A1-55)+(B1-2), (A1-55)+(B1-3), (A1-55)+(B1-4), (A1-55)+(B1-5), (A1-55)+(B1-6), (A1-55)+(B2-1), (A1-55)+(B2-2), (A1-55)+(B2-3), (A1-55)+(B2-4), (A1-55)+(B2-5), (A1-55)+(B2-6), (A1-55)+(B2-7), (A1-55)+(B2-8), (A1-55)+(B2-9), (A1-55)+(B2-10), (A1-55)+(B2-11), (A1-55)+(B2-12), (A1-55)+(B2-13), (A1-55)+(B2-14), (A1-55)+(B2-15), (A1-55)+(B2-16), (A1-55)+(B2-17), (A1-55)+(B2-18), (A1-55)+(B2-19), (A1-55)+(B2-20), (A1-55)+(B2-21), (A1-55)+(B2-22), (A1-55)+(B2-23), (A1-55)+(B2-24), (A1-55)+(B2-25), (A1-55)+(B2-26), (A1-55)+(B2-27), (A1-55)+(B2-28), (A1-55)+(B2-29), (A1-55)+(B3-1), (A1-55)+(B3-2), (A1-55)+(B4-1), (A1-55)+(B4-2), (A1-55)+(B4-3), (A1-55)+(B4-4), (A1-55)+(B4-5), (A1-55)+(B4-6), (A1-55)+(B4-7), (A1-55)+(B4-8), (A1-55)+(B4-9), (A1-55)+(B4-10), (A1-55)+(B4-11), (A1-55)+(B5-1), (A1-55)+(B5-2), (A1-55)+(B5-3), (A1-55)+(B5-4), (A1-55)+(B5-5), (A1-55)+(B6-1), (A1-55)+(B6-2), (A1-55)+(B6-3), (A1-55)+(B6-4), (A1-55)+(B6-5), (A1-55)+(B6-6), (A1-55)+(B7-1), (A1-55)+(B7-2), (A1-55)+(B7-3), (A1-55)+(B7-4), (A1-55)+(B7-5), (A1-55)+(B7-6), (A1-55)+(B7-7)), (A1-55)+(B7-7), (A1-55)+(B7-8), (A1-55)+(B8-1), (A1-55)+(B8-2), (A1-55)+(B8-3), (A1-55)+(B8-4), (A1-55)+(B8-5), (A1-55)+(B8-6), (A1-55)+(B8-7), (A1-55)+(B9-1), (A1-55)+(B9-2), (A1-55)+(B10-1), (A1-55)+(B10-2), (A1-55)+(B10-3), (A1-55)+(B10-4), (A1-55)+(B10-5), (A1-55)+(B11-1), (A1-55)+(B1 1-2), (A1-55)+(B11-2),
(A1-56)+(B1-1), (A1-56)+(B1-2), (A1-56)+(B1-3), (A1-56)+(B1-4), (A1-56)+(B1-5), (A1-56)+(B1-6), (A1-56)+(B2-1), (A1-56)+(B2-2), (A1-56)+(B2-3), (A1-56)+(B2-4), (A1-56)+(B2-5), (A1-56)+(B2-6), (A1-56)+(B2-7), (A1-56)+(B2-8), (A1-56)+(B2-9), (A1-56)+(B2-10), (A1-56)+(B2-11), (A1-56)+(B2-12), (A1-56)+(B2-13), (A1-56)+(B2-14), (A1-56)+(B2-15), (A1-56)+(B2-16), (A1-56)+(B2-17), (A1-56)+(B2-18), (A1-56)+(B2-19), (A1-56)+(B2-20), (A1-56)+(B2-21), (A1-56)+(B2-22), (A1-56)+(B2-23), (A1-56)+(B2-24), (A1-56)+(B2-25), (A1-56)+(B2-26), (A1-56)+(B2-27), (A1-56)+(B2-28), (A1-56)+(B2-29), (A1-56)+(B3-1), (A1-56)+(B3-2), (A1-56)+(B4-1), (A1-56)+(B4-2), (A1-56)+(B4-3), (A1-56)+(B4-4), (A1-56)+(B4-5), (A1-56)+(B4-6), (A1-56)+(B4-7), (A1-56)+(B4-8), (A1-56)+(B4-9), (A1-56)+(B4-10), (A1-56)+(B4-11), (A1-56)+(B5-1), (A1-56)+(B5-2), (A1-56)+(B5-3), (A1-56)+(B5-4), (A1-56)+(B5-5), (A1-56)+(B6-1), (A1-56)+(B6-2), (A1-56)+(B6-3), (A1-56)+(B6-4), (A1-56)+(B6-5), (A1-56)+(B6-6), (A1-56)+(B7-1), (A1-56)+(B7-2), (A1-56)+(B7-3), (A1-56)+(B7-4), (A1-56)+(B7-5), (A1-56)+(B7-6), (A1-56)+(B7-7)), (A1-56)+(B7-7), (A1-56)+(B7-8), (A1-56)+(B8-1), (A1-56)+(B8-2), (A1-56)+(B8-3), (A1-56)+(B8-4), (A1-56)+(B8-5), (A1-56)+(B8-6), (A1-56)+(B8-7), (A1-56)+(B9-1), (A1-56)+(B9-2), (A1-56)+(B10-1), (A1-56)+(B10-2), (A1-56)+(B10-3), (A1-56)+(B10-4), (A1-56)+(B10-5), (A1-56)+(B11-1), (A1-56)+(B11-2), (A1-56)+(B11-2),
(A1-57)+(B1-1), (A1-57)+(B1-2), (A1-57)+(B1-3), (A1-57)+(B1-4), (A1-57)+(B1-5), (A1-57)+(B1-6), (A1-57)+(B2-1), (A1-57)+(B2-2), (A1-57)+(B2-3), (A1-57)+(B2-4), (A1-57)+(B2-5), (A1-57)+(B2-6), (A1-57)+(B2-7), (A1-57)+(B2-8), (A1-57)+(B2-9), (A1-57)+(B2-10), (A1-57)+(B2-11), (A1-57)+(B2-12), (A1-57)+(B2-13), (A1-57)+(B2-14), (A1-57)+(B2-15), (A1-57)+(B2-16), (A1-57)+(B2-17), (A1-57)+(B2-18), (A1-57)+(B2-19), (A1-57)+(B2-20), (A1-57)+(B2-21), (A1-57)+(B2-22), (A1-57)+(B2-23), (A1-57)+(B2-24), (A1-57)+(B2-25), (A1-57)+(B2-26), (A1-57)+(B2-27), (A1-57)+(B2-28), (A1-57)+(B2-29), (A1-57)+(B3-1), (A1-57)+(B3-2), (A1-57)+(B4-1), (A1-57)+(B4-2), (A1-57)+(B4-3), (A1-57)+(B4-4), (A1-57)+(B4-5), (A1-57)+(B4-6), (A1-57)+(B4-7), (A1-57)+(B4-8), (A1-57)+(B4-9), (A1-57)+(B4-10), (A1-57)+(B4-11), (A1-57)+(B5-1), (A1-57)+(B5-2), (A1-57)+(B5-3), (A1-57)+(B5-4), (A1-57)+(B5-5), (A1-57)+(B6-1), (A1-57)+(B6-2), (A1-57)+(B6-3), (A1-57)+(B6-4), (A1-57)+(B6-5), (A1-57)+(B6-6), (A1-57)+(B7-1), (A1-57)+(B7-2), (A1-57)+(B7-3), (A1-57)+(B7-4), (A1-57)+(B7-5), (A1-57)+(B7-6), (A1-57)+(B7-7)), (A1-57)+(B7-7), (A1-57)+(B7-8), (A1-57)+(B8-1), (A1-57)+(B8-2), (A1-57)+(B8-3), (A1-57)+(B8-4), (A1-57)+(B8-5), (A1-57)+(B8-6), (A1-57)+(B8-7), (A1-57)+(B9-1), (A1-57)+(B9-2), (A1-57)+(B10-1), (A1-57)+(B10-2), (A1-57)+(B10-3), (A1-57)+(B10-4), (A1-57)+(B10-5), (A1-57)+(B11-1), (A1-57)+(B11-2), (A1-57)+(B11-2),
(A1-58)+(B1-1), (A1-58)+(B1-2), (A1-58)+(B1-3), (A1-58)+(B1-4), (A1-58)+(B1-5), (A1-58)+(B1-6), (A1-58)+(B2-1), (A1-58)+(B2-2), (A1-58)+(B2-3), (A1-58)+(B2-4), (A1-58)+(B2-5), (A1-58)+(B2-6), (A1-58)+(B2-7), (A1-58)+(B2-8), (A1-58)+(B2-9), (A1-58)+(B2-10), (A1-58)+(B2-11), (A1-58)+(B2-12), (A1-58)+(B2-13), (A1-58)+(B2-14), (A1-58)+(B2-15), (A1-58)+(B2-16), (A1-58)+(B2-17), (A1-58)+(B2-18), (A1-58)+(B2-19), (A1-58)+(B2-20), (A1-58)+(B2-21), (A1-58)+(B2-22), (A1-58)+(B2-23), (A1-58)+(B2-24), (A1-58)+(B2-25), (A1-58)+(B2-26), (A1-58)+(B2-27), (A1-58)+(B2-28), (A1-58)+(B2-29), (A1-58)+(B3-1), (A1-58)+(B3-2), (A1-58)+(B4-1), (A1-58)+(B4-2), (A1-58)+(B4-3), (A1-58)+(B4-4), (A1-58)+(B4-5), (A1-58)+(B4-6), (A1-58)+(B4-7), (A1-58)+(B4-8), (A1-58)+(B4-9), (A1-58)+(B4-10), (A1-58)+(B4-11), (A1-58)+(B5-1), (A1-58)+(B5-2), (A1-58)+(B5-3), (A1-58)+(B5-4), (A1-58)+(B5-5), (A1-58)+(B6-1), (A1-58)+(B6-2), (A1-58)+(B6-3), (A1-58)+(B6-4), (A1-58)+(B6-5), (A1-58)+(B6-6), (A1-58)+(B7-1), (A1-58)+(B7-2), (A1-58)+(B7-3), (A1-58)+(B7-4), (A1-58)+(B7-5), (A1-58)+(B7-6), (A1-58)+(B7-7)), (A1-58)+(B7-7), (A1-58)+(B7-8), (A1-58)+(B8-1), (A1-58)+(B8-2), (A1-58)+(B8-3), (A1-58)+(B8-4), (A1-58)+(B8-5), (A1-58)+(B8-6), (A1-58)+(B8-7), (A1-58)+(B9-1), (A1-58)+(B9-2), (A1-58)+(B10-1), (A1-58)+(B10-2), (A1-58)+(B10-3), (A1-58)+(B10-4), (A1-58)+(B10-5), (A1-58)+(B11-1), (A1-58)+(B11-2), (A1-58)+(B11-2),
(A1-59)+(B1-1), (A1-59)+(B1-2), (A1-59)+(B1-3), (A1-59)+(B1-4), (A1-59)+(B1-5), (A1-59)+(B1-6), (A1-59)+(B2-1), (A1-59)+(B2-2), (A1-59)+(B2-3), (A1-59)+(B2-4), (A1-59)+(B2-5), (A1-59)+(B2-6), (A1-59)+(B2-7), (A1-59)+(B2-8), (A1-59)+(B2-9), (A1-59)+(B2-10), (A1-59)+(B2-11), (A1-59)+(B2-12), (A1-59)+(B2-13), (A1-59)+(B2-14), (A1-59)+(B2-15), (A1-59)+(B2-16), (A1-59)+(B2-17), (A1-59)+(B2-18), (A1-59)+(B2-19), (A1-59)+(B2-20), (A1-59)+(B2-21), (A1-59)+(B2-22), (A1-59)+(B2-23), (A1-59)+(B2-24), (A1-59)+(B2-25), (A1-59)+(B2-26), (A1-59)+(B2-27), (A1-59)+(B2-28), (A1-59)+(B2-29), (A1-59)+(B3-1), (A1-59)+(B3-2), (A1-59)+(B4-1), (A1-59)+(B4-2), (A1-59)+(B4-3), (A1-59)+(B4-4), (A1-59)+(B4-5), (A1-59)+(B4-6), (A1-59)+(B4-7), (A1-59)+(B4-8), (A1-59)+(B4-9), (A1-59)+(B4-10), (A1-59)+(B4-11), (A1-59)+(B5-1), (A1-59)+(B5-2), (A1-59)+(B5-3), (A1-59)+(B5-4), (A1-59)+(B5-5), (A1-59)+(B6-1), (A1-59)+(B6-2), (A1-59)+(B6-3), (A1-59)+(B6-4), (A1-59)+(B6-5), (A1-59)+(B6-6), (A1-59)+(B7-1), (A1-59)+(B7-2), (A1-59)+(B7-3), (A1-59)+(B7-4), (A1-59)+(B7-5), (A1-59)+(B7-6), (A1-59)+(B7-7)), (A1-59)+(B7-7), (A1-59)+(B7-8), (A1-59)+(B8-1), (A1-59)+(B8-2), (A1-59)+(B8-3), (A1-59)+(B8-4), (A1-59)+(B8-5), (A1-59)+(B8-6), (A1-59)+(B8-7), (A1-59)+(B9-1), (A1-59)+(B9-2), (A1-59)+(B10-1), (A1-59)+(B10-2), (A1-59)+(B10-3), (A1-59)+(B10-4), (A1-59)+(B10-5), (A1-59)+(B11-1), (A1-59)+(B11-2), (A1-59)+(B11-2),
(A1-60)+(B1-1), (A1-60)+(B1-2), (A1-60)+(B1-3), (A1-60)+(B1-4), (A1-60)+(B1-5), (A1-60)+(B1-6), (A1-60)+(B2-1), (A1-60)+(B2-2), (A1-60)+(B2-3), (A1-60)+(B2-4), (A1-60)+(B2-5), (A1-60)+(B2-6), (A1-60)+(B2-7), (A1-60)+(B2-8), (A1-60)+(B2-9), (A1-60)+(B2-10), (A1-60)+(B2-11), (A1-60)+(B2-12), (A1-60)+(B2-13), (A1-60)+(B2-14), (A1-60)+(B2-15), (A1-60)+(B2-16), (A1-60)+(B2-17), (A1-60)+(B2-18), (A1-60)+(B2-19), (A1-60)+(B2-20), (A1-60)+(B2-21), (A1-60)+(B2-22), (A1-60)+(B2-23), (A1-60)+(B2-24), (A1-60)+(B2-25), (A1-60)+(B2-26), (A1-60)+(B2-27), (A1-60)+(B2-28), (A1-60)+(B2-29), (A1-60)+(B3-1), (A1-60)+(B3-2), (A1-60)+(B4-1), (A1-60)+(B4-2), (A1-60)+(B4-3), (A1-60)+(B4-4), (A1-60)+(B4-5), (A1-60)+(B4-6), (A1-60)+(B4-7), (A1-60)+(B4-8), (A1-60)+(B4-9), (A1-60)+(B4-10), (A1-60)+(B4-11), (A1-60)+(B5-1), (A1-60)+(B5-2), (A1-60)+(B5-3), (A1-60)+(B5-4), (A1-60)+(B5-5), (A1-60)+(B6-1), (A1-60)+(B6-2), (A1-60)+(B6-3), (A1-60)+(B6-4), (A1-60)+(B6-5), (A1-60)+(B6-6), (A1-60)+(B7-1), (A1-60)+(B7-2), (A1-60)+(B7-3), (A1-60)+(B7-4), (A1-60)+(B7-5), (A1-60)+(B7-6), (A1-60)+(B7-7)), (A1-60)+(B7-7), (A1-60)+(B7-8), (A1-60)+(B8-1), (A1-60)+(B8-2), (A1-60)+(B8-3), (A1-60)+(B8-4), (A1-60)+(B8-5), (A1-60)+(B8-6), (A1-60)+(B8-7), (A1-60)+(B9-1), (A1-60)+(B9-2), (A1-60)+(B10-1), (A1-60)+(B10-2), (A1-60)+(B10-3), (A1-60)+(B10-4), (A1-60)+(B10-5), (A1-60)+(B11-1), (A1-60)+(B11-2), (A1-60)+(B11-2),
(A1-61)+(B1-1), (A1-61)+(B1-2), (A1-61)+(B1-3), (A1-61)+(B1-4), (A1-61)+(B1-5), (A1-61)+(B1-6), (A1-61)+(B2-1), (A1-61)+(B2-2), (A1-61)+(B2-3), (A1-61)+(B2-4), (A1-61)+(B2-5), (A1-61)+(B2-6), (A1-61)+(B2-7), (A1-61)+(B2-8), (A1-61)+(B2-9), (A1-61)+(B2-10), (A1-61)+(B2-11), (A1-61)+(B2-12), (A1-61)+(B2-13), (A1-61)+(B2-14), (A1-61)+(B2-15), (A1-61)+(B2-16), (A1-61)+(B2-17), (A1-61)+(B2-18), (A1-61)+(B2-19), (A1-61)+(B2-20), (A1-61)+(B2-21), (A1-61)+(B2-22), (A1-61)+(B2-23), (A1-61)+(B2-24), (A1-61)+(B2-25), (A1-61)+(B2-26), (A1-61)+(B2-27), (A1-61)+(B2-28), (A1-61)+(B2-29), (A1-61)+(B3-1), (A1-61)+(B3-2), (A1-61)+(B4-1), (A1-61)+(B4-2), (A1-61)+(B4-3), (A1-61)+(B4-4), (A1-61)+(B4-5), (A1-61)+(B4-6), (A1-61)+(B4-7), (A1-61)+(B4-8), (A1-61)+(B4-9), (A1-61)+(B4-10), (A1-61)+(B4-11), (A1-61)+(B5-1), (A1-61)+(B5-2), (A1-61)+(B5-3), (A1-61)+(B5-4), (A1-61)+(B5-5), (A1-61)+(B6-1), (A1-61)+(B6-2), (A1-61)+(B6-3), (A1-61)+(B6-4), (A1-61)+(B6-5), (A1-61)+(B6-6), (A1-61)+(B7-1), (A1-61)+(B7-2), (A1-61)+(B7-3), (A1-61)+(B7-4), (A1-61)+(B7-5), (A1-61)+(B7-6), (A1-61)+(B7-7)), (A1-61)+(B7-7), (A1-61)+(B7-8), (A1-61)+(B8-1), (A1-61)+(B8-2), (A1-61)+(B8-3), (A1-61)+(B8-4), (A1-61)+(B8-5), (A1-61)+(B8-6), (A1-61)+(B8-7), (A1-61)+(B9-1), (A1-61)+(B9-2), (A1-61)+(B10-1), (A1-61)+(B10-2), (A1-61)+(B10-3), (A1-61)+(B10-4), (A1-61)+(B10-5), (A1-61)+(B11-1), (A1-61)+(B11-2), (A1-61)+(B11-2),
(A2-1)+(B1-1), (A2-1)+(B1-2), (A2-1)+(B1-3), (A2-1)+(B1-4), (A2-1)+(B1-5), (A2-1)+(B1-6), (A2-1)+(B2-1), (A2-1)+(B2-2), (A2-1)+(B2-3), (A2-1)+(B2-4), (A2-1)+(B2-5), (A2-1)+(B2-6), (A2-1)+(B2-7), (A2-1)+(B2-8), (A2-1)+(B2-9), (A2-1)+(B2-10), (A2-1)+(B2-11), (A2-1)+(B2-12), (A2-1)+(B2-13), (A2-1)+(B2-14), (A2-1)+(B2-15), (A2-1)+(B2-16), (A2-1)+(B2-17), (A2-1)+(B2-18), (A2-1)+(B2-19), (A2-1)+(B2-20), (A2-1)+(B2-21), (A2-1)+(B2-22), (A2-1)+(B2-23), (A2-1)+(B2-24), (A2-1)+(B2-25), (A2-1)+(B2-26), (A2-1)+(B2-27), (A2-1)+(B2-28), (A2-1)+(B2-29), (A2-1)+(B3-1), (A2-1)+(B3-2), (A2-1)+(B4-1), (A2-1)+(B4-2), (A2-1)+(B4-3), (A2-1)+(B4-4), (A2-1)+(B4-5), (A2-1)+(B4-6), (A2-1)+(B4-7), (A2-1)+(B4-8), (A2-1)+(B4-9), (A2-1)+(B4-10), (A2-1)+(B4-11), (A2-1)+(B5-1), (A2-1)+(B5-2), (A2-1)+(B5-3), (A2-1)+(B5-4), (A2-1)+(B5-5), (A2-1)+(B6-1), (A2-1)+(B6-2), (A2-1)+(B6-3), (A2-1)+(B6-4), (A2-1)+(B6-5), (A2-1)+(B6-6), (A2-1)+(B7-1), (A2-1)+(B7-2), (A2-1)+(B7-3), (A2-1)+(B7-4), (A2-1)+(B7-5), (A2-1)+(B7-6), (A2-1)+(B7-7)), (A2-1)+(B7-7), (A2-1)+(B7-8), (A2-1)+(B8-1), (A2-1)+(B8-2), (A2-1)+(B8-3), (A2-1)+(B8-4), (A2-1)+(B8-5), (A2-1)+(B8-6), (A2-1)+(B8-7), (A2-1)+(B9-1), (A2-1)+(B9-2), (A2-1)+(B10-1), (A2-1)+(B10-2), (A2-1)+(B10-3), (A2-1)+(B10-4), (A2-1)+(B10-5), (A2-1)+(B11-1), (A2-1)+(B11-2), (A2-1)+(B11-2),
(A2-2)+(B1-1), (A2-2)+(B1-2), (A2-2)+(B1-3), (A2-2)+(B1-4), (A2-2)+(B1-5), (A2-2)+(B1-6), (A2-2)+(B2-1), (A2-2)+(B2-2), (A2-2)+(B2-3), (A2-2)+(B2-4), (A2-2)+(B2-5), (A2-2)+(B2-6), (A2-2)+(B2-7), (A2-2)+(B2-8), (A2-2)+(B2-9), (A2-2)+(B2-10), (A2-2)+(B2-11), (A2-2)+(B2-12), (A2-2)+(B2-13), (A2-2)+(B2-14), (A2-2)+(B2-15), (A2-2)+(B2-16), (A2-2)+(B2-17), (A2-2)+(B2-18), (A2-2)+(B2-19), (A2-2)+(B2-20), (A2-2)+(B2-21), (A2-2)+(B2-22), (A2-2)+(B2-23), (A2-2)+(B2-24), (A2-2)+(B2-25), (A2-2)+(B2-26), (A2-2)+(B2-27), (A2-2)+(B2-28), (A2-2)+(B2-29), (A2-2)+(B3-1), (A2-2)+(B3-2), (A2-2)+(B4-1), (A2-2)+(B4-2), (A2-2)+(B4-3), (A2-2)+(B4-4), (A2-2)+(B4-5), (A2-2)+(B4-6), (A2-2)+(B4-7), (A2-2)+(B4-8), (A2-2)+(B4-9), (A2-2)+(B4-10), (A2-2)+(B4-11), (A2-2)+(B5-1), (A2-2)+(B5-2), (A2-2)+(B5-3), (A2-2)+(B5-4), (A2-2)+(B5-5), (A2-2)+(B6-1), (A2-2)+(B6-2), (A2-2)+(B6-3), (A2-2)+(B6-4), (A2-2)+(B6-5), (A2-2)+(B6-6), (A2-2)+(B7-1), (A2-2)+(B7-2), (A2-2)+(B7-3), (A2-2)+(B7-4), (A2-2)+(B7-5), (A2-2)+(B7-6), (A2-2)+(B7-7)), (A2-2)+(B7-7), (A2-2)+(B7-8), (A2-2)+(B8-1), (A2-2)+(B8-2), (A2-2)+(B8-3), (A2-2)+(B8-4), (A2-2)+(B8-5), (A2-2)+(B8-6), (A2-2)+(B8-7), (A2-2)+(B9-1), (A2-2)+(B9-2), (A2-2)+(B10-1), (A2-2)+(B10-2), (A2-2)+(B10-3), (A2-2)+(B10-4), (A2-2)+(B10-5), (A2-2)+(B11-1), (A2-2)+(B11-2), (A2-2)+(B11-2),
(A2-3)+(B1-1), (A2-3)+(B1-2), (A2-3)+(B1-3), (A2-3)+(B1-4), (A2-3)+(B1-5), (A2-3)+(B1-6), (A2-3)+(B2-1), (A2-3)+(B2-2), (A2-3)+(B2-3), (A2-3)+(B2-4), (A2-3)+(B2-5), (A2-3)+(B2-6), (A2-3)+(B2-7), (A2-3)+(B2-8), (A2-3)+(B2-9), (A2-3)+(B2-10), (A2-3)+(B2-11), (A2-3)+(B2-12), (A2-3)+(B2-13), (A2-3)+(B2-14), (A2-3)+(B2-15), (A2-3)+(B2-16), (A2-3)+(B2-17), (A2-3)+(B2-18), (A2-3)+(B2-19), (A2-3)+(B2-20), (A2-3)+(B2-21), (A2-3)+(B2-22), (A2-3)+(B2-23), (A2-3)+(B2-24), (A2-3)+(B2-25), (A2-3)+(B2-26), (A2-3)+(B2-27), (A2-3)+(B2-28), (A2-3)+(B2-29), (A2-3)+(B3-1), (A2-3)+(B3-2), (A2-3)+(B4-1), (A2-3)+(B4-2), (A2-3)+(B4-3), (A2-3)+(B4-4), (A2-3)+(B4-5), (A2-3)+(B4-6), (A2-3)+(B4-7), (A2-3)+(B4-8), (A2-3)+(B4-9), (A2-3)+(B4-10), (A2-3)+(B4-11), (A2-3)+(B5-1), (A2-3)+(B5-2), (A2-3)+(B5-3), (A2-3)+(B5-4), (A2-3)+(B5-5), (A2-3)+(B6-1), (A2-3)+(B6-2), (A2-3)+(B6-3), (A2-3)+(B6-4), (A2-3)+(B6-5), (A2-3)+(B6-6), (A2-3)+(B7-1), (A2-3)+(B7-2), (A2-3)+(B7-3), (A2-3)+(B7-4), (A2-3)+(B7-5), (A2-3)+(B7-6), (A2-3)+(B7-7)), (A2-3)+(B7-7), (A2-3)+(B7-8), (A2-3)+(B8-1), (A2-3)+(B8-2), (A2-3)+(B8-3), (A2-3)+(B8-4), (A2-3)+(B8-5), (A2-3)+(B8-6), (A2-3)+(B8-7), (A2-3)+(B9-1), (A2-3)+(B9-2), (A2-3)+(B10-1), (A2-3)+(B10-2), (A2-3)+(B10-3), (A2-3)+(B10-4), (A2-3)+(B10-5), (A2-3)+(B11-1), (A2-3)+(B11-2), (A2-3)+(B11-2),
(A2-4)+(B1-1), (A2-4)+(B1-2), (A2-4)+(B1-3), (A2-4)+(B1-4), (A2-4)+(B1-5), (A2-4)+(B1-6), (A2-4)+(B2-1), (A2-4)+(B2-2), (A2-4)+(B2-3), (A2-4)+(B2-4), (A2-4)+(B2-5), (A2-4)+(B2-6), (A2-4)+(B2-7), (A2-4)+(B2-8), (A2-4)+(B2-9), (A2-4)+(B2-10), (A2-4)+(B2-11), (A2-4)+(B2-12), (A2-4)+(B2-13), (A2-4)+(B2-14), (A2-4)+(B2-15), (A2-4)+(B2-16), (A2-4)+(B2-17), (A2-4)+(B2-18), (A2-4)+(B2-19), (A2-4)+(B2-20), (A2-4)+(B2-21), (A2-4)+(B2-22), (A2-4)+(B2-23), (A2-4)+(B2-24), (A2-4)+(B2-25), (A2-4)+(B2-26), (A2-4)+(B2-27), (A2-4)+(B2-28), (A2-4)+(B2-29), (A2-4)+(B3-1), (A2-4)+(B3-2), (A2-4)+(B4-1), (A2-4)+(B4-2), (A2-4)+(B4-3), (A2-4)+(B4-4), (A2-4)+(B4-5), (A2-4)+(B4-6), (A2-4)+(B4-7), (A2-4)+(B4-8), (A2-4)+(B4-9), (A2-4)+(B4-10), (A2-4)+(B4-11), (A2-4)+(B5-1), (A2-4)+(B5-2), (A2-4)+(B5-3), (A2-4)+(B5-4), (A2-4)+(B5-5), (A2-4)+(B6-1), (A2-4)+(B6-2), (A2-4)+(B6-3), (A2-4)+(B6-4), (A2-4)+(B6-5), (A2-4)+(B6-6), (A2-4)+(B7-1), (A2-4)+(B7-2), (A2-4)+(B7-3), (A2-4)+(B7-4), (A2-4)+(B7-5), (A2-4)+(B7-6), (A2-4)+(B7-7)), (A2-4)+(B7-7), (A2-4)+(B7-8), (A2-4)+(B8-1), (A2-4)+(B8-2), (A2-4)+(B8-3), (A2-4)+(B8-4), (A2-4)+(B8-5), (A2-4)+(B8-6), (A2-4)+(B8-7), (A2-4)+(B9-1), (A2-4)+(B9-2), (A2-4)+(B10-1), (A2-4)+(B10-2), (A2-4)+(B10-3), (A2-4)+(B10-4), (A2-4)+(B10-5), (A2-4)+(B11-1), (A2-4)+(B11-2), (A2-4)+(B11-2).

In den erfindungsgemäßen herbiziden Zusammensetzungen beträgt die Aufwandmenge der Herbizide der allgemeinen Formel (I) (Komponente A) üblicherweise 1 bis 500 g Aktivsusbstanz (a. i.) pro Hektar, bevorzugt 2 bis 300 g a. i./ha, insbesondere bevorzugt 3 bis 200 g a. i./ha. Die Aufwandmenge der Herbizide der Komponente B beträgt üblicherweise 1 bis 5000 g Aktivsusbstanz pro Hektar, bevorzugt 2 bis 3000 g a. i./ha, insbesondere bevorzugt 3 bis 2000 g a. i./ha. Die Aufwandmenge der Safener der Komponente C beträgt üblicherweise 1 bis 500 g Aktivsusbstanz pro Hektar, bevorzugt 2 bis 400 g a. i./ha, insbesondere bevorzugt 3 bis 300 g a. i./ha.

Bei Anwendung der erfindungsgemäßen herbiziden Zusammensetzungen wird im Vor- und Nachauflaufverfahren ein sehr breites Spektrum an Schadpflanzen bekämpft, z.B. annuellen und perennierenden mono- oder dikotylen Unkräutern sowie an unerwünschten Kulturpflanzen. Die erfindungsgemäßen herbiziden Zusammensetzungen eignen sich besonders zum Einsatz in Kulturen wie Getreide, Mais, Reis, Soja, Raps, Zuckerrüben, Baumwolle, Zuckerrohr sowie für den Einsatz in Dauerkulturen, Plantagen und auf Nichtkulturland. Ebenso eigenen sie sich sehr gut für den Einsatz in transgenen Kulturen von Mais, Getreide, Zuckerrüben , Reis, Baumwolle sowie Glycine max. (z.B. RR-Soja oder LL-Soja) und deren Kreuzungen), Phaseolus, Pisum, Vicia und Arachis, oder Gemüsekulturen aus verschiedenen botanischen Gruppen wie Kartoffel, Lauch, Kohl, Karotte, Tomate, Zwiebel, sowie Dauer- und Plantagenkulturen wie Kern- und Steinobst, Beerenobst, Wein, Hevea, Bananen, Zuckerrohr, Kaffee, Tee, Citrus, Nussplantagen, Rasen, Palmenkulturen und Forstkulturen. Für die Anwendung der erfindungsgemäßen Herbizid-Safener-Kombinationen (A)+(B) sind diese Kulturen ebenfalls bevorzugt, besonders bevorzugt ist der Einsatz in Getreide (z.B. Weizen, Gerste, Roggen, Hafer), Reis, Mais, Hirse, Zuckerrübe, Zuckerrohr, Sonnenblume, Raps und Baumwolle. Die Herbizid-Safener-Kombinationen (A)+(B) sind auch einsetzbar in toleranten und nicht toleranten Mutantenkulturen und toleranten und nicht toleranten transgenen Kulturen, vorzugsweise von Mais, Reis, Getreide, Raps, Baumwolle, Zuckerrüben und Soja, z.B. solche die gegen Imidazolinon-Herbizide, Atrazin, Glufosinate, Glyphosate, 2,4 D, Dicamba sowie Herbiziden aus der Gruppe der Inhibitoren der Hydroxyphenylpyruvat-Dioxygenase, wie sulcotrione, mesotrione, tembotrione, tefuryltrione, benzobicyclon, bicyclopyrone und ketospiradox resistent sind.

Herbizid wirksame Menge bedeutet im Sinne der Erfindung eine Menge an einem oder mehreren Herbiziden, die geeignet ist, den Pflanzenwuchs negativ zu beeinflussen. Antidotisch wirksame Menge bedeutet im Sinne der Erfindung eine Menge an einem oder mehreren Safenern, die geeignet ist, die phytotoxische Wirkung von Pflanzenschutzmittelwirkstoffen (z.B. von Herbiziden) an Kulturpflanzen zu reduzieren.

Die in den erfindungsgemäßen herbiziden Zusammensetzungen enthaltenen Safener (C) können je nach ihren Eigenschaften auch zur Vorbehandlung des Saatgutes der Kulturpflanze (z.B. zur Beizung des Saatguts) verwendet werden oder vor der Saat in die Saatfurchen eingebracht oder zusammen mit dem Herbizid vor oder nach dem Auflaufen der Pflanzen angewendet werden. Vorauflaufbehandlung schließt sowohl die Behandlung der Anbaufläche (einschließlich eventuell auf der Anbaufläche befindlichen Wassers, z.B. bei Reisapplikationen) vor der Aussaat als auch die Behandlung der angesäten, aber noch nicht bewachsenen Anbauflächen ein. Bevorzugt ist die gemeinsame Anwendung mit dem Herbizid. Hierzu können Tankmischungen oder Fertigformulierungen eingesetzt werden.

In einer bevorzugten Ausführungsform werden das Saatgut (z.B. Körner, Samen oder vegetative Vermehrungsorgane wie Knollen oder Sprossteile mit Knospen) oder Setzlinge mit den Safenern (C), gegebenenfalls in Kombination mit anderen agrochemischen Wirkstoffen, vorbehandelt. Zur Vorbehandlung des Saatguts können die Wirkstoffe z.B. durch Beizung an das Saatgut gebracht oder die Wirkstoffe und das Saatgut können in Wasser oder andere Lösungsmittel gegeben, und die Wirkstoffe z.B. durch Anlagerung oder Diffusion im Tauchverfahren oder durch Quellen oder Vorkeimen aufgenommen werden. Zur Vorbehandlung von Setzlingen können die jungen Pflanzen z.B. durch Spritzen, Tauchen oder Gießen mit den Safenern, gegebenenfalls in Kombination mit anderen agrochemischen Wirkstoffen, in Kontakt gebracht und anschließend verpflanzt und gegebenenfalls mit den Herbiziden (A) und (B) nachbehandelt werden.

Die Saatgut- oder Setzlingsbehandlung kann mit den Safenern (C) alleine oder gemeinsam mit anderen agrochemischen Wirkstoffen - wie Fungiziden, Insektiziden oder Mitteln zur Pflanzenstärkung, Düngung oder zur Beschleunigung der Quell- und Keimungsvorgänge - erfolgen. Dabei können die Safener nach der Vorbehandlungsanwendung anschließend nochmals vor, nach oder gemeinsam mit einem oder mehreren Herbiziden der Formel (I) (A) und Herbiziden (B) eventuell auch in Kombination mit anderen bekannten Herbiziden angewandt werden. Durch die Vorbehandlung des Saatguts oder der Setzlinge kann eine verbesserte Langzeitwirkung der Safener erzielt werden.

Gegenstand der vorliegenden Erfindung ist somit weiterhin ein Verfahren zur Bekämpfung von unerwünschten Pflanzen in Pflanzenkulturen, das dadurch gekennzeichnet ist, dass die Komponenten (A), (B) und gegebenenfalls (C) der erfindungsgemäßen herbiziden Zusammensetzungen auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden, z.B. gemeinsam oder getrennt. Dabei können einer oder mehrere Safener (C), vor, nach oder gleichzeitig mit dem oder den Herbizid(en) der allgemeinen Formel (I) (A) und den Herbiziden (B) auf die Pflanzen, das Saatgut oder die Fläche, auf der die Pflanzen wachsen (z.B. die Anbaufläche), appliziert werden. In einer bevorzugten Ausführungsform werden die Safener (C) zur Saatgutbehandlung eingesetzt.

Unter unerwünschten Pflanzen sind alle Pflanzen zu verstehen, die an Orten wachsen, wo sie unerwünscht sind. Dies können z.B. Schadpflanzen (z.B. mono- oder dikotyle Unkräuter oder unerwünschte Kulturpflanzen) sein, z.B. auch solche, die gegen bestimmte herbizide Wirkstoffe wie Glyphosate, Atrazin, Glufosinate oder Imidazolinon-Herbizide resistent sind.

Monokotyle Unkräuter entstammen z.B. den Gattungen Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera, Phalaris. Dikotyle Unkräuter entstammen z.B. den Gattungen Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum, Euphorbia, Kochia, Biden, Stellaria.

Gegenstand der Erfindung ist auch die Verwendung der erfindungsgemäßen herbiziden Zusammensetzungen zur Bekämpfung von unerwünschtem Pflanzenwuchs, vorzugsweise in Pflanzenkulturen.

Die erfindungsgemäßen herbiziden Zusammensetzungen können nach bekannten Verfahren z.B. als Mischformulierungen der Einzelkomponenten, gegebenenfalls mit weiteren Wirkstoffen, Zusatzstoffen und/oder üblichen Formulierungshilfsmitteln hergestellt werden, die dann in üblicher Weise mit Wasser verdünnt zur Anwendung gebracht werden, oder als sogenannte Tankmischungen durch gemeinsame Verdünnung der getrennt formulierten oder partiell getrennt formulierten Einzelkomponenten mit Wasser hergestellt werden. Ebenfalls möglich ist die zeitlich versetzte Anwendung (Splitapplikation) der getrennt formulierten oder partiell getrennt formulierten Einzelkomponenten. Möglich ist auch die Anwendung der Einzelkomponenten oder der herbiziden Zusammensetzungen in mehreren Portionen (Sequenzanwendung), z. B. nach Anwendungen im Vorauflauf, gefolgt von Nachauflauf-Applikationen oder nach frühen Nachauflaufanwendungen, gefolgt von Applikationen im mittleren oder späten Nachauflauf. Bevorzugt ist dabei die gemeinsame oder die zeitnahe Anwendung der Wirkstoffe der jeweiligen Kombination.

Die erfindungsgemäßen herbiziden Zusammensetzungen können auch zur Bekämpfung von Schadpflanzen in Kulturen von bekannten oder noch zu entwickelnden gentechnisch veränderten Pflanzen eingesetzt werden.

Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen herbiziden Zusammensetzungen in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624).

Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Zusammensetzungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Zusammensetzungen in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Zusammensetzungen zur Bekämpfung von Schadpflanzen in transgenen Kulturpflanzen.

Bevorzugt ist die Anwendung der erfindungsgemäßen Zusammensetzungen in wirtschaftlich bedeutenden transgenen Kulturen von Nutz- und Zierpflanzen, z. B. von Getreide (z.B. Weizen, Gerste, Roggen, Hafer), Hirse, Reis, Maniok und Mais oder auch Kulturen von Zuckerrübe, Baumwolle, Soja, Raps, Kartoffel, Tomate, Erbse und anderen Gemüsekulturen.

Gegenstand der Erfindung ist deshalb auch die Verwendung der erfindungsgemäßen Zusammensetzungen zur Bekämpfung von Schadpflanzen in transgenen Kulturpflanzen oder Kulturpflanzen, die Toleranz durch Selektionszüchtung aufweisen.

Die Herbizide (A), (B) und die Safener (C) können gemeinsam oder getrennt in übliche Formulierungen z.B. zur Sprüh-, Gieß-, Spritz- und Saatgutbeizanwendung übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen. Die Formulierungen können die üblichen Hilfs- und Zusatzstoffe enthalten.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel könne z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 Gew.-%.

Die Herbizide (A), (B) und die Safener (C) können als solche oder in ihren Formulierungen auch in Mischung mit anderen agrochemischen Wirkstoffen zur Bekämpfung von unerwünschtem Pflanzenwuchs, z.B. zur Unkrautbekämpfung oder zur Bekämpfung von unerwünschten Kulturpflanzen Verwendung finden, wobei z.B. Fertigformulierungen oder Tankmischungen möglich sind.

Auch Mischungen mit anderen bekannten Wirkstoffen wie Fungiziden, Insektiziden, Akariziden, Nematiziden, Schutzstoffen gegen Vogelfraß, Pflanzennährstoffen und Bodenstrukturverbesserungsmitteln sind möglich, ebenso mit im Pflanzenschutz üblichen Zusatzstoffen und Formulierungshilfsmitteln.

Die Herbizide (A), (B) und die Safener (C) können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht üblicherweise, z.B. durch Gießen, Spritzen, Sprühen, Streuen.

Die Wirkstoffe können auf die Pflanzen, Pflanzenteile, das Saatgut oder die Anbaufläche (Ackerboden) ausgebracht werden, vorzugsweise auf das Saatgut oder die grünen Pflanzen und Pflanzenteile und gegebenenfalls zusätzlich auf den Ackerboden. Eine Möglichkeit der Anwendung ist die gemeinsame Ausbringung der Wirkstoffe in Form von Tankmischungen, wobei die optimal formulierten konzentrierten Formulierungen der Einzelwirkstoffe gemeinsam im Tank mit Wasser gemischt und die erhaltene Spritzbrühe ausgebracht wird.

Eine gemeinsame Formulierung der erfindungsgemäßen Kombination an Wirkstoffen (A), (B) und (C) hat den Vorteil der leichteren Anwendbarkeit, weil die Mengen der Komponenten bereits im optimalen Verhältnis zueinander eingestellt werden können. Außerdem können die Hilfsmittel in der Formulierung aufeinander optimal abgestimmt werden.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Biologische Beispiele:
Alle Versuche, deren Ergebnisse dargestellt werden, wurden im Gewächshaus nach der unten genannten Methodik durchgeführt. Die Wirkung der erfindungsgemäßen herbiziden Zusammensetzungen wurde an folgenden Schadpflanzen 14 bzw. 21 Tage nach der Applikation untersucht: Echinochloa crus-galli (ECHCG), Eleusine indica (ELEIN), Setaria viridis (SETVI), Amaranthus retroflexus (AMARE), Chenopodium album (CHEAL), Kochia scoparia (KCHSC), Solanum nigrum (SOLNI), Avena fatua (AVEFA), Bromus sterilis (BROST), Lolium multiflorum (LOLMU), Phalaris minor (PHAMI), Poa annua (POAAN), Galium aparine (GALAP), Matricaria inodora (MATIN) und Polygonum convolvulus (POLCO).

Die Samen der Schadpflanzen wurden in Töpfen (8 cm Durchmesser) mit einem sandigen Lehmboden ausgesät, unter optimalen Bedingungen zur Keimung gebracht. Die Anwendung der erfindungsgemäßen herbiziden Zusammensetzungen beziehungsweise der alleinigen Wirkstoffe erfolgte im Nachauflauf auf die bewachsenen Töpfe mit einem Spritzvolumen von 300 l/ha. Der Versuch wurde im Gewächshaus unter optimalen Wachstumsbedingungen durchgeführt. Die herbiziden Effekte wurden im Vergleich von nicht behandelten und behandelten Pflanzen visuell bewertet. Die prozentualen Werte bedeuten: 0 % = keine Effekte, 100 % = komplettes Absterben der Pflanzen). Die Prozentwerte werden genutzt, um Wechselwirkungen zwischen Einzelbehandlungen und Behandlung in Kombination nach S.R. Colby, Weeds 15, pages 20 to 22 (1967).zu errechnen.

Die Abkürzungen bedeuten:

| | |
|---|---|
| a.i. = | active ingredient |
| E^{C} = | Erwartungswert nach Colby (E^{C} =A + B - A x B/100) |
| Diff: = | Differenz (%) von gemessenem Wert zu Erwartungswert (%) (gemessener Wert minus Erwartungswert) |

Bewertung :

| | |
|---|---|
| - gemessener Wert E ist grösser als E^{C}: -> | Synergismus (+ Diff.) |
| - gemessener Wert E ist gleich E^{C}: -> | Additive Wirkung |
| - gemessener Wert E ist kleiner als E^{C}: -> | Antagonismus(- Diff.) |

Die Ergebnisse sind in den nachfolgenden Tabellen dargestellt.

**Tabelle 1: Wirkung gegen ECHCG, 14 Tage nach Applikation**

| Wirkstoff | Dosierung [g a.i./ha] | Wirkung [%] |
|---|---|---|
| A1-3 | 4 | 40 |
| tembotrione | 0,5 | 10 |
| A1-3 + tembotrione | 4 + 0,5 | 75 (Ec = 46, Diff. = +29) |

**Tabelle 2: Wirkung gegen ECHCG, 14 Tage nach Applikation**

| Wirkstoff | Dosierung [g a.i./ha] | Wirkung [%] |
|---|---|---|
| A1-3 | 1 | 0 |
| tembotrione | 0,5 | 10 |
| A1-3 + tembotrione | 1 + 0,5 | 30 (Ec = 10, Diff. = +20) |

**Tabelle 3: Wirkung gegen ELEIN, 14 Tage nach Applikation**

| Wirkstoff | Dosierung [g a.i./ha] | Wirkung [%] |
|---|---|---|
| A1-3 | 4 | 50 |
| tembotrione | 2 | 40 |
| A1-3 + tembotrione | 4+2 | 75 (Ec = 70, Diff. = +5) |

**Tabelle 4: Wirkung gegen ELEIN, 14 Tage nach Applikation**

| Wirkstoff | Dosierung [g a.i./ha] | Wirkung [%] |
|---|---|---|
| A1-3 | 4 | 50 |
| tembotrione | 0,5 | 30 |
| A1-3 + tembotrione | 4 + 0,5 | 70 (Ec = 65, Diff. = +5) |

**Tabelle 5: Wirkung gegen KCHSC, 14 Tage nach Applikation**

| Wirkstoff | Dosierung [g a.i./ha] | Wirkung [%] |
|---|---|---|
| A1-3 | 4 | 40 |
| tembotrione | 2 | 20 |
| A1-3 + tembotrione | 4+2 | 70 (Ec = 52, Diff. = +18) |

**Tabelle 6: Wirkung gegen DIGSA, 14 Tage nach Applikation**

| Wirkstoff | Dosierung [g a.i./ha] | Wirkung [%] |
|---|---|---|
| A1-13 | 0,4 | 0 |
| foramsulfuron | 4 | 40 |
| A1-13 + foramsulfuron | 0,4 + 4 | 60 (Ec = 40, Diff. = +20) |

**Tabelle 7: Wirkung gegen ECHCG, 14 Tage nach Applikation**

| Wirkstoff | Dosierung [g a.i./ha] | Wirkung [%] |
|---|---|---|
| A1-13 | 0,4 | 0 |
| foramsulfuron | 1 | 60 |
| A1-13 + foramsulfuron | 0,4 + 1 | 75 (Ec = 60, Diff. = +15) |

**Tabelle 8: Wirkung gegen ECHCG, 14 Tage nach Applikation**

| Wirkstoff | Dosierung [g a.i./ha] | Wirkung [%] |
|---|---|---|
| A1-13 | 0,1 | 0 |
| foramsulfuron | 1 | 60 |
| A1-13 + foramsulfuron | 0,1 + 1 | 75 (Ec = 60, Diff. = +15) |

**Tabelle 9: Wirkung gegen SETVI. 14 Tage nach Applikation**

| Wirkstoff | Dosierung [g a.i./ha] | Wirkung [%] |
|---|---|---|
| A1-13 | 0,4 | 10 |
| foramsulfuron | 1 | 40 |
| A1-13 + foramsulfuron | 0,4 + 1 | 60 (Ec = 46, Diff. = +14) |

**Tabelle 10: Wirkung gegen SETVI, 14 Tage nach Applikation**

| Wirkstoff | Dosierung [g a.i./ha] | Wirkung [%] |
|---|---|---|
| A1-13 | 0,1 | 0 |
| foramsulfuron | 1 | 40 |
| A1-13 + foramsulfuron | 0,1 + 1 | 50 (Ec = 40, Diff. = +10) |

**Tabelle 11: Wirkung gegen CHEAL, 14 Tage nach Applikation**

| Wirkstoff | Dosierung [g a.i./ha] | Wirkung [%] |
|---|---|---|
| A1-13 | 0,4 | 10 |
| foramsulfuron | 1 | 50 |
| A1-13 + foramsulfuron | 0,4 + 1 | 65 (Ec = 55, Diff. = +10) |

**Tabelle 12: Wirkung gegen KCHSC, 14 Tage nach Applikation**

| Wirkstoff | Dosierung [g a.i./ha] | Wirkung [%] |
|---|---|---|
| A1-13 | 0,4 | 0 |
| foramsulfuron | 4 | 90 |
| A1-13 + foramsulfuron | 0,4 + 4 | 97 (Ec = 90, Diff. = +7) |

**Tabelle 13: Wirkung gegen KCHSC, 14 Tage nach Applikation**

| Wirkstoff | Dosierung [g a.i./ha] | Wirkung [%] |
|---|---|---|
| A1-13 | 0,1 | 0 |
| foramsulfuron | 1 | 80 |
| A1-13 + foramsulfuron | 0,1 + 1 | 85 (Ec = 80, Diff. = +5) |

**Tabelle 14: Wirkung gegen DIGSA, 14 Tage nach Applikation**

| Wirkstoff | Dosierung [g a.i./ha] | Wirkung [%] |
|---|---|---|
| A1-14 | 0,4 | 0 |
| bromoxynil | 400 | 0 |
| A1-14 + bromoxynil | 0,4 + 400 | 30 (Ec = 0, Diff. = +30) |

**Tabelle 15: Wirkung gegen DIGSA, 14 Tage nach Applikation**

| Wirkstoff | Dosierung [g a.i./ha] | Wirkung [%] |
|---|---|---|
| A1-14 | 0,4 | 0 |
| bromoxynil | 100 | 0 |
| A1-14 + bromoxynil | 0,4 + 100 | 30 (Ec = 0, Diff. = +30) |

**Tabelle 16: Wirkung gegen ECHCG, 14 Tage nach Applikation**

| Wirkstoff | Dosierung [g a.i./ha] | Wirkung [%] |
|---|---|---|
| A1-14 | 0,4 | 0 |
| bromoxynil | 400 | 0 |
| A1-14 + bromoxynil | 0,4 + 400 | 50 (Ec = 0, Diff. = +50) |

**Tabelle 17: Wirkung gegen ECHCG, 14 Tage nach Applikation**

| Wirkstoff | Dosierung [g a.i./ha] | Wirkung [%] |
|---|---|---|
| A1-14 | 0,4 | 0 |
| bromoxynil | 100 | 0 |
| A1-14 + bromoxynil | 0,4 + 100 | 30 (Ec = 0, Diff. = +30) |

**Tabelle 18: Wirkung gegen ELEIN, 14 Tage nach Applikation**

| Wirkstoff | Dosierung [g a.i./ha] | Wirkung [%] |
|---|---|---|
| A1-14 | 0,4 | 20 |
| bromoxynil | 400 | 0 |
| A1-14 + bromoxynil | 0,4 + 400 | 30 (Ec = 20, Diff. = +10) |

**Tabelle 19: Wirkung gegen ELEIN, 14 Tage nach Applikation**

| Wirkstoff | Dosierung [g a.i./ha] | Wirkung [%] |
|---|---|---|
| A1-14 | 0,4 | 20 |
| bromoxynil | 100 | 0 |
| A1-14 + bromoxynil | 0,4 + 100 | 30 (Ec = 20, Diff. = +10) |

**Tabelle 20: Wirkung gegen ELEIN, 14 Tage nach Applikation**

| Wirkstoff | Dosierung [g a.i./ha] | Wirkung [%] |
|---|---|---|
| A1-14 | 0,1 | 0 |
| bromoxynil | 400 | 0 |
| A1-14 + bromoxynil | 0,1 +400 | 20 (Ec = 0, Diff. = +20) |

**Tabelle 21: Wirkung gegen ELEIN, 14 Tage nach Applikation**

| Wirkstoff | Dosierung [g a.i./ha] | Wirkung [%] |
|---|---|---|
| A1-14 | 0,1 | 0 |
| bromoxynil | 100 | 0 |
| A1-14 + bromoxynil | 0,1 + 100 | 20 (Ec = 0, Diff. = +20) |

**Tabelle 22: Wirkung gegen SETVI, 14 Tage nach Applikation**

| Wirkstoff | Dosierung [g a.i./ha] | Wirkung [%] |
|---|---|---|
| A1-14 | 0,1 | 0 |
| bromoxynil | 400 | 0 |
| A1-14 + bromoxynil | 0,1 +400 | 20 (Ec = 0, Diff. = +20) |

**Tabelle 23: Wirkung gegen SETVI, 14 Tage nach Applikation**

| Wirkstoff | Dosierung [g a.i./ha] | Wirkung [%] |
|---|---|---|
| A1-14 | 0,1 | 0 |
| bromoxynil | 100 | 0 |
| A1-14 + bromoxynil | 0,1 + 100 | 20 (Ec = 0, Diff. = +20) |

**Tabelle 24: Wirkung gegen AMARE, 14 Tage nach Applikation**

| Wirkstoff | Dosierung [g a.i./ha] | Wirkung [%] |
|---|---|---|
| A1-14 | 0,1 | 0 |
| bromoxynil | 100 | 40 |
| A1-14 + bromoxynil | 0,1 + 100 | 50 (Ec = 40, Diff. = +10) |

**Tabelle 25: Wirkung gegen CHEAL, 14 Tage nach Applikation**

| Wirkstoff | Dosierung [g a.i./ha] | Wirkung [%] |
|---|---|---|
| A1-14 | 0,1 | 0 |
| bromoxynil | 400 | 85 |
| A1-14 + bromoxynil | 0,1 +400 | 100 (Ec = 85, Diff. = +15) |

**Tabelle 26: Wirkung gegen DIGSA, 14 Tage nach Applikation**

| Wirkstoff | Dosierung [g a.i./ha] | Wirkung [%] |
|---|---|---|
| A1-36 | 0,5 | 10 |
| isoxaflutole | 10 | 10 |
| A1-36 + isoxaflutole | 0,5 + 10 | 30 (Ec = 19, Diff. = +11) |

**Tabelle 27: Wirkung gegen ECHCG, 14 Tage nach Applikation**

| Wirkstoff | Dosierung [g a.i./ha] | Wirkung [%] |
|---|---|---|
| A1-36 | 2 | 40 |
| isoxaflutole | 10 | 60 |
| A1-36 + isoxaflutole | 2 + 10 | 95 (Ec = 76, Diff. = +19) |

**Tabelle 28: Wirkung gegen ECHCG, 14 Tage nach Applikation**

| Wirkstoff | Dosierung [g a.i./ha] | Wirkung [%] |
|---|---|---|
| A1-36 | 2 | 40 |
| isoxaflutole | 2,5 | 10 |
| A1-36 + isoxaflutole | 2 + 2,5 | 70 (Ec = 46, Diff. = +24) |

**Tabelle 29: Wirkung gegen ECHCG, 14 Tage nach Applikation**

| Wirkstoff | Dosierung [g a.i./ha] | Wirkung [%] |
|---|---|---|
| A1-36 | 0,5 | 10 |
| isoxaflutole | 10 | 60 |
| A1-36 + isoxaflutole | 0,5 + 10 | 70 (Ec = 64, Diff. = +6) |

**Tabelle 30: Wirkung gegen ELEIN, 14 Tage nach Applikation**

| Wirkstoff | Dosierung [g a.i./ha] | Wirkung [%] |
|---|---|---|
| A1-36 | 2 | 50 |
| isoxaflutole | 10 | 60 |
| A1-36 + isoxaflutole | 2 + 10 | 93 (Ec = 80, Diff. = +13) |

**Tabelle 31: Wirkung gegen ELEIN, 14 Tage nach Applikation**

| Wirkstoff | Dosierung [g a.i./ha] | Wirkung [%] |
|---|---|---|
| A1-36 | 2 | 50 |
| isoxaflutole | 2,5 | 20 |
| A1-36 + isoxaflutole | 2 + 2,5 | 85 (Ec = 60, Diff. = +25) |

**Tabelle 32: Wirkung aeaen ECHCG, 14 Tage nach Applikation**

| Wirkstoff | Dosierung [g a.i./ha] | Wirkung [%] |
|---|---|---|
| A2-1 | 4 | 50 |
| halauxifen | 2 | 10 |
| A2-1 + halauxifen | 4+2 | 70 (Ec = 55, Diff. = +15) |

**Tabelle 33: Wirkung gegen ECHCG, 14 Tage nach Applikation**

| Wirkstoff | Dosierung [g a.i./ha] | Wirkung [%] |
|---|---|---|
| A2-1 | 4 | 50 |
| halauxifen | 0,5 | 0 |
| A2-1 + halauxifen | 4 + 0,5 | 60 (Ec = 50, Diff. = +10) |

**Tabelle 34: Wirkung gegen DIGSA, 14 Tage nach Applikation**

| Wirkstoff | Dosierung [g a.i./ha] | Wirkung [%] |
|---|---|---|
| A1-55 | 0,5 | 20 |
| dicamba | 40 | 0 |
| A1-55 + dicamba | 0,5 + 40 | 30 (Ec = 20, Diff. = +10) |

**Tabelle 35: Wirkung gegen ELEIN, 14 Tage nach Applikation**

| Wirkstoff | Dosierung [g a.i./ha] | Wirkung [%] |
|---|---|---|
| A1-55 | 2 | 40 |
| dicamba | 40 | 10 |
| A1-55 + dicamba | 2 + 40 | 65 (Ec = 46, Diff. = +19) |

**Tabelle 36: Wirkung gegen ELEIN, 14 Tage nach Applikation**

| Wirkstoff | Dosierung [g a.i./ha] | Wirkung [%] |
|---|---|---|
| A1-55 | 2 | 40 |
| dicamba | 10 | 10 |
| A1-55 + dicamba | 2 + 10 | 65 (Ec = 46, Diff. = +19) |

**Tabelle 37: Wirkung gegen SETVI, 14 Tage nach Applikation**

| Wirkstoff | Dosierung [g a.i./ha] | Wirkung [%] |
|---|---|---|
| A1-55 | 2 | 40 |
| dicamba | 40 | 0 |
| A1-55 + dicamba | 2 + 40 | 65 (Ec = 40, Diff. = +25) |

**Tabelle 38: Wirkung gegen SETVI, 14 Tage nach Applikation**

| Wirkstoff | Dosierung [g a.i./ha] | Wirkung [%] |
|---|---|---|
| A1-55 | 2 | 40 |
| dicamba | 10 | 0 |
| A1-55 + dicamba | 2 + 10 | 65 (Ec = 40, Diff. = +25) |

**Tabelle 39: Wirkung gegen AMARE, 14 Tage nach Applikation**

| Wirkstoff | Dosierung [g a.i./ha] | Wirkung [%] |
|---|---|---|
| A1-55 | 0,5 | 20 |
| dicamba | 40 | 65 |
| A1-55 + dicamba | 0,5 + 40 | 80 (Ec = 72, Diff. = +8) |

**Tabelle 40: Wirkung gegen CHEAL, 14 Tage nach Applikation**

| Wirkstoff | Dosierung [g a.i./ha] | Wirkung [%] |
|---|---|---|
| A1-55 | 2 | 70 |
| dicamba | 10 | 50 |
| A1-55 + dicamba | 2 + 10 | 90 (Ec = 85, Diff. = +5) |

**Tabelle 41: Wirkung gegen KCHSC, 14 Tage nach Applikation**

| Wirkstoff | Dosierung [g a.i./ha] | Wirkung [%] |
|---|---|---|
| A1-57 | 1 | 40 |
| glyphosate | 50 | 30 |
| A1-57 + glyphosate | 1 + 50 | 65 (Ec = 58, Diff. = +7) |

**Tabelle 42: Wirkung gegen DIGSA, 14 Tage nach Applikation**

| Wirkstoff | Dosierung [g a.i./ha] | Wirkung [%] |
|---|---|---|
| A1-7 | 2 | 20 |
| fenoxaprop-P-ethyl | 5 | 30 |
| A1-7 + fenoxaprop-P-ethyl | 2+5 | 70 (Ec = 44, Diff. = +26) |

**Tabelle 43: Wirkung gegen AMARE, 14 Tage nach Applikation**

| Wirkstoff | Dosierung [g a.i./ha] | Wirkung [%] |
|---|---|---|
| A1-7 | 0,5 | 30 |
| fenoxaprop-P-ethyl | 20 | 0 |
| A1-7 + fenoxaprop-P-ethyl | 0,5 + 20 | 40 (Ec = 30, Diff. = +10) |

**Tabelle 44: Wirkung gegen AMARE, 14 Tage nach Applikation**

| Wirkstoff | Dosierung [g a.i./ha] | Wirkung [%] |
|---|---|---|
| A1-7 | 0,5 | 30 |
| fenoxaprop-P-ethyl | 5 | 0 |
| A1-7 + fenoxaprop-P-ethyl | 0,5 + 5 | 40 (Ec = 30, Diff. = +10) |

**Tabelle 45: Wirkung gegen CHEAL, 14 Tage nach Applikation**

| Wirkstoff | Dosierung [g a.i./ha] | Wirkung [%] |
|---|---|---|
| A1-7 | 2 | 50 |
| fenoxaprop-P-ethyl | 20 | 0 |
| A1-7 + fenoxaprop-P-ethyl | 2 + 20 | 70 (Ec = 50, Diff. = +20) |

**Tabelle 46: Wirkung gegen CHEAL, 14 Tage nach Applikation**

| Wirkstoff | Dosierung [g a.i./ha] | Wirkung [%] |
|---|---|---|
| A1-7 | 2 | 50 |
| fenoxaprop-P-ethyl | 5 | 0 |
| A1-7 + fenoxaprop-P-ethyl | 2+5 | 65 (Ec = 50, Diff. = +15) |

**Tabelle 47: Wirkung gegen CHEAL, 14 Tage nach Applikation**

| Wirkstoff | Dosierung [g a.i./ha] | Wirkung [%] |
|---|---|---|
| A1-7 | 0,5 | 0 |
| fenoxaprop-P-ethyl | 20 | 0 |
| A1-7 + fenoxaprop-P-ethyl | 0,5 + 20 | 20 (Ec = 0, Diff. = +20) |

**Tabelle 48: Wirkung gegen CHEAL, 14 Tage nach Applikation**

| Wirkstoff | Dosierung [g a.i./ha] | Wirkung [%] |
|---|---|---|
| A1-7 | 0,5 | 0 |
| fenoxaprop-P-ethyl | 5 | 0 |
| A1-7 + fenoxaprop-P-ethyl | 0,5 + 5 | 20 (Ec = 0, Diff. = +20) |

**Tabelle 49: Wirkung gegen KCHSC, 14 Tage nach Applikation**

| Wirkstoff | Dosierung [g a.i./ha] | Wirkung [%] |
|---|---|---|
| A1-7 | 0,5 | 0 |
| fenoxaprop-P-ethyl | 20 | 0 |
| A1-7 + fenoxaprop-P-ethyl | 0,5 + 20 | 30 (Ec = 0, Diff. = +30) |

**Tabelle 50: Wirkung gegen KCHSC, 14 Tage nach Applikation**

| Wirkstoff | Dosierung [g a.i./ha] | Wirkung [%] |
|---|---|---|
| A1-7 | 0,5 | 0 |
| fenoxaprop-P-ethyl | 5 | 0 |
| A1-7 + fenoxaprop-P-ethyl | 0,5 + 5 | 20 (Ec = 0, Diff. = +20) |

**Tabelle 51: Wirkung gegen SOLNI, 14 Tage nach Applikation**

| Wirkstoff | Dosierung [g a.i./ha] | Wirkung [%] |
|---|---|---|
| A1-7 | 2 | 20 |
| fenoxaprop-P-ethyl | 20 | 0 |
| A1-7 + fenoxaprop-P-ethyl | 2 + 20 | 40 (Ec = 20, Diff. = +20) |

**Tabelle 52: Wirkung gegen SOLNI, 14 Tage nach Applikation**

| Wirkstoff | Dosierung [g a.i./ha] | Wirkung [%] |
|---|---|---|
| A1-7 | 2 | 20 |
| fenoxaprop-P-ethyl | 5 | 0 |
| A1-7 + fenoxaprop-P-ethyl | 2+5 | 40 (Ec = 20, Diff. = +20) |

**Tabelle 53: Wirkung gegen SOLNI, 14 Tage nach Applikation**

| Wirkstoff | Dosierung [g a.i./ha] | Wirkung [%] |
|---|---|---|
| A1-7 | 0,5 | 10 |
| fenoxaprop-P-ethyl | 20 | 0 |
| A1-7 + fenoxaprop-P-ethyl | 0,5 + 20 | 20 (Ec = 10, Diff. = +10) |

**Tabelle 54: Wirkung gegen SOLNI, 14 Tage nach Applikation**

| Wirkstoff | Dosierung [g a.i./ha] | Wirkung [%] |
|---|---|---|
| A1-7 | 0,5 | 10 |
| fenoxaprop-P-ethyl | 5 | 0 |
| A1-7 + fenoxaprop-P-ethyl | 0,5 + 5 | 20 (Ec = 10, Diff. = +10) |

**Tabelle 55: Wirkung gegen DIGSA, 14 Tage nach Applikation**

| Wirkstoff | Dosierung [g a.i./ha] | Wirkung [%] |
|---|---|---|
| A1-60 | 4 | 93 |
| lenacil | 1200 | 0 |
| A1-60 + lenacil | 4 + 1200 | 99 (Ec = 93, Diff. = +6) |

**Tabelle 56: Wirkung gegen DIGSA, 14 Tage nach Applikation**

| Wirkstoff | Dosierung [g a.i./ha] | Wirkung [%] |
|---|---|---|
| A1-60 | 4 | 93 |
| lenacil | 300 | 0 |
| A1-60 + lenacil | 4 + 300 | 99 (Ec = 93, Diff. = +6) |

**Tabelle 57: Wirkung gegen ECHCG, 14 Tage nach Applikation**

| Wirkstoff | Dosierung [g a.i./ha] | Wirkung [%] |
|---|---|---|
| A1-60 | 4 | 90 |
| lenacil | 1200 | 0 |
| A1-60 + lenacil | 4 + 1200 | 99 (Ec = 90, Diff. = +9) |

**Tabelle 58: Wirkung gegen ECHCG, 14 Tage nach Applikation**

| Wirkstoff | Dosierung [g a.i./ha] | Wirkung [%] |
|---|---|---|
| A1-60 | 4 | 90 |
| lenacil | 300 | 0 |
| A1-60 + lenacil | 4 + 300 | 99 (Ec = 90, Diff. = +9) |

**Tabelle 59: Wirkung aeaen ECHCG, 14 Tage nach Applikation**

| Wirkstoff | Dosierung [g a.i./ha] | Wirkung [%] |
|---|---|---|
| A1-60 | 1 | 65 |
| lenacil | 1200 | 0 |
| A1-60 + lenacil | 1 + 1200 | 75 (Ec = 65, Diff. = +10) |

**Tabelle 60: Wirkung gegen ECHCG, 14 Tage nach Applikation**

| Wirkstoff | Dosierung [g a.i./ha] | Wirkung [%] |
|---|---|---|
| A1-60 | 1 | 65 |
| lenacil | 300 | 0 |
| A1-60 + lenacil | 1 + 300 | 75 (Ec = 65, Diff. = +10) |

**Tabelle 61: Wirkung gegen ELEIN, 14 Tage nach Applikation**

| Wirkstoff | Dosierung [g a.i./ha] | Wirkung [%] |
|---|---|---|
| A1-60 | 4 | 90 |
| lenacil | 1200 | 0 |
| A1-60 + lenacil | 4 + 1200 | 99 (Ec = 90, Diff. = +9) |

**Tabelle 62: Wirkung gegen ELEIN, 14 Tage nach Applikation**

| Wirkstoff | Dosierung [g a.i./ha] | Wirkung [%] |
|---|---|---|
| A1-60 | 4 | 90 |
| lenacil | 300 | 0 |
| A1-60 + lenacil | 4 + 300 | 98 (Ec = 90, Diff. = +8) |

**Tabelle 63: Wirkung gegen ELEIN, 14 Tage nach Applikation**

| Wirkstoff | Dosierung [g a.i./ha] | Wirkung [%] |
|---|---|---|
| A1-60 | 1 | 70 |
| lenacil | 1200 | 0 |
| A1-60 + lenacil | 1 + 1200 | 75 (Ec = 70, Diff. = +5) |

**Tabelle 64: Wirkung gegen ELEIN, 14 Tage nach Applikation**

| Wirkstoff | Dosierung [g a.i./ha] | Wirkung [%] |
|---|---|---|
| A1-60 | 1 | 70 |
| lenacil | 300 | 0 |
| A1-60 + lenacil | 1 + 300 | 75 (Ec = 70, Diff. = +5) |

**Tabelle 65: Wirkung gegen SETVI, 14 Tage nach Applikation**

| Wirkstoff | Dosierung [g a.i./ha] | Wirkung [%] |
|---|---|---|
| A1-60 | 4 | 85 |
| lenacil | 1200 | 0 |
| A1-60 + lenacil | 4 + 1200 | 95 (Ec = 85, Diff. = +10) |

**Tabelle 66: Wirkung gegen SETVI, 14 Tage nach Applikation**

| Wirkstoff | Dosierung [g a.i./ha] | Wirkung [%] |
|---|---|---|
| A1-60 | 4 | 85 |
| lenacil | 300 | 0 |
| A1-60 + lenacil | 4 + 300 | 90 (Ec = 85, Diff. = +5) |

**Tabelle 67: Wirkung gegen SETVI, 14 Tage nach Applikation**

| Wirkstoff | Dosierung [g a.i./ha] | Wirkung [%] |
|---|---|---|
| A1-60 | 1 | 70 |
| lenacil | 1200 | 0 |
| A1-60 + lenacil | 1 + 1200 | 75 (Ec = 70, Diff. = +5) |

**Tabelle 68: Wirkung gegen AMARE, 14 Tage nach Applikation**

| Wirkstoff | Dosierung [g a.i./ha] | Wirkung [%] |
|---|---|---|
| A1-60 | 4 | 70 |
| lenacil | 1200 | 0 |
| A1-60 + lenacil | 4 + 1200 | 85 (Ec = 70, Diff. = +15) |

**Tabelle 69: Wirkung gegen SOLNI, 14 Tage nach Applikation**

| Wirkstoff | Dosierung [g a.i./ha] | Wirkung [%] |
|---|---|---|
| A1-60 | 4 | 65 |
| lenacil | 1200 | 0 |
| A1-60 + lenacil | 4 + 1200 | 100 (Ec = 65, Diff. = +35) |

**Tabelle 70: Wirkung gegen SOLNI, 14 Tage nach Applikation**

| Wirkstoff | Dosierung [g a.i./ha] | Wirkung [%] |
|---|---|---|
| A1-60 | 4 | 65 |
| lenacil | 300 | 0 |
| A1-60 + lenacil | 4 + 300 | 95 (Ec = 65, Diff. = +30) |

**Tabelle 71: Wirkung gegen SOLNI, 14 Tage nach Applikation**

| Wirkstoff | Dosierung [g a.i./ha] | Wirkung [%] |
|---|---|---|
| A1-60 | 1 | 65 |
| lenacil | 1200 | 0 |
| A1-60 + lenacil | 1 + 1200 | 80 (Ec = 65, Diff. = +15) |

**Tabelle 72: Wirkung gegen SOLNI, 14 Tage nach Applikation**

| Wirkstoff | Dosierung [g a.i./ha] | Wirkung [%] |
|---|---|---|
| A1-60 | 1 | 65 |
| lenacil | 300 | 0 |
| A1-60 + lenacil | 1 + 300 | 80 (Ec = 65, Diff. = +15) |

**Tabelle 73: Wirkung gegen DIGSA, 14 Tage nach Applikation**

| Wirkstoff | Dosierung [g a.i./ha] | Wirkung [%] |
|---|---|---|
| A1-13 | 0,4 | 0 |
| metribuzin | 20 | 20 |
| A1-13 + metribuzin | 0,4 + 20 | 60 (Ec = 20, Diff. = +40) |

**Tabelle 74: Wirkung gegen DIGSA, 14 Tage nach Applikation**

| Wirkstoff | Dosierung [g a.i./ha] | Wirkung [%] |
|---|---|---|
| A1-13 | 0,4 | 0 |
| metribuzin | 5 | 0 |
| A1-13 + metribuzin | 0,4 + 5 | 60 (Ec = 0, Diff. = +60) |

**Tabelle 75: Wirkung gegen ECHCG, 14 Tage nach Applikation**

| Wirkstoff | Dosierung [g a.i./ha] | Wirkung [%] |
|---|---|---|
| A1-13 | 0,4 | 0 |
| metribuzin | 20 | 30 |
| A1-13 + metribuzin | 0,4 + 20 | 75 (Ec = 30, Diff. = +45) |

**Tabelle 76: Wirkung gegen ECHCG, 14 Tage nach Applikation**

| Wirkstoff | Dosierung [g a.i./ha] | Wirkung [%] |
|---|---|---|
| A1-13 | 0,4 | 0 |
| metribuzin | 5 | 10 |
| A1-13 + metribuzin | 0,4 + 5 | 60 (Ec = 10, Diff. = +50) |

**Tabelle 77: Wirkung gegen ELEIN, 14 Tage nach Applikation**

| Wirkstoff | Dosierung [g a.i./ha] | Wirkung [%] |
|---|---|---|
| A1-13 | 0,4 | 20 |
| metribuzin | 20 | 20 |
| A1-13 + metribuzin | 0,4 + 20 | 80 (Ec = 36, Diff. = +44) |

**Tabelle 78: Wirkung gegen ELEIN, 14 Tage nach Applikation**

| Wirkstoff | Dosierung [g a.i./ha] | Wirkung [%] |
|---|---|---|
| A1-13 | 0,4 | 20 |
| metribuzin | 5 | 10 |
| A1-13 + metribuzin | 0,4 + 5 | 70 (Ec = 28, Diff. = +42) |

**Tabelle 79: Wirkung gegen SETVI, 14 Tage nach Applikation**

| Wirkstoff | Dosierung [g a.i./ha] | Wirkung [%] |
|---|---|---|
| A1-13 | 0,4 | 10 |
| metribuzin | 20 | 30 |
| A1-13 + metribuzin | 0,4 + 20 | 60 (Ec = 37, Diff. = +23) |

**Tabelle 80: Wirkung gegen SETVI, 14 Tage nach Applikation**

| Wirkstoff | Dosierung [g a.i./ha] | Wirkung [%] |
|---|---|---|
| A1-13 | 0,4 | 10 |
| metribuzin | 5 | 10 |
| A1-13 + metribuzin | 0,4 + 5 | 60 (Ec = 19, Diff. = +41) |

**Tabelle 81: Wirkung gegen AMARE, 14 Tage nach Applikation**

| Wirkstoff | Dosierung [g a.i./ha] | Wirkung [%] |
|---|---|---|
| A1-13 | 0,4 | 0 |
| metribuzin | 20 | 30 |
| A1-13 + metribuzin | 0,4 + 20 | 60 (Ec = 30, Diff. = +30) |

**Tabelle 82: Wirkung gegen AMARE, 14 Tage nach Applikation**

| Wirkstoff | Dosierung [g a.i./ha] | Wirkung [%] |
|---|---|---|
| A1-13 | 0,4 | 0 |
| metribuzin | 5 | 20 |
| A1-13 + metribuzin | 0,4 + 5 | 60 (Ec = 20, Diff. = +40) |

**Tabelle 83: Wirkung gegen CHEAL, 14 Tage nach Applikation**

| Wirkstoff | Dosierung [g a.i./ha] | Wirkung [%] |
|---|---|---|
| A1-13 | 0,4 | 10 |
| metribuzin | 20 | 30 |
| A1-13 + metribuzin | 0,4 + 20 | 60 (Ec = 37, Diff. = +23) |

**Tabelle 84: Wirkung gegen CHEAL, 14 Tage nach Applikation**

| Wirkstoff | Dosierung [g a.i./ha] | Wirkung [%] |
|---|---|---|
| A1-13 | 0,4 | 10 |
| metribuzin | 5 | 30 |
| A1-13 + metribuzin | 0,4 + 5 | 60 (Ec = 37, Diff. = +23) |

**Tabelle 85: Wirkung gegen CHEAL, 14 Tage nach Applikation**

| Wirkstoff | Dosierung [g a.i./ha] | Wirkung [%] |
|---|---|---|
| A1-13 | 0,1 | 0 |
| metribuzin | 20 | 30 |
| A1-13 + metribuzin | 0,1 + 20 | 50 (Ec = 30, Diff. = +20) |

**Tabelle 86: Wirkung gegen KCHSC, 14 Tage nach Applikation**

| Wirkstoff | Dosierung [g a.i./ha] | Wirkung [%] |
|---|---|---|
| A1-13 | 0,4 | 0 |
| metribuzin | 20 | 20 |
| A1-13 + metribuzin | 0,4 + 20 | 70 (Ec = 20, Diff. = +50) |

**Tabelle 87: Wirkung gegen KCHSC, 14 Tage nach Applikation**

| Wirkstoff | Dosierung [g a.i./ha] | Wirkung [%] |
|---|---|---|
| A1-13 | 0,4 | 0 |
| metribuzin | 5 | 20 |
| A1-13 + metribuzin | 0,4 + 5 | 70 (Ec = 20, Diff. = +50) |

**Tabelle 88: Wirkung gegen KCHSC, 14 Tage nach Applikation**

| Wirkstoff | Dosierung [g a.i./ha] | Wirkung [%] |
|---|---|---|
| A1-13 | 0,1 | 0 |
| metribuzin | 20 | 20 |
| A1-13 + metribuzin | 0,1 + 20 | 30 (Ec = 20, Diff. = +10) |

**Tabelle 89: Wirkung gegen SOLNI, 14 Tage nach Applikation**

| Wirkstoff | Dosierung [g a.i./ha] | Wirkung [%] |
|---|---|---|
| A1-13 | 0,4 | 20 |
| metribuzin | 20 | 0 |
| A1-13 + metribuzin | 0,4 + 20 | 80 (Ec = 20, Diff. = +60) |

**Tabelle 90: Wirkung gegen SOLNI, 14 Tage nach Applikation**

| Wirkstoff | Dosierung [g a.i./ha] | Wirkung [%] |
|---|---|---|
| A1-13 | 0,4 | 20 |
| metribuzin | 5 | 0 |
| A1-13 + metribuzin | 0,4 + 5 | 80 (Ec = 20, Diff. = +60) |

**Tabelle 91: Wirkung gegen SOLNI, 14 Tage nach Applikation**

| Wirkstoff | Dosierung [g a.i./ha] | Wirkung [%] |
|---|---|---|
| A1-13 | 0,1 | 10 |
| metribuzin | 20 | 0 |
| A1-13 + metribuzin | 0,1 + 20 | 30 (Ec = 10, Diff. = +20) |

**Tabelle 92: Wirkung gegen SOLNI, 14 Tage nach Applikation**

| Wirkstoff | Dosierung [g a.i./ha] | Wirkung [%] |
|---|---|---|
| A1-13 | 0,1 | 10 |
| metribuzin | 5 | 0 |
| A1-13 + metribuzin | 0,1 + 5 | 30 (Ec = 10, Diff. = +20) |

**Tabelle 93: Wirkung gegen ECHCG, 21 Tage nach Applikation**

| Wirkstoff | Dosierung [g a.i./ha] | Wirkung [%] |
|---|---|---|
| A1-3 | 4 | 10 |
| tembotrione | 2 | 60 |
| A1-3 + tembotrione | 4+2 | 70 (Ec = 64, Diff. = +6) |

**Tabelle 94: Wirkung gegen ECHCG, 21 Tage nach Applikation**

| Wirkstoff | Dosierung [g a.i./ha] | Wirkung [%] |
|---|---|---|
| A1-3 | 4 | 10 |
| tembotrione | 0,5 | 10 |
| A1-3 + tembotrione | 4 + 0,5 | 70 (Ec = 19, Diff. = +51) |

**Tabelle 95: Wirkung gegen ECHCG, 21 Tage nach Applikation**

| Wirkstoff | Dosierung [g a.i./ha] | Wirkung [%] |
|---|---|---|
| A1-3 | 1 | 0 |
| tembotrione | 0,5 | 10 |
| A1-3 + tembotrione | 1 + 0,5 | 30 (Ec = 10, Diff. = +20) |

**Tabelle 96: Wirkung gegen ELEIN, 21 Tage nach Applikation**

| Wirkstoff | Dosierung [g a.i./ha] | Wirkung [%] |
|---|---|---|
| A1-3 | 4 | 20 |
| tembotrione | 2 | 10 |
| A1-3 + tembotrione | 4+2 | 40 (Ec = 28, Diff. = +12) |

**Tabelle 97: Wirkung gegen ELEIN, 21 Tage nach Applikation**

| Wirkstoff | Dosierung [g a.i./ha] | Wirkung [%] |
|---|---|---|
| A1-3 | 4 | 20 |
| tembotrione | 0,5 | 0 |
| A1-3 + tembotrione | 4 + 0,5 | 40 (Ec = 20, Diff. = +20) |

**Tabelle 98: Wirkung gegen SETVI, 21 Tage nach Applikation**

| Wirkstoff | Dosierung [g a.i./ha] | Wirkung [%] |
|---|---|---|
| A1-3 | 4 | 20 |
| tembotrione | 0,5 | 0 |
| A1-3 + tembotrione | 4 + 0,5 | 30 (Ec = 20, Diff. = +10) |

**Tabelle 99: Wirkung gegen SETVI, 21 Tage nach Applikation**

| Wirkstoff | Dosierung [g a.i./ha] | Wirkung [%] |
|---|---|---|
| A1-3 | 1 | 10 |
| tembotrione | 2 | 10 |
| A1-3 + tembotrione | 1 + 2 | 30 (Ec = 19, Diff. = +11) |

**Tabelle 100: Wirkung gegen CHEAL, 21 Tage nach Applikation**

| Wirkstoff | Dosierung [g a.i./ha] | Wirkung [%] |
|---|---|---|
| A1-3 | 1 | 20 |
| tembotrione | 2 | 30 |
| A1-3 + tembotrione | 1 + 2 | 93 (Ec = 44, Diff. = +49) |

**Tabelle 101: Wirkung gegen KCHSC, 21 Tage nach Applikation**

| Wirkstoff | Dosierung [g a.i./ha] | Wirkung [%] |
|---|---|---|
| A1-3 | 4 | 20 |
| tembotrione | 2 | 10 |
| A1-3 + tembotrione | 4+2 | 65 (Ec = 28, Diff. = +37) |

**Tabelle 102: Wirkung gegen KCHSC, 21 Tage nach Applikation**

| Wirkstoff | Dosierung [g a.i./ha] | Wirkung [%] |
|---|---|---|
| A1-3 | 4 | 20 |
| tembotrione | 0,5 | 0 |
| A1-3 + tembotrione | 4 + 0,5 | 30 (Ec = 20, Diff. = +10) |

**Tabelle 103: Wirkung gegen ECHCG, 21 Tage nach Applikation**

| Wirkstoff | Dosierung [g a.i./ha] | Wirkung [%] |
|---|---|---|
| A1-13 | 0,4 | 0 |
| foramsulfuron | 1 | 30 |
| A1-13 + foramsulfuron | 0,4 + 1 | 60 (Ec = 30, Diff. = +30) |

**Tabelle 104: Wirkung gegen ECHCG, 21 Tage nach Applikation**

| Wirkstoff | Dosierung [g a.i./ha] | Wirkung [%] |
|---|---|---|
| A1-13 | 0,1 | 0 |
| foramsulfuron | 1 | 30 |
| A1-13 + foramsulfuron | 0,1 + 1 | 60 (Ec = 30, Diff. = +30) |

**Tabelle 105: Wirkung gegen ELEIN, 21 Tage nach Applikation**

| Wirkstoff | Dosierung [g a.i./ha] | Wirkung [%] |
|---|---|---|
| A1-13 | 0,4 | 10 |
| foramsulfuron | 1 | 60 |
| A1-13 + foramsulfuron | 0,4 + 1 | 70 (Ec = 64, Diff. = +6) |

**Tabelle 106: Wirkung gegen ELEIN, 21 Tage nach Applikation**

| Wirkstoff | Dosierung [g a.i./ha] | Wirkung [%] |
|---|---|---|
| A1-13 | 0,1 | 0 |
| foramsulfuron | 1 | 60 |
| A1-13 + foramsulfuron | 0,1 + 1 | 65 (Ec = 60, Diff. = +5) |

**Tabelle 107: Wirkung gegen SETVI, 21 Tage nach Applikation**

| Wirkstoff | Dosierung [g a.i./ha] | Wirkung [%] |
|---|---|---|
| A1-13 | 0,4 | 10 |
| foramsulfuron | 4 | 50 |
| A1-13 + foramsulfuron | 0,4 + 4 | 60 (Ec = 55, Diff. = +5) |

**Tabelle 108: Wirkung gegen SETVI, 21 Tage nach Applikation**

| Wirkstoff | Dosierung [g a.i./ha] | Wirkung [%] |
|---|---|---|
| A1-13 | 0,1 | 0 |
| foramsulfuron | 1 | 20 |
| A1-13 + foramsulfuron | 0,1 + 1 | 30 (Ec = 20, Diff. = +10) |

**Tabelle 109: Wirkung gegen KCHSC, 21 Tage nach Applikation**

| Wirkstoff | Dosierung [g a.i./ha] | Wirkung [%] |
|---|---|---|
| A1-13 | 0,4 | 0 |
| foramsulfuron | 4 | 80 |
| A1-13 + foramsulfuron | 0,4 + 4 | 90 (Ec = 80, Diff. = +10) |

**Tabelle 110: Wirkung gegen AMARE, 21 Tage nach Applikation**

| Wirkstoff | Dosierung [g a.i./ha] | Wirkung [%] |
|---|---|---|
| A1-34 | 100 | 50 |
| thiencarbazone-methyl | 0,25 | 60 |
| A1-34 + thiencarbazone-methyl | 100 + 0,25 | 95 (Ec = 80, Diff. = +15) |

**Tabelle 111: Wirkung gegen AMARE, 21 Tage nach Applikation**

| Wirkstoff | Dosierung [g a.i./ha] | Wirkung [%] |
|---|---|---|
| A1-34 | 25 | 30 |
| thiencarbazone-methyl | 0,25 | 60 |
| A1-34 + thiencarbazone-methyl | 25 + 0,25 | 90 (Ec = 72, Diff. = +18) |

**Tabelle 112: Wirkung gegen CHEAL, 21 Tage nach Applikation**

| Wirkstoff | Dosierung [g a.i./ha] | Wirkung [%] |
|---|---|---|
| A1-34 | 25 | 70 |
| thiencarbazone-methyl | 1 | 70 |
| A1-34 + thiencarbazone-methyl | 25 + 1 | 100 (Ec = 91, Diff. = +9) |

**Tabelle 113: Wirkung gegen CHEAL, 21 Tage nach Applikation**

| Wirkstoff | Dosierung [g a.i./ha] | Wirkung [%] |
|---|---|---|
| A1-34 | 25 | 70 |
| thiencarbazone-methyl | 0,25 | 50 |
| A1-34 + thiencarbazone-methyl | 25 + 0,25 | 90 (Ec = 85, Diff. = +5) |

**Tabelle 114: Wirkung gegen SOLNI, 21 Tage nach Applikation**

| Wirkstoff | Dosierung [g a.i./ha] | Wirkung [%] |
|---|---|---|
| A1-34 | 25 | 10 |
| thiencarbazone-methyl | 1 | 50 |
| A1-34 + thiencarbazone-methyl | 25 + 1 | 60 (Ec = 55, Diff. = +5) |

**Tabelle 115: Wirkung gegen DIGSA, 21 Tage nach Applikation**

| Wirkstoff | Dosierung [g a.i./ha] | Wirkung [%] |
|---|---|---|
| A1-14 | 0,4 | 0 |
| bromoxynil | 400 | 0 |
| A1-14 + bromoxynil | 0,4 + 400 | 10 (Ec = 0, Diff. = +10) |

**Tabelle 116: Wirkung gegen DIGSA, 21 Tage nach Applikation**

| Wirkstoff | Dosierung [g a.i./ha] | Wirkung [%] |
|---|---|---|
| A1-14 | 0,4 | 0 |
| bromoxynil | 100 | 0 |
| A1-14 + bromoxynil | 0,4 + 100 | 10 (Ec = 0, Diff. = +10) |

**Tabelle 117: Wirkung gegen ECHCG, 21 Tage nach Applikation**

| Wirkstoff | Dosierung [g a.i./ha] | Wirkung [%] |
|---|---|---|
| A1-14 | 0,4 | 0 |
| bromoxynil | 400 | 0 |
| A1-14 + bromoxynil | 0,4 + 400 | 30 (Ec = 0, Diff. = +30) |

**Tabelle 118: Wirkung gegen SETVI, 21 Tage nach Applikation**

| Wirkstoff | Dosierung [g a.i./ha] | Wirkung [%] |
|---|---|---|
| A1-14 | 0,4 | 0 |
| bromoxynil | 400 | 0 |
| A1-14 + bromoxynil | 0,4 + 400 | 30 (Ec = 0, Diff. = +30) |

**Tabelle 119: Wirkung gegen SETVI, 21 Tage nach Applikation**

| Wirkstoff | Dosierung [g a.i./ha] | Wirkung [%] |
|---|---|---|
| A1-14 | 0,4 | 0 |
| bromoxynil | 100 | 0 |
| A1-14 + bromoxynil | 0,4 + 100 | 10 (Ec = 0, Diff. = +10) |

**Tabelle 120: Wirkung gegen CHEAL, 21 Tage nach Applikation**

| Wirkstoff | Dosierung [g a.i./ha] | Wirkung [%] |
|---|---|---|
| A1-14 | 0,1 | 0 |
| bromoxynil | 400 | 75 |
| A1-14 + bromoxynil | 0,1 + 400 | 100 (Ec = 75, Diff. = +25) |

**Tabelle 121: Wirkung gegen DIGSA, 21 Tage nach Applikation**

| Wirkstoff | Dosierung [g a.i./ha] | Wirkung [%] |
|---|---|---|
| A1-36 | 0,5 | 10 |
| isoxaflutole | 10 | 0 |
| A1-36 + isoxaflutole | 0,5 + 10 | 20 (Ec = 10, Diff. = +10) |

**Tabelle 122: Wirkung gegen ECHCG, 21 Tage nach Applikation**

| Wirkstoff | Dosierung [g a.i./ha] | Wirkung [%] |
|---|---|---|
| A1-36 | 2 | 20 |
| isoxaflutole | 10 | 60 |
| A1-36 + isoxaflutole | 2 + 10 | 95 (Ec = 68, Diff. = +27) |

**Tabelle 123: Wirkung gegen ECHCG, 21 Tage nach Applikation**

| Wirkstoff | Dosierung [g a.i./ha] | Wirkung [%] |
|---|---|---|
| A1-36 | 2 | 20 |
| isoxaflutole | 2,5 | 10 |
| A1-36 + isoxaflutole | 2 + 2,5 | 70 (Ec = 28, Diff. = +42) |

**Tabelle 124: Wirkung gegen ECHCG, 21 Tage nach Applikation**

| Wirkstoff | Dosierung [g a.i./ha] | Wirkung [%] |
|---|---|---|
| A1-36 | 0,5 | 0 |
| isoxaflutole | 10 | 60 |
| A1-36 + isoxaflutole | 0,5 + 10 | 70 (Ec = 60, Diff. = +10) |

**Tabelle 125: Wirkung gegen ECHCG, 21 Tage nach Applikation**

| Wirkstoff | Dosierung [g a.i./ha] | Wirkung [%] |
|---|---|---|
| A1-36 | 0,5 | 0 |
| isoxaflutole | 2,5 | 10 |
| A1-36 + isoxaflutole | 0,5 + 2,5 | 20 (Ec = 10, Diff. = +10) |

**Tabelle 126: Wirkung gegen ELEIN, 21 Tage nach Applikation**

| Wirkstoff | Dosierung [g a.i./ha] | Wirkung [%] |
|---|---|---|
| A1-36 | 2 | 30 |
| isoxaflutole | 10 | 20 |
| A1-36 + isoxaflutole | 2 + 10 | 75 (Ec = 44, Diff. = +31) |

**Tabelle 127: Wirkung gegen ELEIN, 21 Tage nach Applikation**

| Wirkstoff | Dosierung [g a.i./ha] | Wirkung [%] |
|---|---|---|
| A1-36 | 2 | 30 |
| isoxaflutole | 2,5 | 0 |
| A1-36 + isoxaflutole | 2 + 2,5 | 65 (Ec = 30, Diff. = +35) |

**Tabelle 128: Wirkung gegen SETVI, 21 Tage nach Applikation**

| Wirkstoff | Dosierung [g a.i./ha] | Wirkung [%] |
|---|---|---|
| A1-36 | 2 | 65 |
| isoxaflutole | 10 | 0 |
| A1-36 + isoxaflutole | 2 + 10 | 70 (Ec = 65, Diff. = +5) |

**Tabelle 129: Wirkung gegen SETVI, 21 Tage nach Applikation**

| Wirkstoff | Dosierung [g a.i./ha] | Wirkung [%] |
|---|---|---|
| A1-36 | 2 | 65 |
| isoxaflutole | 2,5 | 0 |
| A1-36 + isoxaflutole | 2 + 2,5 | 70 (Ec = 65, Diff. = +5) |

**Tabelle 130: Wirkung gegen AMARE, 21 Tage nach Applikation**

| Wirkstoff | Dosierung [g a.i./ha] | Wirkung [%] |
|---|---|---|
| A1-36 | 2 | 70 |
| isoxaflutole | 2,5 | 40 |
| A1-36 + isoxaflutole | 2 + 2,5 | 93 (Ec = 82, Diff. = +11) |

**Tabelle 131: Wirkung gegen AMARE, 21 Tage nach Applikation**

| Wirkstoff | Dosierung [g a.i./ha] | Wirkung [%] |
|---|---|---|
| A1-36 | 0,5 | 20 |
| isoxaflutole | 2,5 | 40 |
| A1-36 + isoxaflutole | 0,5 + 2,5 | 70 (Ec = 52, Diff. = +18) |

**Tabelle 132: Wirkung gegen SOLNI, 21 Tage nach Applikation**

| Wirkstoff | Dosierung [g a.i./ha] | Wirkung [%] |
|---|---|---|
| A1-36 | 2 | 50 |
| isoxaflutole | 10 | 65 |
| A1-36 + isoxaflutole | 2 + 10 | 95 (Ec = 82,5, Diff. = +12,5) |

**Tabelle 133: Wirkung gegen ECHCG, 21 Tage nach Applikation**

| Wirkstoff | Dosierung [g a.i./ha] | Wirkung [%] |
|---|---|---|
| A2-1 | 4 | 40 |
| halauxifen | 2 | 20 |
| A2-1 + halauxifen | 4+2 | 65 (Ec = 52, Diff. = +13) |

**Tabelle 134: Wirkung gegen ELEIN, 21 Tage nach Applikation**

| Wirkstoff | Dosierung [g a.i./ha] | Wirkung [%] |
|---|---|---|
| A2-1 | 4 | 60 |
| halauxifen | 2 | 0 |
| A2-1 + halauxifen | 4+2 | 70 (Ec = 60, Diff. = +10) |

**Tabelle 135: Wirkung gegen ELEIN, 21 Tage nach Applikation**

| Wirkstoff | Dosierung [g a.i./ha] | Wirkung [%] |
|---|---|---|
| A2-1 | 4 | 60 |
| halauxifen | 0,5 | 0 |
| A2-1 + halauxifen | 4 + 0,5 | 65 (Ec = 60, Diff. = +5) |

**Tabelle 136: Wirkung gegen SETVI, 21 Tage nach Applikation**

| Wirkstoff | Dosierung [g a.i./ha] | Wirkung [%] |
|---|---|---|
| A2-1 | 4 | 20 |
| halauxifen | 2 | 0 |
| A2-1 + halauxifen | 4+2 | 30 (Ec = 20, Diff. = +10) |

**Tabelle 137: Wirkung gegen KCHSC, 21 Tage nach Applikation**

| Wirkstoff | Dosierung [g a.i./ha] | Wirkung [%] |
|---|---|---|
| A2-1 | 4 | 40 |
| halauxifen | 2 | 30 |
| A2-1 + halauxifen | 4+2 | 65 (Ec = 58, Diff. = +7) |

**Tabelle 138: Wirkung gegen KCHSC, 21 Tage nach Applikation**

| Wirkstoff | Dosierung [g a.i./ha] | Wirkung [%] |
|---|---|---|
| A2-1 | 4 | 40 |
| halauxifen | 0,5 | 20 |
| A2-1 + halauxifen | 4 + 0,5 | 65 (Ec = 52, Diff. = +13) |

**Tabelle 139: Wirkung gegen SOLNI, 21 Tage nach Applikation**

| Wirkstoff | Dosierung [g a.i./ha] | Wirkung [%] |
|---|---|---|
| A2-1 | 1 | 10 |
| halauxifen | 0,5 | 50 |
| A2-1 + halauxifen | 1 + 0,5 | 60 (Ec = 55, Diff. = +5) |

**Tabelle 140: Wirkung gegen DIGSA, 21 Tage nach Applikation**

| Wirkstoff | Dosierung [g a.i./ha] | Wirkung [%] |
|---|---|---|
| A1-55 | 2 | 30 |
| dicamba | 40 | 0 |
| A1-55 + dicamba | 2 + 40 | 40 (Ec = 30, Diff. = +10) |

**Tabelle 141: Wirkung gegen ELEIN, 21 Tage nach Applikation**

| Wirkstoff | Dosierung [g a.i./ha] | Wirkung [%] |
|---|---|---|
| A1-55 | 2 | 30 |
| dicamba | 40 | 0 |
| A1-55 + dicamba | 2 + 40 | 50 (Ec = 30, Diff. = +20) |

**Tabelle 142: Wirkung gegen ELEIN, 21 Tage nach Applikation**

| Wirkstoff | Dosierung [g a.i./ha] | Wirkung [%] |
|---|---|---|
| A1-55 | 2 | 30 |
| dicamba | 10 | 0 |
| A1-55 + dicamba | 2 + 10 | 50 (Ec = 30, Diff. = +20) |

**Tabelle 143: Wirkung gegen SETVI, 21 Tage nach Applikation**

| Wirkstoff | Dosierung [g a.i./ha] | Wirkung [%] |
|---|---|---|
| A1-55 | 2 | 30 |
| dicamba | 40 | 0 |
| A1-55 + dicamba | 2 + 40 | 50 (Ec = 30, Diff. = +20) |

**Tabelle 144: Wirkung gegen SETVI, 21 Tage nach Applikation**

| Wirkstoff | Dosierung [g a.i./ha] | Wirkung [%] |
|---|---|---|
| A1-55 | 2 | 30 |
| dicamba | 10 | 0 |
| A1-55 + dicamba | 2 + 10 | 40 (Ec = 30, Diff. = +10) |

**Tabelle 145: Wirkung gegen AMARE, 21 Tage nach Applikation**

| Wirkstoff | Dosierung [g a.i./ha] | Wirkung [%] |
|---|---|---|
| A1-55 | 0,5 | 10 |
| dicamba | 40 | 65 |
| A1-55 + dicamba | 0,5 + 40 | 75 (Ec = 68,5, Diff. = +6,5) |

**Tabelle 146: Wirkung gegen ELEIN, 21 Tage nach Applikation**

| Wirkstoff | Dosierung [g a.i./ha] | Wirkung [%] |
|---|---|---|
| A1-57 | 1 | 10 |
| glyphosate | 50 | 0 |
| A1-57 + glyphosate | 1 + 50 | 20 (Ec = 10, Diff. = +10) |

**Tabelle 147: Wirkung gegen CHEAL, 21 Tage nach Applikation**

| Wirkstoff | Dosierung [g a.i./ha] | Wirkung [%] |
|---|---|---|
| A1-57 | 1 | 30 |
| glyphosate | 50 | 30 |
| A1-57 + glyphosate | 1 + 50 | 65 (Ec = 51, Diff. = +14) |

**Tabelle 148: Wirkung gegen KCHSC, 21 Tage nach Applikation**

| Wirkstoff | Dosierung [g a.i./ha] | Wirkung [%] |
|---|---|---|
| A1-57 | 1 | 20 |
| glyphosate | 200 | 30 |
| A1-57 + glyphosate | 1 + 200 | 50 (Ec = 44, Diff. = +6) |

**Tabelle 149: Wirkung gegen KCHSC, 21 Tage nach Applikation**

| Wirkstoff | Dosierung [g a.i./ha] | Wirkung [%] |
|---|---|---|
| A1-57 | 1 | 20 |
| glyphosate | 50 | 10 |
| A1-57 + glyphosate | 1 + 50 | 40 (Ec = 28, Diff. = +12) |

**Tabelle 150: Wirkung gegen DIGSA, 21 Tage nach Applikation**

| Wirkstoff | Dosierung [g a.i./ha] | Wirkung [%] |
|---|---|---|
| A1-7 | 2 | 10 |
| fenoxaprop-P-ethyl | 5 | 10 |
| A1-7 + fenoxaprop-P-ethyl | 2+5 | 30 (Ec = 19, Diff. = +11) |

**Tabelle 151: Wirkung gegen SETVI, 21 Tage nach Applikation**

| Wirkstoff | Dosierung [g a.i./ha] | Wirkung [%] |
|---|---|---|
| A1-7 | 2 | 75 |
| fenoxaprop-P-ethyl | 20 | 80 |
| A1-7 + fenoxaprop-P-ethyl | 2 + 20 | 100 (Ec = 95, Diff. = +5) |

**Tabelle 152: Wirkung gegen SETVI, 21 Tage nach Applikation**

| Wirkstoff | Dosierung [g a.i./ha] | Wirkung [%] |
|---|---|---|
| A1-7 | 0,5 | 30 |
| fenoxaprop-P-ethyl | 20 | 80 |
| A1-7 + fenoxaprop-P-ethyl | 0,5 + 20 | 100 (Ec = 86, Diff. = +14) |

**Tabelle 153: Wirkung gegen CHEAL, 21 Tage nach Applikation**

| Wirkstoff | Dosierung [g a.i./ha] | Wirkung [%] |
|---|---|---|
| A1-7 | 2 | 20 |
| fenoxaprop-P-ethyl | 20 | 0 |
| A1-7 + fenoxaprop-P-ethyl | 2 + 20 | 60 (Ec = 20, Diff. = +40) |

**Tabelle 154: Wirkung gegen CHEAL, 21 Tage nach Applikation**

| Wirkstoff | Dosierung [g a.i./ha] | Wirkung [%] |
|---|---|---|
| A1-7 | 2 | 20 |
| fenoxaprop-P-ethyl | 5 | 0 |
| A1-7 + fenoxaprop-P-ethyl | 2+5 | 50 (Ec = 20, Diff. = +30) |

**Tabelle 155: Wirkung gegen CHEAL, 21 Tage nach Applikation**

| Wirkstoff | Dosierung [g a.i./ha] | Wirkung [%] |
|---|---|---|
| A1-7 | 0,5 | 10 |
| fenoxaprop-P-ethyl | 20 | 0 |
| A1-7 + fenoxaprop-P-ethyl | 0,5 + 20 | 30 (Ec = 10, Diff. = +20) |

**Tabelle 156: Wirkung gegen CHEAL, 21 Tage nach Applikation**

| Wirkstoff | Dosierung [g a.i./ha] | Wirkung [%] |
|---|---|---|
| A1-7 | 0,5 | 10 |
| fenoxaprop-P-ethyl | 5 | 0 |
| A1-7 + fenoxaprop-P-ethyl | 0,5 + 5 | 20 (Ec = 10, Diff. = +10) |

**Tabelle 157: Wirkung gegen KCHSC, 21 Tage nach Applikation**

| Wirkstoff | Dosierung [g a.i./ha] | Wirkung [%] |
|---|---|---|
| A1-7 | 0,5 | 10 |
| fenoxaprop-P-ethyl | 20 | 0 |
| A1-7 + fenoxaprop-P-ethyl | 0,5 + 20 | 20 (Ec = 10, Diff. = +10) |

**Tabelle 158: Wirkung gegen SOLNI, 21 Tage nach Applikation**

| Wirkstoff | Dosierung [g a.i./ha] | Wirkung [%] |
|---|---|---|
| A1-7 | 2 | 10 |
| fenoxaprop-P-ethyl | 20 | 0 |
| A1-7 + fenoxaprop-P-ethyl | 2 + 20 | 20 (Ec = 10, Diff. = +10) |

**Tabelle 159: Wirkung gegen SOLNI, 21 Tage nach Applikation**

| Wirkstoff | Dosierung [g a.i./ha] | Wirkung [%] |
|---|---|---|
| A1-7 | 2 | 10 |
| fenoxaprop-P-ethyl | 5 | 0 |
| A1-7 + fenoxaprop-P-ethyl | 2+5 | 20 (Ec = 10, Diff. = +10) |

**Tabelle 160: Wirkung gegen SOLNI, 21 Tage nach Applikation**

| Wirkstoff | Dosierung [g a.i./ha] | Wirkung [%] |
|---|---|---|
| A1-7 | 0,5 | 0 |
| fenoxaprop-P-ethyl | 20 | 0 |
| A1-7 + fenoxaprop-P-ethyl | 0,5 + 20 | 10 ( Ec = 0, Diff. = +10) |

**Tabelle 161: Wirkung gegen SOLNI, 21 Tage nach Applikation**

| Wirkstoff | Dosierung [g a.i./ha] | Wirkung [%] |
|---|---|---|
| A1-7 | 0,5 | 0 |
| fenoxaprop-P-ethyl | 5 | 0 |
| A1-7 + fenoxaprop-P-ethyl | 0,5 + 5 | 10 (Ec = 0, Diff. = +10) |

**Tabelle 162: Wirkung gegen DIGSA, 21 Tage nach Applikation**

| Wirkstoff | Dosierung [g a.i./ha] | Wirkung [%] |
|---|---|---|
| A1-60 | 4 | 70 |
| lenacil | 1200 | 0 |
| A1-60 + lenacil | 4 + 1200 | 97 (Ec = 70, Diff. = +27) |

**Tabelle 163: Wirkung gegen DIGSA, 21 Tage nach Applikation**

| Wirkstoff | Dosierung [g a.i./ha] | Wirkung [%] |
|---|---|---|
| A1-60 | 4 | 70 |
| lenacil | 300 | 0 |
| A1-60 + lenacil | 4 + 300 | 95 (Ec = 70, Diff. = +25) |

**Tabelle 164: Wirkung gegen ECHCG, 21 Tage nach Applikation**

| Wirkstoff | Dosierung [g a.i./ha] | Wirkung [%] |
|---|---|---|
| A1-60 | 4 | 75 |
| lenacil | 1200 | 0 |
| A1-60 + lenacil | 4 + 1200 | 100 (Ec = 75, Diff. = +25) |

**Tabelle 165: Wirkung gegen ECHCG, 21 Tage nach Applikation**

| Wirkstoff | Dosierung [g a.i./ha] | Wirkung [%] |
|---|---|---|
| A1-60 | 4 | 75 |
| lenacil | 300 | 0 |
| A1-60 + lenacil | 4 + 300 | 100 (Ec = 75, Diff. = +25) |

**Tabelle 166: Wirkung gegen ECHCG, 21 Tage nach Applikation**

| Wirkstoff | Dosierung [g a.i./ha] | Wirkung [%] |
|---|---|---|
| A1-60 | 1 | 40 |
| lenacil | 1200 | 0 |
| A1-60 + lenacil | 1 + 1200 | 70 (Ec = 40, Diff. = +30) |

**Tabelle 167: Wirkung gegen ECHCG, 21 Tage nach Applikation**

| Wirkstoff | Dosierung [g a.i./ha] | Wirkung [%] |
|---|---|---|
| A1-60 | 1 | 40 |
| lenacil | 300 | 0 |
| A1-60 + lenacil | 1 + 300 | 70 (Ec = 40, Diff. = +30) |

**Tabelle 168: Wirkung gegen ELEIN, 21 Tage nach Applikation**

| Wirkstoff | Dosierung [g a.i./ha] | Wirkung [%] |
|---|---|---|
| A1-60 | 4 | 75 |
| lenacil | 1200 | 0 |
| A1-60 + lenacil | 4 + 1200 | 98 (Ec = 75, Diff. = +23) |

**Tabelle 169: Wirkung gegen ELEIN, 21 Tage nach Applikation**

| Wirkstoff | Dosierung [g a.i./ha] | Wirkung [%] |
|---|---|---|
| A1-60 | 4 | 75 |
| lenacil | 300 | 0 |
| A1-60 + lenacil | 4 + 300 | 95 (Ec = 75, Diff. = +20) |

**Tabelle 170: Wirkung gegen ELEIN, 21 Tage nach Applikation**

| Wirkstoff | Dosierung [g a.i./ha] | Wirkung [%] |
|---|---|---|
| A1-60 | 1 | 40 |
| lenacil | 1200 | 0 |
| A1-60 + lenacil | 1 + 1200 | 70 (Ec = 40, Diff. = +30) |

**Tabelle 171: Wirkung gegen ELEIN, 21 Tage nach Applikation**

| Wirkstoff | Dosierung [g a.i./ha] | Wirkung [%] |
|---|---|---|
| A1-60 | 1 | 40 |
| lenacil | 300 | 0 |
| A1-60 + lenacil | 1 + 300 | 95 (Ec = 40, Diff. = +55) |

**Tabelle 172: Wirkung gegen SETVI, 21 Tage nach Applikation**

| Wirkstoff | Dosierung [g a.i./ha] | Wirkung [%] |
|---|---|---|
| A1-60 | 4 | 70 |
| lenacil | 1200 | 0 |
| A1-60 + lenacil | 4 + 1200 | 85 (Ec = 70, Diff. = +15) |

**Tabelle 173: Wirkung gegen SETVI, 21 Tage nach Applikation**

| Wirkstoff | Dosierung [g a.i./ha] | Wirkung [%] |
|---|---|---|
| A1-60 | 4 | 70 |
| lenacil | 300 | 0 |
| A1-60 + lenacil | 4 + 300 | 75 (Ec = 70, Diff. = +5) |

**Tabelle 174: Wirkung gegen SETVI, 21 Tage nach Applikation**

| Wirkstoff | Dosierung [g a.i./ha] | Wirkung [%] |
|---|---|---|
| A1-60 | 1 | 50 |
| lenacil | 1200 | 0 |
| A1-60 + lenacil | 1 + 1200 | 80 (Ec = 50, Diff. = +30) |

**Tabelle 175: Wirkung gegen AMARE, 21 Tage nach Applikation**

| Wirkstoff | Dosierung [g a.i./ha] | Wirkung [%] |
|---|---|---|
| A1-60 | 4 | 60 |
| lenacil | 1200 | 0 |
| A1-60 + lenacil | 4 + 1200 | 65 (Ec = 60, Diff. = +5) |

**Tabelle 176: Wirkung gegen AMARE, 21 Tage nach Applikation**

| Wirkstoff | Dosierung [g a.i./ha] | Wirkung [%] |
|---|---|---|
| A1-60 | 1 | 20 |
| lenacil | 1200 | 0 |
| A1-60 + lenacil | 1 + 1200 | 30 (Ec = 20, Diff. = +10) |

**Tabelle 177: Wirkung gegen AMARE, 21 Tage nach Applikation**

| Wirkstoff | Dosierung [g a.i./ha] | Wirkung [%] |
|---|---|---|
| A1-60 | 1 | 20 |
| lenacil | 300 | 0 |
| A1-60 + lenacil | 1 + 300 | 30 (Ec = 20, Diff. = +10) |

**Tabelle 178: Wirkung gegen SOLNI, 21 Tage nach Applikation**

| Wirkstoff | Dosierung [g a.i./ha] | Wirkung [%] |
|---|---|---|
| A1-60 | 4 | 30 |
| lenacil | 1200 | 0 |
| A1-60 + lenacil | 4 + 1200 | 100 (Ec = 30, Diff. = +70) |

**Tabelle 179: Wirkung gegen SOLNI, 21 Tage nach Applikation**

| Wirkstoff | Dosierung [g a.i./ha] | Wirkung [%] |
|---|---|---|
| A1-60 | 4 | 30 |
| lenacil | 300 | 0 |
| A1-60 + lenacil | 4 + 300 | 80 (Ec = 30, Diff. = +50) |

**Tabelle 180: Wirkung gegen SOLNI, 21 Tage nach Applikation**

| Wirkstoff | Dosierung [g a.i./ha] | Wirkung [%] |
|---|---|---|
| A1-60 | 1 | 20 |
| lenacil | 1200 | 0 |
| A1-60 + lenacil | 1 + 1200 | 60 (Ec = 20, Diff. = +40) |

**Tabelle 181: Wirkung gegen SOLNI, 21 Tage nach Applikation**

| Wirkstoff | Dosierung [g a.i./ha] | Wirkung [%] |
|---|---|---|
| A1-60 | 1 | 20 |
| lenacil | 300 | 0 |
| A1-60 + lenacil | 1 + 300 | 60 (Ec = 20, Diff. = +40) |

**Tabelle 182: Wirkung gegen ECHCG, 21 Tage nach Applikation**

| Wirkstoff | Dosierung [g a.i./ha] | Wirkung [%] |
|---|---|---|
| A1-13 | 0,4 | 0 |
| metribuzin | 20 | 0 |
| A1-13 + metribuzin | 0,4 + 20 | 75 (Ec = 0, Diff. = +75) |

**Tabelle 183: Wirkung gegen ECHCG, 21 Tage nach Applikation**

| Wirkstoff | Dosierung [g a.i./ha] | Wirkung [%] |
|---|---|---|
| A1-13 | 0,4 | 0 |
| metribuzin | 5 | 0 |
| A1-13 + metribuzin | 0,4 + 5 | 60 (Ec = 0, Diff. = +60) |

**Tabelle 184: Wirkung gegen ECHCG, 21 Tage nach Applikation**

| Wirkstoff | Dosierung [g a.i./ha] | Wirkung [%] |
|---|---|---|
| A1-13 | 0,1 | 0 |
| metribuzin | 20 | 0 |
| A1-13 + metribuzin | 0,1 + 20 | 20 (Ec = 0, Diff. = +20) |

**Tabelle 185: Wirkung gegen ELEIN, 21 Tage nach Applikation**

| Wirkstoff | Dosierung [g a.i./ha] | Wirkung [%] |
|---|---|---|
| A1-13 | 0,4 | 10 |
| metribuzin | 20 | 0 |
| A1-13 + metribuzin | 0,4 + 20 | 70 (Ec = 10, Diff. = +60) |

**Tabelle 186: Wirkung gegen ELEIN, 21 Tage nach Applikation**

| Wirkstoff | Dosierung [g a.i./ha] | Wirkung [%] |
|---|---|---|
| A1-13 | 0,4 | 10 |
| metribuzin | 5 | 0 |
| A1-13 + metribuzin | 0,4 + 5 | 60 (Ec = 10, Diff. = +50) |

**Tabelle 187: Wirkung gegen ELEIN, 21 Tage nach Applikation**

| Wirkstoff | Dosierung [g a.i./ha] | Wirkung [%] |
|---|---|---|
| A1-13 | 0,1 | 0 |
| metribuzin | 20 | 0 |
| A1-13 + metribuzin | 0,1 + 20 | 10 (Ec = 0, Diff. = +10) |

**Tabelle 188: Wirkung gegen SETVI, 21 Tage nach Applikation**

| Wirkstoff | Dosierung [g a.i./ha] | Wirkung [%] |
|---|---|---|
| A1-13 | 0,4 | 10 |
| metribuzin | 20 | 0 |
| A1-13 + metribuzin | 0,4 + 20 | 50 (Ec = 10, Diff. = +40) |

**Tabelle 189: Wirkung gegen SETVI, 21 Tage nach Applikation**

| Wirkstoff | Dosierung [g a.i./ha] | Wirkung [%] |
|---|---|---|
| A1-13 | 0,4 | 10 |
| metribuzin | 5 | 0 |
| A1-13 + metribuzin | 0,4 + 5 | 40 (Ec = 10, Diff. = +30) |

**Tabelle 190: Wirkung gegen SETVI, 21 Tage nach Applikation**

| Wirkstoff | Dosierung [g a.i./ha] | Wirkung [%] |
|---|---|---|
| A1-13 | 0,1 | 0 |
| metribuzin | 20 | 0 |
| A1-13 + metribuzin | 0,1 + 20 | 10 ( Ec = 0, Diff. = +10) |

**Tabelle 191: Wirkung gegen AMARE, 21 Tage nach Applikation**

| Wirkstoff | Dosierung [g a.i./ha] | Wirkung [%] |
|---|---|---|
| A1-13 | 0,4 | 10 |
| metribuzin | 20 | 10 |
| A1-13 + metribuzin | 0,4 + 20 | 30 (Ec = 19, Diff. = +11) |

**Tabelle 192: Wirkung gegen AMARE, 21 Tage nach Applikation**

| Wirkstoff | Dosierung [g a.i./ha] | Wirkung [%] |
|---|---|---|
| A1-13 | 0,4 | 10 |
| metribuzin | 5 | 0 |
| A1-13 + metribuzin | 0,4 + 5 | 30 (Ec = 10, Diff. = +20) |

**Tabelle 193: Wirkung gegen CHEAL, 21 Tage nach Applikation**

| Wirkstoff | Dosierung [g a.i./ha] | Wirkung [%] |
|---|---|---|
| A1-13 | 0,1 | 0 |
| metribuzin | 20 | 20 |
| A1-13 + metribuzin | 0,1 + 20 | 30 (Ec = 20, Diff. = +10) |

**Tabelle 194: Wirkung gegen CHEAL, 21 Tage nach Applikation**

| Wirkstoff | Dosierung [g a.i./ha] | Wirkung [%] |
|---|---|---|
| A1-13 | 0,1 | 0 |
| metribuzin | 5 | 20 |
| A1-13 + metribuzin | 0,1 + 5 | 30 (Ec = 20, Diff. = +10) |

**Tabelle 195: Wirkung gegen KCHSC, 21 Tage nach Applikation**

| Wirkstoff | Dosierung [g a.i./ha] | Wirkung [%] |
|---|---|---|
| A1-13 | 0,4 | 0 |
| metribuzin | 20 | 10 |
| A1-13 + metribuzin | 0,4 + 20 | 65 (Ec = 10, Diff. = +55) |

**Tabelle 196: Wirkung gegen KCHSC, 21 Tage nach Applikation**

| Wirkstoff | Dosierung [g a.i./ha] | Wirkung [%] |
|---|---|---|
| A1-13 | 0,4 | 0 |
| metribuzin | 5 | 0 |
| A1-13 + metribuzin | 0,4 + 5 | 60 (Ec = 0, Diff. = +60) |

**Tabelle 197: Wirkung gegen KCHSC, 21 Tage nach Applikation**

| Wirkstoff | Dosierung [g a.i./ha] | Wirkung [%] |
|---|---|---|
| A1-13 | 0,1 | 0 |
| metribuzin | 20 | 10 |
| A1-13 + metribuzin | 0,1 + 20 | 20 (Ec = 10, Diff. = +10) |

**Tabelle 198: Wirkung gegen KCHSC, 21 Tage nach Applikation**

| Wirkstoff | Dosierung [g a.i./ha] | Wirkung [%] |
|---|---|---|
| A1-13 | 0,1 | 0 |
| metribuzin | 5 | 0 |
| A1-13 + metribuzin | 0,1 + 5 | 10 (Ec = 0, Diff. = +10) |

**Tabelle 199: Wirkung gegen SOLNI, 21 Tage nach Applikation**

| Wirkstoff | Dosierung [g a.i./ha] | Wirkung [%] |
|---|---|---|
| A1-13 | 0,4 | 10 |
| metribuzin | 20 | 0 |
| A1-13 + metribuzin | 0,4 + 20 | 50 (Ec = 10, Diff. = +40) |

**Tabelle 200: Wirkung gegen SOLNI, 21 Tage nach Applikation**

| Wirkstoff | Dosierung [g a.i./ha] | Wirkung [%] |
|---|---|---|
| A1-13 | 0,4 | 10 |
| metribuzin | 5 | 0 |
| A1-13 + metribuzin | 0,4 + 5 | 65 (Ec = 10, Diff. = +55) |

**Tabelle 201: Wirkung gegen AVEFA, 14 Tage nach Applikation**

| Wirkstoff | Dosierung [g a.i./ha] | Wirkung [%] |
|---|---|---|
| A1-3 | 20 | 0 |
| pinoxaden | 20 | 20 |
| A1-3 + pinoxaden | 20 + 20 | 30 (Ec = 20, Diff. = +10) |

**Tabelle 202: Wirkung gegen AVEFA, 14 Tage nach Applikation**

| Wirkstoff | Dosierung [g a.i./ha] | Wirkung [%] |
|---|---|---|
| A1-3 | 5 | 0 |
| pinoxaden | 5 | 0 |
| A1-3 + pinoxaden | 5+5 | 15 (Ec = 0, Diff. = +15) |

**Tabelle 203: Wirkung gegen BROST, 14 Tage nach Applikation**

| Wirkstoff | Dosierung [g a.i./ha] | Wirkung [%] |
|---|---|---|
| A1-3 | 20 | 5 |
| pinoxaden | 20 | 10 |
| A1-3 + pinoxaden | 20 + 20 | 30 (Ec = 14,5, Diff. = +15,5) |

**Tabelle 204: Wirkung gegen BROST, 14 Tage nach Applikation**

| Wirkstoff | Dosierung [g a.i./ha] | Wirkung [%] |
|---|---|---|
| A1-3 | 20 | 5 |
| pinoxaden | 5 | 0 |
| A1-3 + pinoxaden | 20 + 5 | 25 (Ec = 5, Diff. = +20) |

**Tabelle 205: Wirkung gegen BROST, 14 Tage nach Applikation**

| Wirkstoff | Dosierung [g a.i./ha] | Wirkung [%] |
|---|---|---|
| A1-3 | 5 | 0 |
| pinoxaden | 20 | 10 |
| A1-3 + pinoxaden | 5 + 20 | 30 (Ec = 10, Diff. = +20) |

**Tabelle 206: Wirkung gegen BROST, 14 Tage nach Applikation**

| Wirkstoff | Dosierung [g a.i./ha] | Wirkung [%] |
|---|---|---|
| A1-3 | 5 | 0 |
| pinoxaden | 5 | 0 |
| A1-3 + pinoxaden | 5+5 | 15 (Ec = 0, Diff. = +15) |

**Tabelle 207: Wirkung gegen LOLMU, 14 Tage nach Applikation**

| Wirkstoff | Dosierung [g a.i./ha] | Wirkung [%] |
|---|---|---|
| A1-3 | 5 | 0 |
| pinoxaden | 20 | 85 |
| A1-3 + pinoxaden | 5 + 20 | 90 (Ec = 85, Diff. = +5) |

**Tabelle 208: Wirkung gegen PHAMI, 14 Tage nach Applikation**

| Wirkstoff | Dosierung [g a.i./ha] | Wirkung [%] |
|---|---|---|
| A1-3 | 20 | 20 |
| pinoxaden | 20 | 50 |
| A1-3 + pinoxaden | 20 + 20 | 85 (Ec = 60, Diff. = +25) |

**Tabelle 209: Wirkung gegen PHAMI, 14 Tage nach Applikation**

| Wirkstoff | Dosierung [g a.i./ha] | Wirkung [%] |
|---|---|---|
| A1-3 | 20 | 20 |
| pinoxaden | 5 | 10 |
| A1-3 + pinoxaden | 20 + 5 | 50 (Ec = 28, Diff. = +22) |

**Tabelle 210: Wirkung gegen PHAMI, 14 Tage nach Applikation**

| Wirkstoff | Dosierung [g a.i./ha] | Wirkung [%] |
|---|---|---|
| A1-3 | 5 | 0 |
| pinoxaden | 20 | 50 |
| A1-3 + pinoxaden | 5 + 20 | 60 (Ec = 50, Diff. = +10) |

**Tabelle 211: Wirkung gegen PHAMI, 14 Tage nach Applikation**

| Wirkstoff | Dosierung [g a.i./ha] | Wirkung [%] |
|---|---|---|
| A1-3 | 5 | 0 |
| pinoxaden | 5 | 10 |
| A1-3 + pinoxaden | 5+5 | 15 (Ec = 10, Diff. = +5) |

**Tabelle 212: Wirkung gegen POAAN, 14 Tage nach Applikation**

| Wirkstoff | Dosierung [g a.i./ha] | Wirkung [%] |
|---|---|---|
| A1-3 | 20 | 5 |
| pinoxaden | 20 | 0 |
| A1-3 + pinoxaden | 20 + 20 | 35 (Ec = 5, Diff. = +30) |

**Tabelle 213: Wirkung gegen POAAN, 14 Tage nach Applikation**

| Wirkstoff | Dosierung [g a.i./ha] | Wirkung [%] |
|---|---|---|
| A1-3 | 20 | 5 |
| pinoxaden | 5 | 0 |
| A1-3 + pinoxaden | 20 + 5 | 15 (Ec = 5, Diff. = +10) |

**Tabelle 214: Wirkung gegen POAAN, 14 Tage nach Applikation**

| Wirkstoff | Dosierung [g a.i./ha] | Wirkung [%] |
|---|---|---|
| A1-3 | 5 | 0 |
| pinoxaden | 20 | 0 |
| A1-3 + pinoxaden | 5 + 20 | 20 (Ec = 0, Diff. = +20) |

**Tabelle 215: Wirkung gegen GALAP, 14 Tage nach Applikation**

| Wirkstoff | Dosierung [g a.i./ha] | Wirkung [%] |
|---|---|---|
| A1-3 | 5 | 40 |
| pinoxaden | 20 | 0 |
| A1-3 + pinoxaden | 5 + 20 | 50 (Ec = 40, Diff. = +10) |

**Tabelle 216: Wirkung gegen MATIN, 14 Tage nach Applikation**

| Wirkstoff | Dosierung [g a.i./ha] | Wirkung [%] |
|---|---|---|
| A1-3 | 5 | 30 |
| pinoxaden | 20 | 0 |
| A1-3 + pinoxaden | 5 + 20 | 35 (Ec = 30, Diff. = +5) |

**Tabelle 217: Wirkung gegen POLCO, 14 Tage nach Applikation**

| Wirkstoff | Dosierung [g a.i./ha] | Wirkung [%] |
|---|---|---|
| A1-3 | 20 | 10 |
| pinoxaden | 20 | 0 |
| A1-3 + pinoxaden | 20 + 20 | 40 (Ec = 10, Diff. = +30) |

**Tabelle 218: Wirkung gegen POLCO, 14 Tage nach Applikation**

| Wirkstoff | Dosierung [g a.i./ha] | Wirkung [%] |
|---|---|---|
| A1-3 | 20 | 10 |
| pinoxaden | 5 | 0 |
| A1-3 + pinoxaden | 20 + 5 | 40 (Ec = 10, Diff. = +30) |

**Tabelle 219: Wirkung gegen POLCO, 14 Tage nach Applikation**

| Wirkstoff | Dosierung [g a.i./ha] | Wirkung [%] |
|---|---|---|
| A1-3 | 5 | 0 |
| pinoxaden | 20 | 0 |
| A1-3 + pinoxaden | 5 + 20 | 40 (Ec = 0, Diff. = +40) |

**Tabelle 220: Wirkung gegen POLCO, 14 Tage nach Applikation**

| Wirkstoff | Dosierung [g a.i./ha] | Wirkung [%] |
|---|---|---|
| A1-3 | 5 | 0 |
| pinoxaden | 5 | 0 |
| A1-3 + pinoxaden | 5+5 | 20 (Ec = 0, Diff. = +20) |

**Tabelle 221: Wirkung gegen AVEFA, 14 Tage nach Applikation**

| Wirkstoff | Dosierung [g a.i./ha] | Wirkung [%] |
|---|---|---|
| A1-13 | 10 | 35 |
| mesosulfuron-methyl | 10 | 5 |
| A1-13 + mesosulfuron-methyl | 10 + 10 | 60 (Ec = 38,25, Diff. = +21,75) |

**Tabelle 222: Wirkung gegen PHAMI, 14 Tage nach Applikation**

| Wirkstoff | Dosierung [g a.i./ha] | Wirkung [%] |
|---|---|---|
| A1-13 | 10 | 30 |
| mesosulfuron-methyl | 10 | 5 |
| A1-13 + mesosulfuron-methyl | 10 + 10 | 40 (Ec = 33,5, Diff. = +6,5) |

**Tabelle 223: Wirkung gegen PHAMI, 14 Tage nach Applikation**

| Wirkstoff | Dosierung [g a.i./ha] | Wirkung [%] |
|---|---|---|
| A1-13 | 10 | 30 |
| mesosulfuron-methyl | 2,5 | 0 |
| A1-13 + mesosulfuron-methyl | 10 + 2,5 | 40 (Ec = 30, Diff. = +10) |

**Tabelle 224: Wirkung gegen PHAMI, 14 Tage nach Applikation**

| Wirkstoff | Dosierung [g a.i./ha] | Wirkung [%] |
|---|---|---|
| A1-13 | 2,5 | 30 |
| mesosulfuron-methyl | 10 | 5 |
| A1-13 + mesosulfuron-methyl | 2,5 + 10 | 40 (Ec = 33,5, Diff. = +6,5) |

**Tabelle 225: Wirkung gegen POAAN, 14 Tage nach Applikation**

| Wirkstoff | Dosierung [g a.i./ha] | Wirkung [%] |
|---|---|---|
| A1-13 | 10 | 40 |
| mesosulfuron-methyl | 10 | 40 |
| A1-13 + mesosulfuron-methyl | 10 + 10 | 70 (Ec = 64, Diff. = +6) |

**Tabelle 226: Wirkung gegen POAAN, 14 Tage nach Applikation**

| Wirkstoff | Dosierung [g a.i./ha] | Wirkung [%] |
|---|---|---|
| A1-13 | 2,5 | 20 |
| mesosulfuron-methyl | 10 | 40 |
| A1-13 + mesosulfuron-methyl | 2,5 + 10 | 70 (Ec = 52, Diff. = +18) |

**Tabelle 227: Wirkung gegen POAAN, 14 Tage nach Applikation**

| Wirkstoff | Dosierung [g a.i./ha] | Wirkung [%] |
|---|---|---|
| A1-13 | 2,5 | 20 |
| mesosulfuron-methyl | 2,5 | 15 |
| A1-13 + mesosulfuron-methyl | 2,5 + 2,5 | 40 (Ec = 32, Diff. = +8) |

**Tabelle 228: Wirkung gegen GALAP, 14 Tage nach Applikation**

| Wirkstoff | Dosierung [g a.i./ha] | Wirkung [%] |
|---|---|---|
| A1-13 | 10 | 50 |
| mesosulfuron-methyl | 10 | 30 |
| A1-13 + mesosulfuron-methyl | 10 + 10 | 75 (Ec = 65, Diff. = +10) |

**Tabelle 229: Wirkung gegen GALAP, 14 Tage nach Applikation**

| Wirkstoff | Dosierung [g a.i./ha] | Wirkung [%] |
|---|---|---|
| A1-13 | 10 | 50 |
| mesosulfuron-methyl | 2,5 | 30 |
| A1-13 + mesosulfuron-methyl | 10 + 2,5 | 70 (Ec = 65, Diff. = +5) |

**Tabelle 230: Wirkung gegen MATIN, 14 Tage nach Applikation**

| Wirkstoff | Dosierung [g a.i./ha] | Wirkung [%] |
|---|---|---|
| A1-13 | 10 | 40 |
| mesosulfuron-methyl | 10 | 0 |
| A1-13 + mesosulfuron-methyl | 10 + 10 | 70 (Ec = 40, Diff. = +30) |

**Tabelle 231: Wirkung gegen MATIN, 14 Tage nach Applikation**

| Wirkstoff | Dosierung [g a.i./ha] | Wirkung [%] |
|---|---|---|
| A1-13 | 10 | 40 |
| mesosulfuron-methyl | 2,5 | 0 |
| A1-13 + mesosulfuron-methyl | 10 + 2,5 | 65 (Ec = 40, Diff. = +25) |

**Tabelle 232: Wirkun gegen MATIN, 14 Tage nach Applikation**

| Wirkstoff | Dosierung [g a.i./ha] | Wirkung [%] |
|---|---|---|
| A1-13 | 2,5 | 30 |
| mesosulfuron-methyl | 10 | 0 |
| A1-13 + mesosulfuron-methyl | 2,5 + 10 | 40 (Ec = 30, Diff. = +10) |

**Tabelle 233: Wirkung gegen BROST, 14 Tage nach Applikation**

| Wirkstoff | Dosierung [g a.i./ha] | Wirkung [%] |
|---|---|---|
| A1-34 | 50 | 10 |
| iodosulfuron-methyl-sodium | 2 | 15 |
| A1-34 + iodosulfuron-methyl-sodium | 50 + 2 | 30 (Ec = 23,5, Diff. = +6,5) |

**Tabelle 234: Wirkung gegen LOLMU, 14 Tage nach Applikation**

| Wirkstoff | Dosierung [g a.i./ha] | Wirkung [%] |
|---|---|---|
| A1-34 | 200 | 30 |
| iodosulfuron-methyl-sodium | 2 | 30 |
| A1-34 + iodosulfuron-methyl-sodium | 200 + 2 | 60 (Ec = 51, Diff. = +9) |

**Tabelle 235: Wirkung gegen LOLMU, 14 Tage nach Applikation**

| Wirkstoff | Dosierung [g a.i./ha] | Wirkung [%] |
|---|---|---|
| A1-34 | 200 | 30 |
| iodosulfuron-methyl-sodium | 0,5 | 0 |
| A1-34 + iodosulfuron-methyl-sodium | 200 + 0,5 | 40 (Ec = 30, Diff. = +10) |

**Tabelle 236: Wirkung gegen LOLMU, 14 Tage nach Applikation**

| Wirkstoff | Dosierung [g a.i./ha] | Wirkung [%] |
|---|---|---|
| A1-34 | 50 | 5 |
| iodosulfuron-methyl-sodium | 0,5 | 0 |
| A1-34 + iodosulfuron-methyl-sodium | 50 + 0,5 | 15 (Ec = 5, Diff. = +10) |

**Tabelle 237: Wirkung gegen PHAMI, 14 Tage nach Applikation**

| Wirkstoff | Dosierung [g a.i./ha] | Wirkung [%] |
|---|---|---|
| A1-34 | 200 | 60 |
| iodosulfuron-methyl-sodium | 0,5 | 10 |
| A1-34 + iodosulfuron-methyl-sodium | 200 + 0,5 | 70 (Ec = 64, Diff. = +6) |

**Tabelle 238: Wirkung gegen POAAN, 14 Tage nach Applikation**

| Wirkstoff | Dosierung [g a.i./ha] | Wirkung [%] |
|---|---|---|
| A1-34 | 200 | 85 |
| iodosulfuron-methyl-sodium | 2 | 20 |
| A1-34 + iodosulfuron-methyl-sodium | 200 + 2 | 93 (Ec = 88, Diff. = +5) |

**Tabelle 239: Wirkung gegen POAAN, 14 Tage nach Applikation**

| Wirkstoff | Dosierung [g a.i./ha] | Wirkung [%] |
|---|---|---|
| A1-34 | 200 | 85 |
| iodosulfuron-methyl-sodium | 0,5 | 0 |
| A1-34 + iodosulfuron-methyl-sodium | 200 + 0,5 | 95 (Ec = 85, Diff. = + 10) |

**Tabelle 240: Wirkung gegen GALAP, 14 Tage nach Applikation**

| Wirkstoff | Dosierung [g a.i./ha] | Wirkung [%] |
|---|---|---|
| A1-34 | 50 | 50 |
| iodosulfuron-methyl-sodium | 0,5 | 30 |
| A1-34 + iodosulfuron-methyl-sodium | 50 + 0,5 | 70 (Ec = 65, Diff. = +5) |

**Tabelle 241: Wirkung gegen POLCO, 14 Tage nach Applikation**

| Wirkstoff | Dosierung [g a.i./ha] | Wirkung [%] |
|---|---|---|
| A1-34 | 200 | 30 |
| iodosulfuron-methyl-sodium | 2 | 30 |
| A1-34 + iodosulfuron-methyl-sodium | 200 + 2 | 60 (Ec = 51, Diff. = +9) |

**Tabelle 242: Wirkung gegen POLCO, 14 Tage nach Applikation**

| Wirkstoff | Dosierung [g a.i./ha] | Wirkung [%] |
|---|---|---|
| A1-34 | 200 | 30 |
| iodosulfuron-methyl-sodium | 0,5 | 15 |
| A1-34 + iodosulfuron-methyl-sodium | 200 + 0,5 | 60 (Ec = 40,5, Diff. = +19,5) |

**Tabelle 243: Wirkung gegen BROST, 14 Tage nach Applikation**

| Wirkstoff | Dosierung [g a.i./ha] | Wirkung [%] |
|---|---|---|
| A1-14 | 10 | 3 |
| pyrasulfotole | 25 | 0 |
| A1-14 + pyrasulfotole | 10 + 25 | 10 (Ec = 3, Diff. = +7) |

**Tabelle 244: Wirkung gegen BROST, 14 Tage nach Applikation**

| Wirkstoff | Dosierung [g a.i./ha] | Wirkung [%] |
|---|---|---|
| A1-14 | 2,5 | 0 |
| pyrasulfotole | 25 | 0 |
| A1-14 + pyrasulfotole | 2,5 + 25 | 10 (Ec = 0, Diff. = + 10) |

**Tabelle 245: Wirkung gegen LOLMU, 14 Tage nach Applikation**

| Wirkstoff | Dosierung [g a.i./ha] | Wirkung [%] |
|---|---|---|
| A1-14 | 10 | 0 |
| pyrasulfotole | 100 | 0 |
| A1-14 + pyrasulfotole | 10 + 100 | 20 (Ec = 0, Diff. = +20) |

**Tabelle 246: Wirkung gegen LOLMU, 14 Tage nach Applikation**

| Wirkstoff | Dosierung [g a.i./ha] | Wirkung [%] |
|---|---|---|
| A1-14 | 2,5 | 0 |
| pyrasulfotole | 100 | 0 |
| A1-14 + pyrasulfotole | 2,5 + 100 | 15 (Ec = 0, Diff. = +15) |

**Tabelle 247: Wirkung gegen LOLMU, 14 Tage nach Applikation**

| Wirkstoff | Dosierung [g a.i./ha] | Wirkung [%] |
|---|---|---|
| A1-14 | 2,5 | 0 |
| pyrasulfotole | 25 | 0 |
| A1-14 + pyrasulfotole | 2,5 + 25 | 15 (Ec = 0, Diff. = +15) |

**Tabelle 248: Wirkung gegen PHAMI, 14 Tage nach Applikation**

| Wirkstoff | Dosierung [g a.i./ha] | Wirkung [%] |
|---|---|---|
| A1-14 | 10 | 25 |
| pyrasulfotole | 100 | 5 |
| A1-14 + pyrasulfotole | 10 + 100 | 40 (Ec = 28,75, Diff. = +11,25) |

**Tabelle 249: Wirkung gegen PHAMI, 14 Tage nach Applikation**

| Wirkstoff | Dosierung [g a.i./ha] | Wirkung [%] |
|---|---|---|
| A1-14 | 2,5 | 0 |
| pyrasulfotole | 100 | 5 |
| A1-14 + pyrasulfotole | 2,5 + 100 | 30 (Ec = 5, Diff. = +25) |

**Tabelle 250: Wirkung gegen PHAMI, 14 Tage nach Applikation**

| Wirkstoff | Dosierung [g a.i./ha] | Wirkung [%] |
|---|---|---|
| A1-14 | 2,5 | 0 |
| pyrasulfotole | 25 | 0 |
| A1-14 + pyrasulfotole | 2,5 + 25 | 10 (Ec = 0, Diff. = + 10) |

**Tabelle 251: Wirkung gegen POAAN, 14 Tage nach Applikation**

| Wirkstoff | Dosierung [g a.i./ha] | Wirkung [%] |
|---|---|---|
| A1-14 | 2,5 | 10 |
| pyrasulfotole | 100 | 0 |
| A1-14 + pyrasulfotole | 2,5 + 100 | 25 (Ec = 10, Diff. = + 15) |

**Tabelle 252: Wirkung gegen POAAN, 14 Tage nach Applikation**

| Wirkstoff | Dosierung [g a.i./ha] | Wirkung [%] |
|---|---|---|
| A1-14 | 2,5 | 10 |
| pyrasulfotole | 25 | 0 |
| A1-14 + pyrasulfotole | 2,5 + 25 | 20 (Ec = 10, Diff. = + 10) |

**Tabelle 253: Wirkung gegen MATIN, 14 Tage nach Applikation**

| Wirkstoff | Dosierung [g a.i./ha] | Wirkung [%] |
|---|---|---|
| A1-14 | 10 | 60 |
| pyrasulfotole | 100 | 15 |
| A1-14 + pyrasulfotole | 10 + 100 | 80 (Ec = 66, Diff. = + 14) |

**Tabelle 254: Wirkung gegen MATIN, 14 Tage nach Applikation**

| Wirkstoff | Dosierung [g a.i./ha] | Wirkung [%] |
|---|---|---|
| A1-14 | 10 | 60 |
| pyrasulfotole | 25 | 0 |
| A1-14 + pyrasulfotole | 10 + 25 | 70 (Ec = 60, Diff. = + 10) |

**Tabelle 255: Wirkung gegen MATIN, 14 Tage nach Applikation**

| Wirkstoff | Dosierung [g a.i./ha] | Wirkung [%] |
|---|---|---|
| A1-14 | 2,5 | 50 |
| pyrasulfotole | 100 | 15 |
| A1-14 + pyrasulfotole | 2,5 + 100 | 70 (Ec = 57,5, Diff. = +12,5) |

**Tabelle 256: Wirkung gegen POLCO, 14 Tage nach Applikation**

| Wirkstoff | Dosierung [g a.i./ha] | Wirkung [%] |
|---|---|---|
| A1-14 | 10 | 20 |
| pyrasulfotole | 100 | 60 |
| A1-14 + pyrasulfotole | 10 + 100 | 85 (Ec = 68, Diff. = +17) |

**Tabelle 257: Wirkung gegen POLCO, 14 Tage nach Applikation**

| Wirkstoff | Dosierung [g a.i./ha] | Wirkung [%] |
|---|---|---|
| A1-14 | 10 | 20 |
| pyrasulfotole | 25 | 50 |
| A1-14 + pyrasulfotole | 10 + 25 | 95 (Ec = 60, Diff. = +35) |

**Tabelle 258: Wirkung gegen POLCO, 14 Tage nach Applikation**

| Wirkstoff | Dosierung [g a.i./ha] | Wirkung [%] |
|---|---|---|
| A1-14 | 2,5 | 20 |
| pyrasulfotole | 100 | 60 |
| A1-14 + pyrasulfotole | 2,5 + 100 | 75 (Ec = 68, Diff. = +7) |

**Tabelle 259: Wirkung gegen POLCO, 14 Tage nach Applikation**

| Wirkstoff | Dosierung [g a.i./ha] | Wirkung [%] |
|---|---|---|
| A1-14 | 2,5 | 20 |
| pyrasulfotole | 25 | 50 |
| A1-14 + pyrasulfotole | 2,5 + 25 | 65 (Ec = 60, Diff. = +5) |

**Tabelle 260: Wirkung gegen AVEFA, 14 Tage nach Applikation**

| Wirkstoff | Dosierung [g a.i./ha] | Wirkung [%] |
|---|---|---|
| A1-36 | 10 | 15 |
| flurtamone | 320 | 15 |
| A1-36 + flurtamone | 10 + 320 | 40 (Ec = 27,75, Diff. = +12,25) |

**Tabelle 261: Wirkung gegen AVEFA, 14 Tage nach Applikation**

| Wirkstoff | Dosierung [g a.i./ha] | Wirkung [%] |
|---|---|---|
| A1-36 | 10 | 15 |
| flurtamone | 80 | 0 |
| A1-36 + flurtamone | 10 + 80 | 30 (Ec = 15, Diff. = + 15) |

**Tabelle 262: Wirkung gegen AVEFA, 14 Tage nach Applikation**

| Wirkstoff | Dosierung [g a.i./ha] | Wirkung [%] |
|---|---|---|
| A1-36 | 2,5 | 0 |
| flurtamone | 320 | 15 |
| A1-36 + flurtamone | 2,5 + 320 | 30 (Ec = 15, Diff. = +15) |

**Tabelle 263: Wirkung gegen AVEFA, 14 Tage nach Applikation**

| Wirkstoff | Dosierung [g a.i./ha] | Wirkung [%] |
|---|---|---|
| A1-36 | 2,5 | 0 |
| flurtamone | 80 | 0 |
| A1-36 + flurtamone | 2,5 + 80 | 15 (Ec = 0, Diff. = +15) |

**Tabelle 264: Wirkung gegen BROST, 14 Tage nach Applikation**

| Wirkstoff | Dosierung [g a.i./ha] | Wirkung [%] |
|---|---|---|
| A1-36 | 2,5 | 0 |
| flurtamone | 320 | 10 |
| A1-36 + flurtamone | 2,5 + 320 | 15 (Ec = 10, Diff. = +5) |

**Tabelle 265: Wirkung gegen BROST, 14 Tage nach Applikation**

| Wirkstoff | Dosierung [g a.i./ha] | Wirkung [%] |
|---|---|---|
| A1-36 | 2,5 | 0 |
| flurtamone | 80 | 0 |
| A1-36 + flurtamone | 2,5 + 80 | 15 (Ec = 0, Diff. = +15) |

**Tabelle 266: Wirkung gegen LOLMU, 14 Tage nach Applikation**

| Wirkstoff | Dosierung [g a.i./ha] | Wirkung [%] |
|---|---|---|
| A1-36 | 10 | 3 |
| flurtamone | 80 | 30 |
| A1-36 + flurtamone | 10 + 80 | 60 (Ec = 32,1, Diff. = +27,9) |

**Tabelle 267: Wirkung gegen PHAMI, 14 Tage nach Applikation**

| Wirkstoff | Dosierung [g a.i./ha] | Wirkung [%] |
|---|---|---|
| A1-36 | 10 | 30 |
| flurtamone | 320 | 50 |
| A1-36 + flurtamone | 10 + 320 | 75 (Ec = 65, Diff. = + 10) |

**Tabelle 268: Wirkung gegen PHAMI, 14 Tage nach Applikation**

| Wirkstoff | Dosierung [g a.i./ha] | Wirkung [%] |
|---|---|---|
| A1-36 | 10 | 30 |
| flurtamone | 80 | 40 |
| A1-36 + flurtamone | 10 + 80 | 65 (Ec = 58, Diff. = +7) |

**Tabelle 269: Wirkung gegen PHAMI, 14 Tage nach Applikation**

| Wirkstoff | Dosierung [g a.i./ha] | Wirkung [%] |
|---|---|---|
| A1-36 | 2,5 | 0 |
| flurtamone | 320 | 50 |
| A1-36 + flurtamone | 2,5 + 320 | 85 (Ec = 50, Diff. = +35) |

**Tabelle 270: Wirkung gegen GALAP, 14 Tage nach Applikation**

| Wirkstoff | Dosierung [g a.i./ha] | Wirkung [%] |
|---|---|---|
| A1-36 | 10 | 25 |
| flurtamone | 320 | 80 |
| A1-36 + flurtamone | 10 + 320 | 100 (Ec = 85, Diff. = +15) |

**Tabelle 271: Wirkung gegen GALAP, 14 Tage nach Applikation**

| Wirkstoff | Dosierung [g a.i./ha] | Wirkung [%] |
|---|---|---|
| A1-36 | 10 | 25 |
| flurtamone | 80 | 40 |
| A1-36 + flurtamone | 10 + 80 | 60 (Ec = 55, Diff. = +5) |

**Tabelle 272: Wirkung gegen GALAP, 14 Tage nach Applikation**

| Wirkstoff | Dosierung [g a.i./ha] | Wirkung [%] |
|---|---|---|
| A1-36 | 2,5 | 5 |
| flurtamone | 80 | 40 |
| A1-36 + flurtamone | 2,5 + 80 | 70 (Ec = 43, Diff. = +27) |

**Tabelle 273: Wirkung gegen MATIN, 14 Tage nach Applikation**

| Wirkstoff | Dosierung [g a.i./ha] | Wirkung [%] |
|---|---|---|
| A1-36 | 10 | 40 |
| flurtamone | 320 | 50 |
| A1-36 + flurtamone | 10 + 320 | 97 (Ec = 70, Diff. = +27) |

**Tabelle 274: Wirkung gegen MATIN, 14 Tage nach Applikation**

| Wirkstoff | Dosierung [g a.i./ha] | Wirkung [%] |
|---|---|---|
| A1-36 | 10 | 40 |
| flurtamone | 80 | 50 |
| A1-36 + flurtamone | 10 + 80 | 97 (Ec = 70, Diff. = +27) |

**Tabelle 275: Wirkung gegen MATIN, 14 Tage nach Applikation**

| Wirkstoff | Dosierung [g a.i./ha] | Wirkung [%] |
|---|---|---|
| A1-36 | 2,5 | 15 |
| flurtamone | 320 | 50 |
| A1-36 + flurtamone | 2,5 + 320 | 90 (Ec = 57,5, Diff. = +32,5) |

**Tabelle 276: Wirkung gegen MATIN, 14 Tage nach Applikation**

| Wirkstoff | Dosierung [g a.i./ha] | Wirkung [%] |
|---|---|---|
| A1-36 | 2,5 | 15 |
| flurtamone | 80 | 50 |
| A1-36 + flurtamone | 2,5 + 80 | 80 (Ec = 57,5, Diff. = +22,5) |

**Tabelle 277: Wirkung gegen POLCO, 14 Tage nach Applikation**

| Wirkstoff | Dosierung [g a.i./ha] | Wirkung [%] |
|---|---|---|
| A1-36 | 2,5 | 0 |
| flurtamone | 320 | 30 |
| A1-36 + flurtamone | 2,5 + 320 | 40 (Ec = 30, Diff. = + 10) |

**Tabelle 278: Wirkung gegen POLCO, 14 Tage nach Applikation**

| Wirkstoff | Dosierung [g a.i./ha] | Wirkung [%] |
|---|---|---|
| A1-36 | 2,5 | 0 |
| flurtamone | 80 | 50 |
| A1-36 + flurtamone | 2,5 + 80 | 70 (Ec = 50, Diff. = +20) |

**Tabelle 279: Wirkung gegen AVEFA, 14 Tage nach Applikation**

| Wirkstoff | Dosierung [g a.i./ha] | Wirkung [%] |
|---|---|---|
| A2-1 | 50 | 0 |
| fluroxypyr | 80 | 0 |
| A2-1 + fluroxypyr | 50 + 80 | 20 (Ec = 0, Diff. = +20) |

**Tabelle 280: Wirkung gegen AVEFA, 14 Tage nach Applikation**

| Wirkstoff | Dosierung [g a.i./ha] | Wirkung [%] |
|---|---|---|
| A2-1 | 12,5 | 0 |
| fluroxypyr | 80 | 0 |
| A2-1 + fluroxypyr | 12,5 + 80 | 15 (Ec = 0, Diff. = +15) |

**Tabelle 281: Wirkung gegen BROST, 14 Tage nach Applikation**

| Wirkstoff | Dosierung [g a.i./ha] | Wirkung [%] |
|---|---|---|
| A2-1 | 12,5 | 0 |
| fluroxypyr | 80 | 0 |
| A2-1 + fluroxypyr | 12,5 + 80 | 15 (Ec = 0, Diff. = +15) |

**Tabelle 282: Wirkung gegen LOLMU, 14 Tage nach Applikation**

| Wirkstoff | Dosierung [g a.i./ha] | Wirkung [%] |
|---|---|---|
| A2-1 | 50 | 0 |
| fluroxypyr | 80 | 0 |
| A2-1 + fluroxypyr | 50 + 80 | 20 (Ec = 0, Diff. = +20) |

**Tabelle 283: Wirkung gegen LOLMU, 14 Tage nach Applikation**

| Wirkstoff | Dosierung [g a.i./ha] | Wirkung [%] |
|---|---|---|
| A2-1 | 12,5 | 0 |
| fluroxypyr | 80 | 0 |
| A2-1 + fluroxypyr | 12,5 + 80 | 20 (Ec = 0, Diff. = +20) |

**Tabelle 284: Wirkung gegen PHAMI, 14 Tage nach Applikation**

| Wirkstoff | Dosierung [g a.i./ha] | Wirkung [%] |
|---|---|---|
| A2-1 | 12,5 | 15 |
| fluroxypyr | 80 | 15 |
| A2-1 + fluroxypyr | 12,5 + 80 | 35 (Ec = 27,75, Diff. = +7,25) |

**Tabelle 285: Wirkung gegen POAAN, 14 Tage nach Applikation**

| Wirkstoff | Dosierung [g a.i./ha] | Wirkung [%] |
|---|---|---|
| A2-1 | 12,5 | 15 |
| fluroxypyr | 20 | 0 |
| A2-1 + fluroxypyr | 12,5 + 20 | 20 (Ec = 15, Diff. = +5) |

**Tabelle 286: Wirkung gegen POLCO, 14 Tage nach Applikation**

| Wirkstoff | Dosierung [g a.i./ha] | Wirkung [%] |
|---|---|---|
| A2-1 | 50 | 70 |
| fluroxypyr | 20 | 70 |
| A2-1 + fluroxypyr | 50 + 20 | 100 (Ec = 91, Diff. = +9) |

**Tabelle 287: Wirkung gegen AVEFA, 14 Tage nach Applikation**

| Wirkstoff | Dosierung [g a.i./ha] | Wirkung [%] |
|---|---|---|
| A1-55 | 2,5 | 0 |
| glufosinate-ammonium | 100 | 25 |
| A1-55 + glufosinate-ammonium | 2,5 + 100 | 40 (Ec = 25, Diff. = + 15) |

**Tabelle 288: Wirkung gegen AVEFA, 14 Tage nach Applikation**

| Wirkstoff | Dosierung [g a.i./ha] | Wirkung [%] |
|---|---|---|
| A1-55 | 2,5 | 0 |
| glufosinate-ammonium | 25 | 0 |
| A1-55 + glufosinate-ammonium | 2,5 + 25 | 10 (Ec = 0, Diff. = + 10) |

**Tabelle 289: Wirkung gegen BROST, 14 Tage nach Applikation**

| Wirkstoff | Dosierung [g a.i./ha] | Wirkung [%] |
|---|---|---|
| A1-55 | 2,5 | 5 |
| glufosinate-ammonium | 100 | 15 |
| A1-55 + glufosinate-ammonium | 2,5 + 100 | 40 (Ec = 19,25, Diff. = +20,75) |

**Tabelle 290: Wirkung gegen LOLMU, 14 Tage nach Applikation**

| Wirkstoff | Dosierung [g a.i./ha] | Wirkung [%] |
|---|---|---|
| A1-55 | 10 | 15 |
| glufosinate-ammonium | 25 | 0 |
| A1-55 + glufosinate-ammonium | 10 + 25 | 20 (Ec = 15, Diff. = +5) |

**Tabelle 291: Wirkung gegen LOLMU, 14 Tage nach Applikation**

| Wirkstoff | Dosierung [g a.i./ha] | Wirkung [%] |
|---|---|---|
| A1-55 | 2,5 | 0 |
| glufosinate-ammonium | 100 | 25 |
| A1-55 + glufosinate-ammonium | 2,5 + 100 | 40 (Ec = 25, Diff. = + 15) |

**Tabelle 292: Wirkung gegen LOLMU, 14 Tage nach Applikation**

| Wirkstoff | Dosierung [g a.i./ha] | Wirkung [%] |
|---|---|---|
| A1-55 | 2,5 | 0 |
| glufosinate-ammonium | 25 | 0 |
| A1-55 + glufosinate-ammonium | 2,5 + 25 | 15 (Ec = 0, Diff. = +15) |

**Tabelle 293: Wirkung gegen PHAMI, 14 Tage nach Applikation**

| Wirkstoff | Dosierung [g a.i./ha] | Wirkung [%] |
|---|---|---|
| A1-55 | 10 | 25 |
| glufosinate-ammonium | 100 | 15 |
| A1-55 + glufosinate-ammonium | 10 + 100 | 65 (Ec = 36,25, Diff. = +28,75) |

**Tabelle 294: Wirkung gegen PHAMI, 14 Tage nach Applikation**

| Wirkstoff | Dosierung [g a.i./ha] | Wirkung [%] |
|---|---|---|
| A1-55 | 10 | 25 |
| glufosinate-ammonium | 25 | 0 |
| A1-55 + glufosinate-ammonium | 10 + 25 | 50 (Ec = 25, Diff. = +25) |

**Tabelle 295: Wirkung gegen PHAMI, 14 Tage nach Applikation**

| Wirkstoff | Dosierung [g a.i./ha] | Wirkung [%] |
|---|---|---|
| A1-55 | 2,5 | 0 |
| glufosinate-ammonium | 100 | 15 |
| A1-55 + glufosinate-ammonium | 2,5 + 100 | 65 (Ec = 15, Diff. = +50) |

**Tabelle 296: Wirkung gegen PHAMI, 14 Tage nach Applikation**

| Wirkstoff | Dosierung [g a.i./ha] | Wirkung [%] |
|---|---|---|
| A1-55 | 2,5 | 0 |
| glufosinate-ammonium | 25 | 0 |
| A1-55 + glufosinate-ammonium | 2,5 + 25 | 40 (Ec = 0, Diff. = +40) |

**Tabelle 297: Wirkung gegen POAAN, 14 Tage nach Applikation**

| Wirkstoff | Dosierung [g a.i./ha] | Wirkung [%] |
|---|---|---|
| A1-55 | 10 | 20 |
| glufosinate-ammonium | 100 | 0 |
| A1-55 + glufosinate-ammonium | 10 + 100 | 40 (Ec = 20, Diff. = +20) |

**Tabelle 298: Wirkung gegen POAAN, 14 Tage nach Applikation**

| Wirkstoff | Dosierung [g a.i./ha] | Wirkung [%] |
|---|---|---|
| A1-55 | 2,5 | 5 |
| glufosinate-ammonium | 100 | 0 |
| A1-55 + glufosinate-ammonium | 2,5 + 100 | 30 (Ec = 5, Diff. = +25) |

**Tabelle 299: Wirkung gegen GALAP, 14 Tage nach Applikation**

| Wirkstoff | Dosierung [g a.i./ha] | Wirkung [%] |
|---|---|---|
| A1-55 | 10 | 30 |
| glufosinate-ammonium | 25 | 0 |
| A1-55 + glufosinate-ammonium | 10 + 25 | 60 (Ec = 30, Diff. = +30) |

**Tabelle 300: Wirkung gegen GALAP, 14 Tage nach Applikation**

| Wirkstoff | Dosierung [g a.i./ha] | Wirkung [%] |
|---|---|---|
| A1-55 | 2,5 | 0 |
| glufosinate-ammonium | 25 | 0 |
| A1-55 + glufosinate-ammonium | 2,5 + 25 | 30 (Ec = 0, Diff. = +30) |

**Tabelle 301: Wirkung gegen MATIN, 14 Tage nach Applikation**

| Wirkstoff | Dosierung [g a.i./ha] | Wirkung [%] |
|---|---|---|
| A1-55 | 10 | 30 |
| glufosinate-ammonium | 100 | 30 |
| A1-55 + glufosinate-ammonium | 10 + 100 | 95 (Ec = 51, Diff. = +44) |

**Tabelle 302: Wirkung gegen MATIN, 14 Tage nach Applikation**

| Wirkstoff | Dosierung [g a.i./ha] | Wirkung [%] |
|---|---|---|
| A1-55 | 10 | 30 |
| glufosinate-ammonium | 25 | 0 |
| A1-55 + glufosinate-ammonium | 10 + 25 | 95 (Ec = 30, Diff. = +65) |

**Tabelle 303: Wirkung gegen MATIN, 14 Tage nach Applikation**

| Wirkstoff | Dosierung [g a.i./ha] | Wirkung [%] |
|---|---|---|
| A1-55 | 2,5 | 0 |
| glufosinate-ammonium | 100 | 30 |
| A1-55 + glufosinate-ammonium | 2,5 + 100 | 80 (Ec = 30, Diff. = +50) |

**Tabelle 304: Wirkung gegen MATIN, 14 Tage nach Applikation**

| Wirkstoff | Dosierung [g a.i./ha] | Wirkung [%] |
|---|---|---|
| A1-55 | 2,5 | 0 |
| glufosinate-ammonium | 25 | 0 |
| A1-55 + glufosinate-ammonium | 2,5 + 25 | 70 (Ec = 0, Diff. = +70) |

**Tabelle 305: Wirkung gegen POLCO, 14 Tage nach Applikation**

| Wirkstoff | Dosierung [g a.i./ha] | Wirkung [%] |
|---|---|---|
| A1-55 | 10 | 0 |
| glufosinate-ammonium | 100 | 25 |
| A1-55 + glufosinate-ammonium | 10 + 100 | 50 (Ec = 25, Diff. = +25) |

**Tabelle 306: Wirkung gegen POLCO, 14 Tage nach Applikation**

| Wirkstoff | Dosierung [g a.i./ha] | Wirkung [%] |
|---|---|---|
| A1-55 | 10 | 0 |
| glufosinate-ammonium | 25 | 0 |
| A1-55 + glufosinate-ammonium | 10 + 25 | 70 (Ec = 0, Diff. = +70) |

**Tabelle 307: Wirkung gegen POLCO, 14 Tage nach Applikation**

| Wirkstoff | Dosierung [g a.i./ha] | Wirkung [%] |
|---|---|---|
| A1-55 | 2,5 | 0 |
| glufosinate-ammonium | 100 | 25 |
| A1-55 + glufosinate-ammonium | 2,5 + 100 | 50 (Ec = 25, Diff. = +25) |

**Tabelle 308: Wirkung gegen POLCO, 14 Tage nach Applikation**

| Wirkstoff | Dosierung [g a.i./ha] | Wirkung [%] |
|---|---|---|
| A1-55 | 2,5 | 0 |
| glufosinate-ammonium | 25 | 0 |
| A1-55 + glufosinate-ammonium | 2,5 + 25 | 15 (Ec = 0, Diff. = +15) |

**Tabelle 309: Wirkung gegen AVEFA, 14 Tage nach Applikation**

| Wirkstoff | Dosierung [g a.i./ha] | Wirkung [%] |
|---|---|---|
| A1-57 | 50 | 0 |
| aclonifen | 800 | 0 |
| A1-57 + aclonifen | 50 + 800 | 50 (Ec = 0, Diff. = +50) |

**Tabelle 310: Wirkung gegen AVEFA, 14 Tage nach Applikation**

| Wirkstoff | Dosierung [g a.i./ha] | Wirkung [%] |
|---|---|---|
| A1-57 | 50 | 0 |
| aclonifen | 200 | 0 |
| A1-57 + aclonifen | 50 + 200 | 20 (Ec = 0, Diff. = +20) |

**Tabelle 311: Wirkung gegen AVEFA, 14 Tage nach Applikation**

| Wirkstoff | Dosierung [g a.i./ha] | Wirkung [%] |
|---|---|---|
| A1-57 | 12,5 | 0 |
| aclonifen | 800 | 0 |
| A1-57 + aclonifen | 12,5 + 800 | 35 (Ec = 0, Diff. = +35) |

**Tabelle 312: Wirkung gegen AVEFA, 14 Tage nach Applikation**

| Wirkstoff | Dosierung [g a.i./ha] | Wirkung [%] |
|---|---|---|
| A1-57 | 12,5 | 0 |
| aclonifen | 200 | 0 |
| A1-57 + aclonifen | 12,5 + 200 | 5 (Ec = 0, Diff. = +5) |

**Tabelle 313: Wirkung gegen BROST, 14 Tage nach Applikation**

| Wirkstoff | Dosierung [g a.i./ha] | Wirkung [%] |
|---|---|---|
| A1-57 | 50 | 40 |
| aclonifen | 800 | 0 |
| A1-57 + aclonifen | 50 + 800 | 50 (Ec = 40, Diff. = + 10) |

**Tabelle 314: Wirkung gegen BROST, 14 Tage nach Applikation**

| Wirkstoff | Dosierung [g a.i./ha] | Wirkung [%] |
|---|---|---|
| A1-57 | 12,5 | 0 |
| aclonifen | 800 | 0 |
| A1-57 + aclonifen | 12,5 + 800 | 40 (Ec = 0, Diff. = +40) |

**Tabelle 315: Wirkung gegen BROST, 14 Tage nach Applikation**

| Wirkstoff | Dosierung [g a.i./ha] | Wirkung [%] |
|---|---|---|
| A1-57 | 12,5 | 0 |
| aclonifen | 200 | 0 |
| A1-57 + aclonifen | 12,5 + 200 | 10 (Ec = 0, Diff. = + 10) |

**Tabelle 316: Wirkung gegen LOLMU, 14 Tage nach Applikation**

| Wirkstoff | Dosierung [g a.i./ha] | Wirkung [%] |
|---|---|---|
| A1-57 | 50 | 35 |
| aclonifen | 800 | 0 |
| A1-57 + aclonifen | 50 + 800 | 40 (Ec = 35, Diff. = +5) |

**Tabelle 317: Wirkung gegen LOLMU, 14 Tage nach Applikation**

| Wirkstoff | Dosierung [g a.i./ha] | Wirkung [%] |
|---|---|---|
| A1-57 | 12,5 | 0 |
| aclonifen | 800 | 0 |
| A1-57 + aclonifen | 12,5 + 800 | 10 (Ec = 0, Diff. = + 10) |

**Tabelle 318: Wirkung gegen PHAMI, 14 Tage nach Applikation**

| Wirkstoff | Dosierung [g a.i./ha] | Wirkung [%] |
|---|---|---|
| A1-57 | 50 | 70 |
| aclonifen | 200 | 0 |
| A1-57 + aclonifen | 50 + 200 | 85 (Ec = 70, Diff. = + 15) |

**Tabelle 319: Wirkung gegen PHAMI, 14 Tage nach Applikation**

| Wirkstoff | Dosierung [g a.i./ha] | Wirkung [%] |
|---|---|---|
| A1-57 | 12,5 | 50 |
| aclonifen | 800 | 40 |
| A1-57 + aclonifen | 12,5 + 800 | 85 (Ec = 70, Diff. = + 15) |

**Tabelle 320: Wirkung gegen PHAMI, 14 Tage nach Applikation**

| Wirkstoff | Dosierung [g a.i./ha] | Wirkung [%] |
|---|---|---|
| A1-57 | 12,5 | 50 |
| aclonifen | 200 | 0 |
| A1-57 + aclonifen | 12,5 + 200 | 60 (Ec = 50, Diff. = + 10) |

**Tabelle 321: Wirkung gegen POAAN, 14 Tage nach Applikation**

| Wirkstoff | Dosierung [g a.i./ha] | Wirkung [%] |
|---|---|---|
| A1-57 | 50 | 50 |
| aclonifen | 800 | 30 |
| A1-57 + aclonifen | 50 + 800 | 85 (Ec = 65, Diff. = +20) |

**Tabelle 322: Wirkung gegen POAAN, 14 Tage nach Applikation**

| Wirkstoff | Dosierung [g a.i./ha] | Wirkung [%] |
|---|---|---|
| A1-57 | 50 | 50 |
| aclonifen | 200 | 0 |
| A1-57 + aclonifen | 50 + 200 | 80 (Ec = 50, Diff. = +30) |

**Tabelle 323: Wirkung gegen POAAN, 14 Tage nach Applikation**

| Wirkstoff | Dosierung [g a.i./ha] | Wirkung [%] |
|---|---|---|
| A1-57 | 12,5 | 15 |
| aclonifen | 800 | 30 |
| A1-57 + aclonifen | 12,5 + 800 | 70 (Ec = 40,5, Diff. = +29,5) |

**Tabelle 324: Wirkung gegen POAAN, 14 Tage nach Applikation**

| Wirkstoff | Dosierung [g a.i./ha] | Wirkung [%] |
|---|---|---|
| A1-57 | 12,5 | 15 |
| aclonifen | 200 | 0 |
| A1-57 + aclonifen | 12,5 + 200 | 45 (Ec = 15, Diff. = +30) |

**Tabelle 325: Wirkung gegen GALAP, 14 Tage nach Applikation**

| Wirkstoff | Dosierung [g a.i./ha] | Wirkung [%] |
|---|---|---|
| A1-57 | 50 | 60 |
| aclonifen | 200 | 20 |
| A1-57 + aclonifen | 50 + 200 | 80 (Ec = 68, Diff. = + 12) |

**Tabelle 326: Wirkung gegen GALAP, 14 Tage nach Applikation**

| Wirkstoff | Dosierung [g a.i./ha] | Wirkung [%] |
|---|---|---|
| A1-57 | 12,5 | 30 |
| aclonifen | 200 | 20 |
| A1-57 + aclonifen | 12,5 + 200 | 65 (Ec = 44, Diff. = +21) |

**Tabelle 327: Wirkung gegen MATIN, 14 Tage nach Applikation**

| Wirkstoff | Dosierung [g a.i./ha] | Wirkung [%] |
|---|---|---|
| A1-57 | 50 | 85 |
| aclonifen | 800 | 0 |
| A1-57 + aclonifen | 50 + 800 | 95 (Ec = 85, Diff. = + 10) |

**Tabelle 328: Wirkung gegen MATIN, 14 Tage nach Applikation**

| Wirkstoff | Dosierung [g a.i./ha] | Wirkung [%] |
|---|---|---|
| A1-57 | 12,5 | 60 |
| aclonifen | 800 | 0 |
| A1-57 + aclonifen | 12,5 + 800 | 93 (Ec = 60, Diff. = +33) |

**Tabelle 329: Wirkung gegen MATIN, 14 Tage nach Applikation**

| Wirkstoff | Dosierung [g a.i./ha] | Wirkung [%] |
|---|---|---|
| A1-57 | 12,5 | 60 |
| aclonifen | 200 | 0 |
| A1-57 + aclonifen | 12,5 + 200 | 75 (Ec = 60, Diff. = + 15) |

**Tabelle 330: Wirkung gegen POLCO, 14 Tage nach Applikation**

| Wirkstoff | Dosierung [g a.i./ha] | Wirkung [%] |
|---|---|---|
| A1-57 | 12,5 | 65 |
| aclonifen | 800 | 10 |
| A1-57 + aclonifen | 12,5 + 800 | 85 (Ec = 68,5, Diff. = +16,5) |

**Tabelle 331: Wirkung gegen POLCO, 14 Tage nach Applikation**

| Wirkstoff | Dosierung [g a.i./ha] | Wirkung [%] |
|---|---|---|
| A1-57 | 12,5 | 65 |
| aclonifen | 200 | 0 |
| A1-57 + aclonifen | 12,5 + 200 | 85 (Ec = 65, Diff. = +20) |

**Tabelle 332: Wirkung gegen AVEFA, 14 Tage nach Applikation**

| Wirkstoff | Dosierung [g a.i./ha] | Wirkung [%] |
|---|---|---|
| A1-7 | 2,5 | 15 |
| saflufenacil | 2 | 0 |
| A1-7 + saflufenacil | 2,5 + 2 | 20 (Ec = 15, Diff. = +5) |

**Tabelle 333: Wirkung gegen LOLMU, 14 Tage nach Applikation**

| Wirkstoff | Dosierung [g a.i./ha] | Wirkung [%] |
|---|---|---|
| A1-7 | 2,5 | 0 |
| saflufenacil | 8 | 10 |
| A1-7 + saflufenacil | 2,5 + 8 | 15 (Ec = 10, Diff. = +5) |

**Tabelle 334: Wirkung gegen LOLMU, 14 Tage nach Applikation**

| Wirkstoff | Dosierung [g a.i./ha] | Wirkung [%] |
|---|---|---|
| A1-7 | 2,5 | 0 |
| saflufenacil | 2 | 0 |
| A1-7 + saflufenacil | 2,5 + 2 | 10 (Ec = 0, Diff. = + 10) |

**Tabelle 335: Wirkung gegen POLCO, 14 Tage nach Applikation**

| Wirkstoff | Dosierung [g a.i./ha] | Wirkung [%] |
|---|---|---|
| A1-7 | 10 | 25 |
| saflufenacil | 8 | 10 |
| A1-7 + saflufenacil | 10+8 | 80 (Ec = 32,5, Diff. = +47,5) |

**Tabelle 336: Wirkung gegen POLCO, 14 Tage nach Applikation**

| Wirkstoff | Dosierung [g a.i./ha] | Wirkung [%] |
|---|---|---|
| A1-7 | 10 | 25 |
| saflufenacil | 2 | 0 |
| A1-7 + saflufenacil | 10+2 | 75 (Ec = 25, Diff. = +50) |

**Tabelle 337: Wirkung gegen POLCO, 14 Tage nach Applikation**

| Wirkstoff | Dosierung [g a.i./ha] | Wirkung [%] |
|---|---|---|
| A1-7 | 2,5 | 10 |
| saflufenacil | 8 | 10 |
| A1-7 + saflufenacil | 2,5 + 8 | 93 (Ec = 19, Diff. = +74) |

**Tabelle 338: Wirkung gegen POLCO, 14 Tage nach Applikation**

| Wirkstoff | Dosierung [g a.i./ha] | Wirkung [%] |
|---|---|---|
| A1-7 | 2,5 | 10 |
| saflufenacil | 2 | 0 |
| A1-7 + saflufenacil | 2,5 + 2 | 75 (Ec = 10, Diff. = +65) |

**Tabelle 339: Wirkung gegen AVEFA, 14 Tage nach Applikation**

| Wirkstoff | Dosierung [g a.i./ha] | Wirkung [%] |
|---|---|---|
| A1-60 | 10 | 0 |
| isoproturon | 1600 | 35 |
| A1-60 + isoproturon | 10 + 1600 | 50 (Ec = 35, Diff. = + 15) |

**Tabelle 340: Wirkung gegen AVEFA, 14 Tage nach Applikation**

| Wirkstoff | Dosierung [g a.i./ha] | Wirkung [%] |
|---|---|---|
| A1-60 | 10 | 0 |
| isoproturon | 400 | 0 |
| A1-60 + isoproturon | 10 + 400 | 50 (Ec = 0, Diff. = +50) |

**Tabelle 341: Wirkung gegen AVEFA, 14 Tage nach Applikation**

| Wirkstoff | Dosierung [g a.i./ha] | Wirkung [%] |
|---|---|---|
| A1-60 | 2,5 | 0 |
| isoproturon | 400 | 0 |
| A1-60 + isoproturon | 2,5 + 400 | 15 (Ec = 0, Diff. = +15) |

**Tabelle 342: Wirkung gegen BROST, 14 Tage nach Applikation**

| Wirkstoff | Dosierung [g a.i./ha] | Wirkung [%] |
|---|---|---|
| A1-60 | 10 | 15 |
| isoproturon | 1600 | 5 |
| A1-60 + isoproturon | 10 + 1600 | 60 (Ec = 19,25, Diff. = +40,75) |

**Tabelle 343: Wirkung gegen BROST, 14 Tage nach Applikation**

| Wirkstoff | Dosierung [g a.i./ha] | Wirkung [%] |
|---|---|---|
| A1-60 | 10 | 15 |
| isoproturon | 400 | 0 |
| A1-60 + isoproturon | 10 + 400 | 40 (Ec = 15, Diff. = +25) |

**Tabelle 344: Wirkung gegen BROST, 14 Tage nach Applikation**

| Wirkstoff | Dosierung [g a.i./ha] | Wirkung [%] |
|---|---|---|
| A1-60 | 2,5 | 0 |
| isoproturon | 1600 | 5 |
| A1-60 + isoproturon | 2,5 + 1600 | 20 (Ec = 5, Diff. = + 15) |

**Tabelle 345: Wirkung gegen BROST, 14 Tage nach Applikation**

| Wirkstoff | Dosierung [g a.i./ha] | Wirkung [%] |
|---|---|---|
| A1-60 | 2,5 | 0 |
| isoproturon | 400 | 0 |
| A1-60 + isoproturon | 2,5 + 400 | 15 (Ec = 0, Diff. = +15) |

**Tabelle 346: Wirkung gegen LOLMU, 14 Tage nach Applikation**

| Wirkstoff | Dosierung [g a.i./ha] | Wirkung [%] |
|---|---|---|
| A1-60 | 10 | 0 |
| isoproturon | 1600 | 15 |
| A1-60 + isoproturon | 10 + 1600 | 50 (Ec = 15, Diff. = +35) |

**Tabelle 347: Wirkung gegen LOLMU, 14 Tage nach Applikation**

| Wirkstoff | Dosierung [g a.i./ha] | Wirkung [%] |
|---|---|---|
| A1-60 | 10 | 0 |
| isoproturon | 400 | 0 |
| A1-60 + isoproturon | 10 + 400 | 30 (Ec = 0, Diff. = +30) |

**Tabelle 348: Wirkung gegen LOLMU, 14 Tage nach Applikation**

| Wirkstoff | Dosierung [g a.i./ha] | Wirkung [%] |
|---|---|---|
| A1-60 | 2,5 | 0 |
| isoproturon | 1600 | 15 |
| A1-60 + isoproturon | 2,5 + 1600 | 25 (Ec = 15, Diff. = + 10) |

**Tabelle 349: Wirkung gegen LOLMU, 14 Tage nach Applikation**

| Wirkstoff | Dosierung [g a.i./ha] | Wirkung [%] |
|---|---|---|
| A1-60 | 2,5 | 0 |
| isoproturon | 400 | 0 |
| A1-60 + isoproturon | 2,5 + 400 | 15 (Ec = 0, Diff. = +15) |

**Tabelle 350: Wirkung gegen PHAMI, 14 Tage nach Applikation**

| Wirkstoff | Dosierung [g a.i./ha] | Wirkung [%] |
|---|---|---|
| A1-60 | 10 | 25 |
| isoproturon | 1600 | 80 |
| A1-60 + isoproturon | 10 + 1600 | 95 (Ec = 85, Diff. = + 10) |

**Tabelle 351: Wirkung gegen PHAMI, 14 Tage nach Applikation**

| Wirkstoff | Dosierung [g a.i./ha] | Wirkung [%] |
|---|---|---|
| A1-60 | 10 | 25 |
| isoproturon | 400 | 35 |
| A1-60 + isoproturon | 10 + 400 | 85 (Ec = 51,25, Diff. = +33,75) |

**Tabelle 352: Wirkung gegen PHAMI, 14 Tage nach Applikation**

| Wirkstoff | Dosierung [g a.i./ha] | Wirkung [%] |
|---|---|---|
| A1-60 | 2,5 | 10 |
| isoproturon | 400 | 35 |
| A1-60 + isoproturon | 2,5 + 400 | 50 (Ec = 41,5, Diff. = +8,5) |

**Tabelle 353: Wirkung gegen POAAN, 14 Tage nach Applikation**

| Wirkstoff | Dosierung [g a.i./ha] | Wirkung [%] |
|---|---|---|
| A1-60 | 10 | 10 |
| isoproturon | 1600 | 80 |
| A1-60 + isoproturon | 10 + 1600 | 90 (Ec = 82, Diff. = +8) |

**Tabelle 354: Wirkung gegen POAAN, 14 Tage nach Applikation**

| Wirkstoff | Dosierung [g a.i./ha] | Wirkung [%] |
|---|---|---|
| A1-60 | 10 | 10 |
| isoproturon | 400 | 60 |
| A1-60 + isoproturon | 10 + 400 | 95 (Ec = 64, Diff. = +31) |

**Tabelle 355: Wirkung gegen POAAN, 14 Tage nach Applikation**

| Wirkstoff | Dosierung [g a.i./ha] | Wirkung [%] |
|---|---|---|
| A1-60 | 2,5 | 0 |
| isoproturon | 1600 | 80 |
| A1-60 + isoproturon | 2,5 + 1600 | 85 (Ec = 80, Diff. = +5) |

**Tabelle 356: Wirkung gegen GALAP, 14 Tage nach Applikation**

| Wirkstoff | Dosierung [g a.i./ha] | Wirkung [%] |
|---|---|---|
| A1-60 | 10 | 30 |
| isoproturon | 1600 | 20 |
| A1-60 + isoproturon | 10 + 1600 | 65 (Ec = 44, Diff. = +21) |

**Tabelle 357: Wirkung gegen GALAP, 14 Tage nach Applikation**

| Wirkstoff | Dosierung [g a.i./ha] | Wirkung [%] |
|---|---|---|
| A1-60 | 10 | 30 |
| isoproturon | 400 | 10 |
| A1-60 + isoproturon | 10 + 400 | 65 (Ec = 37, Diff. = +28) |

**Tabelle 358: Wirkung gegen GALAP, 14 Tage nach Applikation**

| Wirkstoff | Dosierung [g a.i./ha] | Wirkung [%] |
|---|---|---|
| A1-60 | 2,5 | 25 |
| isoproturon | 1600 | 20 |
| A1-60 + isoproturon | 2,5 + 1600 | 65 (Ec = 40, Diff. = +25) |

**Tabelle 359: Wirkung gegen GALAP, 14 Tage nach Applikation**

| Wirkstoff | Dosierung [g a.i./ha] | Wirkung [%] |
|---|---|---|
| A1-60 | 2,5 | 25 |
| isoproturon | 400 | 10 |
| A1-60 + isoproturon | 2,5 + 400 | 60 (Ec = 32,5, Diff. = +27,5) |

**Tabelle 360: Wirkung gegen PHAMI, 14 Tage nach Applikation**

| Wirkstoff | Dosierung [g a.i./ha] | Wirkung [%] |
|---|---|---|
| A1-13 | 10 | 30 |
| indaziflam | 40 | 10 |
| A1-13 + indaziflam | 10 + 40 | 60 (Ec = 37, Diff. = +23) |

**Tabelle 361: Wirkung gegen PHAMI, 14 Tage nach Applikation**

| Wirkstoff | Dosierung [g a.i./ha] | Wirkung [%] |
|---|---|---|
| A1-13 | 2,5 | 30 |
| indaziflam | 40 | 10 |
| A1-13 + indaziflam | 2,5 + 40 | 70 (Ec = 37, Diff. = +33) |

**Tabelle 362: Wirkung gegen GALAP, 14 Tage nach Applikation**

| Wirkstoff | Dosierung [g a.i./ha] | Wirkung [%] |
|---|---|---|
| A1-13 | 10 | 50 |
| indaziflam | 40 | 60 |
| A1-13 + indaziflam | 10 + 40 | 95 (Ec = 80, Diff. = + 15) |

**Tabelle 363: Wirkung gegen GALAP, 14 Tage nach Applikation**

| Wirkstoff | Dosierung [g a.i./ha] | Wirkung [%] |
|---|---|---|
| A1-13 | 2,5 | 40 |
| indaziflam | 10 | 50 |
| A1-13 + indaziflam | 2,5 + 10 | 75 (Ec = 70, Diff. = +5) |

**Tabelle 364: Wirkung gegen MATIN, 14 Tage nach Applikation**

| Wirkstoff | Dosierung [g a.i./ha] | Wirkung [%] |
|---|---|---|
| A1-13 | 10 | 40 |
| indaziflam | 10 | 30 |
| A1-13 + indaziflam | 10 + 10 | 70 (Ec = 58, Diff. = +12) |

**Tabelle 365: Wirkung gegen MATIN, 14 Tage nach Applikation**

| Wirkstoff | Dosierung [g a.i./ha] | Wirkung [%] |
|---|---|---|
| A1-13 | 2,5 | 30 |
| indaziflam | 10 | 30 |
| A1-13 + indaziflam | 2,5 + 10 | 70 (Ec = 51, Diff. = +19) |

**Tabelle 366: Wirkung gegen LOLMU, 21 Tage nach Applikation**

| Wirkstoff | Dosierung [g a.i./ha] | Wirkung [%] |
|---|---|---|
| A1-3 | 20 | 5 |
| pinoxaden | 5 | 30 |
| A1-3 + pinoxaden | 20 + 5 | 40 (Ec = 33,5, Diff. = +6,5) |

**Tabelle 367: Wirkung gegen LOLMU, 21 Tage nach Applikation**

| Wirkstoff | Dosierung [g a.i./ha] | Wirkung [%] |
|---|---|---|
| A1-3 | 5 | 0 |
| pinoxaden | 20 | 80 |
| A1-3 + pinoxaden | 5 + 20 | 90 (Ec = 80, Diff. = +10) |

**Tabelle 368: Wirkung gegen PHAMI, 21 Tage nach Applikation**

| Wirkstoff | Dosierung [g a.i./ha] | Wirkung [%] |
|---|---|---|
| A1-3 | 20 | 5 |
| pinoxaden | 20 | 20 |
| A1-3 + pinoxaden | 20 + 20 | 80 (Ec = 24, Diff. = +56) |

**Tabelle 369: Wirkung gegen PHAMI, 21 Tage nach Applikation**

| Wirkstoff | Dosierung [g a.i./ha] | Wirkung [%] |
|---|---|---|
| A1-3 | 20 | 5 |
| pinoxaden | 5 | 10 |
| A1-3 + pinoxaden | 20 + 5 | 20 (Ec = 14,5, Diff. = +5,5) |

**Tabelle 370: Wirkung gegen POAAN, 21 Tage nach Applikation**

| Wirkstoff | Dosierung [g a.i./ha] | Wirkung [%] |
|---|---|---|
| A1-3 | 20 | 3 |
| pinoxaden | 20 | 0 |
| A1-3 + pinoxaden | 20 + 20 | 20 (Ec = 3, Diff. = +17) |

**Tabelle 371: Wirkung gegen POAAN, 21 Tage nach Applikation**

| Wirkstoff | Dosierung [g a.i./ha] | Wirkung [%] |
|---|---|---|
| A1-3 | 5 | 0 |
| pinoxaden | 20 | 0 |
| A1-3 + pinoxaden | 5 + 20 | 15 (Ec = 0, Diff. = +15) |

**Tabelle 372: Wirkung gegen GALAP, 21 Tage nach Applikation**

| Wirkstoff | Dosierung [g a.i./ha] | Wirkung [%] |
|---|---|---|
| A1-3 | 20 | 5 |
| pinoxaden | 20 | 0 |
| A1-3 + pinoxaden | 20 + 20 | 30 (Ec = 5, Diff. = +25) |

**Tabelle 373: Wirkung gegen GALAP, 21 Tage nach Applikation**

| Wirkstoff | Dosierung [g a.i./ha] | Wirkung [%] |
|---|---|---|
| A1-3 | 20 | 5 |
| pinoxaden | 5 | 0 |
| A1-3 + pinoxaden | 20 + 5 | 30 (Ec = 5, Diff. = +25) |

**Tabelle 374: Wirkung gegen GALAP, 21 Tage nach Applikation**

| Wirkstoff | Dosierung [g a.i./ha] | Wirkung [%] |
|---|---|---|
| A1-3 | 5 | 5 |
| pinoxaden | 20 | 0 |
| A1-3 + pinoxaden | 5 + 20 | 20 (Ec = 5, Diff. = +15) |

**Tabelle 375: Wirkung gegen POLCO, 21 Tage nach Applikation**

| Wirkstoff | Dosierung [g a.i./ha] | Wirkung [%] |
|---|---|---|
| A1-3 | 20 | 5 |
| pinoxaden | 20 | 0 |
| A1-3 + pinoxaden | 20 + 20 | 15 (Ec = 5, Diff. = +10) |

**Tabelle 376: Wirkung gegen POLCO, 21 Tage nach Applikation**

| Wirkstoff | Dosierung [g a.i./ha] | Wirkung [%] |
|---|---|---|
| A1-3 | 20 | 5 |
| pinoxaden | 5 | 0 |
| A1-3 + pinoxaden | 20 + 5 | 15 (Ec = 5, Diff. = +10) |

**Tabelle 377: Wirkung gegen AVEFA, 21 Tage nach Applikation**

| Wirkstoff | Dosierung [g a.i./ha] | Wirkung [%] |
|---|---|---|
| A1-13 | 10 | 3 |
| mesosulfuron-methyl | 10 | 0 |
| A1-13 + mesosulfuron-methyl | 10 + 10 | 15 (Ec = 3, Diff. = +12) |

**Tabelle 378: Wirkung gegen AVEFA, 21 Tage nach Applikation**

| Wirkstoff | Dosierung [g a.i./ha] | Wirkung [%] |
|---|---|---|
| A1-13 | 2,5 | 0 |
| mesosulfuron-methyl | 10 | 0 |
| A1-13 + mesosulfuron-methyl | 2,5 + 10 | 15 (Ec = 0, Diff. = +15) |

**Tabelle 379: Wirkung gegen BROST, 21 Tage nach Applikation**

| Wirkstoff | Dosierung [g a.i./ha] | Wirkung [%] |
|---|---|---|
| A1-13 | 10 | 5 |
| mesosulfuron-methyl | 10 | 0 |
| A1-13 + mesosulfuron-methyl | 10 + 10 | 20 (Ec = 5, Diff. = +15) |

**Tabelle 380: Wirkung gegen BROST, 21 Tage nach Applikation**

| Wirkstoff | Dosierung [g a.i./ha] | Wirkung [%] |
|---|---|---|
| A1-13 | 2,5 | 0 |
| mesosulfuron-methyl | 10 | 0 |
| A1-13 + mesosulfuron-methyl | 2,5 + 10 | 35 (Ec = 0, Diff. = +35) |

**Tabelle 381: Wirkung gegen BROST, 21 Tage nach Applikation**

| Wirkstoff | Dosierung [g a.i./ha] | Wirkung [%] |
|---|---|---|
| A1-13 | 2,5 | 0 |
| mesosulfuron-methyl | 2,5 | 0 |
| A1-13 + mesosulfuron-methyl | 2,5 + 2,5 | 20 (Ec = 0, Diff. = +20) |

**Tabelle 382: Wirkung gegen LOLMU, 21 Tage nach Applikation**

| Wirkstoff | Dosierung [g a.i./ha] | Wirkung [%] |
|---|---|---|
| A1-13 | 10 | 5 |
| mesosulfuron-methyl | 10 | 15 |
| A1-13 + mesosulfuron-methyl | 10 + 10 | 35 (Ec = 19,25, Diff. = + 15,75) |

**Tabelle 383: Wirkung gegen PHAMI, 21 Tage nach Applikation**

| Wirkstoff | Dosierung [g a.i./ha] | Wirkung [%] |
|---|---|---|
| A1-13 | 10 | 20 |
| mesosulfuron-methyl | 10 | 5 |
| A1-13 + mesosulfuron-methyl | 10 + 10 | 40 (Ec = 24, Diff. = +16) |

**Tabelle 384: Wirkung gegen PHAMI, 21 Tage nach Applikation**

| Wirkstoff | Dosierung [g a.i./ha] | Wirkung [%] |
|---|---|---|
| A1-13 | 2,5 | 15 |
| mesosulfuron-methyl | 10 | 5 |
| A1-13 + mesosulfuron-methyl | 2,5 + 10 | 30 (Ec = 19,25, Diff. = +10,75) |

**Tabelle 385: Wirkung gegen PHAMI, 21 Tage nach Applikation**

| Wirkstoff | Dosierung [g a.i./ha] | Wirkung [%] |
|---|---|---|
| A1-13 | 2,5 | 15 |
| mesosulfuron-methyl | 2,5 | 0 |
| A1-13 + mesosulfuron-methyl | 2,5 + 2,5 | 20 (Ec = 15, Diff. = +5) |

**Tabelle 386: Wirkung gegen POAAN, 21 Tage nach Applikation**

| Wirkstoff | Dosierung [g a.i./ha] | Wirkung [%] |
|---|---|---|
| A1-13 | 2,5 | 10 |
| mesosulfuron-methyl | 10 | 40 |
| A1-13 + mesosulfuron-methyl | 2,5 + 10 | 60 (Ec = 46, Diff. = +14) |

**Tabelle 387: Wirkung gegen POLCO, 21 Tage nach Applikation**

| Wirkstoff | Dosierung [g a.i./ha] | Wirkung [%] |
|---|---|---|
| A1-13 | 10 | 0 |
| mesosulfuron-methyl | 10 | 0 |
| A1-13 + mesosulfuron-methyl | 10 + 10 | 15 (Ec = 0, Diff. = +15) |

**Tabelle 388: Wirkung gegen POLCO, 21 Tage nach Applikation**

| Wirkstoff | Dosierung [g a.i./ha] | Wirkung [%] |
|---|---|---|
| A1-13 | 2,5 | 0 |
| mesosulfuron-methyl | 10 | 0 |
| A1-13 + mesosulfuron-methyl | 2,5 + 10 | 10 (Ec = 0, Diff. = +10) |

**Tabelle 389: Wirkung gegen AVEFA, 21 Tage nach Applikation**

| Wirkstoff | Dosierung [g a.i./ha] | Wirkung [%] |
|---|---|---|
| A1-34 | 200 | 5 |
| iodosulfuron-methyl-sodium | 2 | 5 |
| A1-34 + iodosulfuron-methyl-sodium | 200 + 2 | 15 (Ec = 9,75, Diff. = +5,25) |

**Tabelle 390: Wirkung gegen BROST, 21 Tage nach Applikation**

| Wirkstoff | Dosierung [g a.i./ha] | Wirkung [%] |
|---|---|---|
| A1-34 | 200 | 35 |
| iodosulfuron-methyl-sodium | 2 | 0 |
| A1-34 + iodosulfuron-methyl-sodium | 200 + 2 | 40 (Ec = 35, Diff. = +5) |

**Tabelle 391: Wirkung gegen BROST, 21 Tage nach Applikation**

| Wirkstoff | Dosierung [g a.i./ha] | Wirkung [%] |
|---|---|---|
| A1-34 | 50 | 15 |
| iodosulfuron-methyl-sodium | 2 | 0 |
| A1-34 + iodosulfuron-methyl-sodium | 50 + 2 | 20 (Ec = 15, Diff. = +5) |

**Tabelle 392: Wirkung gegen GALAP, 21 Tage nach Applikation**

| Wirkstoff | Dosierung [g a.i./ha] | Wirkung [%] |
|---|---|---|
| A1-34 | 200 | 40 |
| iodosulfuron-methyl-sodium | 2 | 25 |
| A1-34 + iodosulfuron-methyl-sodium | 200 + 2 | 70 (Ec = 55, Diff. = +15) |

**Tabelle 393: Wirkung gegen GALAP, 21 Tage nach Applikation**

| Wirkstoff | Dosierung [g a.i./ha] | Wirkung [%] |
|---|---|---|
| A1-34 | 50 | 20 |
| iodosulfuron-methyl-sodium | 0,5 | 0 |
| A1-34 + iodosulfuron-methyl-sodium | 50 + 0,5 | 40 (Ec = 20, Diff. = +20) |

**Tabelle 394: Wirkung gegen MATIN, 21 Tage nach Applikation**

| Wirkstoff | Dosierung [g a.i./ha] | Wirkung [%] |
|---|---|---|
| A1-34 | 50 | 50 |
| iodosulfuron-methyl-sodium | 0,5 | 70 |
| A1-34 + iodosulfuron-methyl-sodium | 50 + 0,5 | 90 (Ec = 85, Diff. = +5) |

**Tabelle 395: Wirkung gegen POLCO, 21 Tage nach Applikation**

| Wirkstoff | Dosierung [g a.i./ha] | Wirkung [%] |
|---|---|---|
| A1-34 | 50 | 0 |
| iodosulfuron-methyl-sodium | 2 | 0 |
| A1-34 + iodosulfuron-methyl-sodium | 50 + 2 | 15 (Ec = 0, Diff. = +15) |

**Tabelle 396: Wirkung gegen POLCO, 21 Tage nach Applikation**

| Wirkstoff | Dosierung [g a.i./ha] | Wirkung [%] |
|---|---|---|
| A1-34 | 50 | 0 |
| iodosulfuron-methyl-sodium | 0,5 | 0 |
| A1-34 + iodosulfuron-methyl-sodium | 50 + 0,5 | 15 (Ec = 0, Diff. = +15) |

**Tabelle 397: Wirkung gegen BROST, 21 Tage nach Applikation**

| Wirkstoff | Dosierung [g a.i./ha] | Wirkung [%] |
|---|---|---|
| A1-14 | 10 | 0 |
| pyrasulfotole | 100 | 0 |
| A1-14 + pyrasulfotole | 10 + 100 | 5 (Ec = 0, Diff. = +5) |

**Tabelle 398: Wirkung gegen PHAMI, 21 Tage nach Applikation**

| Wirkstoff | Dosierung [g a.i./ha] | Wirkung [%] |
|---|---|---|
| A1-14 | 2,5 | 0 |
| pyrasulfotole | 100 | 0 |
| A1-14 + pyrasulfotole | 2,5 + 100 | 30 (Ec = 0, Diff. = +30) |

**Tabelle 399: Wirkung gegen PHAMI, 21 Tage nach Applikation**

| Wirkstoff | Dosierung [g a.i./ha] | Wirkung [%] |
|---|---|---|
| A1-14 | 2,5 | 0 |
| pyrasulfotole | 25 | 0 |
| A1-14 + pyrasulfotole | 2,5 + 25 | 20 (Ec = 0, Diff. = +20) |

**Tabelle 400: Wirkung gegen POAAN, 21 Tage nach Applikation**

| Wirkstoff | Dosierung [g a.i./ha] | Wirkung [%] |
|---|---|---|
| A1-14 | 2,5 | 0 |
| pyrasulfotole | 100 | 0 |
| A1-14 + pyrasulfotole | 2,5 + 100 | 15 (Ec = 0, Diff. = +15) |

**Tabelle 401: Wirkung gegen POAAN, 21 Tage nach Applikation**

| Wirkstoff | Dosierung [g a.i./ha] | Wirkung [%] |
|---|---|---|
| A1-14 | 2,5 | 0 |
| pyrasulfotole | 25 | 0 |
| A1-14 + pyrasulfotole | 2,5 + 25 | 15 (Ec = 0, Diff. = +15) |

**Tabelle 402: Wirkung gegen GALAP, 21 Tage nach Applikation**

| Wirkstoff | Dosierung [g a.i./ha] | Wirkung [%] |
|---|---|---|
| A1-14 | 10 | 15 |
| pyrasulfotole | 25 | 40 |
| A1-14 + pyrasulfotole | 10 + 25 | 60 (Ec = 49, Diff. = +11) |

**Tabelle 403: Wirkung gegen GALAP, 21 Tage nach Applikation**

| Wirkstoff | Dosierung [g a.i./ha] | Wirkung [%] |
|---|---|---|
| A1-14 | 2,5 | 0 |
| pyrasulfotole | 25 | 40 |
| A1-14 + pyrasulfotole | 2,5 + 25 | 50 (Ec = 40, Diff. = +10) |

**Tabelle 404: Wirkung gegen MATIN, 21 Tage nach Applikation**

| Wirkstoff | Dosierung [g a.i./ha] | Wirkung [%] |
|---|---|---|
| A1-14 | 2,5 | 30 |
| pyrasulfotole | 100 | 3 |
| A1-14 + pyrasulfotole | 2,5 + 100 | 40 (Ec = 32,1, Diff. = +7,9) |

**Tabelle 405: Wirkung gegen POLCO, 21 Tage nach Applikation**

| Wirkstoff | Dosierung [g a.i./ha] | Wirkung [%] |
|---|---|---|
| A1-14 | 10 | 3 |
| pyrasulfotole | 100 | 30 |
| A1-14 + pyrasulfotole | 10 + 100 | 70 (Ec = 32,1, Diff. = +37,9) |

**Tabelle 406: Wirkung gegen POLCO, 21 Tage nach Applikation**

| Wirkstoff | Dosierung [g a.i./ha] | Wirkung [%] |
|---|---|---|
| A1-14 | 10 | 3 |
| pyrasulfotole | 25 | 0 |
| A1-14 + pyrasulfotole | 10 + 25 | 80 (Ec = 3, Diff. = +77) |

**Tabelle 407: Wirkung gegen POLCO, 21 Tage nach Applikation**

| Wirkstoff | Dosierung [g a.i./ha] | Wirkung [%] |
|---|---|---|
| A1-14 | 2,5 | 0 |
| pyrasulfotole | 100 | 30 |
| A1-14 + pyrasulfotole | 2,5 + 100 | 60 (Ec = 30, Diff. = +30) |

**Tabelle 408: Wirkung gegen POLCO, 21 Tage nach Applikation**

| Wirkstoff | Dosierung [g a.i./ha] | Wirkung [%] |
|---|---|---|
| A1-14 | 2,5 | 0 |
| pyrasulfotole | 25 | 0 |
| A1-14 + pyrasulfotole | 2,5 + 25 | 50 (Ec = 0, Diff. = +50) |

**Tabelle 409: Wirkung gegen AVEFA, 21 Tage nach Applikation**

| Wirkstoff | Dosierung [g a.i./ha] | Wirkung [%] |
|---|---|---|
| A1-36 | 10 | 15 |
| flurtamone | 320 | 0 |
| A1-36 + flurtamone | 10 + 320 | 40 (Ec = 15, Diff. = +25) |

**Tabelle 410: Wirkung gegen AVEFA, 21 Tage nach Applikation**

| Wirkstoff | Dosierung [g a.i./ha] | Wirkung [%] |
|---|---|---|
| A1-36 | 10 | 15 |
| flurtamone | 80 | 0 |
| A1-36 + flurtamone | 10 + 80 | 30 (Ec = 15, Diff. = +15) |

**Tabelle 411: Wirkung gegen AVEFA, 21 Tage nach Applikation**

| Wirkstoff | Dosierung [g a.i./ha] | Wirkung [%] |
|---|---|---|
| A1-36 | 2,5 | 10 |
| flurtamone | 320 | 0 |
| A1-36 + flurtamone | 2,5 + 320 | 30 (Ec = 10, Diff. = +20) |

**Tabelle 412: Wirkung gegen AVEFA, 21 Tage nach Applikation**

| Wirkstoff | Dosierung [g a.i./ha] | Wirkung [%] |
|---|---|---|
| A1-36 | 2,5 | 10 |
| flurtamone | 80 | 0 |
| A1-36 + flurtamone | 2,5 + 80 | 20 (Ec = 10, Diff. = +10) |

**Tabelle 413: Wirkung gegen BROST, 21 Tage nach Applikation**

| Wirkstoff | Dosierung [g a.i./ha] | Wirkung [%] |
|---|---|---|
| A1-36 | 2,5 | 5 |
| flurtamone | 320 | 10 |
| A1-36 + flurtamone | 2,5 + 320 | 20 (Ec = 14,5, Diff. = +5,5) |

**Tabelle 414: Wirkung gegen BROST, 21 Tage nach Applikation**

| Wirkstoff | Dosierung [g a.i./ha] | Wirkung [%] |
|---|---|---|
| A1-36 | 2,5 | 5 |
| flurtamone | 80 | 0 |
| A1-36 + flurtamone | 2,5 + 80 | 15 (Ec = 5, Diff. = +10) |

**Tabelle 415: Wirkung gegen LOLMU, 21 Tage nach Applikation**

| Wirkstoff | Dosierung [g a.i./ha] | Wirkung [%] |
|---|---|---|
| A1-36 | 10 | 0 |
| flurtamone | 80 | 20 |
| A1-36 + flurtamone | 10 + 80 | 60 (Ec = 20, Diff. = +40) |

**Tabelle 416: Wirkung gegen PHAMI, 21 Tage nach Applikation**

| Wirkstoff | Dosierung [g a.i./ha] | Wirkung [%] |
|---|---|---|
| A1-36 | 10 | 20 |
| flurtamone | 80 | 40 |
| A1-36 + flurtamone | 10 + 80 | 80 (Ec = 52, Diff. = +28) |

**Tabelle 417: Wirkung gegen GALAP, 21 Tage nach Applikation**

| Wirkstoff | Dosierung [g a.i./ha] | Wirkung [%] |
|---|---|---|
| A1-36 | 10 | 25 |
| flurtamone | 320 | 70 |
| A1-36 + flurtamone | 10 + 320 | 95 (Ec = 77,5, Diff. = +17,5) |

**Tabelle 418: Wirkung gegen GALAP, 21 Tage nach Applikation**

| Wirkstoff | Dosierung [g a.i./ha] | Wirkung [%] |
|---|---|---|
| A1-36 | 10 | 25 |
| flurtamone | 80 | 0 |
| A1-36 + flurtamone | 10 + 80 | 60 (Ec = 25, Diff. = +35) |

**Tabelle 419: Wirkung gegen GALAP, 21 Tage nach Applikation**

| Wirkstoff | Dosierung [g a.i./ha] | Wirkung [%] |
|---|---|---|
| A1-36 | 2,5 | 0 |
| flurtamone | 80 | 0 |
| A1-36 + flurtamone | 2,5 + 80 | 70 (Ec = 0, Diff. = +70) |

**Tabelle 420: Wirkung gegen MATIN, 21 Tage nach Applikation**

| Wirkstoff | Dosierung [g a.i./ha] | Wirkung [%] |
|---|---|---|
| A1-36 | 10 | 15 |
| flurtamone | 320 | 30 |
| A1-36 + flurtamone | 10 + 320 | 97 (Ec = 40,5, Diff. = +56,5) |

**Tabelle 421: Wirkung gegen MATIN, 21 Tage nach Applikation**

| Wirkstoff | Dosierung [g a.i./ha] | Wirkung [%] |
|---|---|---|
| A1-36 | 10 | 15 |
| flurtamone | 80 | 25 |
| A1-36 + flurtamone | 10 + 80 | 98 (Ec = 36,25, Diff. = +61,75) |

**Tabelle 422: Wirkung gegen MATIN, 21 Tage nach Applikation**

| Wirkstoff | Dosierung [g a.i./ha] | Wirkung [%] |
|---|---|---|
| A1-36 | 2,5 | 0 |
| flurtamone | 320 | 30 |
| A1-36 + flurtamone | 2,5 + 320 | 80 (Ec = 30, Diff. = +50) |

**Tabelle 423: Wirkung gegen MATIN, 21 Tage nach Applikation**

| Wirkstoff | Dosierung [g a.i./ha] | Wirkung [%] |
|---|---|---|
| A1-36 | 2,5 | 0 |
| flurtamone | 80 | 25 |
| A1-36 + flurtamone | 2,5 + 80 | 60 (Ec = 25, Diff. = +35) |

**Tabelle 424: Wirkung gegen POLCO, 21 Tage nach Applikation**

| Wirkstoff | Dosierung [g a.i./ha] | Wirkung [%] |
|---|---|---|
| A1-36 | 10 | 3 |
| flurtamone | 320 | 30 |
| A1-36 + flurtamone | 10 + 320 | 40 (Ec = 32,1, Diff. = +7,9) |

**Tabelle 425: Wirkung gegen POLCO, 21 Tage nach Applikation**

| Wirkstoff | Dosierung [g a.i./ha] | Wirkung [%] |
|---|---|---|
| A1-36 | 10 | 3 |
| flurtamone | 80 | 0 |
| A1-36 + flurtamone | 10 + 80 | 30 (Ec = 3, Diff. = +27) |

**Tabelle 426: Wirkung gegen AVEFA, 21 Tage nach Applikation**

| Wirkstoff | Dosierung [g a.i./ha] | Wirkung [%] |
|---|---|---|
| A2-1 | 50 | 0 |
| fluroxypyr | 80 | 0 |
| A2-1 + fluroxypyr | 50 + 80 | 15 (Ec = 0, Diff. = +15) |

**Tabelle 427: Wirkung gegen AVEFA, 21 Tage nach Applikation**

| Wirkstoff | Dosierung [g a.i./ha] | Wirkung [%] |
|---|---|---|
| A2-1 | 12,5 | 0 |
| fluroxypyr | 80 | 0 |
| A2-1 + fluroxypyr | 12,5 + 80 | 15 (Ec = 0, Diff. = +15) |

**Tabelle 428: Wirkung gegen BROST, 21 Tage nach Applikation**

| Wirkstoff | Dosierung [g a.i./ha] | Wirkung [%] |
|---|---|---|
| A2-1 | 50 | 0 |
| fluroxypyr | 80 | 0 |
| A2-1 + fluroxypyr | 50 + 80 | 15 (Ec = 0, Diff. = +15) |

**Tabelle 429: Wirkung gegen LOLMU, 21 Tage nach Applikation**

| Wirkstoff | Dosierung [g a.i./ha] | Wirkung [%] |
|---|---|---|
| A2-1 | 50 | 0 |
| fluroxypyr | 80 | 0 |
| A2-1 + fluroxypyr | 50 + 80 | 10 (Ec = 0, Diff. = +10) |

**Tabelle 430: Wirkung gegen PHAMI, 21 Tage nach Applikation**

| Wirkstoff | Dosierung [g a.i./ha] | Wirkung [%] |
|---|---|---|
| A2-1 | 50 | 15 |
| fluroxypyr | 80 | 0 |
| A2-1 + fluroxypyr | 50 + 80 | 30 (Ec = 15, Diff. = +15) |

**Tabelle 431: Wirkung gegen PHAMI, 21 Tage nach Applikation**

| Wirkstoff | Dosierung [g a.i./ha] | Wirkung [%] |
|---|---|---|
| A2-1 | 12,5 | 0 |
| fluroxypyr | 80 | 0 |
| A2-1 + fluroxypyr | 12,5 + 80 | 20 (Ec = 0, Diff. = +20) |

**Tabelle 432: Wirkung gegen POAAN, 21 Tage nach Applikation**

| Wirkstoff | Dosierung [g a.i./ha] | Wirkung [%] |
|---|---|---|
| A2-1 | 12,5 | 0 |
| fluroxypyr | 80 | 10 |
| A2-1 + fluroxypyr | 12,5 + 80 | 15 (Ec = 10, Diff. = +5) |

**Tabelle 433: Wirkung gegen POAAN, 21 Tage nach Applikation**

| Wirkstoff | Dosierung [g a.i./ha] | Wirkung [%] |
|---|---|---|
| A2-1 | 12,5 | 0 |
| fluroxypyr | 20 | 0 |
| A2-1 + fluroxypyr | 12,5 + 20 | 10 (Ec = 0, Diff. = +10) |

**Tabelle 434: Wirkung gegen MATIN, 21 Tage nach Applikation**

| Wirkstoff | Dosierung [g a.i./ha] | Wirkung [%] |
|---|---|---|
| A2-1 | 50 | 85 |
| fluroxypyr | 80 | 50 |
| A2-1 + fluroxypyr | 50 + 80 | 98 (Ec = 92,5, Diff. = +5,5) |

**Tabelle 435: Wirkung gegen MATIN, 21 Tage nach Applikation**

| Wirkstoff | Dosierung [g a.i./ha] | Wirkung [%] |
|---|---|---|
| A2-1 | 50 | 85 |
| fluroxypyr | 20 | 20 |
| A2-1 + fluroxypyr | 50 + 20 | 95 (Ec = 88, Diff. = +7) |

**Tabelle 436: Wirkung gegen MATIN, 21 Tage nach Applikation**

| Wirkstoff | Dosierung [g a.i./ha] | Wirkung [%] |
|---|---|---|
| A2-1 | 12,5 | 80 |
| fluroxypyr | 80 | 50 |
| A2-1 + fluroxypyr | 12,5 + 80 | 95 (Ec = 90, Diff. = +5) |

**Tabelle 437: Wirkung gegen MATIN, 21 Tage nach Applikation**

| Wirkstoff | Dosierung [g a.i./ha] | Wirkung [%] |
|---|---|---|
| A2-1 | 12,5 | 80 |
| fluroxypyr | 20 | 20 |
| A2-1 + fluroxypyr | 12,5 + 20 | 93 (Ec = 84, Diff. = +9) |

**Tabelle 438: Wirkung gegen POLCO, 21 Tage nach Applikation**

| Wirkstoff | Dosierung [g a.i./ha] | Wirkung [%] |
|---|---|---|
| A2-1 | 50 | 60 |
| fluroxypyr | 20 | 80 |
| A2-1 + fluroxypyr | 50 + 20 | 100 (Ec = 92, Diff. = +8) |

**Tabelle 439: Wirkung gegen POLCO, 21 Tage nach Applikation**

| Wirkstoff | Dosierung [g a.i./ha] | Wirkung [%] |
|---|---|---|
| A2-1 | 12,5 | 20 |
| fluroxypyr | 20 | 80 |
| A2-1 + fluroxypyr | 12,5 + 20 | 90 (Ec = 84, Diff. = +6) |

**Tabelle 440: Wirkung gegen AVEFA, 21 Tage nach Applikation**

| Wirkstoff | Dosierung [g a.i./ha] | Wirkung [%] |
|---|---|---|
| A1-55 | 10 | 10 |
| glufosinate-ammonium | 100 | 20 |
| A1-55 + glufosinate-ammonium | 10 + 100 | 40 (Ec = 28, Diff. = +12) |

**Tabelle 441: Wirkung gegen AVEFA, 21 Tage nach Applikation**

| Wirkstoff | Dosierung [g a.i./ha] | Wirkung [%] |
|---|---|---|
| A1-55 | 2,5 | 0 |
| glufosinate-ammonium | 100 | 20 |
| A1-55 + glufosinate-ammonium | 2,5 + 100 | 35 (Ec = 20, Diff. = +15) |

**Tabelle 442: Wirkung gegen BROST, 21 Tage nach Applikation**

| Wirkstoff | Dosierung [g a.i./ha] | Wirkung [%] |
|---|---|---|
| A1-55 | 10 | 10 |
| glufosinate-ammonium | 25 | 0 |
| A1-55 + glufosinate-ammonium | 10 + 25 | 20 (Ec = 10, Diff. = +10) |

**Tabelle 443: Wirkung gegen BROST, 21 Tage nach Applikation**

| Wirkstoff | Dosierung [g a.i./ha] | Wirkung [%] |
|---|---|---|
| A1-55 | 2,5 | 0 |
| glufosinate-ammonium | 100 | 15 |
| A1-55 + glufosinate-ammonium | 2,5 + 100 | 20 (Ec = 15, Diff. = +5) |

**Tabelle 444: Wirkung gegen LOLMU, 21 Tage nach Applikation**

| Wirkstoff | Dosierung [g a.i./ha] | Wirkung [%] |
|---|---|---|
| A1-55 | 10 | 10 |
| glufosinate-ammonium | 100 | 15 |
| A1-55 + glufosinate-ammonium | 10 + 100 | 30 (Ec = 23,5, Diff. = +6,5) |

**Tabelle 445: Wirkung gegen LOLMU, 21 Tage nach Applikation**

| Wirkstoff | Dosierung [g a.i./ha] | Wirkung [%] |
|---|---|---|
| A1-55 | 2,5 | 0 |
| glufosinate-ammonium | 100 | 15 |
| A1-55 + glufosinate-ammonium | 2,5 + 100 | 20 (Ec = 15, Diff. = +5) |

**Tabelle 446: Wirkung gegen PHAMI, 21 Tage nach Applikation**

| Wirkstoff | Dosierung [g a.i./ha] | Wirkung [%] |
|---|---|---|
| A1-55 | 10 | 5 |
| glufosinate-ammonium | 100 | 10 |
| A1-55 + glufosinate-ammonium | 10 + 100 | 40 (Ec = 14,5, Diff. = +25,5) |

**Tabelle 447: Wirkung gegen PHAMI, 21 Tage nach Applikation**

| Wirkstoff | Dosierung [g a.i./ha] | Wirkung [%] |
|---|---|---|
| A1-55 | 10 | 5 |
| glufosinate-ammonium | 25 | 0 |
| A1-55 + glufosinate-ammonium | 10 + 25 | 20 (Ec = 5, Diff. = +15) |

**Tabelle 448: Wirkung gegen PHAMI, 21 Tage nach Applikation**

| Wirkstoff | Dosierung [g a.i./ha] | Wirkung [%] |
|---|---|---|
| A1-55 | 2,5 | 0 |
| glufosinate-ammonium | 100 | 10 |
| A1-55 + glufosinate-ammonium | 2,5 + 100 | 40 (Ec = 10, Diff. = +30) |

**Tabelle 449: Wirkung gegen PHAMI, 21 Tage nach Applikation**

| Wirkstoff | Dosierung [g a.i./ha] | Wirkung [%] |
|---|---|---|
| A1-55 | 2,5 | 0 |
| glufosinate-ammonium | 25 | 0 |
| A1-55 + glufosinate-ammonium | 2,5 + 25 | 20 (Ec = 0, Diff. = +20) |

**Tabelle 450: Wirkung gegen POAAN, 21 Tage nach Applikation**

| Wirkstoff | Dosierung [g a.i./ha] | Wirkung [%] |
|---|---|---|
| A1-55 | 10 | 0 |
| glufosinate-ammonium | 100 | 5 |
| A1-55 + glufosinate-ammonium | 10 + 100 | 15 (Ec = 5, Diff. = +10) |

**Tabelle 451: Wirkung gegen POAAN, 21 Tage nach Applikation**

| Wirkstoff | Dosierung [g a.i./ha] | Wirkung [%] |
|---|---|---|
| A1-55 | 2,5 | 0 |
| glufosinate-ammonium | 100 | 5 |
| A1-55 + glufosinate-ammonium | 2,5 + 100 | 15 (Ec = 5, Diff. = +10) |

**Tabelle 452: Wirkung gegen GALAP, 21 Tage nach Applikation**

| Wirkstoff | Dosierung [g a.i./ha] | Wirkung [%] |
|---|---|---|
| A1-55 | 10 | 15 |
| glufosinate-ammonium | 25 | 0 |
| A1-55 + glufosinate-ammonium | 10 + 25 | 30 (Ec = 15, Diff. = +15) |

**Tabelle 453: Wirkung gegen MATIN, 21 Tage nach Applikation**

| Wirkstoff | Dosierung [g a.i./ha] | Wirkung [%] |
|---|---|---|
| A1-55 | 10 | 20 |
| glufosinate-ammonium | 100 | 15 |
| A1-55 + glufosinate-ammonium | 10 + 100 | 90 (Ec = 32, Diff. = +58) |

**Tabelle 454: Wirkung gegen MATIN, 21 Tage nach Applikation**

| Wirkstoff | Dosierung [g a.i./ha] | Wirkung [%] |
|---|---|---|
| A1-55 | 10 | 20 |
| glufosinate-ammonium | 25 | 0 |
| A1-55 + glufosinate-ammonium | 10 + 25 | 80 (Ec = 20, Diff. = +60) |

**Tabelle 455: Wirkung gegen MATIN, 21 Tage nach Applikation**

| Wirkstoff | Dosierung [g a.i./ha] | Wirkung [%] |
|---|---|---|
| A1-55 | 2,5 | 0 |
| glufosinate-ammonium | 100 | 15 |
| A1-55 + glufosinate-ammonium | 2,5 + 100 | 70 (Ec = 15, Diff. = +55) |

**Tabelle 456: Wirkung gegen MATIN, 21 Tage nach Applikation**

| Wirkstoff | Dosierung [g a.i./ha] | Wirkung [%] |
|---|---|---|
| A1-55 | 2,5 | 0 |
| glufosinate-ammonium | 25 | 0 |
| A1-55 + glufosinate-ammonium | 2,5 + 25 | 40 (Ec = 0, Diff. = +40) |

**Tabelle 457: Wirkung gegen POLCO, 21 Tage nach Applikation**

| Wirkstoff | Dosierung [g a.i./ha] | Wirkung [%] |
|---|---|---|
| A1-55 | 10 | 0 |
| glufosinate-ammonium | 100 | 5 |
| A1-55 + glufosinate-ammonium | 10 + 100 | 15 (Ec = 5, Diff. = +10) |

**Tabelle 458: Wirkung gegen POLCO, 21 Tage nach Applikation**

| Wirkstoff | Dosierung [g a.i./ha] | Wirkung [%] |
|---|---|---|
| A1-55 | 10 | 0 |
| glufosinate-ammonium | 25 | 0 |
| A1-55 + glufosinate-ammonium | 10 + 25 | 20 (Ec = 0, Diff. = +20) |

**Tabelle 459: Wirkung gegen AVEFA, 21 Tage nach Applikation**

| Wirkstoff | Dosierung [g a.i./ha] | Wirkung [%] |
|---|---|---|
| A1-57 | 12,5 | 0 |
| aclonifen | 800 | 0 |
| A1-57 + aclonifen | 12,5 + 800 | 15 (Ec = 0, Diff. = +15) |

**Tabelle 460: Wirkung gegen BROST, 21 Tage nach Applikation**

| Wirkstoff | Dosierung [g a.i./ha] | Wirkung [%] |
|---|---|---|
| A1-57 | 50 | 15 |
| aclonifen | 800 | 0 |
| A1-57 + aclonifen | 50 + 800 | 20 (Ec = 15, Diff. = +5) |

**Tabelle 461: Wirkuna gegen BROST, 21 Tage nach Applikation**

| Wirkstoff | Dosierung [g a.i./ha] | Wirkung [%] |
|---|---|---|
| A1-57 | 12,5 | 0 |
| aclonifen | 800 | 0 |
| A1-57 + aclonifen | 12,5 + 800 | 10 (Ec = 0, Diff. = +10) |

**Tabelle 462: Wirkung gegen PHAMI, 21 Tage nach Applikation**

| Wirkstoff | Dosierung [g a.i./ha] | Wirkung [%] |
|---|---|---|
| A1-57 | 12,5 | 20 |
| aclonifen | 800 | 15 |
| A1-57 + aclonifen | 12,5 + 800 | 40 (Ec = 32, Diff. = +8) |

**Tabelle 463: Wirkung gegen PHAMI, 21 Tage nach Applikation**

| Wirkstoff | Dosierung [g a.i./ha] | Wirkung [%] |
|---|---|---|
| A1-57 | 12,5 | 20 |
| aclonifen | 200 | 0 |
| A1-57 + aclonifen | 12,5 + 200 | 30 (Ec = 20, Diff. = +10) |

**Tabelle 464: Wirkung gegen POAAN, 21 Tage nach Applikation**

| Wirkstoff | Dosierung [g a.i./ha] | Wirkung [%] |
|---|---|---|
| A1-57 | 50 | 35 |
| aclonifen | 800 | 10 |
| A1-57 + aclonifen | 50 + 800 | 85 (Ec = 41,5, Diff. = +43,5) |

**Tabelle 465: Wirkung gegen POAAN, 21 Tage nach Applikation**

| Wirkstoff | Dosierung [g a.i./ha] | Wirkung [%] |
|---|---|---|
| A1-57 | 50 | 35 |
| aclonifen | 200 | 0 |
| A1-57 + aclonifen | 50 + 200 | 60 (Ec = 35, Diff. = +25) |

**Tabelle 466: Wirkung gegen POAAN, 21 Tage nach Applikation**

| Wirkstoff | Dosierung [g a.i./ha] | Wirkung [%] |
|---|---|---|
| A1-57 | 12,5 | 10 |
| aclonifen | 800 | 10 |
| A1-57 + aclonifen | 12,5 + 800 | 40 (Ec = 19, Diff. = +21) |

**Tabelle 467: Wirkung gegen GALAP, 21 Tage nach Applikation**

| Wirkstoff | Dosierung [g a.i./ha] | Wirkung [%] |
|---|---|---|
| A1-57 | 50 | 40 |
| aclonifen | 800 | 20 |
| A1-57 + aclonifen | 50 + 800 | 70 (Ec = 52, Diff. = +18) |

**Tabelle 4: Wirkuna gegen MATIN, 21 Tage nach Applikation**

| Wirkstoff | Dosierung [g a.i./ha] | Wirkung [%] |
|---|---|---|
| A1-57 | 50 | 80 |
| aclonifen | 800 | 0 |
| A1-57 + aclonifen | 50 + 800 | 85 (Ec = 80, Diff. = +5) |

**Tabelle 469: Wirkung gegen POLCO, 21 Tage nach Applikation**

| Wirkstoff | Dosierung [g a.i./ha] | Wirkung [%] |
|---|---|---|
| A1-57 | 12,5 | 50 |
| aclonifen | 200 | 0 |
| A1-57 + aclonifen | 12,5 + 200 | 60 (Ec = 50, Diff. = +10) |

**Tabelle 470: Wirkung gegen BROST, 21 Tage nach Applikation**

| Wirkstoff | Dosierung [g a.i./ha] | Wirkung [%] |
|---|---|---|
| A1-7 | 10 | 5 |
| saflufenacil | 8 | 5 |
| A1-7 + saflufenacil | 10 + 8 | 15 (Ec = 9,75, Diff. = +5,25) |

**Tabelle 471: Wirkung gegen POLCO, 21 Tage nach Applikation**

| Wirkstoff | Dosierung [g a.i./ha] | Wirkung [%] |
|---|---|---|
| A1-7 | 10 | 10 |
| saflufenacil | 8 | 0 |
| A1-7 + saflufenacil | 10 + 8 | 60 (Ec = 10, Diff. = +50) |

**Tabelle 472: Wirkung gegen POLCO, 21 Tage nach Applikation**

| Wirkstoff | Dosierung [g a.i./ha] | Wirkung [%] |
|---|---|---|
| A1-7 | 10 | 10 |
| saflufenacil | 2 | 0 |
| A1-7 + saflufenacil | 10 + 2 | 40 (Ec = 10, Diff. = +30) |

**Tabelle 473: Wirkung gegen POLCO, 21 Tage nach Applikation**

| Wirkstoff | Dosierung [g a.i./ha] | Wirkung [%] |
|---|---|---|
| A1-7 | 2,5 | 0 |
| saflufenacil | 8 | 0 |
| A1-7 + saflufenacil | 2,5 + 8 | 80 (Ec = 0, Diff. = +80) |

**Tabelle 474: Wirkung gegen POLCO, 21 Tage nach Applikation**

| Wirkstoff | Dosierung [g a.i./ha] | Wirkung [%] |
|---|---|---|
| A1-7 | 2,5 | 0 |
| saflufenacil | 2 | 0 |
| A1-7 + saflufenacil | 2,5 + 2 | 50 (Ec = 0, Diff. = +50) |

**Tabelle 475: Wirkung gegen AVEFA, 21 Tage nach Applikation**

| Wirkstoff | Dosierung [g a.i./ha] | Wirkung [%] |
|---|---|---|
| A1-60 | 10 | 5 |
| isoproturon | 1600 | 15 |
| A1-60 + isoproturon | 10 + 1600 | 40 (Ec = 19,25, Diff. = +20,75) |

**Tabelle 476: Wirkung gegen AVEFA, 21 Tage nach Applikation**

| Wirkstoff | Dosierung [g a.i./ha] | Wirkung [%] |
|---|---|---|
| A1-60 | 10 | 5 |
| isoproturon | 400 | 0 |
| A1-60 + isoproturon | 10 + 400 | 40 (Ec = 5, Diff. = +35) |

**Tabelle 477: Wirkung gegen AVEFA, 21 Tage nach Applikation**

| Wirkstoff | Dosierung [g a.i./ha] | Wirkung [%] |
|---|---|---|
| A1-60 | 2,5 | 0 |
| isoproturon | 400 | 0 |
| A1-60 + isoproturon | 2,5 + 400 | 10 (Ec = 0, Diff. = +10) |

**Tabelle 478: Wirkung gegen BROST, 21 Tage nach Applikation**

| Wirkstoff | Dosierung [g a.i./ha] | Wirkung [%] |
|---|---|---|
| A1-60 | 10 | 5 |
| isoproturon | 1600 | 0 |
| A1-60 + isoproturon | 10 + 1600 | 30 (Ec = 5, Diff. = +25) |

**Tabelle 479: Wirkung gegen BROST, 21 Tage nach Applikation**

| Wirkstoff | Dosierung [g a.i./ha] | Wirkung [%] |
|---|---|---|
| A1-60 | 10 | 5 |
| isoproturon | 400 | 0 |
| A1-60 + isoproturon | 10 + 400 | 15 (Ec = 5, Diff. = +10) |

**Tabelle 480: Wirkung gegen LOLMU, 21 Tage nach Applikation**

| Wirkstoff | Dosierung [g a.i./ha] | Wirkung [%] |
|---|---|---|
| A1-60 | 10 | 3 |
| isoproturon | 1600 | 0 |
| A1-60 + isoproturon | 10 + 1600 | 30 (Ec = 3, Diff. = +27) |

**Tabelle 481: Wirkung gegen LOLMU, 21 Tage nach Applikation**

| Wirkstoff | Dosierung [g a.i./ha] | Wirkung [%] |
|---|---|---|
| A1-60 | 10 | 3 |
| isoproturon | 400 | 0 |
| A1-60 + isoproturon | 10 + 400 | 15 (Ec = 3, Diff. = +12) |

**Tabelle 482: Wirkung gegen PHAMI, 21 Tage nach Applikation**

| Wirkstoff | Dosierung [g a.i./ha] | Wirkung [%] |
|---|---|---|
| A1-60 | 10 | 20 |
| isoproturon | 400 | 15 |
| A1-60 + isoproturon | 10 + 400 | 80 (Ec = 32, Diff. = +48) |

**Tabelle 483: Wirkung gegen PHAMI, 21 Tage nach Applikation**

| Wirkstoff | Dosierung [g a.i./ha] | Wirkung [%] |
|---|---|---|
| A1-60 | 2,5 | 0 |
| isoproturon | 400 | 15 |
| A1-60 + isoproturon | 2,5 + 400 | 20 (Ec = 15, Diff. = +5) |

**Tabelle 484: Wirkung gegen POAAN, 21 Tage nach Applikation**

| Wirkstoff | Dosierung [g a.i./ha] | Wirkung [%] |
|---|---|---|
| A1-60 | 10 | 20 |
| isoproturon | 400 | 30 |
| A1-60 + isoproturon | 10 + 400 | 80 (Ec = 44, Diff. = +36) |

**Tabelle 485: Wirkung gegen GALAP, 21 Tage nach Applikation**

| Wirkstoff | Dosierung [g a.i./ha] | Wirkung [%] |
|---|---|---|
| A1-60 | 10 | 0 |
| isoproturon | 1600 | 0 |
| A1-60 + isoproturon | 10 + 1600 | 40 (Ec = 0, Diff. = +40) |

**Tabelle 486: Wirkung gegen GALAP, 21 Tage nach Applikation**

| Wirkstoff | Dosierung [g a.i./ha] | Wirkung [%] |
|---|---|---|
| A1-60 | 10 | 0 |
| isoproturon | 400 | 0 |
| A1-60 + isoproturon | 10 + 400 | 50 (Ec = 0, Diff. = +50) |

**Tabelle 487: Wirkung gegen GALAP, 21 Tage nach Applikation**

| Wirkstoff | Dosierung [g a.i./ha] | Wirkung [%] |
|---|---|---|
| A1-60 | 2,5 | 0 |
| isoproturon | 1600 | 0 |
| A1-60 + isoproturon | 2,5 + 1600 | 40 (Ec = 0, Diff. = +40) |

**Tabelle 488: Wirkung gegen GALAP, 21 Tage nach Applikation**

| Wirkstoff | Dosierung [g a.i./ha] | Wirkung [%] |
|---|---|---|
| A1-60 | 2,5 | 0 |
| isoproturon | 400 | 0 |
| A1-60 + isoproturon | 2,5 + 400 | 40 (Ec = 0, Diff. = +40) |

**Tabelle 489: Wirkung gegen MATIN, 21 Tage nach Applikation**

| Wirkstoff | Dosierung [g a.i./ha] | Wirkung [%] |
|---|---|---|
| A1-60 | 10 | 40 |
| isoproturon | 400 | 90 |
| A1-60 + isoproturon | 10 + 400 | 100 (Ec = 94, Diff. = +6) |

**Tabelle 490: Wirkung gegen MATIN, 21 Tage nach Applikation**

| Wirkstoff | Dosierung [g a.i./ha] | Wirkung [%] |
|---|---|---|
| A1-60 | 2,5 | 0 |
| isoproturon | 400 | 90 |
| A1-60 + isoproturon | 2,5 + 400 | 100 (Ec = 90, Diff. = +10) |

**Tabelle 491: Wirkung gegen AVEFA, 21 Tage nach Applikation**

| Wirkstoff | Dosierung [g a.i./ha] | Wirkung [%] |
|---|---|---|
| A1-13 | 10 | 3 |
| indaziflam | 40 | 10 |
| A1-13 + indaziflam | 10 + 40 | 20 (Ec = 12,7, Diff. = +7,3) |

**Tabelle 492: Wirkung gegen AVEFA, 21 Tage nach Applikation**

| Wirkstoff | Dosierung [g a.i./ha] | Wirkung [%] |
|---|---|---|
| A1-13 | 2,5 | 0 |
| indaziflam | 40 | 10 |
| A1-13 + indaziflam | 2,5 + 40 | 30 (Ec = 10, Diff. = +20) |

**Tabelle 493: Wirkung gegen BROST, 21 Tage nach Applikation**

| Wirkstoff | Dosierung [g a.i./ha] | Wirkung [%] |
|---|---|---|
| A1-13 | 10 | 5 |
| indaziflam | 10 | 0 |
| A1-13 + indaziflam | 10 + 10 | 10 (Ec = 5, Diff. = +5) |

**Tabelle 494: Wirkung gegen LOLMU, 21 Tage nach Applikation**

| Wirkstoff | Dosierung [g a.i./ha] | Wirkung [%] |
|---|---|---|
| A1-13 | 10 | 5 |
| indaziflam | 10 | 0 |
| A1-13 + indaziflam | 10 + 10 | 10 (Ec = 5, Diff. = +5) |

**Tabelle 495: Wirkung gegen PHAMI, 21 Tage nach Applikation**

| Wirkstoff | Dosierung [g a.i./ha] | Wirkung [%] |
|---|---|---|
| A1-13 | 10 | 20 |
| indaziflam | 40 | 10 |
| A1-13 + indaziflam | 10 + 40 | 40 (Ec = 28, Diff. = +12) |

**Tabelle 496: Wirkung gegen PHAMI, 21 Tage nach Applikation**

| Wirkstoff | Dosierung [g a.i./ha] | Wirkung [%] |
|---|---|---|
| A1-13 | 2,5 | 15 |
| indaziflam | 40 | 10 |
| A1-13 + indaziflam | 2,5 + 40 | 40 (Ec = 23,5, Diff. = +16,5) |

**Tabelle 497: Wirkung gegen POAAN, 21 Tage nach Applikation**

| Wirkstoff | Dosierung [g a.i./ha] | Wirkung [%] |
|---|---|---|
| A1-13 | 10 | 20 |
| indaziflam | 10 | 0 |
| A1-13 + indaziflam | 10 + 10 | 40 (Ec = 20, Diff. = +20) |

**Tabelle 498: Wirkung gegen GALAP, 21 Tage nach Applikation**

| Wirkstoff | Dosierung [g a.i./ha] | Wirkung [%] |
|---|---|---|
| A1-13 | 10 | 60 |
| indaziflam | 40 | 35 |
| A1-13 + indaziflam | 10 + 40 | 85 (Ec = 74, Diff. = +11) |

## Patentansprüche

1. Herbizide Zusammensetzungen, enthaltend
(A) die Verbindung der Formel (Ib) (Komponente A) oder deren Salze, 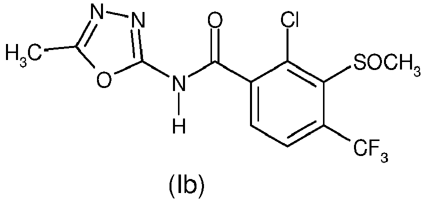 sowie
(B) ein oder mehrere Herbizide (Komponente B) ausgewählt aus den Gruppen (B1) bis (B11):
B1 1,3-Diketoverbindungen, umfassend
prohexadione, prohexadione-calcium, trinexapac-ethyl,
alloxydim, alloxydim-sodium, butroxydim, clethodim, cycloxydim, ketospiradox, profoxydim, sethoxydim, tepraloxydim, tralkoxydim,
mesotrione, sulcotrione, tefuryltrione, tembotrione, bicyclopyrone, pinoxaden,
B2 (Sulfon)Amide, umfassend
beflubutamide, bromobutide, dimethenamide, dimethenamide-P, diphenamide, napropamide, pethoxamid, N-[3-Chlor-4-(1-methylethyl)-phenyl]-2-methylpentanamid, propyzamide,
diflufenican, etobenzanid, flufenacet, mefenacet, mefluidide, picolinafen, propanil, N-phenylphtalamic acid,
acetochlor, alachlor, amidochlor, butachlor, butenachlor, dimethachlor, metazachlor, metolachlor, S-metolachlor, pretilachlor, propachlor, propisochlor, (2-chloro-6'-ethyl-N-isopropoxymethylaceto-o-toluidide), thenylchlor,
asulam, carbaryl, carbetamide, chlorpropham, desmedipham, phenmedipham, propham,
butylate, cycloate, dimepiperate, EPTC, esprocarb, methasulfocarb, molinate, orbencarb, pebulate, prosulfocarb, pyributicarb, thiobencarb, tri-allate, vernolate, amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron-methyl-sodium, foramsulfuron, halosulfuron-methyl, imazosulfuron, iodosulfuron, iodosulfuron-methyl-sodium, mesosulfuron, mesosulfuron-methyl, metazosulfuron, methiopyrsulfuron, metsulfuron, metsulfuron-methyl, monosulfuron, monosulfuronester, nicosulfuron, orthosulfamuron, oxasulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, trifloxysulfuron (sodium), triflusulfuron, triflusulfuron-methyl, tritosulfuron, (benzoic acid, 2-[[[[[4-methoxy-6-(methylthio)-2-pyrimidinyl]amino]carbonyl]amino]-sulfonyl]methyl-ester), flucarbazone, flucarbazone-sodium, ipfencarbazone, propoxycarbazone, propoxycarbazone-sodium, thiencarbazone, thiencarbazone-methyl, cloransulam, cloransulam-methyl, diclosulam, florasulam, flumetsulam, metosulam, penoxsulam, pyroxsulam,
3-chloro-N-[(4,6-dimethoxypyrimidin-2-yl)carbamoyl]-1-methyl-4-(5-methyl-5,6-dihydro-1,4,2-dioxazin-3-yl)-1H-pyrazole-5-sulfonamide,
B3 Arylnitrile, umfassend
bromoxynil, bromoxynil-butyrate, bromoxynil-potassium, bromoxynil-heptanoate, bromoxynil-octanoate, detosyl-pyrazolate (DTP), dichlobenil, ioxynil, ioxynil-octanoate, ioxynil-potassium, ioxynil-sodium, pyraclonil,
B4 Azole, umfassend
benzofenap , pyrazolynate (pyrazolate), pyrazoxyfen, pyroxasulfone, topramezone, pyrasulfotole, 3-(3-chloro-5-{[1-methyl-3-(trifluormethyl)-1H-pyrazol-5-yl]oxy}phenoxy)-1-methyl-5-(trifluormethyl)-1H-pyrazole, 3-(3-iodo-5-{[1-methyl-3-(trifluormethyl)-1H-pyrazol-5-yl]oxy}phenoxy)-1-methyl-5-(trifluormethyl)-1H-pyrazole, 1-ethyl-3-(3-fluoro-5-{[1-methyl-3-(trifluormethyl)-1H-pyrazol-5-yl]oxy}phenoxy)-5-(trifluormethyl)-1H-pyrazole, pyraflufen, pyraflufen-ethyl, fenoxasulfone, isouron, isoxaben, isoxaflutole, imazamethabenz, imazamethabenz-methyl, imazamox, imazamox-ammonium, imazapic, imazapic-ammonium, imazapyr, imazapyr-isopropyl-ammonium, imazaquin, imazaquin-ammonium, imazethapyr, imazethapyr-immonium, azafenidin, oxadiargyl, oxadiazon, amicarbazone, carfentrazone, carfentrazone-ethyl, sulfentrazone, amitrole, paclobutrazol, uniconazole, uniconazole-P, cafenstrole, fentrazamide,
B5 weitere Herbizide, umfassend
allidochlor, aminocyclopyrachlor, aminocyclopyrachlor-potassium, aminocyclopyrachlor-methyl, N-acetylthiazolidine-4-carboxylic acid, aminopyralid, ammonium pelargonate, ammonium sulfamat, aviglycine, benazolin, benazolin-ethyl, benfluralin, benfuresate, bentazone, benzobicyclon, 6-benzylaminopurine, brassinolide, bromofenoxim, butralin, chlorfenac, chlorfenac-sodium, chlorfenprop, chlorflurenol, chlorflurenol-methyl, chloridazon, chlormequat chloride, chlorphthalim, chlorthal-dimethyl, cinidon, cinidon-ethyl, cinmethylin, clofencet, clomazone, cloxyfonac, cyanamide, cyclanilide, cyclopyrimorate, 6-isopentylamino-purin, kinetin, zeatin, dalapon, daminozide, dazomet, n-decanol, difenzoquat metilsulfate, 2,6-diisopropylnaphtalene, dikegulac, dikegulac-sodium, dimethipin, dinitramine, dinoterb, diquat, diquat dibromide, dithiopyr, DNOC, endothal, endothal-dipotassium, endothaldisodium, endothal-mono(N,N-dimethylalkylammonium), ethafluralin, ethofumesate, ethylchlozate, flamprop, flamprop-M-isopropyl, flamprop-M-methyl, fluchloralin, flufenpyr, flufenpyr-ethyl, flumetralin, flumichlorac, flumiclorac-pentyl, flumioxazin, flupropanate, flurenol, flurenol-butyl, flurenol-dimethylammonium-methyl, fluridone, flurochloridone, flurtamone, fluthiacet, fluthiacet-methyl, gibberillic acid, halauxifen, indanofan, isoprothiolane, maleic hydrazide, mepiquat chloride, metam, methiozolin, methylarsonic acid, 1-methylcyclopropene, methyl isothiocyanate, nitrophenolate mixture, nonanoic acid, norflurazon, oleic acid, oryzalin, oxaziclomefone, paraquat, paraquat dichloride, pendimethalin, pentachlorophenol, pentoxazone, petroleum oils, prodiamine, n-propyl-dihydrojasmonate, pyridafol, pyridate, quinoclamine, sintofen, TCA, TCA sodium, tecnazene, thiazopyr, triacontanol, triafamone, trifluralin, urea sulfate,
B6 (Het)Arylcarbonsäuren, umfassend
chloramben, dicamba, 2,3,6-TBA, clopyralid, fluroxypyr, fluroxypyr-meptyl, inabenfide, picloram, triclopyr, quinclorac, quinmerac, indol-3-ylacetic acid, 4-indol-3-yl-butyric acid, 2-(1-naphthyl)acetamide, 1-naphtylacetic acid, 2-naphthyloxyacetic acid,
B7 organische Phosphorverbindungen, umfassend
anilofos, bensulide, bilanafos, bilanafos-sodium, butamifos, clacyfos, fosamine, glufosinate, glufosinate-Salze, glufosinate-ammonium, glufosinate-sodium, glufosinate-P, L-glufosinate-ammonium, L-glufosinate-sodium, glyphosate, glyphosate-Salze, glyphosate-isopropyl-ammonium, glyphosate-ammonium, glyphosatedimethylammonium, glyphosate-trimesium (=sulfosate), glyphosate-diammonium, glyphosate-potassium, glyphosate-sodium, piperophos, ethephon, tribufos,
B8 Phenylether, umfassend
acifluorfen-sodium, aclonifen, fluoroglycofen, fluoroglycofen-ethyl, fomesafen, fomesafen-sodium, halosafen, lactofen, oxyfluorfen, acifluorfen, bifenox, ethoxyfenethyl, clomeprop, cloprop, dichlorprop, dichlorprop-P, mecoprop, mecoprop-sodium, mecoprop-butotyl, mecoprop-P, mecoprop-P-butotyl, mecoprop-P-dimethylammonium, mecoprop-P-2-ethylhexyl, mecoprop-P-patassium, 4-CPA, 2,4-D, 2,4-D-butotyl, 2,4-D-butyl, 2,4-D-dimethylammonium, 2,4-D-diolamin, 2,4-D-ethyl, 2,4-D-2-ethylhexyl, 2,4-D-isobutyl, 2,4-D-isooctyl, 2,4-D- iso-propyl-ammonium, 2,4-D-potassium, 2,4-D-triisopropanolammonium, 2,4-D-trolamine, MCPA, MCPA-butotyl, MCPAdimethylammonium, MCPA-2-ethylhexyl. MCPA-isopropylammonium, MCPApotassium, MCPA-sodium, 2,4-DB, MCPB, MCPB-methyl, MCPB-ethyl-sodium, clodinafop-ethyl, clodinafop-propargyl, cyhalofop, cyhalofop-butyl, diclofop, diclofop-P, diclofop-methyl, diclofop-P-methyl, fenoxaprop, fenoxaprop-P, fenoxaprop-P-ethyl, fluazifop, fluazifop-P, fluazifop-butyl, fluazifop-P-butyl, haloxyfop, haloxyfop-P, metamifop, propaquizafop quizalofop, quizalofop-ethyl, quizalofop-P, quizalofop-P-ethyl, quizalofop-P-tefuryl,
B9- Pyrimidine, umfassend
ancymidol, flurprimidol, pyrimisulfan, bispyribac, bispyribac-sodium, pyribenzoxim, pyriminobac, pyriminobac-methyl, pyribambenz, pyribambenz-isopropyl, pyribambenzpropyl, pyriftalid, pyrithiobac, pyrithiobac-sodium, bromacil, butafenacil, lenacil, saflufenacil, terbacil, tifenacil, 2-chloro-4-fluoro-5-[3-methyl-2,6-dioxo-4-(trifluoromethyl)-3,6-dihydropyrimidin-1 (2H)-yl]-N-[methyl(1-methylethyl)-sulfamoyl]benzamide, ethyl[(3-{2-chloro-5-[2,6-dioxo-4-(trifluoromethyl)-3,6-dihydropyrimidin-1 (2H)-yl]-4-fluorophenoxy}pyridin-2-yl)oxy]acetate,
B10 (Thio)harnstoffe, umfassend
cumyluron, chlorbromuron, chlorotoluron, daimuron, diflufenzopyr, diflufenzopyrsodium, dimefuron, diuron, fluometuron, forchlorfenuron, isoproturon, karbutilate, linuron, metobromuron, metoxuron, monolinuron, neburon, siduron, terbucarb, thidiazuron, tebuthiuron, methabenzthiazuron,
B11 Triazine, umfassend
triaziflam, indaziflam, atrazine, cyanazine, cyprazine, propazine, simazine, terbumeton, terbuthylazine, trietazine, prometon, ametryn, dimethametryn, prometryn, simetryn, terbutryn, ethiozin, hexazinon, metamitron, metribuzin.

2. Herbizide Zusammensetzungen nach Anspruch 1, worin
A bedeutet N oder CY,
R bedeutet Wasserstoff, (C₁-C₆)-Alkyl, (C₃-C₇)-Cycloalkyl, Halogen-(C₁-C₆)-alkyl, (C₃-C₇)-Cycloalkylmethyl, Methoxycarbonylmethyl, Ethoxycarbonylmethyl, Acetylmethyl, Methoxymethyl, Methoxyethyl, Benzyl, Pyrazin.2-yl, Furan-2-yl, Tetrahydrofuran-2-yl, Morpholin, Dimethylamino oder durch s Reste aus der Gruppe Methyl, Methoxy, Trifluormethyl und Halogen substituiertes Phenyl;
X bedeutet Nitro, Halogen, Cyano, (C₁-C₆)-Alkyl, Halogen-(C₁-C₆)-alkyl, (C₃-C₆)-Cycloalkyl, OR¹, S(O)ₙR², (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Heteroaryl, (C₁-C₆)-Alkyl-Heterocyclyl, wobei die beiden letzt genannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy substituiert sind, und wobei Heterocyclyl n Oxogruppen trägt,
Y Wasserstoff, Nitro, Halogen, Cyano, (C₁-C₆)-Alkyl, (C₁-C₆)-Halogenalkyl, OR¹, S(O)ₙR², SO₂N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Nitro, Methyl, Halogen-(C₁-C₆)-alkyl, (C₃-C₆)-Cycloalkyl, S(O)ₙR², 1,2,4-Triazol-1-yl, Pyrazol-1-yl, oder
Z kann auch Wasserstoff bedeuten, falls Y für den Rest S(O)ₙR² steht,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 16 letztgenannten Reste durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₃-C₆)-Cycloalkyl oder (C₃-C₆)-Cycloalkyl-(C₁-C₆)-alkyl, wobei diese drei vorstehend genannten Reste jeweils durch s Reste aus der Gruppe bestehend aus Halogen und OR³ substituiert sind,
R³ bedeutet Wasserstoff oder (C₁-C₆)-Alkyl,
R⁴ bedeutet (C₁-C₆)-Alkyl,
n bedeutet 0, 1 oder 2,
s bedeutet 0, 1, 2 oder 3.

3. Herbizide Zusammensetzungen nach Anspruch 1 oder 2, worin
A bedeutet N oder CY,
R bedeutet Wasserstoff, (C₁-C₄)-Alkyl, Cyclopropyl, Halogen-(C₁-C₄)-alkyl, (C₃-C₆)-Cycloalkylmethyl, Methoxymethyl, Methoxyethyl, Benzyl,
X bedeutet Nitro, Halogen, Cyano, (C₁-C₆)-Alkyl, Halogen-(C₁-C₆)-alkyl, Cyclopropyl, OR¹, S(O)ₙR², (C₁-C₆)-Alkyl-S(O)ₙR², (C₁-C₆)-Alkyl-OR¹, (C₁-C₂)-Alkyl-Heteroaryl, (C₁- C₂)-Alkyl-Heterocyclyl, wobei die beiden letzt genannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy substituiert sind, und wobei Heterocyclyl n Oxogruppen trägt,
Y Wasserstoff, Nitro, Halogen, Cyano, (C₁-C₆)-Alkyl, (C₁-C₆)-Halogenalkyl, OR¹, S(O)ₙR², SO₂N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-CON(R₁)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Nitro, Methyl, Halogen-(C₁-C₆)-alkyl, (C₃-C₆)-Cycloalkyl, S(O)ₙR², 1,2,4-Triazol-1-yl, Pyrazol-1-yl, oder
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 16 letztgenannten Reste durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₃-C₆)-Cycloalkyl oder (C₃-C₆)-Cycloalkyl-(C₁-C₆)-alkyl, wobei diese drei vorstehend genannten Reste jeweils durch s Reste aus der Gruppe bestehend aus Halogen und OR³ substituiert sind,
R³ bedeutet Wasserstoff oder (C₁-C₆)-Alkyl,
R⁴ bedeutet (C₁-C₆)-Alkyl,
n bedeutet 0, 1 oder 2,
s bedeutet 0, 1, 2 oder 3.

4. Herbizide Zusammensetzungen nach einem der Ansprüche 1 bis 3, enthaltend als Komponente B ein oder mehrere Herbizide ausgewählt aus den Gruppen B1 bis B11:
B1 umfassend clethodim, sethoxydim, tepraloxydim, mesotrione, sulcotrione, tefuryltrione, tembotrione, bicyclopyrone, pinoxaden, tralkoxydim,
B2 umfassend dimethenamide, dimethenamide-P, napropamide, pethoxamid, propyzamide, diflufenican, flufenacet, mefenacet, picolinafen, propanil, acetochlor, alachlor, butachlor, metazachlor, metolachlor, S-metolachlor, pretilachlor, thenylchlor, asulam, carbetamide, desmedipham, phenmedipham, esprocarb, molinate, prosulfocarb, thiobencarb, amidosulfuron, chlorimuron-ethyl, cyclosulfamuron, ethoxysulfuron, flupyrsulfuron-methyl-sodium, foramsulfuron, iodosulfuron-methyl-sodium, mesosulfuron-methyl, nicosulfuron, orthosulfamuron, prosulfuron, pyrazosulfuron-ethyl, rimsulfuron, trifloxysulfuron (sodium), flucarbazone-sodium, propoxycarbazone-sodium, thiencarbazone-methyl, florasulam, metosulam, penoxsulam, metsulfuron-methyl, sulfosulfuron, thifensulfuron-methyl, tribenuron-methyl, tritosulfuron, pyroxsulam,
B3 umfassend bromoxynil und ioxynil.
B4 umfassend benzofenap, topramezone, pyrasulfotole, isoxaflutole, imazamox, imazethapyr, oxadiargyl, oxadiazon, amicarbazone, carfentrazone-ethyl, sulfentrazone, uniconazole, cafenstrole, fentrazamide, 3-(3-chloro-5-{[1-methyl-3-(trifluormethyl)-1H-pyrazol-5-yl]oxy}phenoxy)-1-methyl-5-(trifluormethyl)-1H-pyrazole, 3-(3-iodo-5-{[1-methyl-3-(trifluormethyl)-1H-pyrazol-5-yl]oxy}phenoxy)-1-methyl-5-(trifluormethyl)-1H-pyrazole, 1-ethyl-3-(3-fluoro-5-{[1-methyl-3-(trifluormethyl)-1H-pyrazol-5-yl]oxy}phenoxy)-5-(trifluormethyl)-1H-pyrazole, pyraflufen-ethyl,
B5 umfassend aminopyralid, benazolin, benfuresate, bentazone, cinidon-ethyl, clomazone, diquat dibromide, ethofumesate, flumiclorac-pentyl, flumioxazin, flurtamone, oxaziclomefone, pendimethalin, pyridate und trifluralin,
B6 umfassend dicamba, clopyralid, fluroxypyr, picloram, triclopyr, quinclorac,
B7 umfassend anilofos, glufosinate-ammonium und L-glufosinate-ammonium, glyphosate, glyphosate-isopropyl-ammonium, glyphosate-ammonium, glyphosate-trimesium (=sulfosate), glyphosate-diammonium, glyphosate-potassium,
B8 umfassend acifluorfen-sodium, aclonifen, fluoroglycofen-ethyl, oxyfluorfen, bifenox, dichlorprop-P, mecoprop-P, 2,4-D, MCPA, clodinafop-propargyl, cyhalofop-butyl, diclofop-methyl, diclofop-P-methyl, fenoxaprop-P-ethyl, fluazifop-P-butyl, quizalofop-P,
B9- umfassend bispyribac (sodium), pyriftalid, bromacil, lenacil, 2-chloro-4-fluoro-5-[3-methyl-2,6-dioxo-4-(trifluoromethyl)-3,6-dihydropyrimidin-1 (2H)-yl]-N-[methyl(1 -methylethyl)-sulfamoyl]benzamide,
B10 umfassend cumyluron, daimuron, diuron, isoproturon, diflufenzopyr,
B11 umfassend atrazine, simazine, terbuthylazine, ametryn, terbutryn, metamitron, metribuzin.

5. Herbizide Zusammensetzungen nach einem der Ansprüche 1 bis 4, enthaltend als Komponente B ein oder mehrere Herbizide ausgewählt aus den Gruppen B1, B2, B4, B5, B8, B9- und B11:
B1 umfassend clethodim, sulcotrione, tefuryltrione, tembotrione, bicyclopyrone, pinoxaden,
B2 umfassend dimethenamide-P, napropamide, diflufenican, flufenacet, mefenacet, acetochlor, metazachlor, S-metolachlor, asulam, desmedipham, phenmedipham, molinate, prosulfocarb, amidosulfuron, ethoxysulfuron, foramsulfuron, iodosulfuron-methyl-sodium, mesosulfuron-methyl, flucarbazone-sodium, propoxycarbazone-sodium, thiencarbazone-methyl, florasulam, metosulam, metsulfuron-methyl, sulfosulfuron, thifensulfuron-methyl, tribenuron-methyl, tritosulfuron, pyroxsulam,
B4 umfassend pyrasulfotole, isoxaflutole, oxadiargyl, oxadiazon, amicarbazone, fentrazamide, 3-(3-chloro-5-{[1-methyl-3-(trifluormethyl)-1H-pyrazol-5-yl]oxy}phenoxy)-1-methyl-5-(trifluormethyl)-1H-pyrazole, 3-(3-iodo-5-{[1-methyl-3-(trifluormethyl)-1H-pyrazol-5-yl]oxy}phenoxy)-1-methyl-5-(trifluormethyl)-1H-pyrazole, 1-ethyl-3-(3-fluoro-5-{[1-methyl-3-(trifluormethyl)-1H-pyrazol-5-yl]oxy}phenoxy)-5-(trifluormethyl)-1H-pyrazole, pyraflufen-ethyl, imazamox,
B5 umfassend aminopyralid, benfuresate, ethofumesate, flurtamone und oxaziclomefone,
B8 umfassend aclonifen, diclofop-methyl, diclofop-P-methyl, fenoxaprop-P-ethyl, MCPA, 2,4-D, clodinafop-ethyl,
B9- umfassend bispyribac (sodium), bromacil,
B11 umfassend metamitron, metribuzin, terbuthylazine.

6. Herbizide Zusammensetzungen nach einem der Ansprüche 1 bis 5, enthaltend zusätzlich als Komponente C einen oder mehrere Safener aus der Gruppe bestehend aus benoxacor, cloquintocet-mexyl, cyprosulfamide, dichlormid, fenclorim, fenchlorazole, furilazole, isoxadifen-ethyl, mefenpyr-diethyl, 4-(dichloroacetyl)-1-oxa-4-azaspiro[4.5]decane, 2,2,5-trimethyl-3-(dechloroacetyl)-1,3-oxazolidine.

7. Verfahren zur Bekämpfung von Schadpflanzen in Kulturen, **dadurch gekennzeichnet, daß** man eine herbizid wirksame Menge einer herbiziden Zusammensetzung gemäß einem oder mehreren der Ansprüche 1 bis 6 auf die Schadpflanzen, Pflanzen, Pflanzensamen oder die Fläche, auf der die Pflanzen wachsen, aufbringt.

8. Verfahren gemäß Anspruch 7, **dadurch gekennzeichnet, daß** die Pflanzen aus der Gruppe Zuckerrohr, Mais, Weizen, Roggen, Gerste, Hafer, Reis, Sorghum, Baumwolle und Soja stammen.

9. Verfahren gemäß Anspruch 7 oder 8, **dadurch gekennzeichnet, daß** die Pflanzen gentechnisch verändert sind.
